(19) Europäisches Patentamt
European Patent Office
Office européen des brevets

(11) Publication number: **0 390 558 B1**

(12) # EUROPEAN PATENT SPECIFICATION

(45) Date of publication of patent specification: **07.09.94**

(51) Int. Cl.$^5$: **C07D 233/64**, A61K 31/415

(21) Application number: **90303362.9**

(22) Date of filing: **29.03.90**

(54) **Aromatase inhibiting 4(5)-imidazoles.**

(30) Priority: **30.03.89 GB 8907218**
**31.03.89 GB 8907309**

(43) Date of publication of application:
**03.10.90 Bulletin 90/40**

(45) Publication of the grant of the patent:
**07.09.94 Bulletin 94/36**

(84) Designated Contracting States:
**AT BE CH DE DK ES FR GB GR IT LI LU NL SE**

(56) References cited:
**EP-A- 058 047**
**EP-A- 081 324**
**EP-A- 194 984**
**EP-A- 311 447**

(73) Proprietor: **ORION-YHTYMÄ OY**
**Orionintie 1,**
**PL 65**
**SF-02101 Espoo (FI)**

(72) Inventor: **Karjalainen, Arto Johannes**
**Myllyojantie 13 H 37**
**SF-90650 Oulu (FI)**
Inventor: **Pelkonen, Reino Olavi**
**Hamalantie 3 C**
**SF-90800 Oulu (FI)**
Inventor: **Sodervall, Marja-Liisa**
**Annantie 9-13 C 7**
**SF-90560 Oulu (FI)**
Inventor: **Lahde, Matti Antero**
**Lehmustie 31 as. 14**
**SF-20720 Turku (FI)**
Inventor: **Lammintausta, Risto Arvo Sakari**
**Meltoistentie**
**SF-20900 Turku (FI)**
Inventor: **Karjalainen, Arja Leena**
**Tiilitie 15 C 24**
**SF-90650 Oulu (FI)**
Inventor: **Kalapudas, Arja Marketta**
**Notkolantie 13**
**SF-90240 Oulu (FI)**

(74) Representative: **Sexton, Jane Helen et al**
**J.A. KEMP & CO.**
**14 South Square**
**Gray's Inn**
**London WC1R 5LX (GB)**

EP 0 390 558 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to substituted imidazole derivatives and their non-toxic, pharmaceutically acceptable acid addition salts, and their preparation, to pharmaceutical compositions containing the same and their use.

EP-A-194984 describes diphenyl substituted imidazoles which have $\alpha_2$-blocking and/or anti-convulsive activities. EP-A-311447, which form part of the prior art under Art 54(3) and (4) EPC, describes diphenyl substituted imidazoles such as 4(3,3-diphenylpropen-2-yl)-1H-imidazole, which inhibit aromatase.

The 4(5)-imidazole derivatives of the present invention have the general formula (Ia) and (Ib):

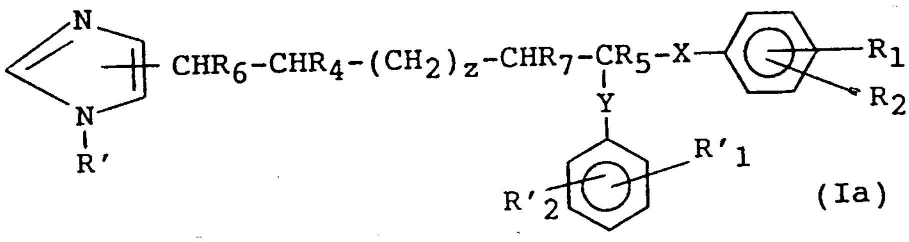

(Ia)

wherein $R_1$, $R_2$, $R'_1$ and $R'_2$ which can be the same or different, are H, $CH_3$, $C_2H_5$, $C_3H_7$, $OCH_3$, OH, $CH_2OH$, $NO_2$, $NH_2$, CN, $CF_3$, $CHF_2$, $CH_2F$ or halogen; R' is H or

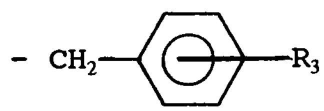

where $R_3$ is H, $CH_3$ or halogen; $R_4$ is H, $R_5$ is H or OH, $R_6$ is H or OH and $R_7$ is H or $R_4$ and $R_6$ together form a bond or $R_5$ and $R_7$ together form a bond; X and Y, which can be the same or different, are a bond, a straight $C_{1-2}$-alkyl or the corresponding alkenyl and z is 0 to 2 and

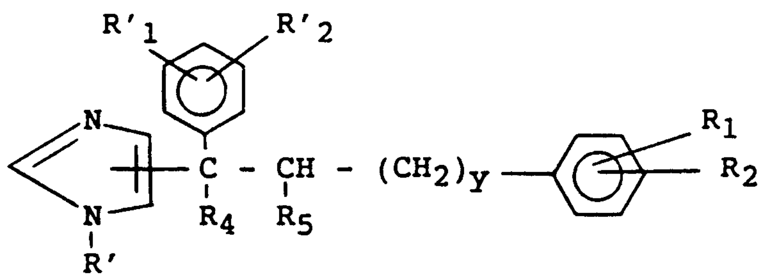

(Ib)

wherein $R_1$, $R_2$, $R'_1$ and $R'_2$, which can be the same or different, are H, $CH_3$, $C_2H_5$, $C_3H_7$, $OCH_3$, OH, $CH_2OH$, $NO_2$, $NH_2$, CN, $CF_3$, $CHF_2$, $CH_2F$ or halogen; R′ is H or

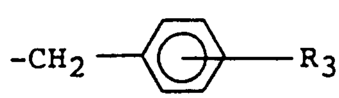

where $R_3$ is H, $CH_3$ or halogen; $R_4$ is H or OH and $R_5$ is H or $R_4$ and $R_5$ together form a bond and y is 0 to 4.

The non-toxic pharmaceutically acceptable acid addition salts of these compounds are also within the scope of the invention.

The compounds of the formulae (Ia) and (Ib) form acid addition salts with both organic and inorganic acids. They can thus form many pharmaceutically usable acid addition salts, as, for instance, chlorides,

bromides, sulfates, nitrates, phosphates, sulfonates, formates, tartrates, maleates, citrates, benzoates, salicylates, ascorbates and the like.

The invention includes within its scope pharmaceutical compositions comprising at least one of the compounds of formula (Ia) or (Ib) or a non-toxic, pharmaceutically acceptable salt thereof, and a compatible pharmaceutically acceptable carrier therefor.

The compounds of the present invention have been found, depending on the substituents R′, $R_1$, $R_2$, $R'_1$ and $R'_2$, to possess varying degrees of aromatase and desmolase inhibiting properties. Among them there are very selective enzyme aromatase inhibiting compounds which are valuable in the treatment of estrogen dependent diseases, e.g. breast cancer.

Compounds of formula (Ia) can be prepared by the following methods. Compounds of formula (Ia) wherein the branches

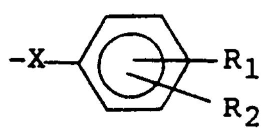

and

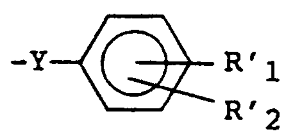

are identical, can be prepared by a successive sequence of reactions comprising a Grignard reaction of 4-(5)-imidazole derivative of the formula (IIa)

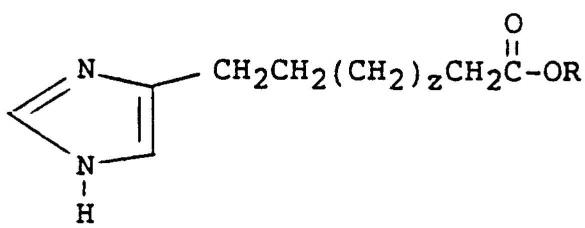

(IIa)

or its 1-benzyl derivative (IIIa) with an appropriate aryl- or arylalkylmagnesium halide (IVa) following the loss of water and hydrogenation.

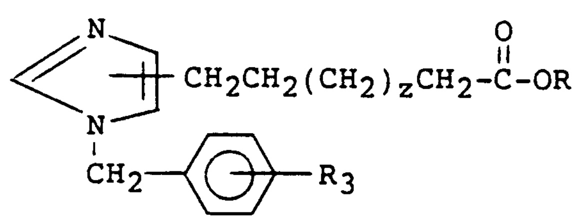

(IIIa)

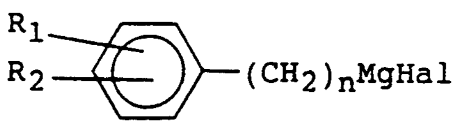

(IVa)

In the formulae (IIa) to (IVa) R is alkyl, preferably lower alkyl, n is 0 to 2 and Hal is halogen. The first reaction step, the Grignard reaction, leads to the following compounds of formula (Va):

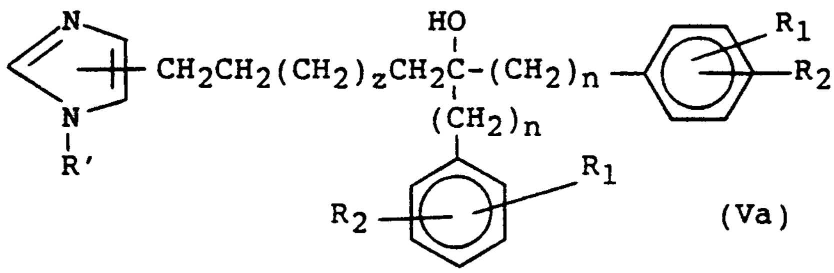

(Va)

In this reaction the aryl- or arylalkylmagnesium halide derivative can be, for example, an aryl- or arylalkylmagnesiumbromide derivative, which is prepared by reacting the corresponding aryl- or arylalkylbromide derivative with magnesium. The Grignard reagent (IVa) cannot be prepared if $R_1$ and/or $R_2$ are OH, $CH_2OH$ or $NH_2$. Suitable solvents for the reaction include a variety of ethers, preferably tetrahydrofuran.

The aryl- or arylalkylmagnesiumhalide derivative is prepared in the usual way by adding the aryl- or arylalkylhalide derivative in a suitable solvent, e.g. tetrahydrofuran, dropwise onto magnesium turnings covered by tetrahydrofuran, at the boiling point of the reaction mixture. When the magnesium turnings have reacted, the mixture is cooled slightly and the 4(5)-imidazole derivative (IIa) or its 1-benzylsubstituted derivative (IIIa) is added in solid form in small portions or dropwise in tetrahydrofuran. After the addition, the reaction mixture is refluxed until all of the 4(5)-imidazole derivative has reacted. The reaction time varies between one and five hours.

According to the feature of the invention, the compounds of formula (Ia), wherein $R_7$ and $R_5$ both are hydrogen or together form a bond, are prepared by dehydration of the compounds of formula (Ia), where $R_5$ is OH, and by catalytic addition of hydrogen in the second step. Water is eliminated by usual methods, i.e. by heating with concentrated hydrochloric acid or by heating with dry potassium hydrogen sulfate. The unsaturated compounds (VIa) (the compounds of formula (Ia) wherein $R_7$ and $R_5$ together form a bond) are isolated and after that hydrogenated. Alternatively they can be hydrogenated directly in an acidic medium without previous isolation. The hydrogenation is conveniently carried out at room temperature with good stirring in alcohol, e.g. ethanol in the presence of a catalyst in a hydrogen atmosphere. Suitable catalysts are for example platinium oxide, palladium-on-carbon or Raney-nickel.

The reaction scheme for these steps can be illustrated as follows:

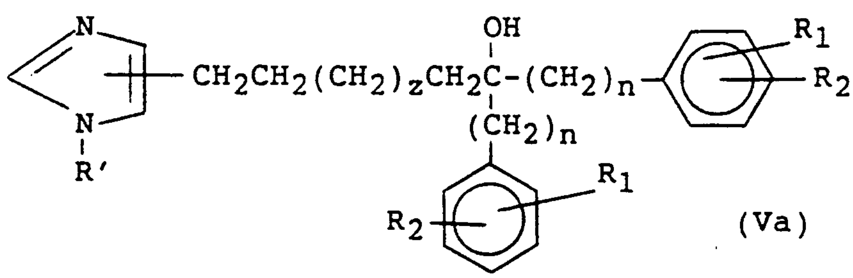

(Va)

wherein R′, $R_1$, $R_2$, z and n are as defined before

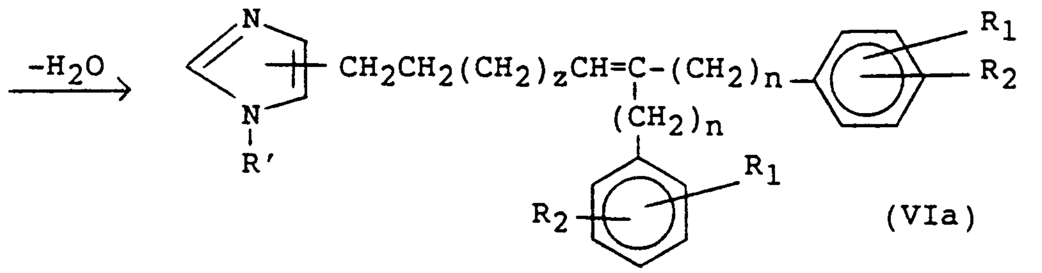


(VIa)

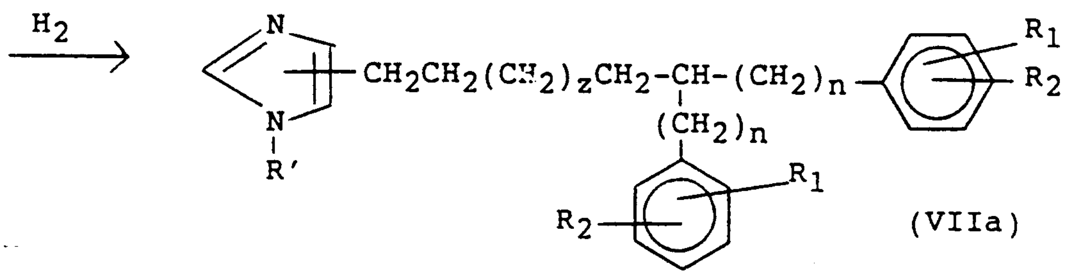


(VIIa)

If R′ is a substituted or unsubstituted benzyl, this group may be removed by hydrogenation as well. In this case the hydrogenation is performed in an acidic medium such as hydrochloric acid-ethanol mixture at elevated temperature.

The reaction scheme of this hydrogenation which leads to compounds of formula (Ia) wherein R′, $R_7$ and $R_5$ each are hydrogen can be illustrated as follows:

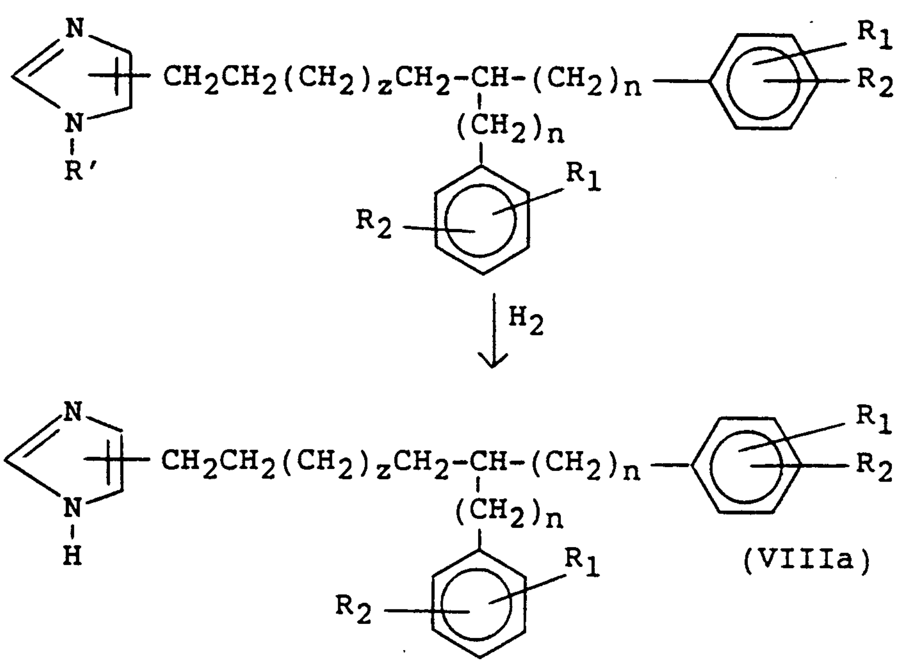


(VIIIa)

wherein $R_1$, $R_2$, z and n are as defined before.

Another method to remove the benzylic R′ group is a hydrogen transfer reaction in which the starting compound (VIIa) is refluxed with ammonium formate and 10 % Pd/C in an appropriate lower alcohol, such as methanol or ethanol, or its aqueous solution. The compounds (VIIIa) can also be prepared directly from the compounds (VIa) by hydrogen transfer reaction with ammonium formate or by hydrogenating both the double bond and the protecting benzyl group at the same time. The compounds (VIIIa) can also be prepared directly from the compounds (Va) by hydrogen transfer reaction in which the starting compound

(Va) is refluxed with ammonium formate and 10 % Pd/C in an acidic medium such as acetic acid.

The compounds of formula (VIIIa) can also be prepared from the compounds of formula (Va) wherein R′ is a substituted or unsubstituted benzyl by removing first the benzylic R′ by a hydrogen transfer reaction as described before to give the compounds of formula (IXa)

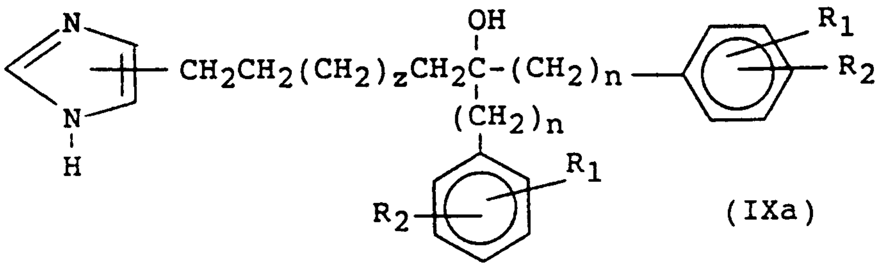

(IXa)

wherein $R_1$, $R_2$, z and n are as defined before.

The benzylic R′ can be removed by hydrogenation as well. The compounds of formula (IXa) are further dehydrated by the methods described before to form the compounds of formula (Ia) where $R_5$ and $R_7$ together form a bond (Xa).

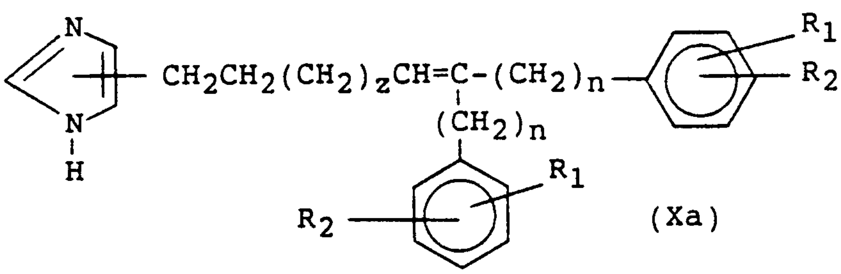

(Xa)

The compounds of formula (Xa) are further hydrogenated by the methods described before to give the compounds of formula (VIIIa).

When the compounds of formula (Ia) wherein $R_1$ and/or $R_2$ are OH, $CH_2OH$ or $NH_2$ are wanted, they can be prepared by the following reactions.

The compounds of formula (Ia) wherein $R_1$ and/or $R_2$ are OH can be prepared by reacting the 4(5)-imidazole derivative (IIa) or (IIIa) with a Grignard reagent (IVa) where $R_1$ and/or $R_2$ are $OCH_2Ph$ or OTHP (THP = tetrahydropyranyl) and then hydrogenating catalytically by the methods described to hydrogenate the benzylic R′ group. If R′ is a protecting benzyl group it will be removed conveniently at the same time. Another method is to dealkylate the compounds of formula (Ia) where $R_1$ and/or $R_2$ are $OCH_3$ by allowing them to react with $BBr_3$, for example.

The compounds of formula (Ia) wherein $R_1$ and/or $R_2$ are $CH_2OH$ may be prepared from the corresponding compounds where $R_1$ and/or $R_2$ are CN by conventional methods, i.e. by hydrolyzing the nitrile group and then catalytical reduction of the acid group.

The compounds of formula (Ia) wherein $R_1$ and/or $R_2$ are $NH_2$ can be prepared by hydrogenating the corresponding compounds where $R_1$ and/or $R_2$ are $NO_2$. The protecting benzyl group will be hydrogenated as well.

The compounds of formula (Ia) wherein $R_1$ and/or $R_2$ are CN can be prepared from the corresponding compounds where $R_1$ and/or $R_2$ are $NH_2$ by diazotization. Compounds of formula (Ia) wherein $R_1$ and/or $R_2$ are halogen may also optionally be prepared by the same method.

Compounds of formula (Ia) where R′ is a benzyl group can be prepared by benzylating the corresponding compounds where R′ is hydrogen. The starting compound is first treated with a strong base such as sodium hydroxide in water or sodium hydride in an appropriate solvent, e.g. dimethyl formamide, to give the alkali metal salt of the imidazole and then in the second step adding to this benzyl halide. The reaction scheme can be illustrated as follows:

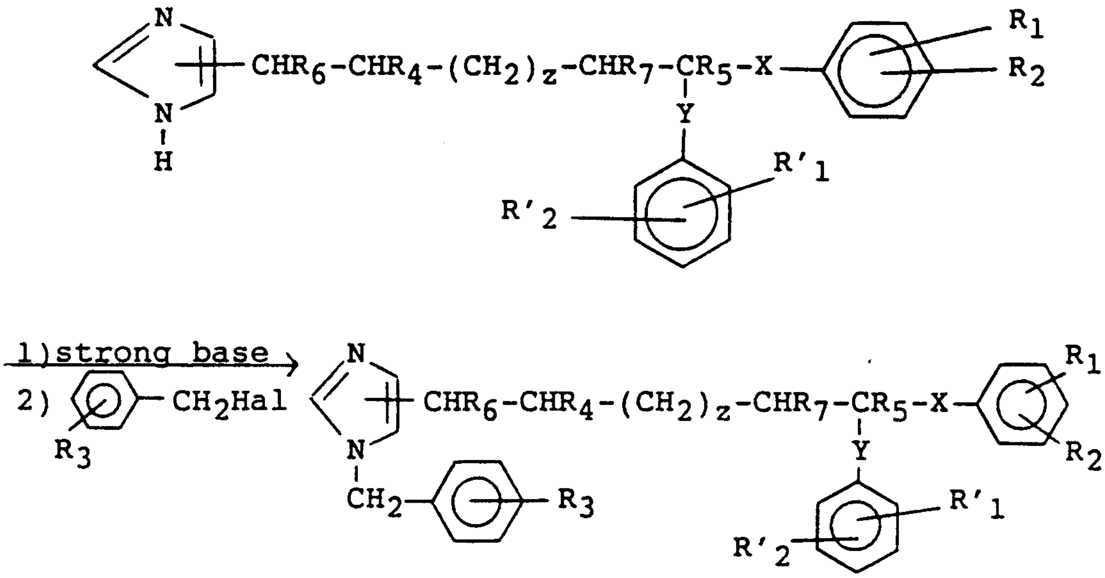


The free OH, $CH_2OH$ and $NH_2$ substituents must be protected during the benzylation reaction.

Another process for the preparations of compounds of formula (Ia) wherein the branches

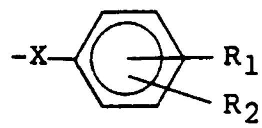

and

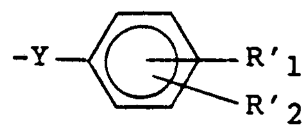

are different, comprises in the first stage a series of two successive Grignard reactions starting from 4(5)-imidazole derivative (IIa) or its 1-benzyl substituted derivative (IIIa) as previously. Now, however, the amount of the Grignard reagent is reduced as well as the reaction temperature, to stop the reaction at the ketone stage to give the ketone (XIa), which further is reacted with another Grignard reagent (XIIa) to give a compound of formula (Ia) where $R_5$ is OH. The reactions are illustrated as follows:

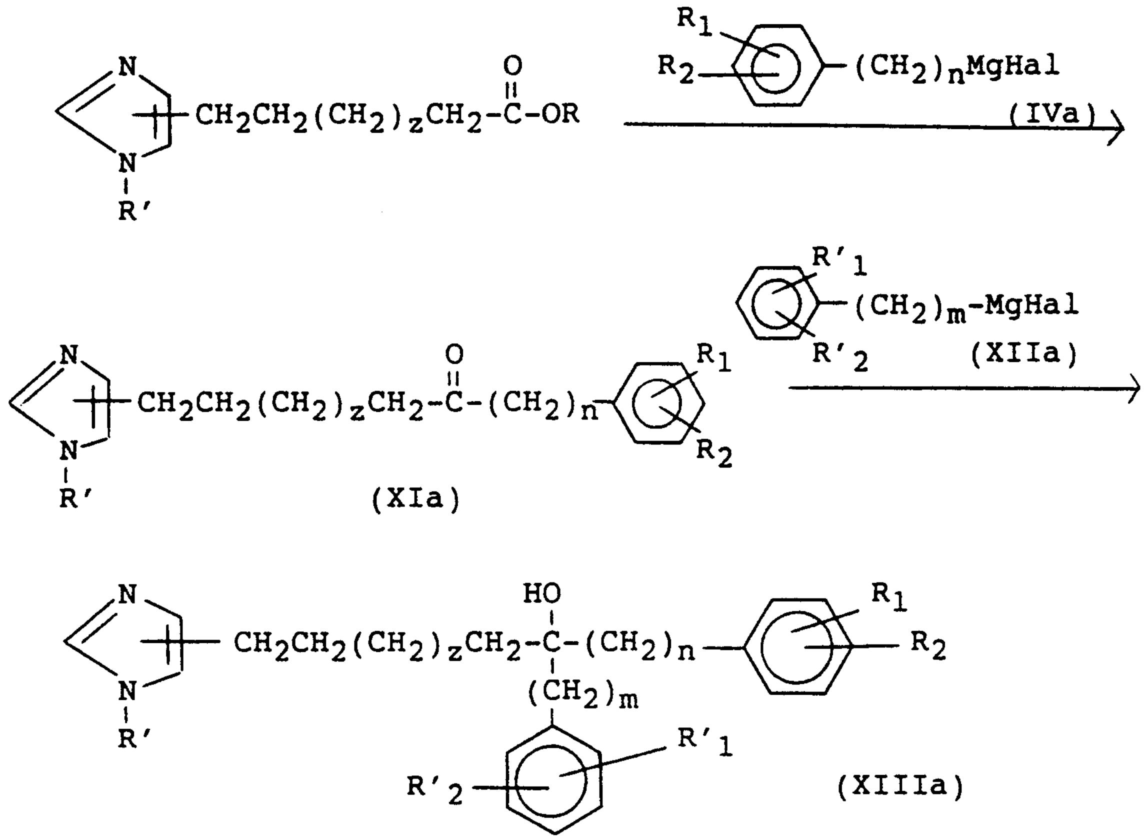


In the reaction scheme above m and n, which can be the same or different, are 0 to 2, z is 0 to 2 and R is an alkyl group, preferably a lower alkyl. The compounds of formula (XIIIa) are further dehydrated and hydrogenated by the methods described before to give the compounds of formula

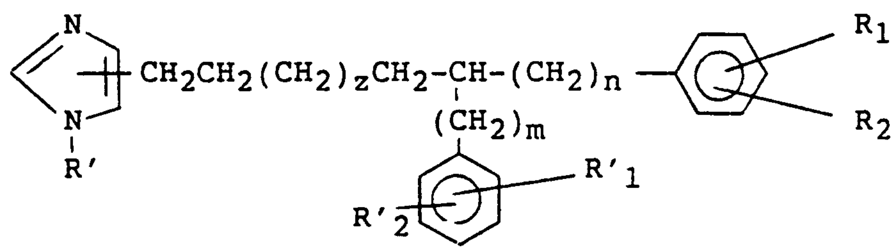

wherein R′, $R_1$, $R_2$, $R'_1$, $R'_2$, z, n and m are as defined before.

The Grignard reagents (IVa) and (XIIa) cannot be prepared when the substituents are OH, $CH_2OH$ or $NH_2$. When the compounds of formula (Ia), wherein one or more of the substituents $R_1$, $R_2$, $R'_1$ and $R'_2$ are OH are wanted they can be prepared by the following method.

The compounds of formula (Ia) wherein one or more of the substituents $R_1$, $R_2$, $R'_1$ and $R'_2$ are OH can be prepared by reacting the 4(5)-imidazole derivative (IIa) or (IIIa) first with a Grignard reagent (IVa) and then with reagent (XIIa) where the substituent/substituents of either compound (IVa) or (XIIa) or both of them are $OCH_2Ph$ or OTHP and hydrogenating catalytically by the methods described to hydrogenate the benzylic R′ group. The protecting benzyl group will be removed conveniently at the same time. Another method is to dealkylate the compounds of formula (Ia) where the substituent/substituents are $OCH_3$ by allowing them to react with $BBr_3$, for example.

The compounds of formula (Ia) wherein one or more of the substituents $R_1$, $R_2$, $R'_1$ and $R'_2$ are $CH_2OH$, $NH_2$ or CN can be prepared by the methods described before.

In order to achieve a better control of the reactions above, the starting material may be an amide

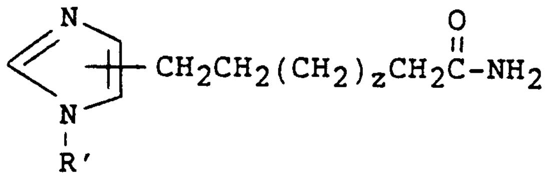

or a nitrile

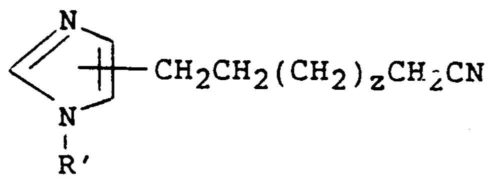

as well.

Choosing appropriate conditions for the dehydration of the compounds of formula (Ia) where $R_5$ is OH results in the corresponding compounds of formula (Ia) where one of the alkyl chains X or Y is transformed to the corresponding alkenyl chain.

The starting compounds of the formulae (IIa) and (IIIa) may be prepared for example from 4(5)-imidazole alkyl acid of the formula

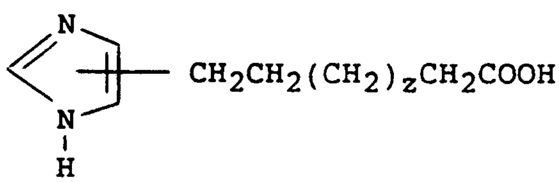

and its 1-benzyl derivative of the formula

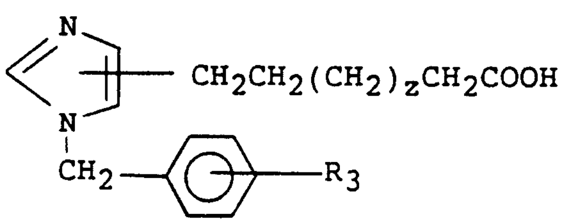

wherein z and $R_3$ are as defined before, by esterifying them according to the method described in US patent No. 3759944.

A further method of preparing the compounds of formula (Ia) is the Wittig reaction where the starting compound is an 4(5)-imidazole aldehyde (XIVa). In the formula (XIVa) R′ is as defined before.

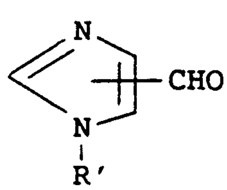

(XIVa)

In the Wittig reaction the first step is to prepare a phosphonium salt (XVa) from the corresponding halogenated hydrocarbon (XVIa) by reacting it with triphenylphosphine. The reaction scheme can be illustrated as follows:

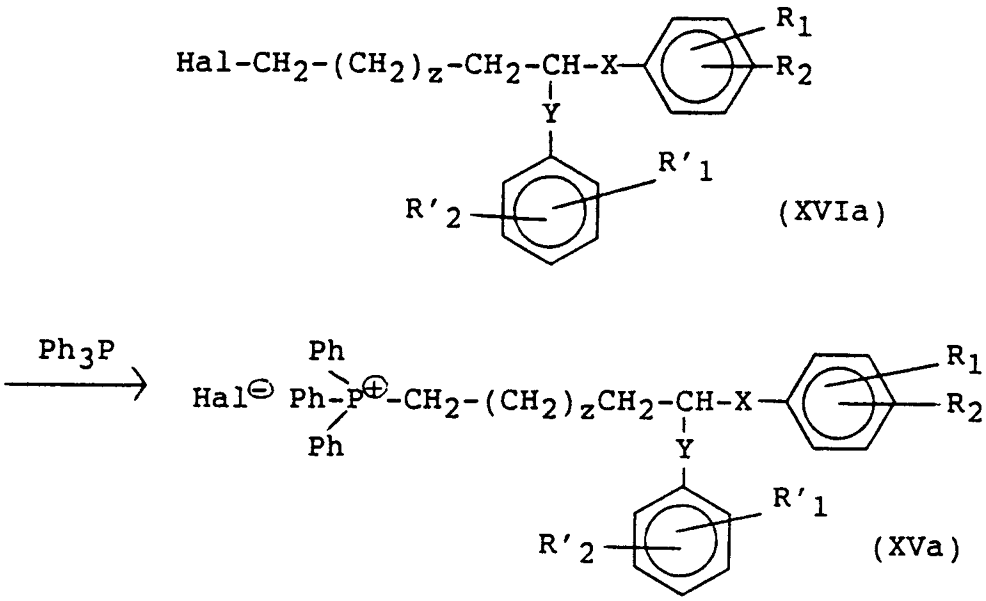


(XVIa)

(XVa)

in which $R_1$, $R_2$, $R'_1$, $R'_2$, X, Y and z are as defined before and Hal is halogen.

In the second step of the Wittig reaction the compound (XVa) is treated with a strong base to form a phosphorus ylide which is further allowed to react with the 4(5)-imidazole aldehyde (XIVa) to achieve the compounds of formula (Ia) wherein $R_4$ and $R_6$ together form a bond (XVIIa). The strong base can be NaH or BuLi in a proper solvent such as dimethoxyethane, tetrahydrofuran or DMF. Further alkali metal alkoxides the corresponding alcohols as solvent and NaH in DMSO can be used as proton acceptors. The compounds (XVIIa) are isolated and after that hydrogenated as has been described before to achieve the compounds of formula (Ia) wherein $R_4$ and $R_6$ both are hydrogen. The reaction scheme for these steps can be illustrated as follows:

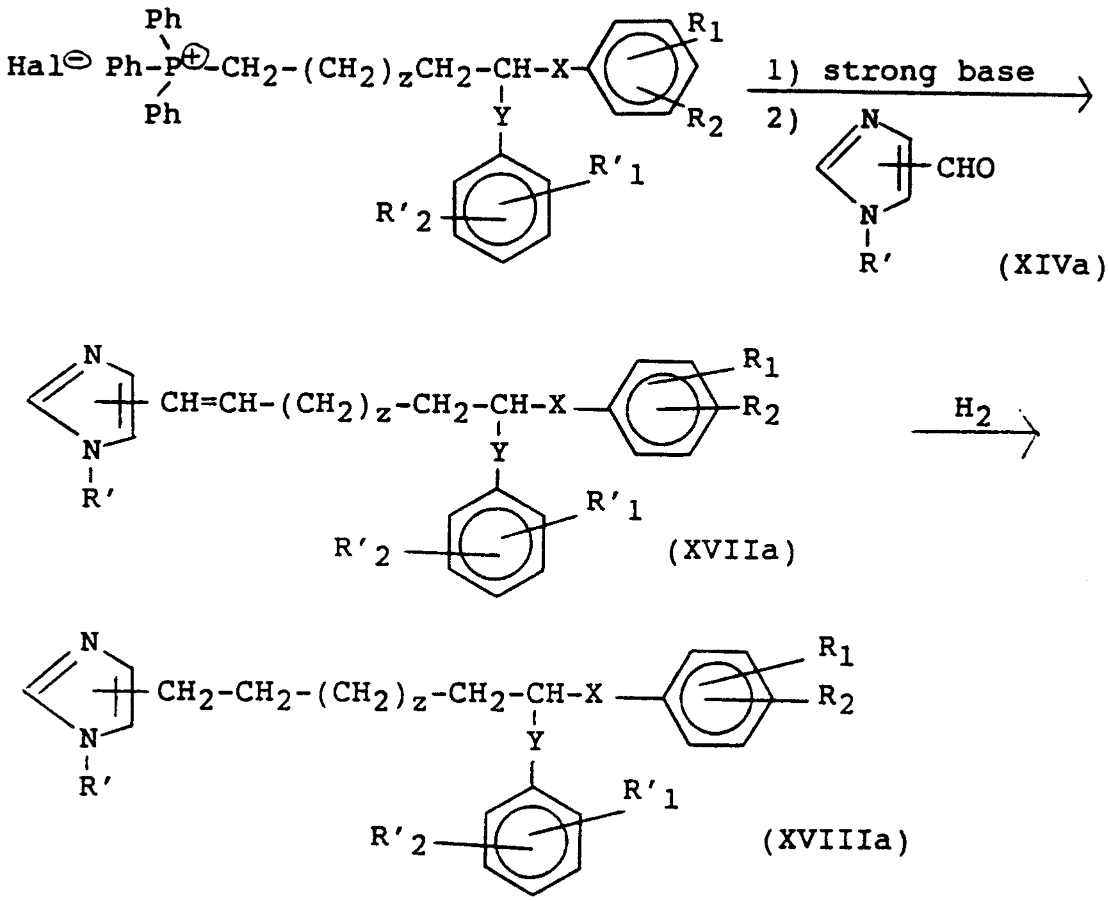


(XIVa)

(XVIIa)

(XVIIIa)

The Wittig reaction can be performed in another order i.e. a phosphonium salt (XIXa) is prepared from the corresponding halogenated 4(5)-imidazole derivative (XXa) by reacting it with triphenylphosphine. In the second step the compound (XIXa) is allowed to react with a strong base and then with a substituted ketone of the formula (XXIa) as described before to give the compounds of formula (Ia) wherein $R_5$ and $R_7$ together form a bond (XXIIa). The reaction scheme for these steps can be illustrated as follows:

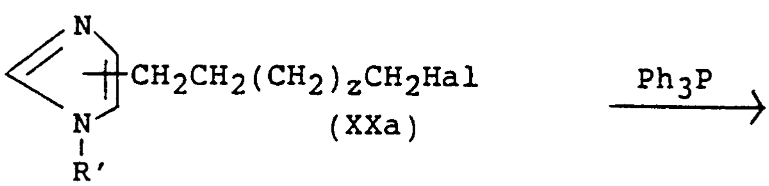


(XXa)

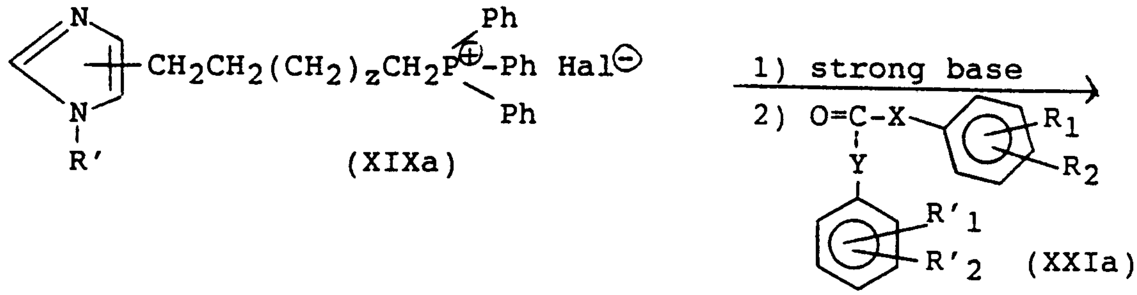


(XIXa)

(XXIa)

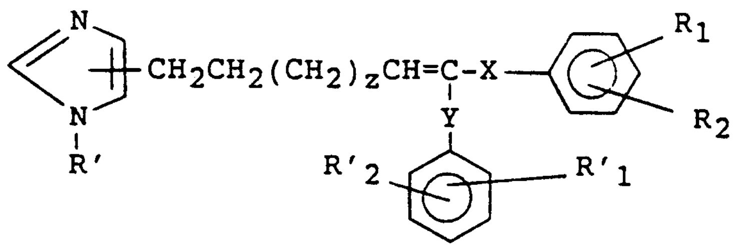

(XXIIa)

wherein R', $R_1$, $R_2$, $R'_1$, $R'_2$, z, X and Y are as defined before.

The compounds of formula (Ia) can also be prepared by a modified Wittig reaction, namely the Horner-Emmons or Wadsworth-Emmons reaction where the phosphonate (XXIIIa) which is prepared from the halogenated hydrocarbon (XVIa) and a triester of phosphonic acid (e.g. $(EtO)_3P$) by the Arbuzow reaction reacts firstly with a base (e.g. NaH in DMSO or in dimethoxyethane) and then with the aldehyde (XIVa). The product (XVIIa) formed is a compound of formula (Ia) where $R_4$ and $R_6$ together form a bond. The reaction scheme can be illustrated as follows:

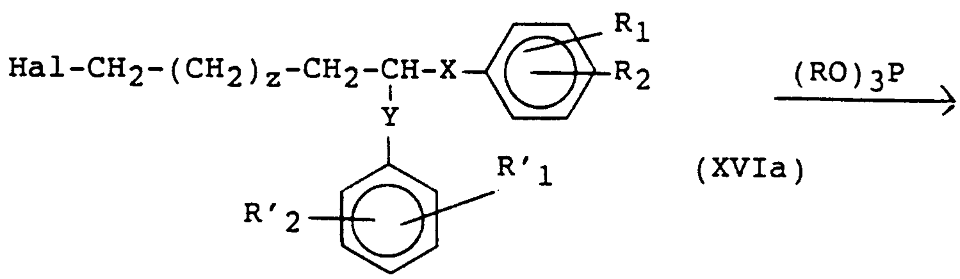


(XVIa)

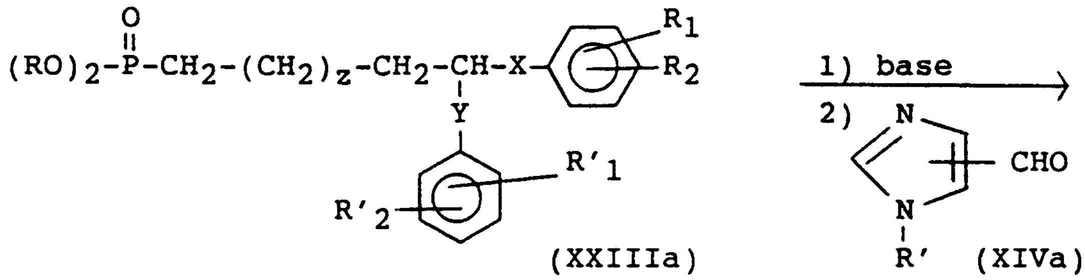


(XXIIIa) (XIVa)

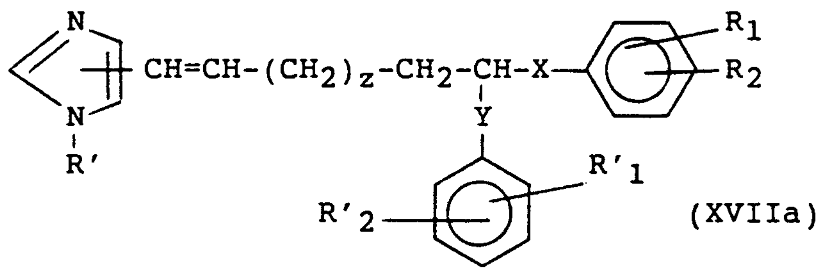

(XVIIa)

In the formula (XXIIIa) R is alkyl with 1-4 carbon atoms and $R_1$, $R_2$, $R'_1$, $R'_2$, X, Y and z are as defined before. The unsaturated compounds (XVIIa) are further hydrogenated to form the compounds of formula (Ia) wherein $R_4$ and $R_6$ both are hydrogen. The benzylic R′ group and the double bond of the compounds of formula (XVIIa) can be removed at the same time by the methods described before.

Another useful method to prepare compounds of formula (Ia) is the Grignard reaction in which the 4(5)-imidazole aldehyde (XIVa) is allowed to react with a Grignard reagent (XXIVa) to give a compound of formula (Ia) where $R_6$ is OH (XXVa). The Grignard reagent is prepared by reacting the corresponding halogenated hydrocarbon with magnesium turnings in the usual way. The compound (XXVa) is further dehydrated by heating with $KHSO_4$ to achieve the compounds of formula (Ia) where $R_4$ and $R_6$ together form a bond (XVIIa).

The unsaturated derivatives are then hydrogenated to form the compounds of formula (Ia) wherein $R_4$ and $R_6$ both are hydrogen. The reaction scheme for these steps can be illustrated as follows:

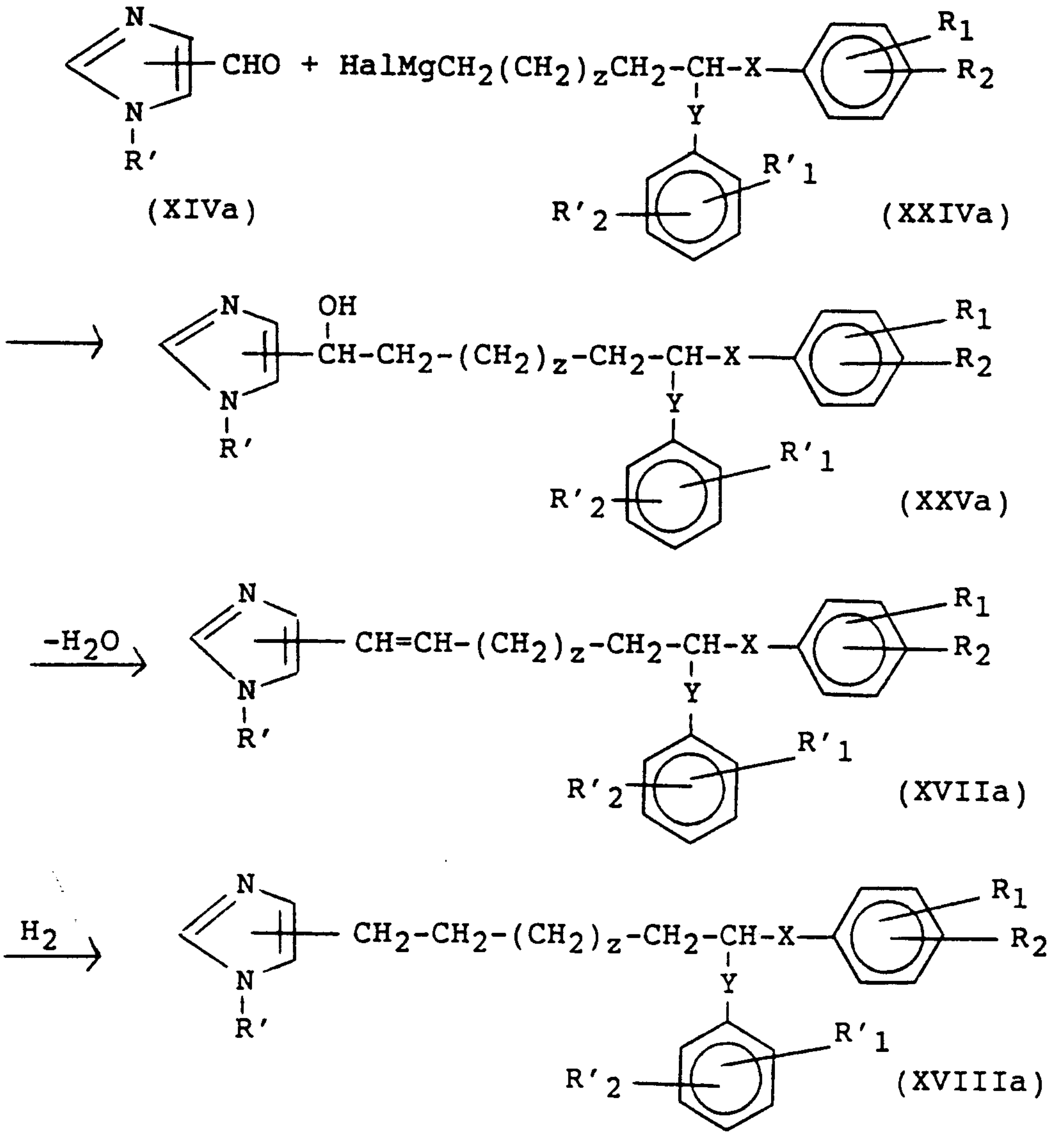


(XIVa) (XXIVa)

(XXVa)

(XVIIa)

(XVIIIa)

In the formulae (XXVa), (XVIIa) and (XVIIIa) R′, $R_1$, $R_2$, $R'_1$, $R'_2$, X, Y and z are as defined before.

If R′ is substituted or unsubstituted benzyl, this group may be removed by hydrogenation and hydrogen transfer reaction as described before to give the compounds of formula (XXVIa).

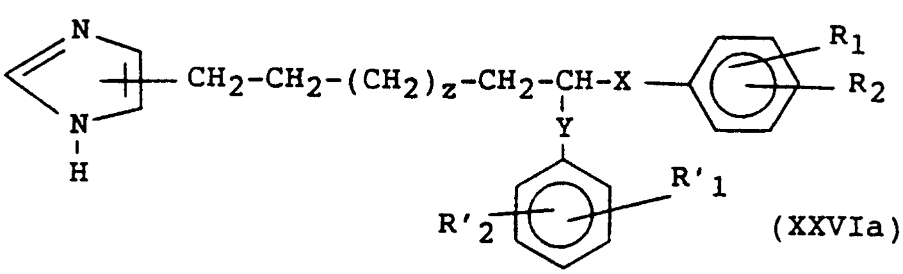

(XXVIa)

The compounds of formula (XXVIa) can also be prepared directly from the compounds (XVIIa) and (XXVa) by the methods described before.

The Grignard reagent (XXIVa) cannot be prepared when the substituents are OH, $CH_2OH$ or $NH_2$. The compounds of formula (Ia) wherein one or more of the substituents $R_1$, $R_2$, $R'_1$ and $R'_2$ are OH, $CH_2OH$ or $NH_2$ can be prepared according to the methods described before.

Further method to prepare the compounds of formula (Ia) wherein one or more of the substituents $R_1$, $R_2$, $R'_1$ and $R'_2$ are $NO_2$ is nitration of the corresponding compounds wherein one or more of the substituents $R_1$, $R_2$, $R'_1$ and $R'_2$ are H.

Compounds of formula (Ib) can be prepared by the following methods.

Compounds of formula (Ib) can be prepared by a successive sequence of reactions comprising firstly a Grignard reaction of 4(5)-imidazole aldehyde (IIb)

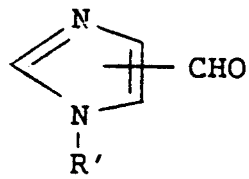

(IIb)

with an appropriate arylalkylmagnesiumhalide (IIIb)

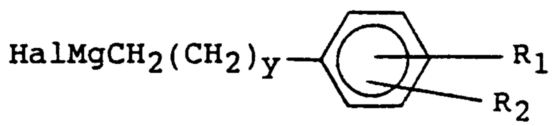

which leads to following compounds (IVb)

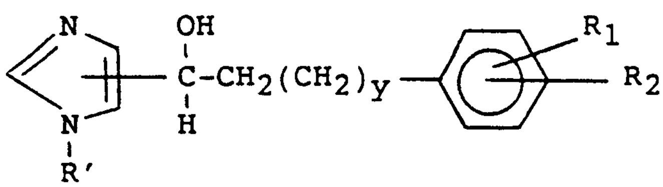

(IVb)

In the reaction the arylalkylmagnesium halide derivative can be, for example, an arylalkylmagnesiumbromide derivative, which is prepared by reacting the corresponding arylalkylbromide derivative with magnesium. Suitable solvents for the reaction include a variety of ethers, preferably tetrahydrofuran.

The arylalkylmagnesiumhalide derivative is prepared in the usual way by adding the arylalkylhalide derivative in a suitable solvent, e.g. tetrahydrofuran, dropwise onto magnesium turnings covered by tetrahydrofuran, at the boiling point of the reaction mixture. When the magnesium turnings have reacted, the mixture is cooled slightly and the 4(5)-imidazole aldehyde (IIb) is added in solid form in small portions or dropwise in tetrahydrofuran. After the addition, the reaction mixture is refluxed until all of the 4(5)-imidazole aldehyde (IIb) has reacted. The reaction time varies between one and five hours.

The compounds (IVb) are further oxidized for example with manganese dioxide to achieve compounds of formula (Vb) which are allowed to react with another Grignard reagent (VIb) to give the compounds of formula (Ib) where $R_4$ is OH (VIIb). The reaction scheme for these steps can be illustrated as follows:

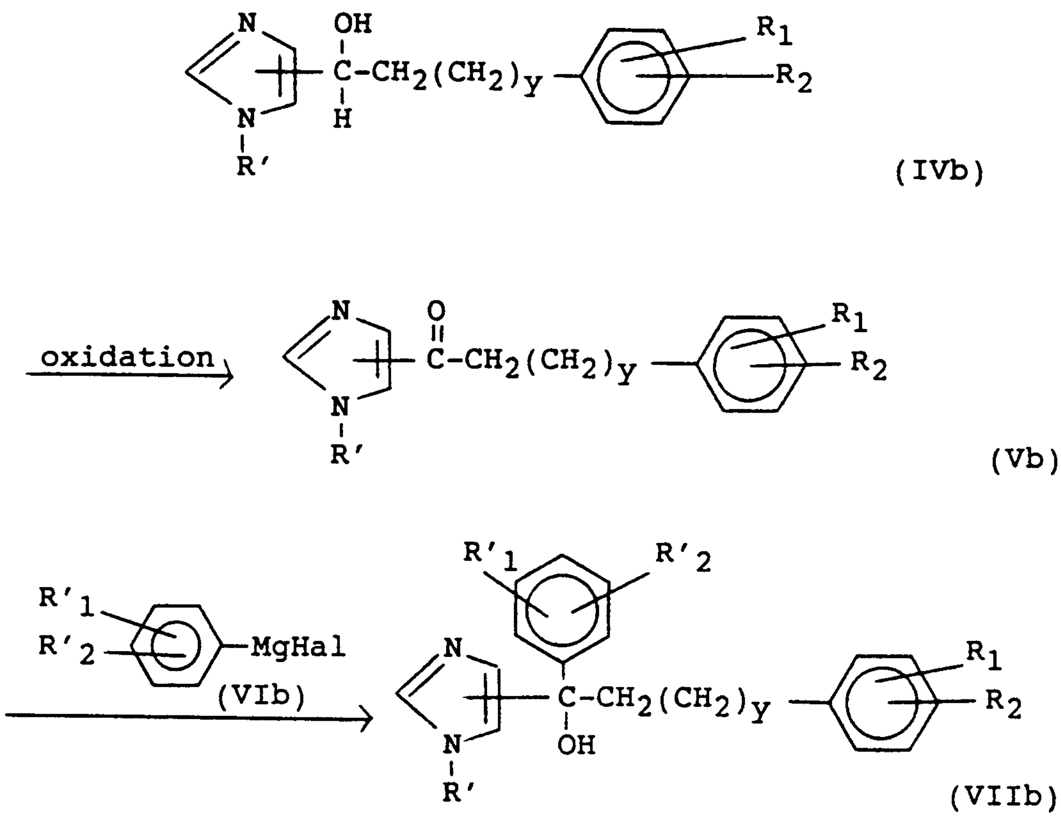


(IVb)

(Vb)

(VIIb)

The arylmagnesium halide (VIb) is prepared by reacting the corresponding halogenated aromatic compound with magnesium turnings in the usual way.

Compounds of formula (Ib) can also be prepared by reacting an 4(5)-imidazole aldehyde (IIb) with an arylmagnesiumhalide (VIb) which leads to following compounds (VIIIb)

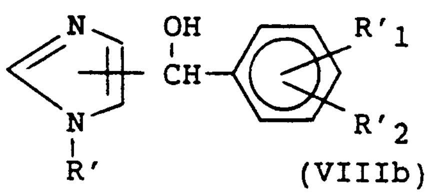

(VIIIb)

The compounds (VIIIb) are further oxidized for example with manganese dioxide to achieve the compounds of formula (IXb) which are further allowed to react with the Grignard reagent (IIIb) to give the compounds of formula (Ib) where $R_4$ is OH (VIIb). The reaction scheme for these steps can be illustrated as follows:

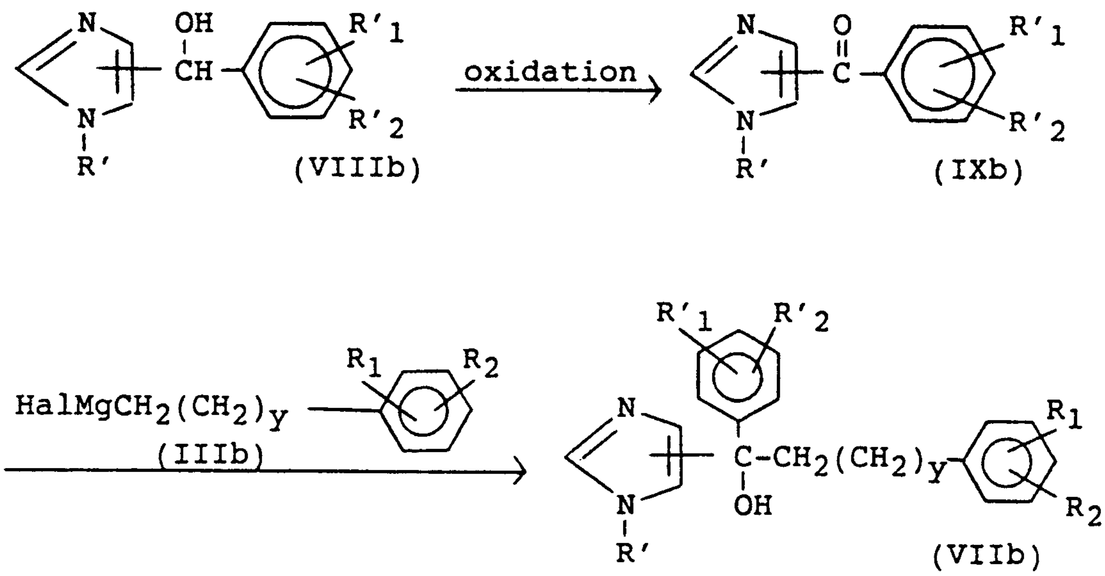


(VIIIb) (IXb) (VIIb)

According to the feature of the invention, the compounds of formula (Ib), wherein $R_4$ and $R_5$ both are hydrogen or together form a bond, are prepared by dehydration of the compounds of formula (Ib), where $R_4$ is OH, and by catalytic addition of hydrogen in the second step. Water is eliminated by usual methods, i.e. by heating with concentrated hydrochloric acid or by heating with dry potassium hydrogen sulfate. The unsaturated compounds (Xb) (the compounds of formula (Ib) wherein $R_4$ and $R_5$ together form a bond) are isolated and after that hydrogenated. Alternatively they can be hydrogenated directly in an acidic medium without previous isolation. The hydrogenation is conveniently carried out at room temperature with good stirring in alcohol, e.g. ethanol in the presence of a catalyst in a hydrogen atmosphere. Suitable catalysts are for example platinium oxide, palladium-on-carbon or Raney-nickel.

The reaction scheme for these steps can be illustrated as follows:

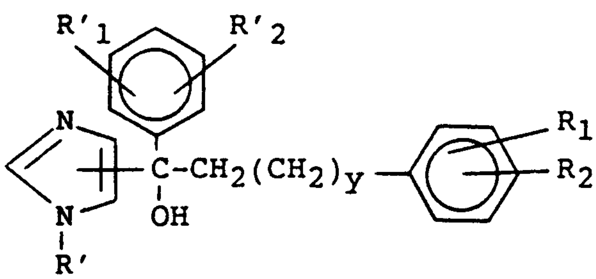

(VIIb)

wherein R′, $R_1$, $R_2$, $R'_1$, $R'_2$ and y are as defined before

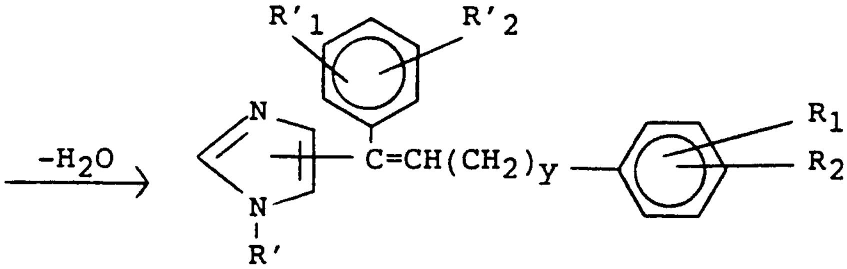


(Xb)

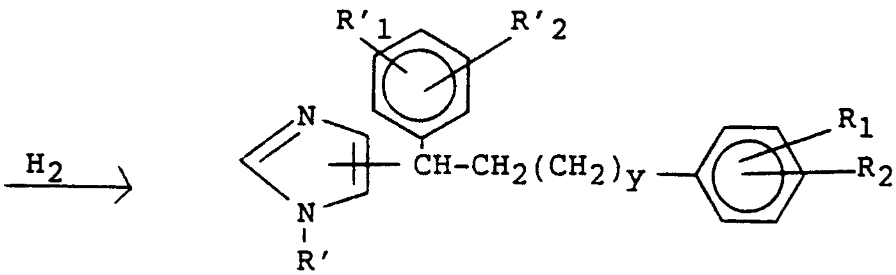


(XIb)

wherein R′, $R_1$, $R_2$, $R'_1$, $R'_2$ and y are as defined before.

If R′ is a substituted or unsubstituted benzyl, this group may be removed by hydrogenation as well. In this case the hydrogenation is performed in an acidic medium such as hydrochloric acid-ethanol mixture at elevated temperature.

The reaction scheme of this hydrogenation which leads to compounds of formula (Ib) wherein R′, $R_4$ and $R_5$ each are hydrogen can be illustrated as follows:

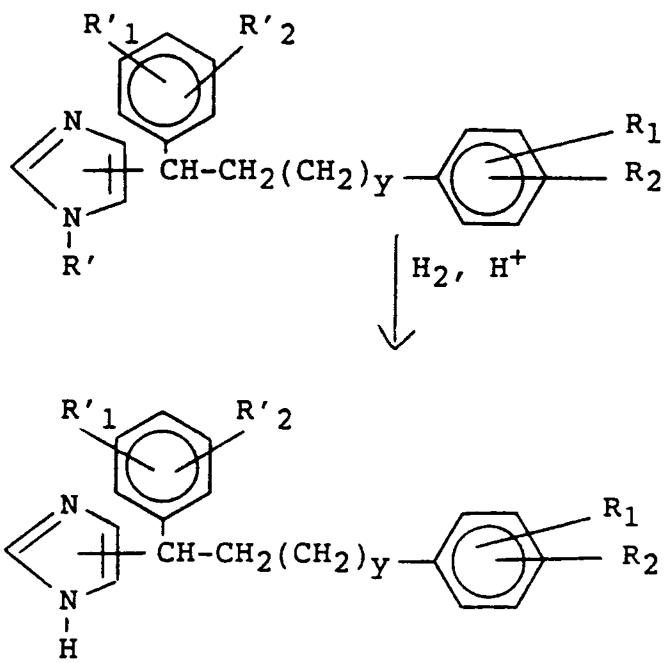


(XIb)

(XIIb)

Another method to remove the benzylic R′ group is a hydrogen transfer reaction in which the starting compound (XIb) is refluxed with ammonium formate and 10 % Pd/C in an appropriate alcohol, such as

methanol or ethanol, or its aqueous solution. The compounds (XIIb) can also be prepared directly from the compounds (Xb) by hydrogen transfer reaction with ammonium formate or by hydrogenating both the double bond and the protecting benzyl group at the same time. The compounds (XIIb) can also be prepared directly from the compounds (VIIb) by hydrogen transfer reaction in which the starting compound (VIIb) is refluxed with ammonium formate and 10 % Pd/C in an acidic medium such as acetic acid.

The compounds of formula (XIIb) can also be prepared from the compounds of formula (VIIb) wherein R′ is a substituted or unsubstituted benzyl by removing first the benzylic R′ by a hydrogen transfer reaction by the method described before to give the compounds of formula (XIIIb)

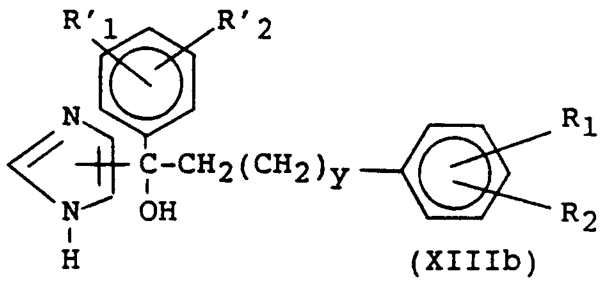

(XIIIb)

wherein $R_1$, $R_2$, $R'_1$, $R'_2$ and y are as defined before. The benzylic R′ can be removed by hydrogenation as well. The compounds of formula (XIIIb) are further dehydrated by the methods described before to form the compounds of formula (Ib) where $R_4$ and $R_5$ together form a bond (XIVb).

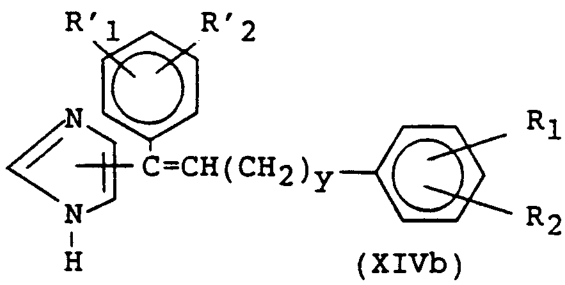

(XIVb)

The compounds of formula (XIVb) are further hydrogenated by the methods described before to give the compounds of formula (XIIb).

The benzylic R′ can be removed already before the oxidizing reaction by the methods described before. The reaction scheme for this hydrogenation can be illustrated as follows:

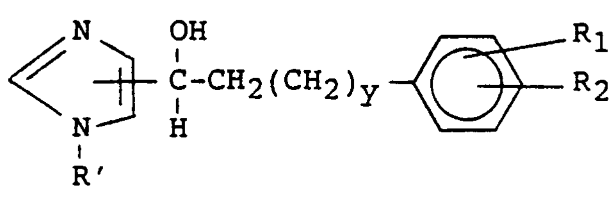

(IVb)

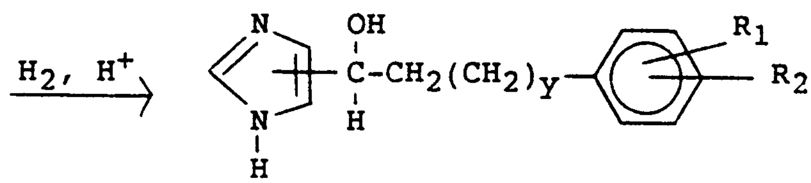


The Grignard reagents (IIIb) and (VIb) cannot be prepared when the substituents are OH, $CH_2OH$ or $NH_2$. The compounds of formula (Ib) wherein one or more of the substituents $R_1$, $R_2$, $R'_1$ and $R'_2$ are OH, $CH_2OH$ or $NH_2$ can be prepared by the following methods.

The compounds of formula (Ib) wherein one or more of the substituents $R_1$, $R_2$, $R'_1$ and $R'_2$ are OH can be prepared by reacting the 4(5)-imidazole derivative (IIb) first with a Grignard reagent (IIIb) and then with reagent (VIb) or revise order where the substituent/substituents of either compound (IIIb) or (VIb) or both of them are $OCH_2Ph$ or OTHP (THP = tetrahydropyranyl) and then hydrogenating catalytically by the methods described to hydrogenate the benzylic R′ group. If R′ is a protecting benzyl group it will be removed conveniently at the same time. Another method is to dealkylate the compounds of formula (Ib) where the substituent/substituents are $OCH_3$ by allowing them to react with $BBr_3$, for example.

The compounds of formula (Ib) wherein one or more of the substituents $R_1$, $R_2$, $R'_1$ and $R'_2$ are $CH_2OH$ may be prepared from the corresponding compounds where the substituent/substituents are CN by conventional methods, i.e. by hydrolyzing the nitrile group and then reducing the acid group.

The compounds of formula (Ib) wherein one or more of the substituents $R_1$, $R_2$, $R'_1$ and $R'_2$ are $NH_2$ can be prepared by hydrogenating the corresponding compounds where the substituent/substituents are $NO_2$. The protecting benzyl group will be hydrogenated as well.

The compounds of formula (Ib) wherein one or more of the substituents $R_1$, $R_2$, $R'_1$ and $R'_2$ are CN can be prepared from the corresponding compounds where one or more of the substituents are $NH_2$ by diazotization. Compounds of formula (Ib) wherein one or more of the substituents $R_1$, $R_2$, $R'_1$ and $R'_2$ are halogen may also optionally be prepared by the same method.

Another method of preparing compounds of formula (Ib) is a McMurry reaction which comprises a reductive coupling of a benzoylimidazole (IXb)

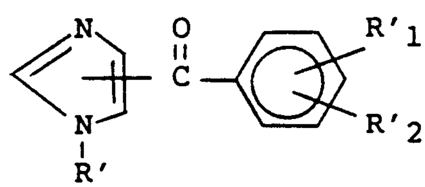

(IXb)

and an appropriate aldehyde of the formula (XVb)

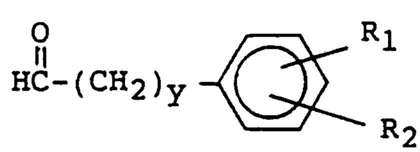

(XVb)

in an appropriate solvent, such as tetrahydrofuran or dimethoxyethane, in the presence of a low valent titanium reagent in an inert atmosphere, e.g. in nitrogen or argon, to give the compounds of formula (Ib) where $R_4$ and $R_5$ together form a bond (Xb)

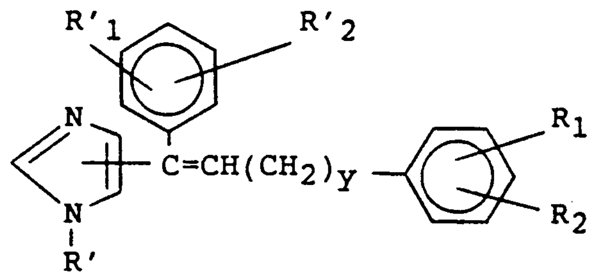

(Xb)

The unsaturated compounds (Xb) are further hydrogenated as described before. The aldehyde (XVb) is prepared from the corresponding alcohol in the usual way.

When the compounds of formula (Xb) where $R'_1$ and/or $R'_2$ are $CH_2OH$, $NH_2$ or CN are wanted they can be prepared by the methods described before.

The compounds of formula (Xb) where $R'_1$ and/or $R'_2$ are OH and $R_1$ and $R_2$ are as defined before can be prepared by a McMurry reaction from the compounds of formula (IXb) where $R'_1$ and/or $R'_2$ are OH which can be prepared by hydrogenating catalytically the compounds of formula (IXb) wherein $R'_1$ and/or $R'_2$ are $OCH_2Ph$ or OTHP by the methods described before. Alternatively the hydrogenation can be done before the oxidizing reaction. If R' is a protecting benzyl group it will be removed conveniently at the same time. Another method is to dealkylate the compounds of formula (IXb) where $R'_1$ and/or $R'_2$ are $OCH_3$ by allowing them to react with $BBr_3$, for example.

Compounds of formula (Ib) wherein $R'_1$ and/or $R'_2$ are OH and $R_1$ and $R_2$ are as defined before can be prepared by a McMurry reaction in which the aldehyde (XVb) is allowed to react with a ketone (IXb) wherein $R'_1$ and/or $R'_2$ are $OCH_2Ph$ or OTHP to give a compound of formula (Xb) where $R'_1$ and/or $R'_2$ are $OCH_2Ph$ or OTHP and $R_1$ and $R_2$ are as defined before which is further hydrogenated to give compounds of formula (XIIb) where $R'_1$ and/or $R'_2$ are OH.

Compounds of formula (Ib) where R' is a benzyl can be prepared by benzylating the corresponding compounds where R' is hydrogen. The starting compound is first treated with a strong base such as sodium hydroxide in water or sodium hydride in an appropriate solvent, e.g. dimethyl formamide, to give the alkali metal salt of the imidazole and then in the second step adding to this benzyl halide. The reaction scheme can be illustrated as follows:

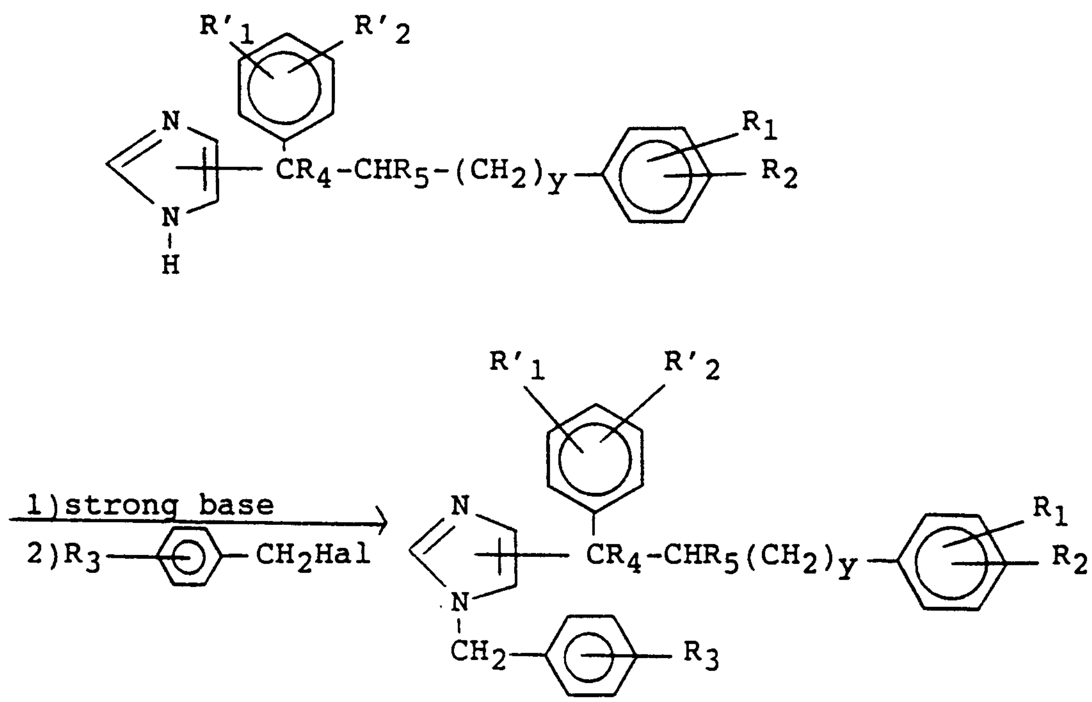


The free OH, $CH_2OH$ and $NH_2$ substituents must be protected during the benzylation reaction.

Further method to prepare the compounds of formula (Ib) wherein one or more of the substituents $R_1$, $R_2$, $R'_1$ and $R'_2$ are $NO_2$ is nitration of the corresponding compounds where one or more of the substituents are H.

The compounds of formula (Ia) and (Ib), their non-toxic, pharmaceutically acceptable acid salts or mixtures thereof may be administered parenterally, intravenously or orally. Typically, an effective amount of the compound is combined with a suitable pharmaceutical carrier. As used herein, the term "effective amount" encompasses those amounts which yield the desired activity without causing adverse side-effects. The precise amount employed in a particular situation is dependent upon numerous factors such as method of administration, type of mammal, condition for which the derivative is administered, etc., and of course the structure of the compound.

The pharmaceutical carriers which are typically employed with the compounds of the present invention may be solid or liquid and are generally selected with the planned manner of administration in mind. Thus, for example, solid carriers include lactose, sucrose, gelatin and agar, while liquid carriers include water, syrup, peanut oil and olive oil. Other suitable carriers are well-known to those skilled in the art of pharmaceutical formulations. The combination of the compound and the carrier may be fashioned into numerous acceptable forms, such as tablets, capsules, suppositories, solutions, emulsions and powders.

The compounds of the invention are especially valuable as aromatase inhibiting agents and are therefore useful in the treatment of estrogen dependent diseases, e.g. breast cancer.

Estrogens are essential steroids in the physiology and function of normal development of breast and sex organs in women. On the other hand estrogens are known to stimulate the growth of estrogen dependent cancers, especially breast and endometrial cancers, and they may increase the risk of development of breast cancer if given at pharmacological doses for a long time. Excessive production of estradiol may also cause other, benign disorders in hormone dependent organs. The importance of estrogens as cancer growth stimulators and/or regulators is clearly stressed by the fact that antiestrogens have reached a central position in the treatment of estrogen receptor rich breast cancers. Antiestrogens act by binding to estrogen receptors and thereby inhibiting the biological effects of estrogens. Another approach for blocking estrogen effect is to inhibit the synthesis of estrogens. This has been achieved clinically by the unspecific steroid synthesis inhibitor aminoglutethimide. The estrogen synthesis could be blocked specifically by inhibiting the enzyme aromatase, which is the key enzyme in biochemical estrogen synthesis pathway. Aromatase inhibition is important because several breast tumors synthesize estradiol and estrone in situ and exhibit therefore continuous growth stimulation (Alan Lipton et al., Cancer 59:779-782, 1987).

The ability of the compounds of the invention to inhibit the enzyme aromatase has been tested by in vitro assay according to M. Pasanen (Biological Research in Pregnancy, vol. 6, No. 2, 1985, pp. 94-99). Human aromatase enzyme was used. The enzyme was prepared from human placenta, which is rich of the enzyme. Microsomal fraction (100000 x g precipitate) was prepared by centrifugation. The enzyme preparation was used without further purification. Test compounds were added together with 100000 dpm of 1,2[$^3$H]-androstene-3,17-dione and NADPH generating system. The concentrations of the test compounds were 0,001; 0,01; 0,1 and 1,0 mM. The incubation was carried out at 37 °C for 40 min. Aromatization of 1,2-[$^3$H]-androstene-3,17-dione results in the production of $^3H_2O$. The tritiated water and the tritiated substrate are easily separated by a Sep-Pak$^R$ minicolumn, which absorbs the steroid but allows free water elution. Radioactivity was counted by a liquid scintillation counter. Aromatase inhibition was evaluated by comparing the $^3H_2O$-radioactivity of inhibitor treated samples to controls containing no inhibitor. IC-50 values were calculated as concentrations which inhibited the enzyme activity 50 %. These concentrations are presented in Table 2.

Cholesterol side chain cleavage (CSCC) activity (desmolase) was measured according to the method of Pasanen and Pelkonen (Steroids 43:517-527, 1984). Incubations were carried out in 1,5 ml Eppendorf plastic tubes, and an Eppendorf shaker, centrifuge and incubator were used as a unit. In a 300 $\mu$l incubation volume, the substrate (5 $\mu$M) was prepared according to Hanukoglu and Jefcoate (J. Chromatogr. 190:256-262, 1980), and 100000 dpm of radioactive $^3$H-4-cholesterol (the purity of the compound was checked by TLC) in 0,5 % Tween 20, 10 mM $MgCl_2$, 5 $\mu$M cyanoketone and 2 mM NADPH was added. Controls contained all the above substances but the enzyme preparation was inactivated prior to the incubation by the addition of 900 $\mu$l of methanol. The mitochondrial fraction (1 mg protein) from human placenta or bovine adrenals was used as a source of enzyme. After 30 min incubation at 37 °C, the reaction was terminated by the addition of 900 $\mu$l of methanol; 1500 dpm of marker $^{14}$C-4-pregnenolone was added to each incubate and the tubes were vigorously shaken. After 10 min equilibration, the methanol-precipitated proteins were separated by centrifugation (8000 x g for 2 min) and the supernatant was sucked into 1 ml plastic injection syringe and loaded onto the pre-equilibrated (75 % methanol) minicolumn. The column was washed with

one ml of 75 % methanol and then with 3 ml of 80 % methanol. The 80 % methanol eluate was run into the counting vial and 10 ml of scintillation liquid was added. Radioactivity was counted using a double-label program on a liquid scintillation counter (LKB RackBeta). Typical activities for placental and bovine adrenal enzyme preparation were 0,5-3 and 50-100 pmol pregnenolone formed/mg protein/min, respectively.

In inhibition experiments, the substance (final concentration range from 1 to 1000 μM) was added into incubation mixture in a volume of 10-20 μl, usually as methanol or ethanol solution. The same volume of the solute was added into control incubation vial. The IC-50 values (concentration causing a 50 % inhibition) were determined graphically and are presented in Table 2.

**Table 1:** Compounds tested

| No. | Name |
|---|---|
| 1a. | 4-[4-(4-methylphenyl)-4-phenylbutyl]-1H-imidazole |
| 2a\ | 1-benzyl-5-[4-(4-methylphenyl)-4-phenylbutyl]-1H-imidazole |
| 3a. | 1-benzyl-5-[4-(3-methylphenyl)-4-phenylbutyl]-1H-imidazole |
| 4a. | 4-[4-(3-methylphenyl)-4-phenylbutyl]-1H-imidazole |
| 5a. | 4-[4-(2-methylphenyl)-4-phenylbutyl]-1H-imidazole |
| 6a. | 1-benzyl-5-[4-(2-methylphenyl)-4-phenylbutyl]-1H-imidazole |
| 7a. | 1-benzyl-5-(4,5-diphenylpentyl)-1H-imidazole |

8a. 4-(4,5-diphenylpentyl)-1H-imidazole

9a. 4-(4,4-diphenylbutyl)-1H-imidazole

10a. 1-benzyl-5-(4,4-diphenylbutyl)-1H-imidazole

11a. 4-[4-(4-methoxyphenyl)-4-phenylbutyl]-1H-imidazole

12a. 4-[4,4-bis(4-methoxyphenyl)butyl]-1H-imidazole

13a. 4-[4,4-bis(3-methylphenyl)butyl]-1H-imidazole

14a. 4-[4-(3,5-dimethylphenyl)-4-phenylbutyl]-1H-imidazole

15a. 4-[4-(3,4-dimethylphenyl)-4-phenylbutyl]-1H-imidazole

16a. 4-[4-(3,5-dimethylphenyl)-4-(3-methylphenyl)butyl]-1H-imidazole

17a. 4-[4,4-bis(4-methylphenyl)butyl]-1H-imidazole

18a. 4-(5,5-diphenylpentyl)-1H-imidazole

19a. 4-(6,6-diphenylhexyl)-1H-imidazole

20a. 4-[4-(2-fluorophenyl)-4-phenylbutyl]-1H-imidazole

21a. 4-[4-(4-fluorophenyl)-4-phenylbutyl]-1H-imidazole

22a. 4-(4,4-diphenyl-1-butenyl)-1H-imidazole

23a. 4-[4,4-bis(4-fluorophenyl)butyl]-1H-imidazole

24a. 4-[4,4-bis(4-nitrophenyl)butyl]-1H-imidazole

25a. 4-[4,4-bis(4-aminophenyl)butyl]-1H-imidazole

26a. 4-[4-(4-ethylphenyl)-4-phenylbutyl]-1H-imidazole

1b. 4-[1-(4-fluorophenyl)-5-phenylpentyl]-1H-imidazole

2b. 4-[1-(4-fluorophenyl)-5-(2-methylphenyl)pentyl]-1H-imidazole

3b. 4-[1-(4-fluorophenyl)-5-(3-methylphenyl)pentyl]-1H-imidazole

4b. 4-[1-(4-fluorophenyl)-5-(4-methylphenyl)pentyl]-1H-imidazole

5b. 4-[5-(3,5-dimethylphenyl)-1-(4-fluorophenyl)-pentyl]-1H-imidazole

6b. 4-(1,5-diphenylpentyl)-1H-imidazole

7b. 4-(1,3-diphenylpropyl)-1H-imidazole

8b. 1-benzyl-5-(1,3-diphenylpropyl)-1H-imidazole

9b. 4-[1-(4-fluorophenyl)-3-phenylpropyl]-1H-imidazole

10b. 4-[1-(4-fluorophenyl)-4-phenylbutyl]-1H-imidazole

11b. 4-[1-(4-fluorophenyl)-5-(3-methoxyphenyl)pentyl]-1H-imidazole

12b. 4-[1-(4-fluorophenyl)-5-(4-methoxyphenyl)pentyl]-1H-imidazole

13b. 4-[1-(4-fluorophenyl)-5-(2,6-dimethylphenyl)-pentyl]-1H-imidazole

14b. 4-[1,3-bis(4-fluorophenyl)propyl]-1H-imidazole

15b. 4-[1,4-bis(4-fluorophenyl)butyl]-1H-imidazole

Table 2: Inhibition of human aromatase and desmolase (CSCC) by test compounds. IC-50 represents the concentration which inhibits the enzyme 50 %.

| Compound No. | IC-50 µmol/l | IC-50 CSCC µmol/l |
|---|---|---|
| 1a | 2,9 | 96 |
| 2a | 19 | 50 |
| 3a | 13 | 38 |
| 4a | 2,5 | 7,5 |
| 5a | 3,4 | 29 |
| 6a | 8,5 | 32 |
| 7a | 15 | |
| 8a | 4,7 | 27 |
| 9a | 2 | 320 |
| 10a | 7 | |
| 11a | 3,5 | 190 |
| 12a | 10 | 46 |
| 13a | 28 | 48 |
| 14a | 7,5 | 65 |
| 15a | 5,0 | 39 |
| 16a | 125 | 95 |
| 17a | 3,5 | 110 |
| 18a | 1,7 | 68 |
| 19a | 14,5 | 61 |
| 20a | 16 | 38 |
| 21a | 2,8 | 80 |
| 22a | 8,5 | 165 |
| 23a | 3,3 | 175 |
| 24a | 20 | 37 |

| | | |
|---|---|---|
| 25a | 26 | 210 |
| 26a | 8,5 | 65 |
| 1b | 2,8 | 19 |
| 2b | 3,5 | 16 |
| 3b | 3,8 | 57 |
| 4b | 8,5 | 170 |
| 5b | 7,5 | 80 |
| 6b | 25 | |
| 7b | 4,5 | 7,5 |
| 8b | 30 | |
| 9b | 0,72 | 22 |
| 10b | 0,75 | 31 |
| 11b | 2,6 | 12 |
| 12b | 3 | 22,5 |
| 13b | 7,7 | 31 |
| 14b | 2,2 | 22 |
| 15b | 0,63 | 29 |

The anti-tumour effect was investigated in vivo against DMBA-induced rat mammary adenocarcinomas by the following method. Mammary adenocarcinoma was induced with DMBA in 50±2 days old female rats. Treatment with the compound under test was started after palpable tumours had appeared. Tumour size and amount of tumours were evaluated once a week. Tumour sizes in the control group, treated with solvent, were compared with the test groups. Daily administration schedule was employed for five weeks and animals were sacrificied. The change in tumour sizes was evaluated.

Results were evaluated as changes in the sizes of tumours and divided into three groups, namely increasing, stable and decreasing sizes of tumours. The anti-tumour effect of 4-(4,4-diphenylbutyl)-1H-imidazole (compound 9a in Table 2) was tested and the results are presented in Table 3.

Table 3

| Number of different tumour types in control and 4-(4,4-diphenylbutyl)-1H-imidazole treated groups of DMBA-induced mammary tumour rats. | | | |
|---|---|---|---|
| group | decreasing | stable | increasing |
| control | 3 | 21 | 42 |
| 4-(4,4-diphenylbutyl)-1H-imidazole 3 mg/kg | 2 | 14 | 37 |
| 4-(4,4-diphenylbutyl)-1H-imidazole 30 mg/kg | 21 | 3 | 15 |

Acute toxicity, $LD_{50}$, was determined by using young adult female mice of NMRI-Strain. The administration of the test compounds was oral. The $LD_{50}$ values of the test compounds of formula (Ia) were 350 mg/kg or more and of formula (Ib) 400 mg/kg or more.

The daily dose for a patient varies from about 20 to 200 mg, administered orally.

The following examples illustrate the invention.

$^1H$ NMR spectra were determined with a Bruker WP 80 DS apparatus (80 MHz). The reference substance was tetramethylsilane. MS spectra were determined with Kratos MS80RF Autoconsole apparatus.

Example 1

4-(4,4-diphenylbutyl)-1H-imidazole

a) 1-benzyl-5-(1-hydroxy-4,4-diphenylbutyl)-1H-imidazole

2,0 g of magnesium turnings are covered with 60 ml of dry tetrahydrofuran. To the mixture is then added dropwise a solution of 1-bromo-3,3-diphenylpropane (22,9 g) in 20 ml of dry tetrahydrofuran at such a rate that a smooth reaction is maintained. After the addition is complete, the reaction mixture is refluxed for one additional hour and cooled to room temperature. The reaction mixture is then added dropwise to a solution of 1-benzyl-5-imidazolecarbaldehyde (7,35 g) in 80 ml of tetrahydrofuran at 60 °C. After the addition is complete, the reaction mixture is refluxed for 2 hours, cooled and poured into cold water. Tetrahydrofuran is evaporated and to the solution is added conc. hydrochloric acid. The solution is cooled and the precipitate which contains the product as hydrochloride salt is removed by filtration, washed with water and dried. Yield 14,1 g. M.p. 160-168 °C.
$^1$H NMR (as HCl-salt, MeOH-$d_4$):
1.50-2.30 (m, 4H), 3.82 (t, 1H), 4.65 (t, 1H), 5.43 (s, 2H), 7.05-7.50 (m, 16H), 8.62 (d, 1H)
Using the same method for example the following compounds included in the invention were prepared:
1-benzyl-5-[1-hydroxy-4-(2-methylphenyl)-4-phenylbutyl]-1H-imidazole
$^1$H NMR (as HCl-salt, MeOH-$d_4$):
1.50-2.40 (m, 4H), 2.21 and 2.15 (2s, 3H), 4.07 (t, 1H), 4.70 (m, 1H), 5.49 and 5.46 (2s, 2H), 6.80-7.50 (m, 15H), 8.88 (s, 1H)
1-benzyl-5-[1-hydroxy-4-(3-methylphenyl)-4-phenylbutyl]-1H-imidazole
$^1$H NMR (as HCl-salt, $CDCl_3$):
1.35-2.35 (m, 4H), 2.26 (s, 3H), 3.73 (t, 1H), 4.62 (m, 1H), 5.38 (s, 2H), 6.80-7.40 (m, 15H), 8.51 (s, 1H)
1-benzyl-5-[1-hydroxy-4-(4-methylphenyl)-4-phenylbutyl]-1H-imidazole
$^1$H NMR (as HCl-salt, $CDCl_3$):
1.40-2.40 (m, 4H), 2.23 and 2.24 (2s, 3H), 3.74 (t, 1H), 4.65 (broad t, 2H), 5.38 (s, 2H), 6.80-7.40 (m, 15H), 8.55 (s, 1H)
1-benzyl-5-[1-hydroxy-4,4-bis(4-methylphenyl)butyl]-1H-imidazole. M.p. of hydrochloride 155-158 °C.
1-benzyl-5-(1-hydroxy-4,5-diphenylpentyl)-1H-imidazole
$^1$H NMR (as hydrogen sulphate salt, MeOH-$d_4$):
1.35-1.90 (m, 4H), 2.82 (broad s, 3H), 4.54 (t, 1H), 5.42 (s, 2H), 6.85-7.50 (m, 16H), 8.80 (s, 1H)
1-benzyl-5-[1-hydroxy-4-(4-methoxyphenyl)-4-phenylbutyl]-1H-imidazole
$^1$H NMR (as HCl-salt, MeOH-$d_4$):
1.50-2.30 (m, 4H), 3.70 (s, 3H), 3.79 (t, 1H), 4.69 (t, 1H), 5.46 (s, 2H), 6.79 (d, 2H), 7.0-7.55 (m, 13H), 8.85 (d, 1H)
1-benzyl-5-[1-hydroxy-4,4-bis(4-methoxyphenyl)butyl]-1H-imidazole
$^1$H NMR (as base, MeOH-$d_4$):
1.50-2.20 (m, 4H), 3.66 (t, 1H), 3.74 (s, 6H), 4.50 (t, 1H), 5.24 (s, 2H), 6.70-7.60 (m, 15H)
1-benzyl-5-[4-(2-fluorophenyl)-1-hydroxy-4-phenylbutyl]-1H-imidazole. M.p. of hydrochloride 160-163 °C.
$^1$H NMR (as HCl-salt, $CDCl_3$):
1.45-2.45 (m, 4H), 4.17 (t, 1H), 4.67 (t, 1H), 5.43 (s, 2H), 6.80-7.50 (m, 15H), 8.43 (s, 1H)
1-benzyl-5-[4-(4-fluorophenyl)-1-hydroxy-4-phenylbutyl]-1H-imidazole. M.p. of hydrochloride 168-171 °C.
$^1$H NMR (as HCl-salt, MeOH-$d_4$):
1.50-2.30 (m, 4H), 3.84 (t, 1H), 4.68 (t, 1H), 5.49 (s, 2H), 6.80-7.55 (m, 15H), 8.87 (d, 1H)
1-benzyl-5-(1-hydroxy-5,5-diphenylpentyl)-1H-imidazole
$^1$H NMR (as base, $CDCl_3$):
1.2-2.2 (m, 6H), 3.70 (t, 1H), 4.54 (t, 1H), 5.46 (s, 2H), 6.8-7.3 (m, 16H), 8.58 (s, 1H)
1-benzyl-5-(1-hydroxy-6,6-diphenylhexyl)-1H-imidazole. M.p. of hydrochloride 147-149 °C.
$^1$H NMR (as HCl-salt, MeOH-$d_4$):
1.6-2.2 (m, 8H), 3.85 (t, 1H), 4.60 (t, 1H), 5.53 (s, 2H), 7.0-7.4 (m, 15H), 7.47 (s, 1H), 8.90 (s, 1H)
1-benzyl-5-[4-(4-ethylphenyl)-1-hydroxy-4-phenylbutyl]-1H-imidazole
$^1$H NMR (as HCl-salt, MeOH-$d_4$):
1.71 (t, 3H), 1.50-2.35 (m, 4H), 2.57 (q, 2H), 3.78 (t, 1H), 4.68 (t, 1H), 5.46 (s, 2H), 6.9-7.5 (m, 15H), 8.82 (d, 1H)
1-benzyl-5-[4,4-bis(4-fluorophenyl)-1-hydroxybutyl]-1H-imidazole
$^1$H NMR (as HCl-salt, MeOH-$d_4$):

1.50-2.00 (m, 4H), 3.86 (t, 1H), 4.70 (t, 1H), 5.52 (s, 2H), 6.80-7.50 (m, 14 H), 8.89 (d, 1H)

b) 1-benzyl-5-(4,4-diphenyl-1-butenyl)-1H-imidazole

1-benzyl-5-(1-hydroxy-4,4-diphenylbutyl)-1H-imidazole hydrochloride (5,0 g) and 30,0 g of anhydrous potassium hydrogen sulphate are heated at 150 °C for 4 hours. The mixture is cooled, 90 ml of ethanol is added to dissolve the product. The mixture is then filtered and the filtrate is evaporated to minor volume. Water is added and the mixture is made alkaline with sodium hydroxide. The product is extracted in methylene chloride, washed with water and evaporated to dryness. The product is then made to hydrochloride salt with dry hydrochloric acid in dry ethylacetate. Yield is 2,9 g. M.p. 204-206 °C.
$^1$H NMR (as HCl-salt, MeOH-$d_4$):
2.88-3.05 (m, 2H), 4.08 (t, 1H), 5.29 (s, 2H), 6.22-6.32 (m, 2H), 7.00-7.50 (m, 16H), 8.87 (d, 1H)
Using the same method for example the following compounds included in the invention were prepared:
1-benzyl-5-[4,4-bis(3-methylphenyl)-1-butenyl]-1H-imidazole. M.p. of hydrochloride 152-156 °C.
$^1$H NMR (as HCl-salt, MeOH-$d_4$):
2.26 (s, 6H), 2.85-3.05 (m, 2H), 3.99 (t, 1H), 5.27 (s, 2H), 6.21-6.31 (m, 2H), 6.80-7.50 (m, 14H), 8.86 (d, 1H)
1-benzyl-5-[4-(3,5-dimethylphenyl)-4-(3-methylphenyl)-1-butenyl]-1H-imidazole
$^1$H NMR (as HCl-salt, MeOH-$d_4$):
2.22 (s, 3H), 2.23 (s, 3H), 2.26 (s, 3H), 2.85-3.05 (m, 2H), 3.94 (t, 1H), 5.26 (s, 2H), 6.20-6.30 (m, 2H), 6.75-7.50 (m, 13H), 8.84 (d, 1H)
1-benzyl-5-[4-(3,5-dimethylphenyl)-4-phenyl-1-butenyl]-1H-imidazole. M.p. 110-112 °C.
$^1$H NMR (as HCl-salt, MeOH-$d_4$):
2.22 (s, 6H), 2.85-3.05 (m, 2H), 3.98 (t, 1H), 5.27 (s, 2H), 6.20-6.30 (m, 2H), 6.75-7.5 (m, 14H), 8.87 (d, 1H)
1-benzyl-5-[4-(2-methylphenyl)-4-phenyl-1-butenyl]-1H-imidazole
$^1$H NMR (as hydrogen sulphate, MeOH-$d_4$):
2.19 (s, 3H), 2.80-3.05 (m, 2H), 4.28 (t, 1H), 5.29 (s, 2H), 6.0-6.60 (m, 2H), 7.0-7.5 (m, 14H), 7.52 (d, 1H), 8.85 (d, 1H)
1-benzyl-5-[4-(3-methylphenyl)-4-phenyl-1-butenyl]-1H-imidazole
$^1$H NMR (as base, $CDCl_3$):
2.27 (s, 3H), 2.75-3.95 (m, 2H), 3.93 (t, 1H), 4.89 (s, 2H), 5.70-6.10 (m, 2H), 6.80-7.40 (m, 16H)
1-benzyl-5-[4-(4-methylphenyl)-4-phenyl-1-butenyl]-1H-imidazole
$^1$H NMR (as base, $CDCl_3$):
2.27 (s, 3H), 2.70-2.95 (m, 2H), 3.93 (t, 1H), 4.89 (s, 2H), 5.60-6.20 (m, 2H), 6.80-7.50 (m, 16H)
1-benzyl-5-[4,4-bis(4-methylphenyl)-1-butenyl]-1H-imidazole. M.p. of hydrochloride 128-132 °C.
1-benzyl-5-[4-(4-methoxyphenyl)-4-phenyl-1-butenyl]-1H-imidazole
$^1$H NMR (as HCl-salt, $CDCl_3$):
2.70-2.95 (m, 2H), 3.95 (t, 1H), 5.16 (s, 2H), 5.70-6.20 (m, 2H), 6.70-7.40 (m, 15H), 8.99 (s, 1H)
1-benzyl-5-[4,4-bis(4-methoxyphenyl)-1-butenyl]-1H-imidazole
$^1$H NMR (as base, $CDCl_3$):
2.60-2.90 (m, 2H), 3.74 (s, 6H), 3.78 (t, 1H), 4.92 (s, 2H), 5.80-6.00 (m, 2H), 6.65-7.50 (m, 15H)
1-benzyl-5-(4,5-diphenyl-1-pentenyl)-1H-imidazole
$^1$H NMR (as base, $CDCl_3$):
2.30-2.55 (m, 2H), 2.88 (m, 3H), 4.93 (s, 2H), 5.5-6.1 (m, 2H), 6.8-7.5 (m, 17H)
1-benzyl-5-[4-(2-fluorophenyl)-4-phenyl-1-butenyl]-1H-imidazole. M.p. of hydrochloride 195-201 °C.
$^1$H NMR (as base, $CDCl_3$):
2.75-3.00 (m, 2H), 4.34 (t, 1H), 5.97 (s, 2H), 5.80-6.10 (m, 2H), 6.75-7.50 (m, 16H)
1-benzyl-5-[4-(4-fluorophenyl)-4-phenyl-1-butenyl]-1H-imidazole
$^1$H NMR (as base, $CDCl_3$):
2.60-2.95 (m, 2H), 3.96 (t, 1H), 4.93 (s, 2H), 5.80-6.05 (m, 2H), 6.75-7.50 (m, 16H)
1-benzyl-5-(6,6-diphenyl-1-hexenyl)-1H-imidazole. M.p. of hydrochloride 184-186 °C.
1-benzyl-5-[4-(4-ethylphenyl)-4-phenyl-1-butenyl]-1H-imidazole
$^1$H NMR (as HCl-salt, MeOH-$d_4$):
1.17 (t, 3H), 2.56 (q, 2H), 2.93-2.98 (m, 2H), 4.03 (t, 1H), 5.28 (s, 2H), 6.20-6.34 (m, 2H), 7.08-7.41 (m, 14H), 7.52 (d, 1H), 8.87 (d, 1H)
1-benzyl-5-[4,4-bis(4-fluorophenyl)-1-butenyl]-1H-imidazole
$^1$HNMR (as HCl-salt, $CDCl_3$):
2.78-2.94 (m, 2H), 4.01 (t, 1H), 5.27 (s, 2H), 5.82-6.34 (m, 2H), 6.83-7.40 (m, 14H), 9.21 (d, 1H)

c) 1-benzyl-5-(4,4-diphenylbutyl)-1H-imidazole

1-benzyl-5-(4,4-diphenyl-1-butenyl)-1H-imidazole hydrochloride (2,0 g) is dissolved in ethanol and a catalytic amount of Pd/C (10 %) is added. The reaction mixture is agitated vigorously at room temperature in a hydrogen atmosphere until the uptake of hydrogen ceases. The mixture is filtered and the filtrate is evaporated to dryness. The residue which is the product is purified by flash chromatography eluting with methylene chloride-methanol mixture. Yield 1,3 g. M.p. of the hydrochloride salt is 200-202 °C.
$^1$H NMR (as HCl-salt, MeOH-$d_4$):
1.30-1.70 (m, 2H), 1.85-2.20 (m, 2H), 2.61 (t, 2H), 3.83 (t, 1H), 5.35 (s, 2H), 7.05-7.50 (m, 16H), 8.89 (d, 1H)
Using the same method for example the following compounds included in the invention were prepared:
1-benzyl-5-[4-(2-methylphenyl)-4-phenylbutyl]-1H-imidazole. Mp. of hydrochloride 200-205 °C.
$^1$H NMR (as HCl-salt, $CDCl_3$):
1.30-1.80 (m, 2H), 1.80-2.15 (m, 2H), 2.22 (s, 3H), 2.48 (t, 2H), 4.02 (t, 1H), 5.31 (s, 2H), 6.96 (s, 1H), 7.0-7.5 (m, 14H), 9.28 (s, 1H)
1-benzyl-5-[4-(3-methylphenyl)-4-phenylbutyl]-1H-imidazole. M.p. of hydrochloride 148-158 °C.
$^1$H NMR (as HCl-salt, $CDCl_3$):
1.30-1.80 (m, 2H), 1.80-2.25 (m, 2H), 2.30 (s, 3H), 2.49 (t, 2H), 3.78 (t, 1H), 5.29 (s, 2H), 6.90-7.50 (m, 15H), 9.24 (s, 1H)
1-benzyl-5-[4-(4-methylphenyl)-4-phenylbutyl]-1H-imidazole. M.p. of hydrochloride 164-170 °C.
$^1$H NMR (as HCl-salt, $CDCl_3$):
1.25-1.75 (m, 2H), 1.80-2.25 (m, 2H), 2.29 (s, 3H), 2.48 (t, 2H), 3.78 (t, 1H), 5.31 (s, 2H), 6.80-7.50 (m, 15H), 9.35 (s, 1H)
1-benzyl-5-(4,5-diphenylpentyl)-1H-imidazole. M.p. of hydrochloride 166-170 °C.
$^1$H NMR (as HCl-salt, MeOH-$d_4$):
1.15-1.90 (m, 4H), 2.49 (t, 2H), 2.81 (m, 3H), 5.30 (s, 2H), 6.90-7.50 (m, 16H), 8.82 (s, 1H)
1-benzyl-5-[4-(4-methoxyphenyl)-4-phenylbutyl]-1H-imidazole. M.p. of hydrochloride 180-187 °C.
$^1$H NMR (as HCl-salt, MeOH-$d_4$):
1.25-1.70 (m, 2H), 1.85-2.20 (m, 2H), 2.62 (t, 2H), 3.74 (s, 3H), 3.78 (t, 1H), 5.34 (s, 2H), 6.81 (d, 2H), 7.0-7.5 (m, 13H), 8.84 (d, 1H)
1-benzyl-5-[4-(2-fluorophenyl)-4-phenylbutyl]-1H-imidazole. M.p. of hydrochloride 185-196 °C.
$^1$H NMR (as HCl-salt, MeOH-$d_4$):
1.25-1.75 (m, 2H), 1.80-2.25 (m, 2H), 2.65 (t, 2H), 4.18 (t, 1H), 5.37 (s, 2H), 6.80-7.5 (m, 15H), 8.89 (d, 1H)
1-benzyl-5-[4-(4-fluorophenyl)-4-phenylbutyl]-1H-imidazole. M.p. of hydrochloride 172-174 °C.
$^1$H NMR (as HCl-salt, MeOH-$d_4$):
1.25-1.70 (m, 2H), 1.80-2.25 (m, 2H), 2.64 (t, 2H), 3.85 (t, 1H), 5.37 (s, 2H), 6.80-7.50 (m, 15H), 8.90 (d, 1H)
1-benzyl-5-(5,5-diphenylpentyl)-1H-imidazole
$^1$H NMR (as base, MeOH-$d_4$):
1.1-1.6 (m, 4H), 1.8-2.1 (m, 2H), 2.37 (t, 2H), 3.72 (t, 1H), 5.11 (s, 2H), 6.67 (s, 1H), 6.9-7.3 (m, 15H), 7.59 (s, 1H)
1-benzyl-5-[4-(4-ethylphenyl)-4-phenylbutyl]-1H-imidazole
$^1$H NMR (as HCl-salt, MeOH-$d_4$):
1.17 (t, 3H), 1.40-1.70 (m, 2H), 1.90-2.20 (m, 2H), 2.57 (q, 2H), 3.80 (t, 1H), 5.34 (s, 2H), 7.00-7.50 (m, 15 H), 8.87 (d, 1H)
1-benzyl-5-[4,4-bis(4-fluorophenyl)butyl]-1H-imidazole
$^1$H NMR (as HCl-salt, MeOH-$d_4$):
1.30-1.70 (m, 2H), 1.80-2.25 (m, 2H), 2.64 (t, 2H), 3.87 (t, 1H), 5.40 (s, 2H), 6.80-7.50 (m, 14 H), 8.92 (d, 1H)

d) 4-(4,4-diphenylbutyl)-1H-imidazole

1-benzyl-5-(4,4-diphenylbutyl)-1H-imidazole hydrochloride (0,6 g) is hydrogenated in the mixture of 20 ml of 2 N hydrochloric acid and 10 ml of ethanol at 80 °C Pd/C (10 %) as catalyst. When the uptake of the hydrogen ceases, the reaction mixture is cooled, filtered and evaporated to dryness. Water is added and the mixture is made alkaline with sodium hydroxide. The product is then extracted to methylene chloride which is washed with water, dried with sodium sulphate and evaporated to dryness. The residue is the product as base and it is made to its hydrochloride in ethyl acetate using dry hydrochloric acid. Yield 0,2 g. M.p. 204-206 °C.
$^1$H NMR (as HCl-salt, MeOH-$d_4$):

1.40-1.90 (m, 2H), 1.90-2.30 (m, 2H), 2.75 (t, 2H), 3.95 (t, 1H), 7.00-7.40 (m, 11H), 8.72 (d, 1H)
Using the same method for example the following compounds included in the invention were prepared:
4-[4,4-bis(3-methylphenyl)butyl]-1H-imidazole. M.p. of hydrochloride 122-129 °C.
$^1$H NMR (as HCl-salt, MeOH-$d_4$):
1.40-1.90 (m, 2H), 1.90-2.30 (m, 2H), 2.26 (s, 6H), 2.73 (t, 2H), 3.85 (t, 1H), 6.80-7.25 (m, 9H), 8.73 (d, 1H)
4-[4-(3,5-dimethylphenyl)-4-(3-methylphenyl)butyl]-1H-imidazole. M.p. of hydrochloride 75-82 °C.
$^1$H NMR (as HCl-salt, MeOH-$d_4$):
1.40-1.90 (m, 2H), 1.90-2.30 (m, 2H), 2.22 (s, 3H), 2.23 (s, 3H), 2.27 (s, 3H), 2.74 (t, 2H), 3.81 (t, 1H), 6.75-7.30 (m, 8H), 8.72 (d, 1H)
4-[4-(3,5-dimethylphenyl)-4-phenylbutyl]-1H-imidazole. M.p. of hydrochloride 104-106 °C.
$^1$H NMR (HCl-salt, MeOH-$d_4$):
1.40-1.90 (m, 2H), 1.90-2.30 (m, 2H), 2.23 (s, 6H), 2.74 (t, 2H), 3.85 (t, 1H), 6.84 (m, 3H), 7.22 (m, 6H), 8.72 (d, 1H)
4-[4-(3,4-dimethylphenyl)-4-phenylbutyl]-1H-imidazole. M.p. of hydrochloride 118-121 °C.
4-[4-(2-methylphenyl)-4-phenylbutyl]-1H-imidazole. M.p. of hydrochloride 151-154,5 °C.
$^1$H NMR (as HCl-salt, $CDCl_3$):
1.50-2.20 (m, 4H), 2.22 (s, 3H), 2.71 (t, 2H), 4.09 (t, 1H), 6.81 (s, 1H), 7.0-7.4 (m, 9H), 9.04 (s, 1H)
5-[4-(3-methylphenyl)-4-phenylbutyl]-1H-imidazole. M.p. of hydrochloride 140-153 °C.
$^1$H NMR (as HCl-salt, MeOH-$d_4$):
1.40-1.85 (m, 2H), 1.85-2.25 (m, 2H), 2.27 (s, 3H), 2.74 (t, 2H), 3.90 (t, 1H), 6.80-7.30 (m, 10H), 8.69 (d, 1H)
4-[4-(4-methylphenyl)-4-phenylbutyl]-1H-imidazole. M.p. of hydrochloride 173-177 °C.
$^1$H NMR (as HCl-salt, $CDCl_3$ + 2 drops of MeOH-$d_4$): 1.40-1.80 (m, 2H), 1.80-2.25 (m, 2H), 2.28 (s, 3H), 2.71 (t, 2H), 3.87 (t, 1H), 6.86 (d, 1H), 7.09 (s, 4H), 7.21 (s, 5H), 8.71 (d, 1H)
4-[4-(4-methoxyphenyl)-4-phenylbutyl]-1H-imidazole. M.p. of hydrochloride 156-159 °C.
$^1$H NMR (as HCl-salt, $CDCl_3$):
1.40-1.90 (m, 2H), 1.90-2.30 (m, 2H), 2.71 (t, 2H), 3.76 (s, 3H), 3.87 (t, 1H), 6.82 (d, 2H), 6.90 (s, 1H), 7.13 (d, 2H), 7.21 (m, 5H), 8.68 (s, 1H)
4-[4,4-bis(4-methoxyphenyl)butyl]-1H-imidazole. M.p. of hydrochloride 138-142 °C.
$^1$H NMR (as HCl-salt, $CDCl_3$):
1.40-2.25 (m, 4H), 2.71 (t, 2H), 3.75 (s, 6H) under which there is (t, 1H), 6.78 (d, 4H), 6.83 (s, 1H), 7.08 (d, 4H), 9.02 (s, 1H)
4-(4,5-diphenylpentyl)-1H-imidazole
$^1$H NMR (as HCl-salt, $CDCl_3$):
1.20-1.90 (m, 4H), 2.57 (t, 2H), 2.83 (m, 3H), 6.71 (s, 1H), 6.80-7.40 (m, 10H), 8.84 (s, 1H)
4-(5,5-diphenylpentyl)-1H-imidazole
$^1$H NMR (as HCl-salt, MeOH-$d_4$):
1.3-1.5 (m, 2H), 1.5-1.7 (m, 2H), 1.8-2.3 (m, 2H), 2.656 (t, 2H), 3.746 (t, 1H), 7.06-7.2 (m, 11H), 8.716 (d, 1H)
4-(6,6-diphenylhexyl)-1H-imidazole
$^1$H NMR (as base, $CDCl_3$):
1.1-1.7 (m, 6H), 1.8-2.2 (m, 2H), 2.530 (t, 2H), 3.847 (t, 1H), 6.685 (s, 1H), 7.2 (s, 10H), 7.470 (s, 1H), 9.6 (broad s, 1H)
4-[4,4-bis(4-methylphenyl)butyl]-1H-imidazole. M.p. of hydrochloride 176-179 °C.
4-[4-(4-fluorophenyl)-4-phenylbutyl]-1H-imidazole. M.p. of hydrochloride 175-182 °C.
4-[4-(2-fluorophenyl)-4-phenylbutyl]-1H-imidazole. M.p. of hydrochloride 182-190 °C.
4-[4-(4-ethylphenyl)-4-phenylbutyl]-1H-imidazole
$^1$H NMR (as HCl-salt, MeOH-$d_4$):
1.18 (t, 3H), 1.40-1.90 (m, 2H), 1.90-2.30 (m, 2H), 2.57 (g, 2H), 2.75 (t, 2H), 3.91 (t, 1H), 6.95-7.30 (m, 10H), 8.73 (d, 1H)
4-[4,4-bis(4-fluorophenyl)butyl]-1H-imidazole
$^1$H NMR (as HCl-salt, MeOH-$d_4$):
1.40-1.85 (m, 2H), 1.90-2.30 (m, 2H), 2.77 (t, 2H), 3.98 (t, 1H), 6.80-7.40 (m, 9H), 8.72 (d, 1H)

Example 2

4-[4-(4-fluorophenyl)-4-phenylbutyl]-1H-imidazole

A concentrated water solution of ammoniumformate (0,98 g, 15,6 mmol) is added dropwise to the boiling mixture of 1-benzyl-5-[4-(4-fluorophenyl)-4-phenylbutyl]-1H-imidazole (1,5 g, 3,9 mmol) and 10 % Pd/C (0,156 g) in 16 ml of 50 % ethanol. The mixture is refluxed for 2 hours. The catalyst is filtrated off and the solvent is evaporated. 2 M NaOH is added and the product is extracted into ethyl acetate. The ethyl acetate phase is dried and evaporated to dryness to give the product. Yield 1,02 g. Melting point of the hydrochloride salt (from ethyl acetate) is 175-182 °C.
$^1$H NMR (as HCl-salt, MeOH-$d_4$):
1.40-1.90 (m, 2H), 1.90-2.30 (m, 2H), 2.75 (t, 2H), 3.96 (t, 1H), 6.85-7.36 (m, 10H), 8.74 (d, 1H)
According to the same procedure as the example the following substituted derivative was prepared:
4-[4-(2-fluorophenyl)-4-phenylbutyl]-1H-imidazole. M.p. of hydrochloride 182-190 °C.
$^1$H NMR (as HCl-salt, MeOH-$d_4$):
1.45-1.95 (m, 2H), 1.95-2.30 (m, 2H), 2.77 (t, 2H), 4.29 (t, 1H), 6.85-7.45 (m, 10H), 8.74 (d, 1H)

Example 3

4-(4,4-diphenylbutyl)-1H-imidazole

a) 1-benzyl-5-(4-hydroxy-4,4-diphenylbutyl)-1H-imidazole

Magnesium turnings (0,49 g) are covered with 4 ml of dry tetrahydrofuran. Brombenzene (3,18 g) in 7 ml of dry tetrahydrofuran is added dropwise to the mixture at such a rate that a smooth reaction is maintained. The reaction mixture is refluxed for an additional hour. Ethyl 4-(1-benzyl-1H-imidazol-5-yl)-butyrate (1,10 g) in 15 ml of dry tetrahydrofuran is then added dropwise to the Grignard reagent and the reaction mixture is refluxed for 2 hours. Saturated ammonium chloride is added to the cooled reaction mixture. Tetrahydrofuran is evaporated and the precipitated product is collected. Yield 1,41 g. Melting point of the hydrochloride salt is 197-201 °C.
$^1$H NMR (as HCl-salt, MeOH-$d_4$):
1.35-1.80 (m, 2H), 2.20-2.45 (m, 2H), 2.61 (t, 2H), 5.33 (s, 2H), 7.0-7.5 (m, 16H), 8.81 (d, 1H)

b) 1-benzyl-5-(4,4-diphenyl-3-butenyl)-1H-imidazole

1-benzyl-5-(4-hydroxy-4,4-diphenylbutyl)-1H-imidazole (1,3 g) is refluxed in 20 ml of ethanol containing 5 % (w/w) hydrogen chloride for 1 hour. The solvent is evaporated and the HCl-salt of the product is precipitated with ethyl acetate. Yield 1,1 g, m.p. 161-168 °C.
$^1$H NMR (as HCl-salt, MeOH-$d_4$):
2.22-2.60 (m, 2H), 2.60-2.90 (m, 2H), 5.29 (s, 2H), 6.06 (t, 1H), 6.90-7.60 (m, 16H), 8.88 (d, 1H)

c) 1-benzyl-5-(4,4-diphenylbutyl)-1H-imidazole

1-benzyl-5-(4,4-diphenyl-3-butenyl)-1H-imidazole hydrochloride salt is hydrogenated in ethanol as described in Example 1 c). M.p. of the product as hydrochloride is 200-202 °C.

d) 4-(4,4-diphenylbutyl)-1H-imidazole

The benzyl group of 1-benzyl-5-(4,4-diphenylbutyl)-1H-imidazole is hydrogenated as is described in Example 1 d).

Example 4

4-[4,4-bis(4-nitrophenyl)butyl]-1H-imidazole

1,8 g (14,6 mmol) of urea nitrate is added in small portions to a mixture of 2,0 g (7,3 mmol) of 4-(4,4-diphenylbutyl)-1H-imidazole in 6,4 ml of concentrated sulphuric acid under 10 °C. The reaction mixture is stirred for 2 hours at room temperature. The mixture is made alkaline with 2 M sodium hydroxide and the

product is extracted into ethyl acetate. The product is purified by flash chromatography using methylene chloride-methanol (95:5) as eluent.
$^1H$ NMR (as HCl-salt, MeOH-$d_4$):
1.4-1.95 (m, 2H), 2.0-2.45 (m, 2H), 2.81 (t, 2H), 4.34 (t, 1H), 7.27 (broad s, 1H), 7.5 (d, 4H), 8.17 (d, 4H), 8.73 (d, 1H)

Example 5

4-[4,4-bis(4-aminophenyl)butyl]-1H-imidazole

4-[4,4-bis(4-nitrophenyl)butyl]-1H-imidazole is hydrogenated in ethanol using 10 % palladium on carbon (Pd/C) as a catalyst.
$^1H$ NMR (as HCl-salt, MeOH-d4):
1.4-2.25 (m, 4H), 2.69 (t, 2H), 3.70 (t, 1H), 6.77 (d, 4H), 6.95 (d, 4H), 7.08 (broad s, 1H), 8.57 (d, 1H)

Example 6

4-(4,4-diphenyl-1-butenyl)-1H-imidazole

a) 4-(1-hydroxy-4,4-diphenylbutyl)-1H-imidazole

A concentrated water solution of ammonium formate (4,0 g) is added dropwise to the boiling mixture of 1-benzyl-5-(1-hydroxy-4,4-diphenylbutyl)-1H-imidazole (4,5 g) and 10 % Pd/C (0,5 g) in 50 ml of 50 % ethanol. The mixture is refluxed for 2 hours. The catalyst is filtrated and the solvent is evaporated. 2 M NaOH is added and the product is extracted into ethyl acetate. The ethyl acetate phase is dried and evaporated to dryness to give the product which is used in the following step b).

b) 4-(4,4-diphenyl-1-butenyl)-1H-imidazole

4-(1-hydroxy-4,4-diphenylbutyl)-1H-imidazole (3,0 g) and 20 g of anhydrous potassium hydrogen sulfate are heated at 150 °C for 4 hours. The mixture is cooled and 90 ml ethanol is added to dissolve the product. The mixture is made alkaline with sodium hydroxide. The product is extracted into methylene chloride, washed with water and evaporated to dryness. The product is then made to hydrochloride salt with dry hydrogen chloride in ethyl acetate. M.p. above 240 °C.
$^1H$ NMR (as HCl-salt, $CDCl_3$):
2.904-3.068 (m, 2H), 4.116 (t, 1H), 6.05-6.35 (m, 2H), 6.998 (d, 1H), 7.22-7.25 (m, 10H), 8.719 (d, 1H)

Example 7

4-[1-(4-fluorophenyl)-5-phenylpentyl]-1H-imidazole

a) 1-benzyl-5-(1-hydroxy-5-phenylpentyl)-1H-imidazole

2,1 g of magnesium turnings are covered with 60 ml of dry tetrahydrofuran. A solution of 4-phenylbutylbromide (18,8 g) in 20 ml of dry tetrahydrofuran is then added dropwise to the mixture at such a rate that a smooth reaction is maintained. After the addition is complete, the reaction mixture is refluxed for one additional hour and cooled to room temperature. The reaction mixture is then added dropwise to a solution of 1-benzyl-5-imidazolecarbaldehyde (6,5 g) in 80 ml of tetrahydrofuran at 60 °C. After the addition is complete, the reaction mixture is refluxed for 2 hours, cooled and poured into cold water. Tetrahydrofuran is evaporated and conc. hydrochloric acid is added to the solution. The product which is separated as an oil, is extracted with methylene chloride and evaporated to dryness.

b) 4-(1-hydroxy-5-phenylpentyl)-1H-imidazole

1-benzyl-5-(1-hydroxy-5-phenylpentyl)-1H-imidazole hydrochloride (8,5 g), prepared in the Step a, is hydrogenated in the mixture of 100 ml of 2 N hydrochloric acid and 10 ml of ethanol at 60 °C Pd/C (10 %) as catalyst. When the uptake of the hydrogen ceases, the reaction mixture is cooled, filtered and evaporated to dryness. Water is added and the mixture is made alkaline with sodium hydroxide. The

product is then extracted to methylene chloride which is washed with water, dried with sodium sulphate and evaporated to dryness. The residue is the product as base, and it is used as such in Step c.

$^1H$ NMR (as base, MeOH-$d_4$ + a drop of $CDCl_3$):

1.2-2.0 (m, 6H), 2.61 (distorted t, 2H), 4.65 (t, 1H), 6.91 (dd, 1H), 7.0-7.3 (m, 5H), 7.56 (d, 1H)

c) 4-(1-oxo-5-phenylpentyl)-1H-imidazole

5,5 g of 4-(1-hydroxy-5-phenylpentyl)-1H-imidazole and 7,0 g of manganese dioxide are refluxed stirring in tetrachloroethylene for four hours. The reaction mixture is filtered and the filtrate is evaporated to dryness. Water is added and the product is extracted into methylene chloride. The combined extracts are washed with water and evaporated to dryness.

d) 4-[1-(4-fluorophenyl)-1-hydroxy-5-phenylpentyl]-1H-imidazole

0,52 g of magnesium turnings are covered with 60 ml of dry tetrahydrofuran. Then a solution of 1-bromo-4-fluorobenzene (3,8 g) in 60 ml of dry tetrahydrofuran is added dropwise to the mixture at such a rate that a smooth reaction is maintained. After the addition is complete, the reaction mixture is refluxed for one additional hour and cooled to room temperature. The reaction mixture is then added dropwise to a solution of 4-(1-oxo-5-phenylpentyl)-1H-imidazole (3,8 g) in 40 ml of tetrahydrofuran at 60 °C. After the addition is complete, the reaction mixture is refluxed for 3 hours, cooled and poured into cold water. Tetrahydrofuran is evaporated and conc. hydrochloric acid is added to the solution. The product is extracted as hydrochloric salt into methylene chloride. Combined methylene chloride extracts are then evaporated to dryness.

e) 4-[1-(4-fluorophenyl)-5-phenyl-1-pentenyl]-1H-imidazole

4-[1-(4-fluorophenyl)-1-hydroxy-5-phenylpentyl]-1H-imidazole hydrochloride (5,0 g) and 30,0 g of anhydrous potassium hydrogen sulphate are heated at 150 °C for 4 hours. The mixture is cooled and 90 ml of ethanol is added to dissolve the product. The mixture is then filtered and the filtrate is evaporated to minor volume. Water is added and the mixture is made alkaline with sodium hydroxide. The product is extracted into methylene chloride, washed with water and evaporated to dryness. The product is then made to hydrochloride salt with dry hydrogen chloride in dry ethylacetate.

$^1H$ NMR (as base, $CDCl_3$):

1.5-2.7 (m, 6H), 4.8 (broad s, 1H), 6.34 (t, 1H), 6.48 (broad s, 1H), 6.9-7.4 (m, 9H), 7.52 (broad s, 1H)

Using the same method for example the following compounds included in the invention were prepared:

4-[1-(4-fluorophenyl)-5-(3-methylphenyl)-1-pentenyl]-1H-imidazole

4-[1-(4-fluorophenyl)-5-(4-methylphenyl)-1-pentenyl]-1H-imidazole

4-[1-(4-fluorophenyl)-5-(2-methylphenyl)-1-pentenyl]-1H-imidazole

4-[5-(3,5-dimethylphenyl)-1-(4-fluorophenyl)-1-pentenyl]-1H-imidazole

4-[1-(4-fluorophenyl)-5-(3-methoxyphenyl)-1-pentenyl]-1H-imidazole

4-[5-(3,5-dimethoxyphenyl)-1-(4-fluorophenyl)-1-pentenyl]-1H-imidazole

f) 4-[1-(4-fluorophenyl)-5-phenylpentyl]-1H-imidazole

4-[1-(4-fluorophenyl)-5-phenyl-1-pentenyl]-1H-imidazole hydrochloride (2,0 g) is dissolved in ethanol and a catalytic amount 10 % Pd/C is added. The reaction mixture is agitated vigorously at room temperature in a hydrogen atmosphere until the uptake of hydrogen ceases. The mixture is filtered and the filtrate is evaporated to dryness. The residue which is the product is purified by flash chromatography eluting with methylene chloride-methanol mixture. Yield 82 %.

$^1H$ NMR (as base, $CDCl_3$):

1.1-2.7 (m, 8H), 3.84 (t, 1H), 6.71 (broad s, 1H), 6.80-7.38 (m, 9H), 7.47 (broad s, 1H), 9.22 (broad s, 1H)

Using the same method for example the following compounds included in the invention were prepared:

4-[1-(4-fluorophenyl)-5-(3-methylphenyl)pentyl]-1H-imidazole

MS: 322 (20, $M^{+\cdot}$), 189 (28), 176 (38), 175 (72), 149 (100), 125 (20), 121 (14), 109 (42), 105 (16), 97 (21)

$^1H$ NMR (as HCl-salt, MeOH-$d_4$):

1.1-2.7 (m, 8H), 2.27 (s, 3H), 4.06 (t, 1H), 6.7-7.5 (m, 8H), 7.37 (d, 1H), 8.77 (d, 1H)

4-[1-(4-fluorophenyl)-5-(4-methylphenyl)pentyl]-1H-imidazole

$^1H$ NMR (as HCl-salt, MeOH-$d_4$):

1.1-2.7 (m, 8H), 2.26 (s, 3H), 4.05 (t, 1H), 6.8-7.6 (m, 9H), 8.78 (d, 1H)
4-[1-(4-fluorophenyl)-5-(2-methylphenyl)pentyl]-1H-imidazole
MS: 322 (53, $M^{+\cdot}$), 189 (30), 176 (55), 175 (100), 148 (18), 121 (12), 105 (42), 101 (11), 79 (12), 77 (13)
$^1$H NMR (as HCl-salt, MeOH-$d_4$):
1.1-2.7 (m, 8H), 2.24 (s, 3H), 4.11 (t, 1H), 6.9-7.5 (m, 9H), 8.79 (d, 1H)
4-[5-(3,5-dimethylphenyl)-1-(4-fluorophenyl)pentyl]-1H-imidazole
MS: 336 (47, $M^{+\cdot}$), 189 (90), 176 (67), 175 (100), 166 (13), 148 (16), 121 (12), 119 (16), 91 (14)
$^1$H NMR (as HCl-salt, MeOH-$d_4$):
1.1-2.6 (m, 8H), 2.22 (s, 6H), 4.09 (t, 1H), 6.6-7.4 (m, 7H), 7.40 (broad s, 1H), 8.80 (broad s, 1H)
4-[1-(4-fluorophenyl)-5-(3-methoxyphenyl)pentyl]-1H-imidazole
4-[5-(3,5-dimethoxyphenyl)-1-(4-fluorophenyl)pentyl]-1H-imidazole

Example 8

4-(1,3-diphenylpropyl)-1H-imidazole

a) 1-benzyl-5-(1-oxo-3-phenylpropyl)-1H-imidazole

1-benzyl-5-(1-hydroxy-3-phenylpropyl)-1H-imidazole is oxidized with manganese dioxide in tetrachloroethylene, as it is described in Example 7 c).
$^1$H NMR (as base, $CDCl_3$):
3.03 (m, 4H), 5.53 (s, 2H), 7.07-7.4 (m, 10H), 7.60 (s, 1H), 7.77 (s, 1H)
Using the same method for example the following compounds included in the invention were prepared:
1-benzyl-5-[5-(2,6-dimethylphenyl)-1-oxopentyl]-1H-imidazole. M.p. of hydrochloride 175-180 °C.
1-benzyl-5-(1-oxo-5-phenylpentyl)-1H-imidazole. M.p. of hydrochloride 185-189 °C.

b) 1-benzyl-5-(1-hydroxy-1,3-diphenylpropyl)-1H-imidazole

Grignard reagent is prepared from 5,0 g of bromobenzene and 0,76 g of Mg turnings in tetrahydrofuran. This solution is then added to 3,1 g of 1-benzyl-5-(1-oxo-3-phenylpropyl)-1H-imidazole in tetrahydrofuran and the reaction mixture is refluxed for 3 hours. The mixture is then poured into cold water, tetrahydrofuran is evaporated and the solution is made acidic with hydrochloric acid. The hydrochloride of the product is filtered, washed with toluene and dried. M.p. 196-198 °C.

Using the same method for example the following compounds included in the invention were prepared:
1-benzyl-5-(1-hydroxy-1,5-diphenylpentyl)-1H-imidazole. M.p. of hydrochloride 193-196 °C.
1-benzyl-5-[5-(2,6-dimethylphenyl)-1-hydroxy-1-phenylpentyl]-1H-imidazole. M.p. of hydrochloride 190-192 °C.

c) 1-benzyl-5-(1,3-diphenylpropyl)-1H-imidazole

1-benzyl-5-(1-hydroxy-1,3-diphenylpropyl)-1H-imidazole is treated with anhydrous potassium hydrogen sulphate at 150 °C as described in Example 7 e). The double bond of the obtained intermediate, 1-benzyl-5-(1,3-diphenyl-1-propenyl)-1H-imidazole, is hydrogenated as described in Example 7 f ). M.p. of the hydrochloride salt is 154-174 °C (from diethylether).
$^1$H NMR (as HCl-salt, MeOH-$d_4$):
2.2-2.7 (m, 4H), 3.90 (t, 1H), 5.12 (AB q, the middle of the quartet, 2H), 6.90-7.37 (m, 15H), 7.70 (broad s, 1H), 8.88 (d, 1H)
Using the same method for example the following compounds were prepared:
1-benzyl-5-(1,5-diphenylpentyl)-1H-imidazole
$^1$H NMR (as base, $CDCl_3$):
1.18-2.61 (m, 8H), 3.56 (t, 1H), 4.59 and 4.81 (AB q, 2H), 6.76-7.40 (m, 17H)
1-benzyl-5-[5-(2,6-dimethylphenyl)-1-phenylpentyl]-1H-imidazole

d) 4-(1,3-diphenylpropyl)-1H-imidazole

1-benzyl-5-(1,3-diphenylpropyl)-1H-imidazole is hydrogenated in the mixture of 2N hydrochloric acid and ethanol at 60 °C 10 % Pd/C as catalyst. The product is isolated as in Example 7 b) and is purified by flash chromatography methylene chloride-methanol (9,5:0,5) as eluent. Yield 73 %.

$^1H$ NMR (as base, $CDCl_3$):
2.1-2.7 (m, 4H), 3.88 (t, 1H), 6.71 (broad s, 1H), 7.01-7.26 (m, 10H), 7.30 (d, 1H), 10.5 (broad s, 1H)
Using the same method for example the following compound was prepared:
4-(1,5-diphenylpentyl)-1H-imidazole
$^1H$ NMR (as HCl-salt, MeOH-$d_4$):
1.2-2.3 (m, 6H), 2.57 (distorted t, 2H), 4.05 (t, 1H), 7.05-7.40 (m, 11H), 8.73 (d, 1H)

Example 9

1-benzyl-4-(1,3-diphenylpropyl)-1H-imidazole

2,0 g of benzylbromide in 5 ml of toluene is added dropwise to the mixture of 4-(1,3-diphenylpropyl)-1H-imidazole (2,6 g), 48 % NaOH (10 ml), toluene (20 ml) and tetrabutylammoniumbromide (0,2 g) at room temperature. After addition the reaction mixture is stirred at room temperature for 3 hours. Water is added and the toluene layer is separated. The toluene phase is then washed with water and evaporated to dryness. The residue contains the isomers 1-benzyl-4-(1,3-diphenylpropyl)-1H-imidazole and 1-benzyl-5-(1,3-diphenylpropyl)-1H-imidazole and the former is separated and purified by flash chromatography (methylene chloride-methanol 9,5:0,5).

Example 10

4-[1,4-bis(4-fluorophenyl)butyl]-1H-imidazole

a) 1-benzyl-5-[1-(4-fluorophenyl)-1-hydroxymethyl]-1H-imidazole

Grignard reaction of 4-bromofluorobenzene and 1-benzyl-5-imidazolecarbaldehyde is performed analogously to Example 7 a). The product is crystallized as hydrochloride salt from ethylacetate. Yield 94 %.
$^1H$ NMR (as base, $CDCl_3$ + MeOH-$d_4$):
5.05 and 5.21 (AB q, 2H), 5.64 (s, 1H), 6.61 (s, 1H), 6.97-7.1 (m, 4H), 7.26-7.33 (m, 5H), 7.41 (s, 1H)
MS: 282 (22, $M^{+\cdot}$), 265 (5), 191 (18), 91 (100)

b) 1-benzyl-5-[1-(4-fluorophenyl)-1-oxomethyl]-1H-imidazole

Oxidation of 1-benzyl-5-[1-(4-fluorophenyl)-1-hydroxymethyl]-1H-imidazole is performed analogously to Example 7 C). The crude product is recrystallized as a base from methanol. Yield 72 %.
$^1H$ NMR (as base, $CDCl_3$):
5.62 (s, 2H), 7.1-7.4 (m, 7H), 7.5-8.0 (m, 4H)
MS: 280 (46, $M^{+\cdot}$), 123 (13), 107 (4), 95 (19), 91 (100)

c) 1-benzyl-5-[1,4-bis(4-fluorophenyl)-1-hydroxybutyl]-1H-imidazole

1-benzyl-5-[1,4-bis(4-fluorophenyl)-1-hydroxybutyl]-1H-imidazole is prepared analogously to Example 7 a) starting from 3-(4-fluorophenyl)-1-bromopropane and 1-benzyl-5-[1-(4-fluorophenyl)-1-oxomethyl]-1H-imidazole. The product is purified by flash chromatography.
$^1H$ NMR (as base, $CDCl_3$):
1.2-1.4 (m, 1H), 1.65-1.85 (m, 1H), 2.1-2.25 (m, 2H), 2.52 (t, 2H), 4.73 and 4.81 (AB q, 2H), 6.75-7.3 (m, 15H)
Using the same method for example the following compounds included in the invention were prepared:
1-benzyl-5-[1-(4-fluorophenyl)-1-hydroxy-3-phenylpropyl]-1H-imidazole
MS: 386 ($M^{+\cdot}$, 8), 368 (22), 281 (100), 159 (26), 91 (99), 65 (34)
1-benzyl-5-[1,3-bis(4-fluorophenyl)-1-hydroxypropyl]-1H-imidazole
MS: 404 ($M^{+\cdot}$, 2), 386 (18), 195 (16), 281 (33), 123 (34), 91 (100), 65 (20)
1-benzyl-5-[1-(4-fluorophenyl)-1-hydroxy-4-phenylbutyl]-1H-imidazole
MS: 400 ($M^{+\cdot}$, 2), 382 (2), 281 (38), 191 (3), 91 (100), 65 (9)
1-benzyl-5-[1-(4-fluorophenyl)-1-hydroxy-5-(3-methoxyphenyl)pentyl]-1H-imidazole
$^1H$ NMR (as HCl-salt, MeOH-$d_4$):
1.0-1.8 (m, 4H), 2.26 (t, 2H), 2.52 (t, 2H), 4.71 (s, 3H), 5.06 and 5.31 (ABq, 2H), 6.6-7.5 (m, 13H), 7.7 (s, 1H), 8.6 (s, 1H)

1-benzyl-5-[5-(2,6-dimethylphenyl)-1-(4-fluorophenyl)-1-hydroxypentyl]-1H-imidazole
MS: 442 ($M^{+\cdot}$, 19), 281 (71), 256 (5), 191 (7), 119 (21), 91 (100)

d) 4-[1,4-bis(4-fluorophenyl)butyl]-1H-imidazole

1-benzyl-5-[1,4-bis(4-fluorophenyl)-1-hydroxybutyl]-1H-imidazole (10 g) is dissolved in conc. acetic acid (100 ml). Into the solution is added 0,1 g of palladium on carbon and 0,8 g of ammoniumformate. The mixture is refluxed for an hour and cooled to room temperature. The solution is filtered through silicous earth. The acetic acid filtrate is evaporated, the residue is dissolved in methylene chloride and washed once with 2 M aqueous sodium hydroxide solution and once with water. The methylene chloride solution is dried with sodium sulphate and evaporated under reduced pressure. The product is then made to hydrochloride salt with dry hydrogen chloride in diethylether. M.p. 135-137 °C.
$^1$H NMR (as base, $CDCl_3$):
1.4-1.65 (m, 2H), 1.8-1.95 (m, 1H), 2.05-2.2 (m, 1H), 2.5-2.65 (m, 2H), 3.87 (t, 1H), 6.7 (s, 1H), 6.8-7.2 (m, 8H), 7.5 (s, 1H)
Using the same method for example the following compounds included in the invention were prepared:
4-[1-(4-fluorophenyl)-3-phenylpropyl]-1H-imidazole
$^1$H NMR (as base, $CDCl_3$):
2.0-2.7 (m, 4H), 3.7-4.0 (m, 1H), 6.7 (s, 1H), 6.75-7.45 (m, 9H), 7.57 (s, 1H)
MS: 280 (4, $M^{+\cdot}$), 189 (9), 176 (100), 148 (15), 121 (9), 91 (12)
4-[1,3-bis(4-fluorophenyl)propyl]-1H-imidazole
$^1$H NMR (as base, $CDCl_3$):
2.1-2.25 (m, 1H), 2.3-2.6 (m, 3H), 3.88 (t, 1H), 6.76 (s, 1H), 6.9-7.2 (m, 8H), 7.63 (s, 1H)
MS: 298 (5, $M^{+\cdot}$), 189 (8), 176 (100), 122 (22), 109 (42)
4-[1-(4-fluorophenyl)-4-phenylbutyl]-1H-imidazole
$^1$H NMR (as base, $CDCl_3$):
1.3-2.3 (m, 4H), 2.6 (t, 2H), 3.88 (t, 1H), 6.67 (s, 1H), 6.7-7.3 (m, 9H), 7.39 (s, 1H)
MS: 294 (31, $M^{+\cdot}$), 189 (24), 175 (100), 91 (31)
4-[1-(4-fluorophenyl)-5-(3-methoxyphenyl)pentyl]-1H-imidazole
$^1$H NMR (as base, $CDCl_3$):
1.1-2.3 (m, 6H), 2.5 (t, 2H), 3.76 (s, 3H), 3.85 (distorted t, 1H), 6.6-7.6 (m, 10H)
MS: 338 (19, $M^{+\cdot}$), 189 (43), 175 (70), 148 (12), 121 (20), 36 (100)
4-[1-(4-fluorophenyl)-5-(4-methoxyphenyl)pentyl]-1H-imidazole
$^1$H NMR (as base, $CDCl_3$):
1.1-2.3 (m, 6H), 2.49 (t, 2H), 3.75 (s, 3H), 3.8 (distorted t, 1H), 6.6-7.6 (m, 10H)
MS: 338 (10, $M^{+\cdot}$), 189 (20), 175 (22), 148 (6), 121 (22), 36 (100)
4-[5-(2,6-dimethylphenyl)-1-(4-fluorophenyl)pentyl]-1H-imidazole
$^1$H NMR (as base, $CDCl_3$):
1.25-1.5 (m, 4H), 1.8-1.95 (m, 1H), 2.05-2.2 (m, 1H), 2.25 (s, 6H), 2.52 (t, 2H), 3.86 (t, 1H), 6.71 (s, 1H), 6.9-7.0 (m, 5H), 7.1-7.2 (m, 2H), 7.4 (s, 1H)
MS: 336 (50, $M^{+\cdot}$), 217 (20), 189 (75), 175 (100), 148 (13), 119 (23)

Example 11

1-benzyl-5-[1-(4-fluorophenyl)-3-phenyl-1-propenyl]-1H-imidazole

Titanium tetrachloride (0,03 mol) is added dropwise to a stirred suspension of zink powder (0,06 mol) in tetrahydrofuran (30 ml) at -10 °C under dry nitrogen. The mixture is heated to reflux and refluxing is continued for 1 hour. The solution is cooled to 0 °C and 1-benzyl-5-[1-(4-fluorophenyl)-1-oxomethyl]-1H-imidazole (0,005 mol) in tetrahydrofuran (10 ml) and phenylacetaldehyde (0,006 mol) in tetrahydrofuran (10 ml) are added into the mixture, respectively. The mixture is refluxed with stirring for 1 hour. The dark mixture is poured into water (60 ml), tetrahydrofuran is evaporated and the mixture is extracted with methylene chloride (2 x 100 ml). The methylene chloride solution is washed with 2 N sodium hydroxide and water, dried with sodium sulphate and evaporated to dryness. The residue is purified by flash chromatography.
$^1$H NMR (as base, $CDCl_3$):
3.35 and 3.45 (2d, 2H), 4.62 and 4.65 (2s, 2H), 6.03 and 6.20 (2t, 1H), 6,8-7.3 (m, 15H), 7.50 and 7.64 (2s, 1H).

Example 12

4-[1,3-bis(4-nitrophenyl)propyl]-1H-imidazole

1,8 g (14,6 mmol) of urea nitrate is added in small portions to a mixture of 2,7 g (7,3 mmol) of 4-(1,3-diphenylpropyl)-1H-imidazole in 6,4 ml of concentrated sulphuric acid under 10 °C. The reaction mixture is stirred for 2 hours at room temperature. The mixture is made alkaline with 2 M sodium hydroxide and the product is purified by flash chromatography using methylene chloride-methanol (95:5) as eluent.

In formula Ia, preferably $R_1$, $R_2$, $R'_1$ and $R'_2$ are each H and $R_4$, $R_5$, $R_6$ and $R_7$ are each H. If one phenyl group in formula Ia is mono-substituted then preferably $R_1$, $R'_2$ and $R_2$ are each H and $R_1$ is not hydrogen and is in the ortho, meta or para position of the phenyl group, preferably the para position. When one phenyl group is mono-substituted in the para position and the other group is unsubstituted, preferably the substituent is $OCH_3$.

If both phenyl groups in formula Ia are mono-substituted, preferably $R_2$ and $R'_2$ are both H, $R_1$ and $R'_1$ are both not H and are each in the para position on the respective phenyl groups. $R_1$ and $R'_1$ are preferably both F.

If a phenyl group is di-substituted, preferably $R'_1$ and $R'_2$ are both H, $R_1$ and $R_2$ are both not H and are in the 3 and 5 or 3 and 4 positions on the phenyl group.

Preferred compounds of formula Ia also include those in which $R_4$ and $R_6$ together form a bond, and those in which R' is H.

In formula Ib, preferably $R_4$, $R_5$ and R' are each H. If one phenyl group in formula Ib is mono-substituted then preferably $R_1$, $R_2$ and $R'_2$ are each H and $R'_1$ is not hydrogen and is in the para position of the phenyl group. Preferably $R'_1$ is F.

If both phenyl groups in formula Ib are monosubstituted, preferably $R_2$ and $R'_2$ are both H, $R_1$ and $R'_1$ are both not H, $R_1$ is in the ortho, meta or para position of the phenyl group and $R'_1$ is the para position of the phenyl group. Preferably $R_1$ is in the para position. Preferred substituents are $CH_3$ and F, in particular it is preferred that $R_1$ and $R'_1$ are both F, or $R_1$ is $CH_3$ and $R'_1$ is F.

If one phenyl group is di-substituted and the other is mono-substituted, preferably $R'_2$ is H, $R_1$, $R_2$ and $R'_1$ are each not H and $R'_1$ is the para position of the phenyl group and $R_1$ and $R_2$ are in the 3 and 5 or 2 and 6 positions of the phenyl group.

Preferred compounds of formula Ib include those in which

(i) $R_2$ and $R'_2$ are both H, and $R_1$ and $R'_1$ are both in the para position of the respective phenyl group;

(ii) $R'_2$ is H, $R'_1$ is in the para position and $R_1$ and $R_2$ are in the 3 and 5 positions of the phenyl group; and

(iii) $R'_2$ is H, $R'_1$ is in the para position and $R_1$ and $R_2$ are in the 2 and 6 positions of the phenyl group.

**Claims**

**Claims for the following Contracting States : AT, BE, CH, DE, DK, FR, GB, IT, LI, LU, NL, SE**

**1.** A 4(5)-substituted imidazole of the formula

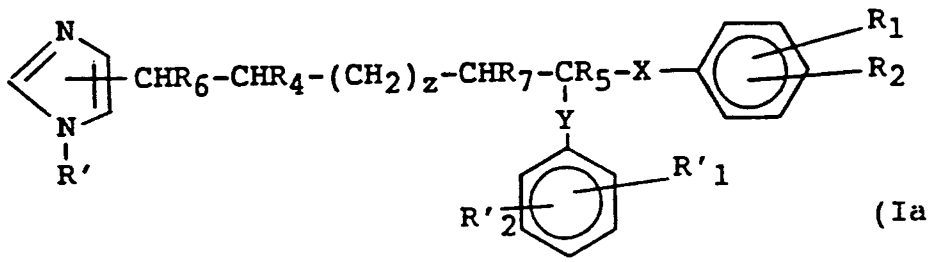

(Ia)

or a non-toxic pharmaceutically acceptable acid addition salt thereof wherein $R_1$, $R_2$, $R'_1$ and $R'_2$, which can be the same or different, are H, $CH_3$, $C_2H_5$, $C_3H_7$, $OCH_3$, OH, $CH_2OH$, $NO_2$, $NH_2$, CN, $CF_3$, $CHF_2$, $CH_2F$ or halogen; R' is H or

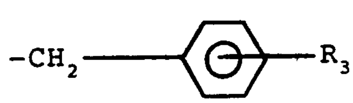

where $R_3$ is H, $CH_3$ or halogen; $R_4$ is H, $R_5$ is H or OH, $R_6$ is H or OH and $R_7$ is H or $R_4$ and $R_6$ together form a bond or $R_5$ and $R_7$ together form a bond; X and Y, which can be the same or different, are a bond, a straight $C_{1-2}$-alkyl or the corresponding alkenyl and z is 0 to 2.

**2.** A substituted imidazole according to claim 1 wherein $R_4$, $R_5$, $R_6$ and $R_7$ are H.

**3.** A substituted imidazole according to claim 1 or 2 wherein $R_1$, $R_2$, $R'_1$ and $R'_2$ each are H.

**4.** A substituted imidazole according to claim 1 or 2 wherein $R'_1$, $R'_2$ and $R_2$ each are H and $R_1$, which is as defined in claim 1 but is not H, is in the ortho, meta or para position of the phenyl group.

**5.** A substituted imidazole according to claim 4 wherein $R_1$ is in the para position of the phenyl group.

**6.** A substituted imidazole according to claim 5 wherein $R_1$ is $OCH_3$.

**7.** A substituted imidazole according to claim 1 or 2 wherein $R_2$ and $R'_2$ both are H and $R_1$ and $R'_1$, which are as defined in claim 1 but are each not H, are each in the para position of the phenyl group.

**8.** A substituted imidazole according to claim 7 wherein $R_1$ and $R'_1$ both are F.

**9.** A substituted imidazole according to claim 1 or 2 wherein $R'_1$ and $R'_2$ both are H and $R_1$ and $R_2$, which are as defined in claim 1 but are each not H, are in the 3 and 5 positions of the phenyl group.

**10.** A substituted imidazole according to claim 1 or 2 wherein $R'_1$ and $R'_2$ are H and $R_1$ and $R_2$, which are as defined in claim 1 but are each not H, are in the 3 and 4 positions of the phenyl group.

**11.** A substituted imidazole according to any one of claims 1 to 10 wherein $R_4$ and $R_6$ together form a bond.

**12.** A substituted imidazole according to any one of claims 1 to 11 wherein R′ is H.

**13.** A compound according to claim 1, which is 4-[4-(4-methylphenyl)-4-phenylbutyl]-1H-imidazole or a non-toxic pharmaceutically acceptable acid addition salt thereof.

**14.** A compound according to claim 1, which is 1-benzyl-5-[4-(4-methylphenyl)-4-phenylbutyl]-1H-imidazole or a non-toxic pharmaceutically acceptable acid addition salt thereof.

**15.** A compound according to claim 1, which is 1-benzyl-5-[4-(3-methylphenyl)-4-phenylbutyl]-1H-imidazole or a non-toxic pharmaceutically acceptable acid addition salt thereof.

**16.** A compound according to claim 1, which is 4-[4-(3-methylphenyl)-4-phenylbutyl]-1H-imidazole or a non-toxic pharmaceutically acceptable acid addition salt thereof.

**17.** A compound according to claim 1, which is 4-[4-(2-methylphenyl)-4-phenylbutyl]-1H-imidazole or a non-toxic pharmaceutically acceptable acid addition salt thereof.

**18.** A compound according to claim 1, which is 1-benzyl-5-[4-(2-methylphenyl)-4-phenylbutyl]-1H-imidazole or a non-toxic pharmaceutically acceptable acid addition salt thereof.

**19.** A compound according to claim 1, which is 1-benzyl-5-(4,5-diphenylpentyl)-1H-imidazole or a non-toxic pharmaceutically acceptable acid addition salt thereof.

**20.** A compound according to claim 1, which is 4-(4,5-diphenylpentyl)-1H-imidazole or a non-toxic pharmaceutically acceptable acid addition salt thereof.

**21.** A compound according to claim 1, which is 4-(4,4-diphenylbutyl)-1H-imidazole or a non-toxic pharmaceutically acceptable acid addition salt thereof.

**22.** A compound according to claim 1, which is 1-benzyl-5-(4,4-diphenylbutyl)-1H-imidazole or a non-toxic pharmaceutically acceptable acid addition salt thereof.

**23.** A compound according to claim 1, which is 4-[4-(4-methoxyphenyl)-4-phenylbutyl]-1H-imidazole or a non-toxic pharmaceutically acceptable acid addition salt thereof.

**24.** A compound according to claim 1, which is 4-[4,4-bis(4-methoxyphenyl)butyl]-1H-imidazole or a non-toxic pharmaceutically acceptable acid addition salt thereof.

**25.** A compound according to claim 1, which is 4-[4,4-bis(3-methylphenyl)butyl]-1H-imidazole or a non-toxic pharmaceutically acceptable acid addition salt thereof.

**26.** A compound according to claim 1, which is 4-[4-(3,5-dimethylphenyl)-4-phenylbutyl]-1H-imidazole or a non-toxic pharmaceutically acceptable acid addition salt thereof.

**27.** A compound according to claim 1, which is 4-[4-(3,4-dimethylphenyl)-4-phenylbutyl]-1H-imidazole or a non-toxic pharmaceutically acceptable acid addition salt thereof.

**28.** A compound according to claim 1, which is 4-[4-(3,5-dimethylphenyl)-4-(3-methylphenyl)butyl]-1H-imidazole or a non-toxic pharmaceutically acceptable acid addition salt thereof.

**29.** A compound according to claim 1, which is 4-[4,4-bis(4-methylphenyl)butyl]-1H-imidazole or a non-toxic pharmaceutically acceptable acid addition salt thereof.

**30.** A compound according to claim 1, which is 4-[5,5-diphenylpentyl)-1H-imidazole or a non-toxic pharmaceutically acceptable acid addition salt thereof.

**31.** A compound according to claim 1, which is 4-[6,6-diphenylhexyl)-1H-imidazole or a non-toxic pharmaceutically acceptable acid addition salt thereof.

**32.** A compound according to claim 1, which is 4-[4-(2-fluorophenyl)-4-phenylbutyl]-1H-imidazole or a non-toxic pharmaceutically acceptable acid addition salt thereof.

**33.** A compound according to claim 1, which is 4-[4-(4-fluorophenyl)-4-phenylbutyl]-1H-imidazole or a non-toxic pharmaceutically acceptable acid addition salt thereof.

**34.** A compound according to claim 1, which is 4(4,4-diphenyl-1-butenyl)-1H-imidazole or a non-toxic pharmaceutically acceptable acid addition salt thereof.

**35.** A compound according to claim 1, which is 4-[4,4-bis(4-fluorophenyl)butyl]-1H-imidazole or a non-toxic pharmaceutically acceptable acid addition salt thereof.

**36.** A compound according to claim 1, which is 4-[4,4-bis(4-nitrophenyl)butyl]-1H-imidazole or a non-toxic pharmaceutically acceptable acid addition salt thereof.

**37.** A compound according to claim 1, which is 4-[4,4-bis(4-aminophenyl)butyl]-1H-imidazole or a non-toxic pharmaceutically acceptable acid addition salt thereof.

**38.** A compound according to claim 1, which is 4-[4-(4-ethylphenyl)-4-phenylbutyl]-1H-imidazole or a non-toxic pharmaceutically acceptable acid addition salt thereof.

**39.** A pharmaceutical composition comprising a substituted imidazole as claimed in any one of claims 1 to 38 and a pharmaceutically acceptable carrier.

**40.** An imidazole derivative as claimed in any one of claims 1 to 39 or a non-toxic addition salt for use in therapy as an aromatase inhibiting agent.

**41.** A 4(5)-substituted imidazole of the formula

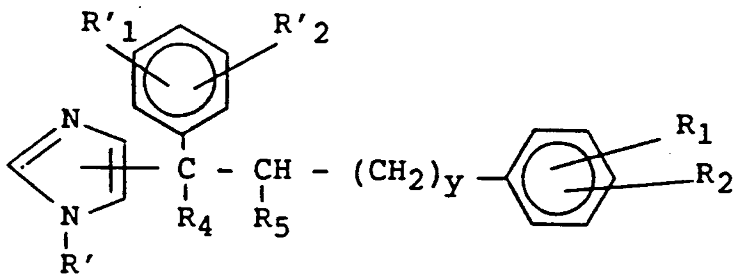

(Ib)

or a non-toxic pharmaceutically acceptable acid addition salt thereof wherein $R_1$, $R_2$, $R'_1$ and $R'_2$, which can be the same or different, are H, $CH_3$, $C_2H_5$, $C_3H_7$, $OCH_3$, OH, $CH_2OH$, $NO_2$, $NH_2$, CN, $CF_3$, $CHF_2$, $CH_2F$ or halogen; R′ is H or

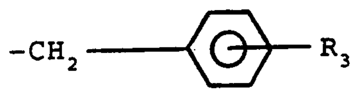

where $R_3$ is H, $CH_3$ or halogen; $R_4$ is H or OH and $R_5$ is H or $R_4$ and $R_5$ together form a bond and y is 0 to 4.

**42.** A substituted imidazole according to claim 41 wherein $R_4$, $R_5$ and R′ each are H.

**43.** A substituted imidazole according to claim 41 or 42 wherein $R_1$, $R_2$ and $R'_2$ each are H and $R'_1$ which is as defined in claim 41, is in the para position of the phenyl group.

**44.** A substituted imidazole according to claim 43 wherein $R'_1$ is F.

**45.** A substituted imidazole according to claim 41 or 42 wherein $R_2$ and $R'_2$ each are H and $R_1$, which is as defined in claim 41, is in the ortho, meta or para position of the phenyl group and $R'_1$, which is as defined in claim 41, is in the para position of the phenyl group.

**46.** A substituted imidazole according to claim 45 wherein $R_2$ and $R'_2$ both are H and $R_1$ and $R'_1$, which are as defined in claim 41, both are in the para position of the phenyl group.

**47.** A substituted imidazole according to claim 46 wherein $R_1$ and $R'_1$ both are F.

**48.** A substituted imidazole according to claim 46 wherein R is $CH_3$ and $R'_1$ is F.

**49.** A substituted imidazole according to claim 41 or 42 wherein $R'_2$ is H, $R'_1$, which is as defined in claim 41, is in the para position of the phenyl group and $R_1$ and $R_2$, which are defined as in claim 41, are in the 3 and 5 positions of the phenyl group.

**50.** A substituted imidazole according to claim 41 or 42 wherein $R'_2$ is H, $R'_1$, which is as defined in claim 41, is in the para position of the phenyl group and $R_1$ and $R_2$, which are as defined in claim 41, are in the 2 and 6 positions of the phenyl group.

**51.** A compound according to claim 41, which is 4-[1-(4-fluorophenyl)-5-phenylpentyl]-1H-imidazole or a non-toxic pharmaceutically acceptable acid addition salt thereof.

**52.** A compound according to claim 41, which is 4-[1-(4-fluorophenyl)-5-(2-methylphenyl)pentyl]-1H-imidazole or a non-toxic pharmaceutically acceptable acid addition salt thereof.

**53.** A compound according to claim 41, which is 4-[1-(4-fluorophenyl)-5-(3-methylphenyl)pentyl]-1H-imidazole or a non-toxic pharmaceutically acceptable acid addition salt thereof.

**54.** A compound according to claim 41, which is 4-[1-(4-fluorophenyl)-5-(4-methylphenyl)pentyl]-1H-imidazole or a non-toxic pharmaceutically acceptable acid addition salt thereof.

**55.** A compound according to claim 41, which is 4-[5-(3,5-dimethylphenyl)-1-(4-fluorophenyl)pentyl]-1H-imidazole or a non-toxic pharmaceutically acceptable acid addition salt thereof.

**56.** A compound according to claim 41, which is 4-(1,5-diphenylpentyl)-1H-imidazole or a non-toxic pharmaceutically acceptable acid addition salt thereof.

**57.** A compound according to claim 41, which is 4-(1,3-diphenylpropyl)-1H-imidazole or a non-toxic pharmaceutically acceptable acid addition salt thereof.

**58.** A compound according to claim 41, which is 1-benzyl-5-(1,3-diphenylpropyl)-1H-imidazole or a non-toxic pharmaceutically acceptable acid addition salt thereof.

**59.** A compound according to claim 41, which is 4-[1-(4-fluorophenyl)-3-phenylpropyl]-1H-imidazole or a non-toxic pharmaceutically acceptable acid addition salt thereof.

**60.** A compound according to claim 41, which is 4-[1-(4-fluorophenyl)-4-phenylbutyl]-1H-imidazole or a non-toxic pharmaceutically acceptable acid addition salt thereof.

**61.** A compound according to claim 41, which is 4-[1-(4-fluorophenyl)-5-(3-methoxyphenyl)pentyl]-1H-imidazole or a non-toxic pharmaceutically acceptable acid addition salt thereof.

**62.** A compound according to claim 41, which is 4-[1-(4-fluorophenyl)-5-(4-methoxyphenyl)pentyl]-1H-imidazole or a non-toxic pharmaceutically acceptable acid addition salt thereof.

**63.** A compound according to claim 41, which is 4-[1-(4-fluorophenyl)-5-(2,6-dimethylphenyl)pentyl]-1H-imidazole or a non-toxic pharmaceutically acceptable acid addition salt thereof.

**64.** A compound according to claim 41, which is 4-[1,3-bis(4-fluorophenyl)propyl]-1H-imidazole or a non-toxic pharmaceutically acceptable acid addition salt thereof.

**65.** A compound according to claim 41, which is 4-[1,4-bis(4-fluorophenyl)butyl]-1H-imidazole or a non-toxic pharmaceutically acceptable acid addition salt thereof.

**66.** A pharmaceutical composition comprising a substituted imidazole as claimed in any one of claims 41 to 65 and a pharmaceutically acceptable carrier.

**67.** An imidazole derivative as claimed in any one of claims 41 to 66 or a non-toxic acid addition salt for use in therapy as an aromatse inhibiting agent.

**Claims for the following Contracting States : ES, GR**

**1.** A process for the preparation of a 4(5)-substituted imidazole of the formula

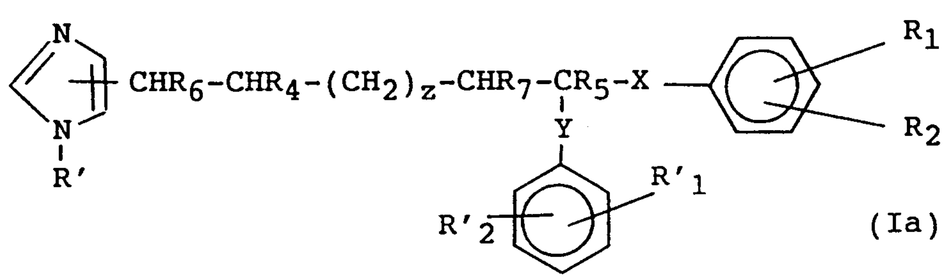

(Ia)

or a non-toxic pharmaceutically acceptable acid addition salt thereof wherein $R_1$, $R_2$, $R'_1$ and $R'_2$, which can be the same or different, are H, $CH_3$, $C_2H_5$, $C_3H_7$, $OCH_3$, $NO_2$, $CF_3$, $CHF_2$, $CH_2F$ or halogen; R′ is H or

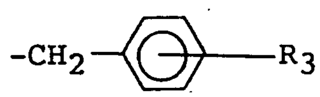

where $R_3$ is H, $CH_3$ or halogen; $R_4$ is H, $R_5$ is H or OH, $R_6$ is H or OH and $R_7$ is H or $R_4$ and $R_6$ together form a bond or $R_5$ and $R_7$ together form a bond; X and Y, which can be the same or different, are a bond, a straight $C_{1-2}$-alkyl or the corresponding alkenyl and z is 0 to 2 which comprises reacting an 4(5)-imidazole derivative of the formula

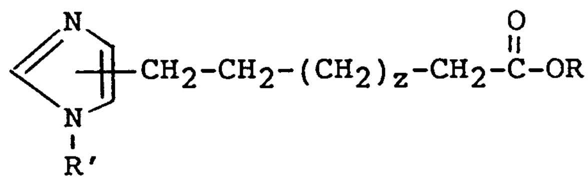

wherein R′ and z are as defined before and R is alkyl with a Grignard reagent of the formula

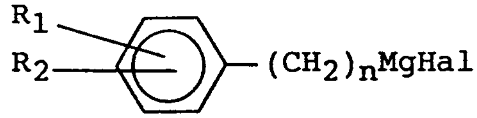

where $R_1$ and $R_2$ are as defined before and n is 0 to 2 to give a compound of formula (Ia) which is

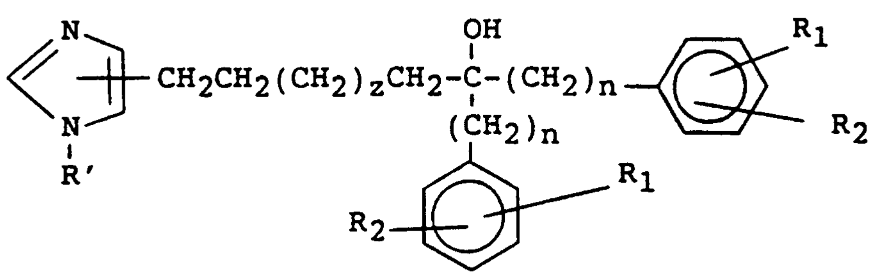

**2.** A process for the preparation of a compound as defined in claim 1 or a non-toxic pharmaceutically acceptable acid addition salt thereof which comprises reacting an 4(5)-imidazole derivative of the formula

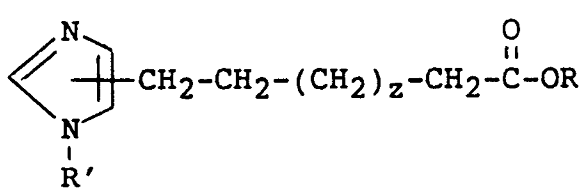

wherein R′ and z are as defined before and R is alkyl with a Grignard reagent

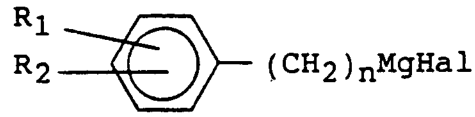

where $R_1$ and $R_2$ are as defined before and n is 0 to 2 to give a compound of the formula

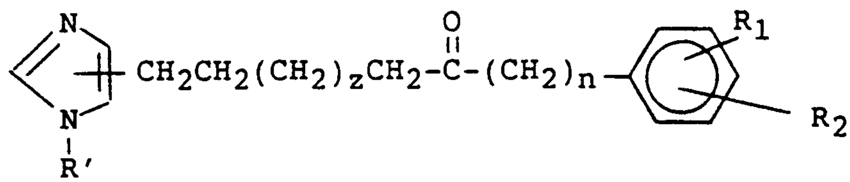

which further is reacted with another Grignard reagent of the formula

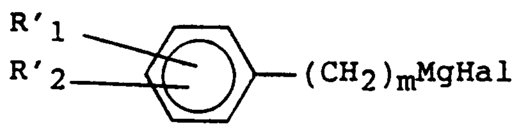

where $R'_1$ and $R'_2$ are as defined before and m is 0 to 2 to give a compound of formula (Ia) which is

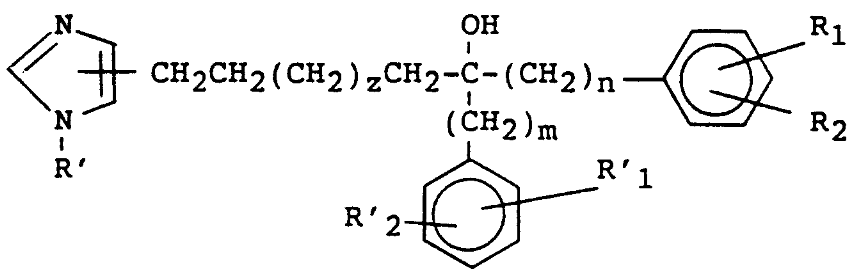

**3.** A process according to claim 1 to 2 for the preparation of a compound as defined in claim 1 or a non-toxic pharmaceutically acceptable acid addition salt thereof wherein an amide of the formula

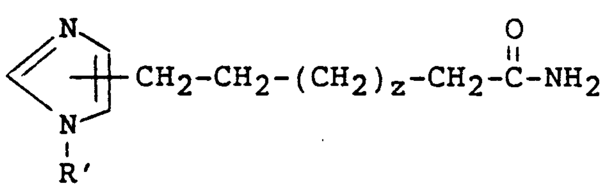

wherein R′ and z are as defined before, is used as starting material instead of the corresponding ester.

**4.** A process according to claim 1 to 2 for the preparation of a compound as defined in claim 1 or a non-toxic pharmaceutically acceptable acid addition salt thereof wherein a nitrile of the formula

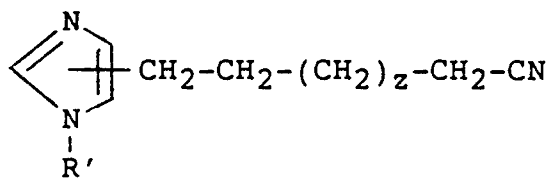

wherein R′ and z are as defined before, is used as starting material instead of the corresponding ester.

5. A process for the preparation of a compound as defined in claim 1 or a non-toxic pharmaceutically acceptable acid addition salt thereof which comprises reacting an 4(5)-imidazole aldehyde of the formula

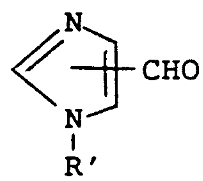

where R′ is as defined before with a Grignard reagent

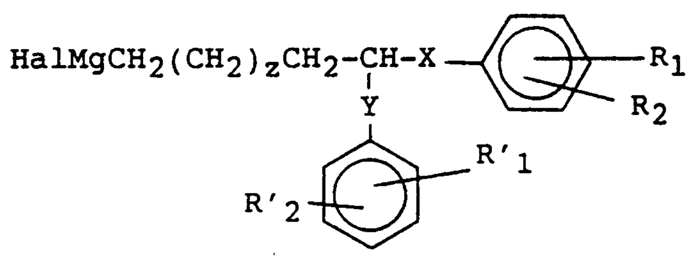

wherein $R_1$, $R_2$, $R'_1$, $R'_2$, X, Y and z are as defined before, to give a compound of formula (Ia) which is

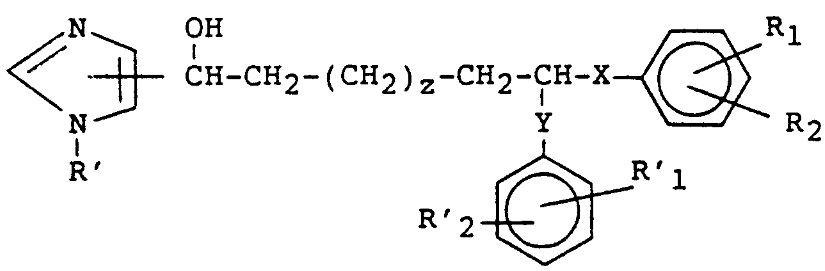

6. A process for the preparation of a 4(5)-substituted imidazole of the formula

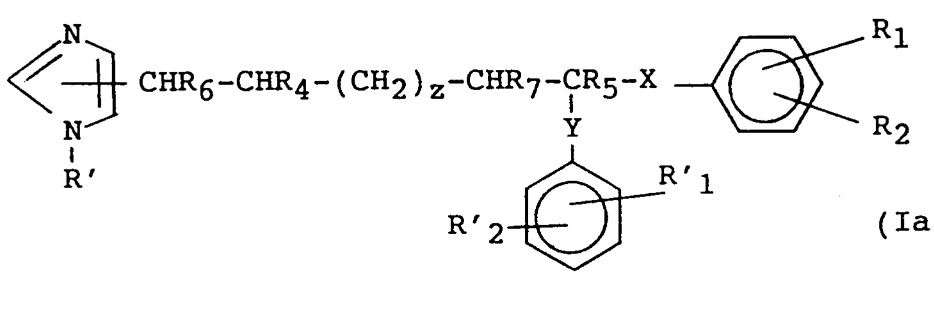

(Ia)

or a non-toxic pharmaceutically acceptable acid addition salt thereof wherein $R_1$, $R_2$, $R'_1$ and $R'_2$, which can be the same or different, are H, $CH_3$, $C_2H_5$, $C_3H_7$, $OCH_3$, OH, $CH_2OH$, $NO_2$, $NH_2$, CN, $CF_3$, $CHF_2$, $CH_2F$ or halogen; R′ is H or

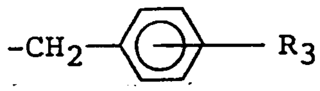

where $R_3$ is H, $CH_3$ or halogen; $R_4$ is H, $R_5$ is H or OH, $R_6$ is H or OH and $R_7$ is H or $R_4$ and $R_6$ together form a bond or $R_5$ and $R_7$ together form a bond; X and Y, which can be the same or different, are a bond, a straight $C_{1-2}$-alkyl or the corresponding alkenyl and z is 0 to 2 which comprises reacting an 4(5)-imidazole derivative of the formula

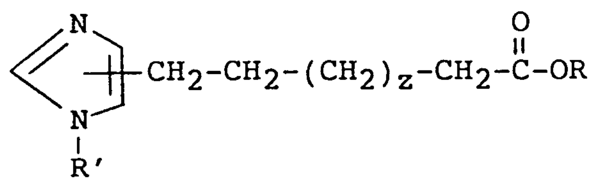

wherein R′ and z are as defined before and R is alkyl with a Grignard reagent

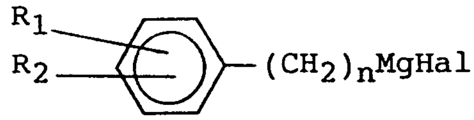

where n is 0 to 2 and $R_1$ and/or $R_2$ are $OCH_2Ph$ or OTHP, to give a compound of formula

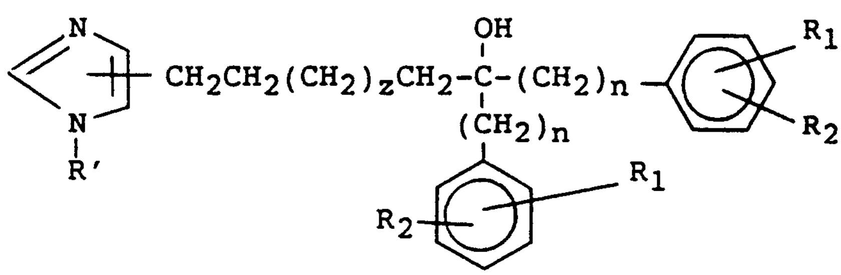

wherein $R_1$ and/or $R_2$ are $OCH_2Ph$ or OTHP, which further is hydrogenated to give a compound of formula (Ia) which is

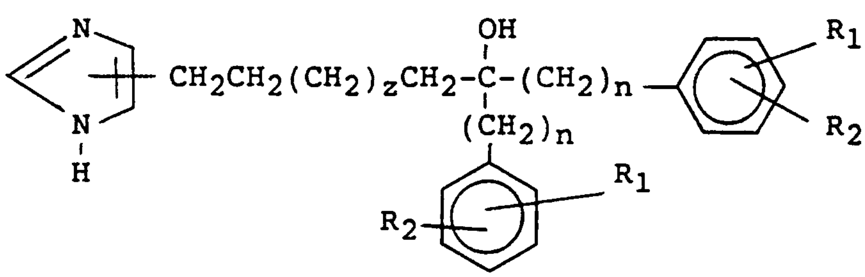

wherein z and n are as defined before and $R_1$ and/or $R_2$ are OH and $R_1$ or $R_2$ that is not OH is H, $CH_3$,

$C_2H_5$, $C_3H_7$, $OCH_3$, $NO_2$, $CF_3$, $CHF_2$, $CH_2F$ or halogen.

**7.** A process for the preparation of a compound as defined in claim 6 or a non-toxic pharmaceutically acceptable acid addition salt thereof which comprises reacting an 4(5)-imidazole derivative of the formula

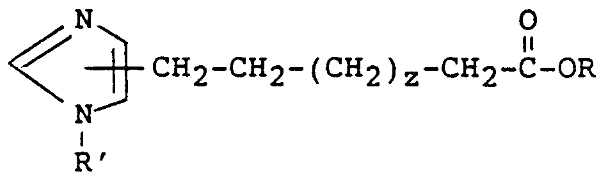

wherein R′ and z are as defined before and R is alkyl with a Grignard reagent

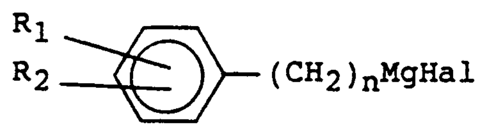

where n is 0 to 2 and

i) $R_1$ and/or $R_2$ are $OCH_2Ph$ or OTHP or

ii) neither $R_1$ nor $R_2$ is $OCH_2Ph$ or OTHP

to give a compound of the formula

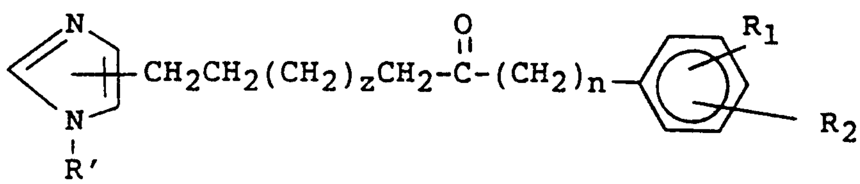

which further is reacted with another Grignard reagent of the formula

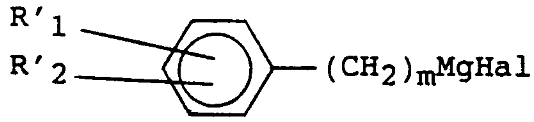

where m is 0 to 2 and

iii) $R'_1$ and/or $R'_2$ are $OCH_2Ph$ or OTHP or

iv) neither $R'_1$ nor $R'_2$ is $OCH_2Ph$ or OTHP

provided that ii) and iv) are not in force at the same time, to give a compound of formula

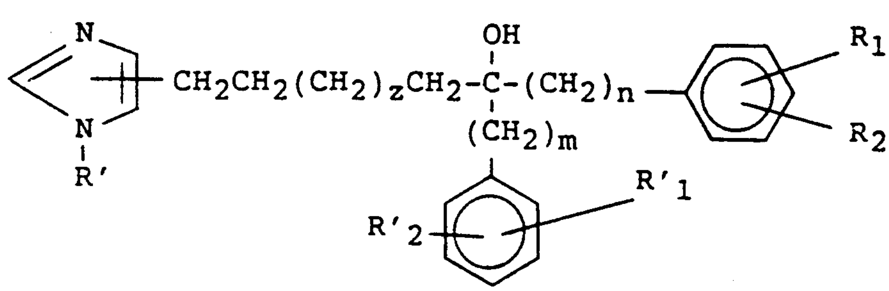

wherein one or more of the substituents $R_1$, $R_2$, $R'_1$ and $R'_2$ are $OCH_2Ph$ or OTHP, which further is hydrogenated to give a compound of formula (Ia) which is

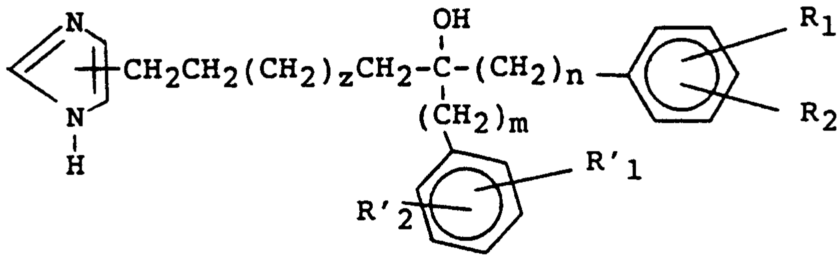

wherein z is 0 to 2, m and n, which can be the same or different, are 0 to 2 and one or more of the substituents $R_1$, $R_2$, $R'_1$ and $R'_2$ are OH and the substituents that are not OH are H, $CH_3$, $C_2H_5$,$C_3H_7$, $OCH_3$, $NO_2$, $CF_3$, $CHF_2$, $CH_2F$ or halogen.

**8.** A process according to claim 6 to 7 for the preparation of a compound of formula (Ia) as defined in claim 6 or a non-toxic pharmaceutically acceptable acid addition salt thereof wherein an amide of the formula

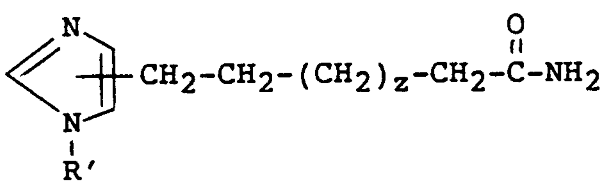

wherein R′ and z are as defined before, is used as starting material instead of the corresponding ester.

**9.** A process according to claim 6 to 7 for the preparation of a compound of formula (Ia) as defined in claim 6 or a non-toxic pharmaceutically acceptable acid addition salt thereof wherein a nitrile of the formula

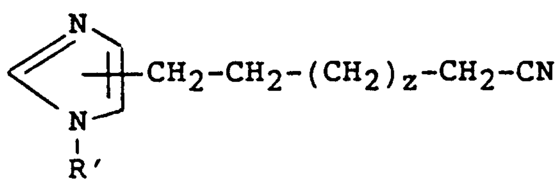

wherein R′ and z are as defined before, is used as starting material instead of the corresponding ester.

**10.** A process for the preparation of a compound as defined in claim 6 or a non-toxic pharmaceutically acceptable acid addition salt thereof which comprises reacting an 4(5)-imidazole aldehyde of the formula

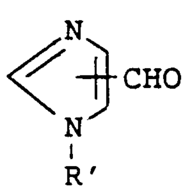

wherein R′ is as defined before, with a Grignard reagent

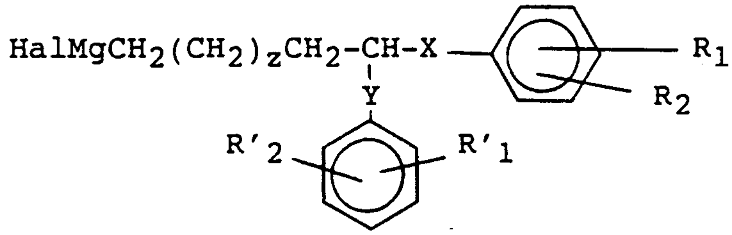

wherein X, Y and z are as defined before and one or more of the substituents $R_1$, $R_2$, $R'_1$ and $R'_2$ are $OCH_2Ph$ or OTHP, to give a compound of formula

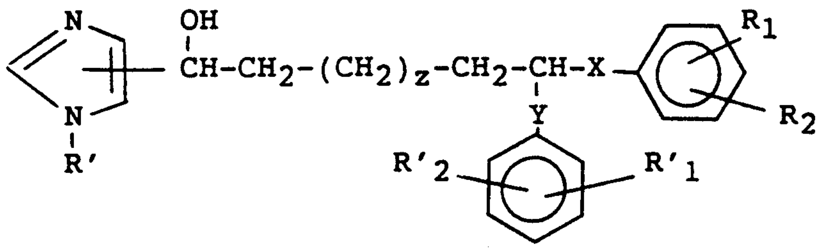

wherein one or more of the substituents $R_1$, $R_2$, $R'_1$ and $R'_2$ are $OCH_2Ph$ or OTHP, which further is hydrogenated to give a compound of formula (Ia) which is

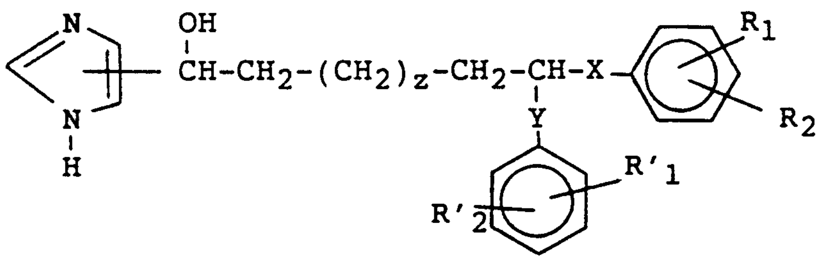

wherein X, Y and z are as defined before and one or more of the substituents $R_1$, $R_2$, $R'_1$ and $R'_2$ are OH and the substituents that are not OH are H, $CH_3$, $C_2H_5$, $C_3H_7$, $OCH_3$, $NO_2$, $CF_3$, $CHF_2$, $CH_2F$ or halogen.

**11.** A process for the preparation of a compound as defined in claim 6 or a non-toxic pharmaceutically acceptable acid addition salt thereof which comprises dealkylating a compound of formula (Ia)

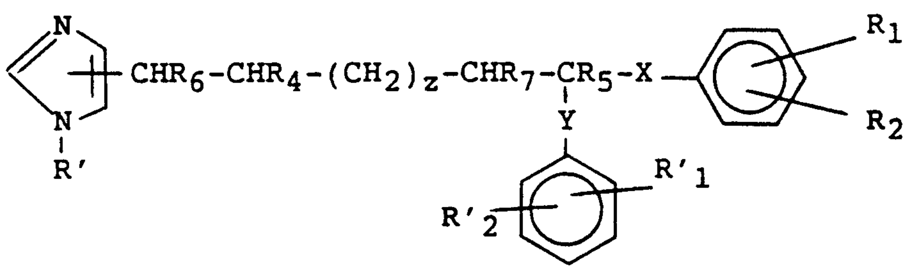

wherein R', $R_4$, $R_5$, $R_6$, $R_7$, X, Y and z are as defined before and one or more of the substituents $R_1$, $R_2$, $R'_1$ and $R'_2$ are $OCH_3$, to give a compound of formula (Ia) wherein one or more of the substituents $R_1$, $R_2$, $R'_1$ and $R'_2$ are OH.

**12.** A process for the preparation of a compound as defined in claim 6 or a non-toxic pharmaceutically acceptable acid addition salt thereof which comprises hydrolyzing a compound of formula (Ia)

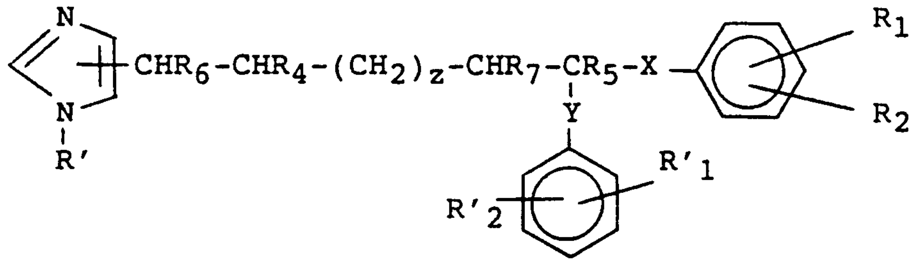

wherein R′, $R_4$, $R_5$, $R_6$, $R_7$, X, Y and z are as defined before and one or more of the substituents $R_1$, $R_2$, $R'_1$ and $R'_2$ are CN and further reducing to give a compound of formula (Ia) wherein one or more of the substituents $R_1$, $R_2$, $R'_1$ and $R'_2$ are $CH_2OH$.

**13.** A process for the preparation of a compound as defined in claim 6 or a non-toxic pharmaceutically acceptable acid addition salt thereof which comprises diazotizing a compound of formula (Ia)

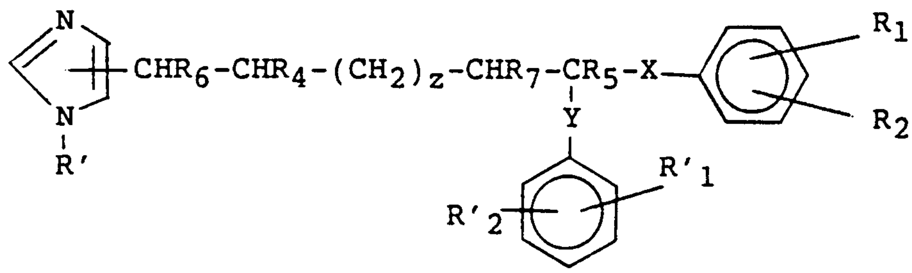

wherein R′, $R_4$, $R_5$, $R_6$, $R_7$, X, Y and z are as defined before and one or more of the substituents $R_1$, $R_2$, $R'_1$ and $R'_2$ are $NH_2$ to give a compound of formula (Ia) wherein one or more of the substituents $R_1$, $R_2$, $R'_1$ and $R'_2$ are CN.

**14.** A process for the preparation of a compound as defined in claim 6 or a non-toxic pharmaceutically acceptable acid addition salt thereof which comprises hydrogenating a compound of formula (Ia)

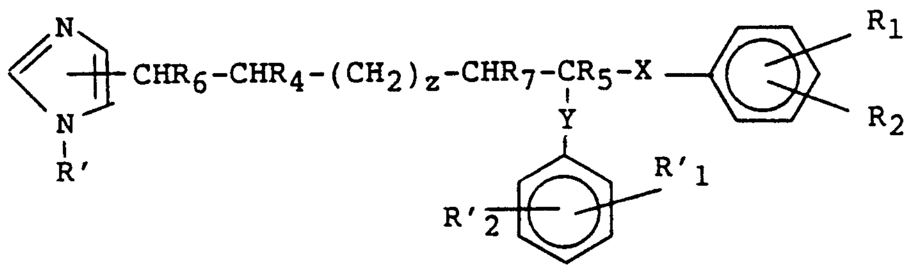

wherein R′, $R_4$, $R_5$, $R_6$, $R_7$, X, Y and z are as defined before and one or more of the substituents $R_1$, $R_2$, $R'_1$ and $R'_2$ are $NO_2$, to give a compound of formula (Ia) wherein R′ is H, $R_4$, $R_5$, $R_6$, $R_7$, X, Y and z are as defined before and one or more of the substituents $R_1$, $R_2$, $R'_1$ and $R'_2$ are $NH_2$.

**15.** A process for the preparation of a compound as defined in claim 6 or a non-toxic pharmaceutically acceptable acid addition salt thereof which comprises dehydrating a compound of formula

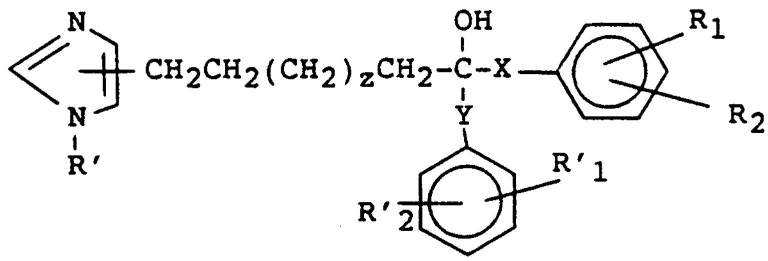

to give a compound of formula (Ia) which is

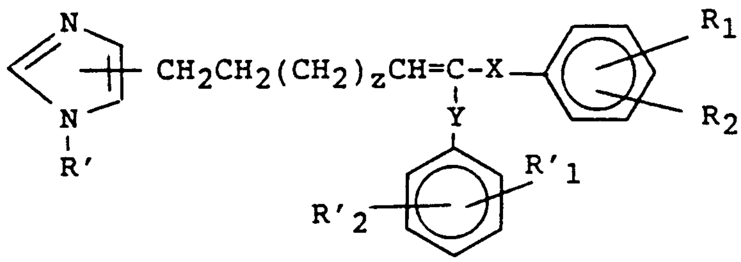

wherein R′, $R_1$, $R_2$, $R'_1$, $R'_2$ and z are as defined before.

**16.** A process for the preparation of a compound as defined in claim 6 or a non-toxic pharmaceutically acceptable acid addition salt thereof which comprises reacting a compound of formula

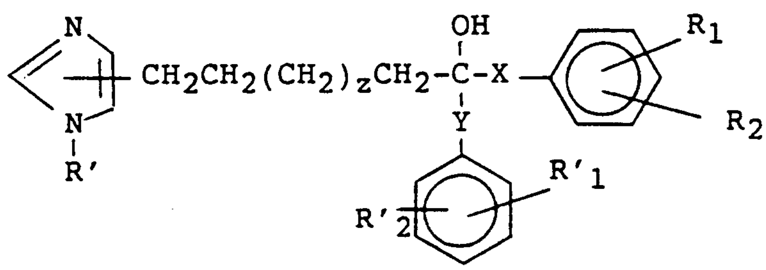

by a hydrogen transfer reaction to give a compound of formula (Ia) which is

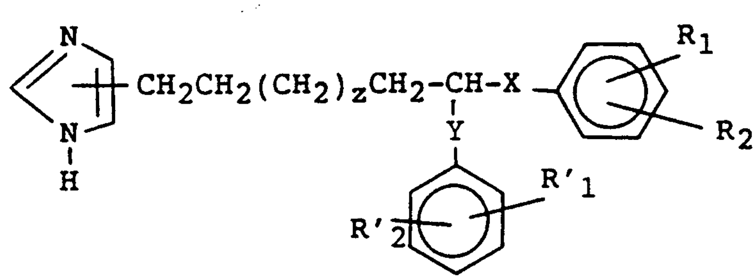

wherein $R_1$, $R_2$, $R'_1$, $R'_2$, X, Y and z are as defined before.

**17.** A process for the preparation of a compound as defined in claim 6 or a non-toxic pharmaceutically acceptable acid addition salt thereof which comprises hydrogenating a compound of formula

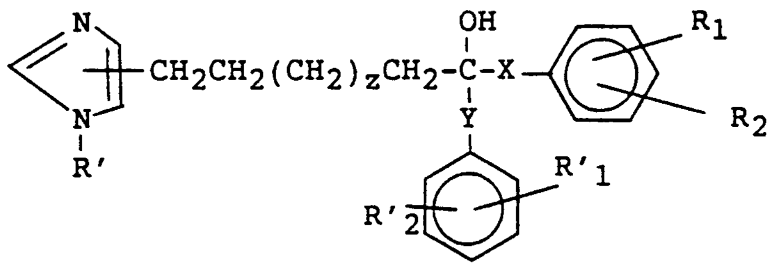

wherein R′ is substituted or unsubstituted benzyl and $R_1$, $R_2$, $R'_1$, $R'_2$, X, Y and z are as defined before to give a compound of formula (Ia) which is

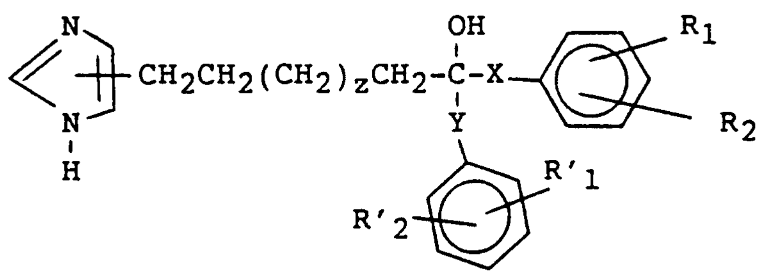

**18.** A process for the preparation of a compound as defined in claim 6 or a non-toxic pharmaceutically acceptable acid addition salt thereof which comprises reacting a compound of formula

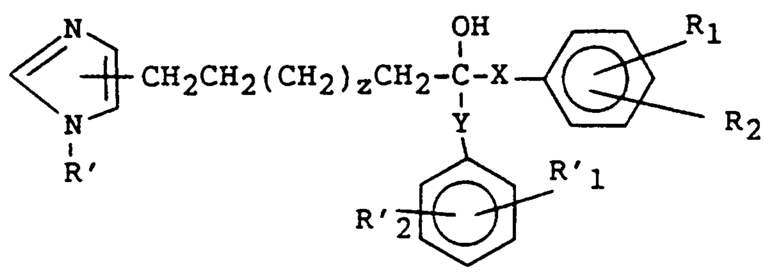

wherein R′ is substituted or unsubstituted benzyl and $R_1$, $R_2$, $R'_1$, $R'_2$, X, Y and z are as defined before, by a hydrogen transfer reaction to give a compound of formula (Ia) which is

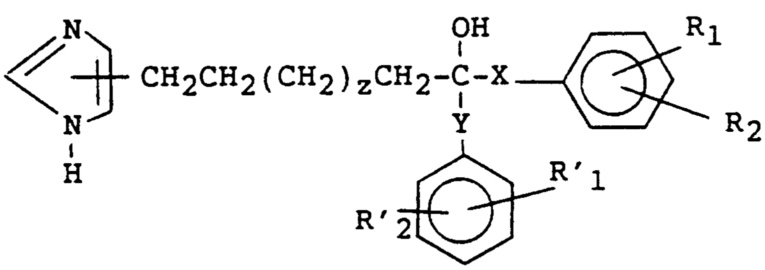

**19.** A process for the preparation of a compound as defined in claim 6 or a non-toxic pharmaceutically acceptable acid addition salt thereof which comprises hydrogenating a compound of formula

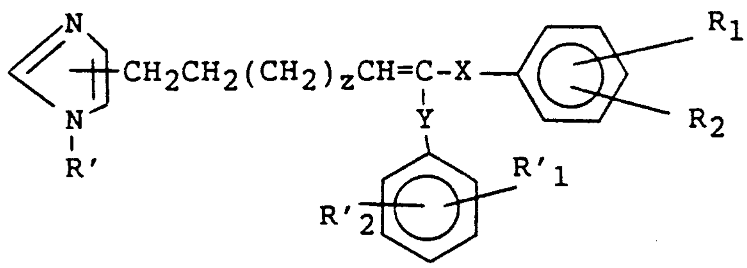

to give a compound of formula (Ia) which is

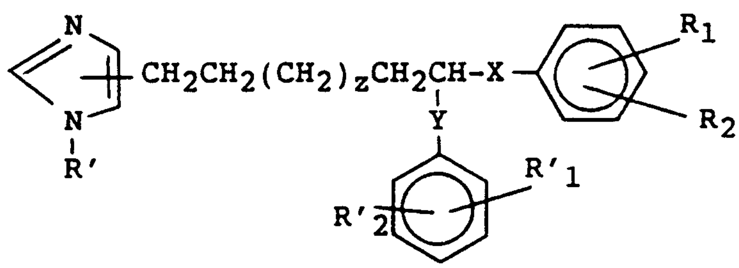

wherein R′, $R_1$, $R_2$, $R'_1$, $R'_2$, X, Y and z are as defined before.

**20.** A process for the preparation of a compound as defined in claim 6 or a non-toxic pharmaceutically acceptable acid addition salt thereof which comprises hydrogenating a compound of formula

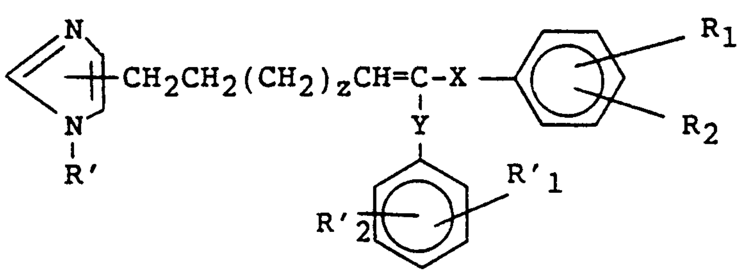

to give a compound of formula (Ia) which is

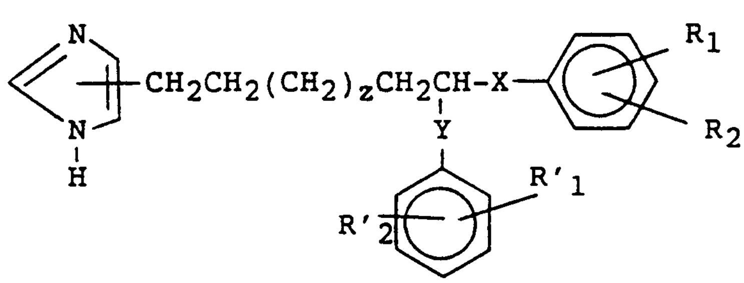

wherein $R_1$, $R_2$, $R'_1$, $R'_2$, X, Y and z are as defined before.

**21.** A process for the preparation of a compound as defined in claim 6 or a non-toxic pharmaceutically acceptable acid addition salt thereof which comprises reacting a compound of formula

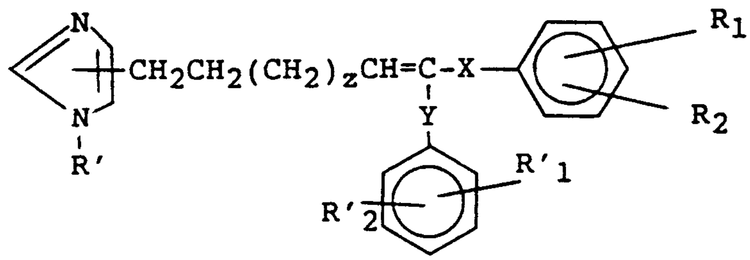

by a hydrogen transfer reaction to give a compound of formula (Ia) which is

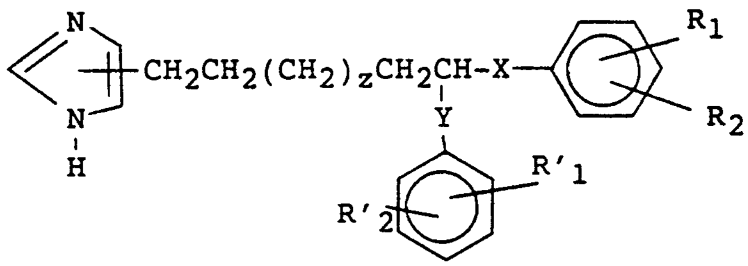

wherein $R_1$, $R_2$, $R'_1$, $R'_2$, X, Y and z are as defined before.

**22.** A process for the preparation of a compound as defined in claim 6 or a non-toxic pharmaceutically acceptable acid addition salt thereof which comprises dehydrating a compound of formula

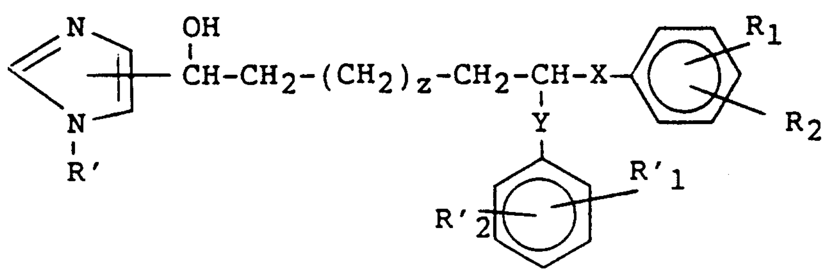

to give a compound of formula (Ia) which is

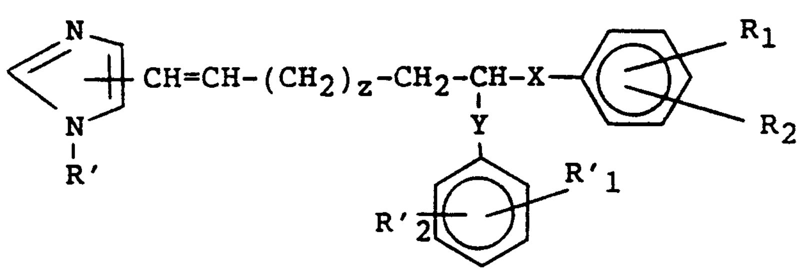

wherein R', $R_1$, $R_2$, $R'_1$, $R'_2$, X, Y and z are as defined before.

**23.** A process for the preparation of a compound as defined in claim 6 or a non-toxic pharmaceutically acceptable acid addition salt thereof which comprises reacting a compound of formula

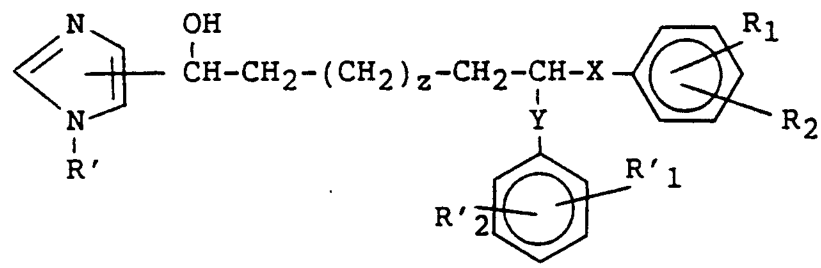

by a hydrogen transfer reaction to give a compound of formula (Ia) which is

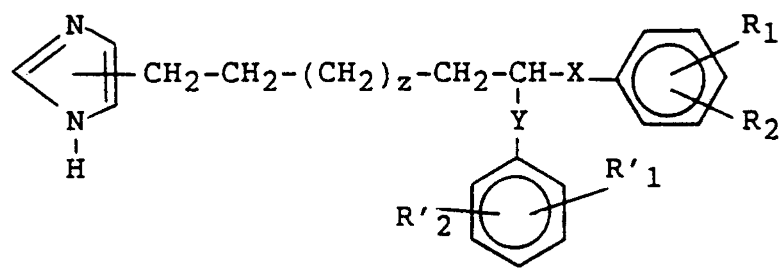

wherein $R_1$, $R_2$, $R'_1$, $R'_2$, X, Y and z are as defined before.

**24.** A process for the preparation of a compound as defined in claim 6 or a non-toxic pharmaceutically acceptable acid addition salt thereof which comprises hydrogenating a compound of formula

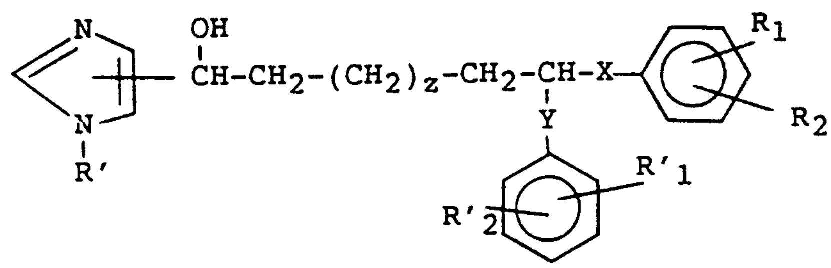

wherein R' is substituted or unsubstituted benzyl and $R_1$, $R_2$, $R'_1$, $R'_2$, X, Y and z are as defined before to give a compound of formula (Ia) which is

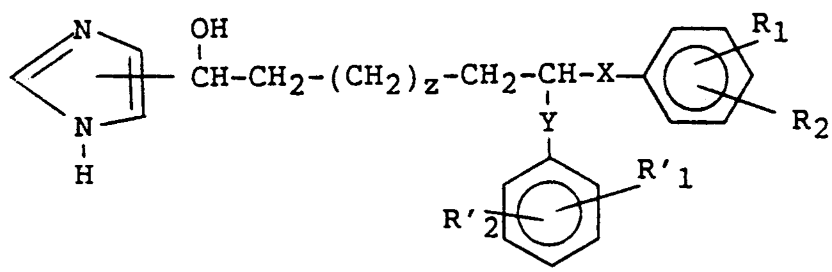

**25.** A process for the preparation of a compound as defined in claim 6 or a non-toxic pharmaceutically acceptable acid addition salt thereof which comprises reacting a compound of formula

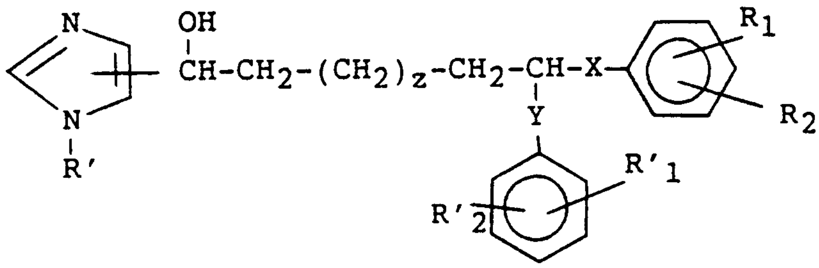

wherein R′ is substituted or unsubstituted benzyl and $R_1$, $R_2$, $R'_1$, $R'_2$, X, Y and z are as defined before, by a hydrogen transfer reaction to give a compound of formula (Ia) which is

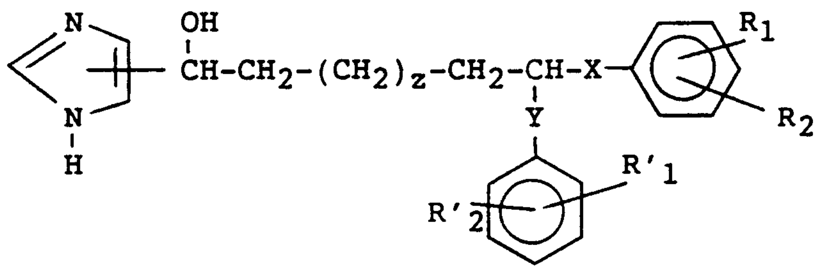

**26.** A process for the preparation of a compound as defined in claim 6 or a non-toxic pharmaceutically acceptable acid addition salt thereof which comprises hydrogenating a compound of formula

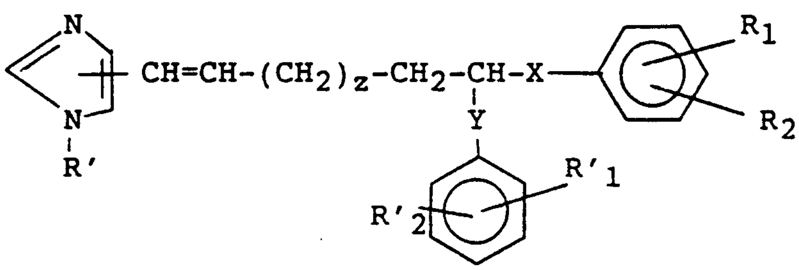

to give a compound of formula (Ia) which is

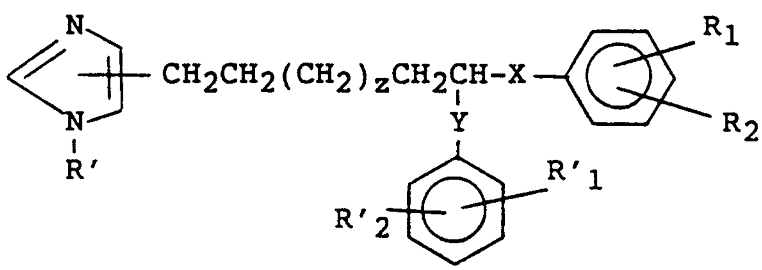

wherein R′, $R_1$, $R_2$, $R'_1$, $R'_2$, X, Y and z are as defined before.

**27.** A process for the preparation of a compound as defined in claim 6 or a non-toxic pharmaceutically acceptable acid addition salt thereof which comprises hydrogenating a compound of formula

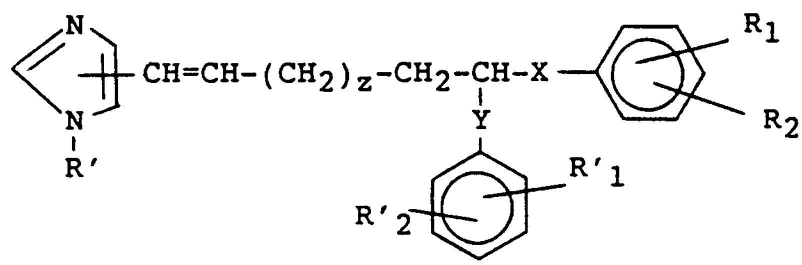

wherein R′, $R_1$, $R_2$, $R'_1$, $R'_2$, X, Y and z are as defined before to give a compound of formula (Ia) which is

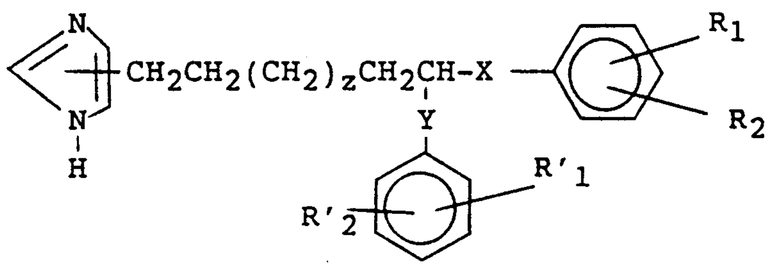

**28.** A process for the preparation of a compound as defined in claim 6 or a non-toxic pharmaceutically acceptable acid addition salt thereof which comprises reacting a compound of formula

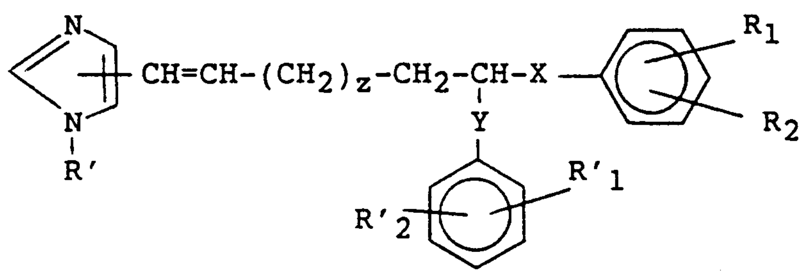

wherein R′, $R_1$, $R_2$, $R'_1$, $R'_2$, X, Y and z are as defined before, by a hydrogen transfer reaction to give a compound of formula (Ia) which is

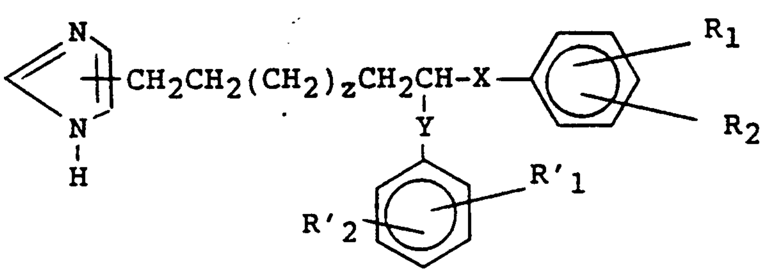

**29.** A process for the preparation of a compound as defined in claim 6 or a non-toxic pharmaceutically acceptable acid addition salt thereof which comprises hydrogenating a compound of formula

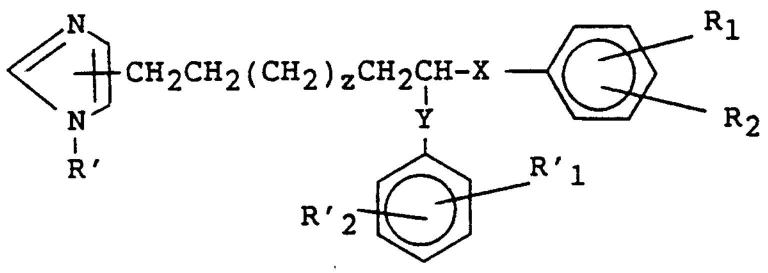

wherein $R_1$, $R_2$, $R'_1$, $R'_2$, X, Y and z are as defined before and R′ is substituted or unsubstituted benzyl to give a compound of formula (Ia) which is

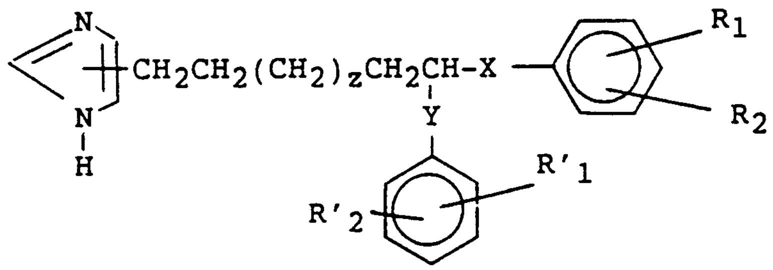

**30.** A process for the preparation of a compound as defined in claim 6 or a non-toxic pharmaceutically acceptable acid addition salt thereof which comprises reacting a compound of formula

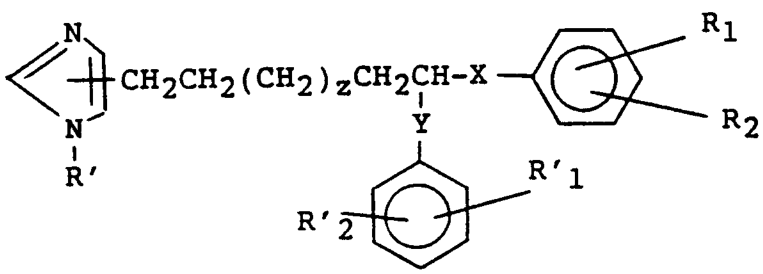

wherein $R_1$, $R_2$, $R'_1$, $R'_2$, X, Y and z are as defined before and R′ is substituted or unsubstituted benzyl by a hydrogen transfer reaction to give a compound of formula (Ia) which is

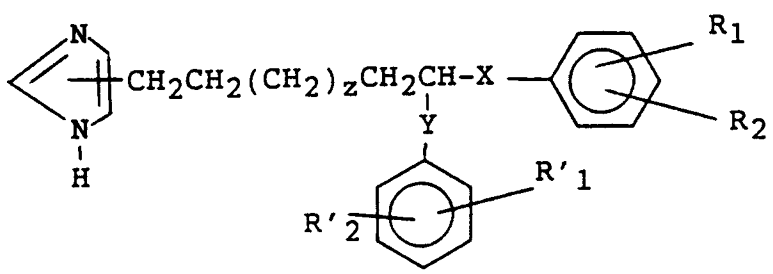

**31.** A process for the preparation of a compound as defined in claim 6 or a non-toxic pharmaceutically acceptable acid addition salt thereof which comprises reacting a compound of formula

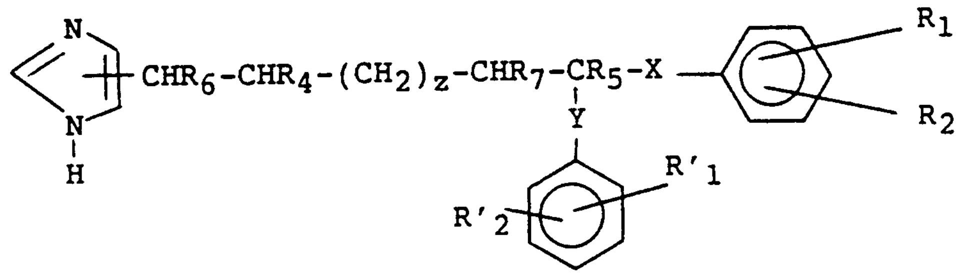

wherein $R_1$, $R_2$, $R'_1$, $R'_2$, $R_4$, $R_5$, $R_6$, $R_7$, X, Y and z are as defined before with a strong base and further with a benzyl halide of the formula

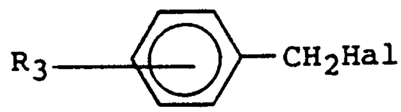

where $R_3$ is as defined before to give a compound of formula (Ia) which is

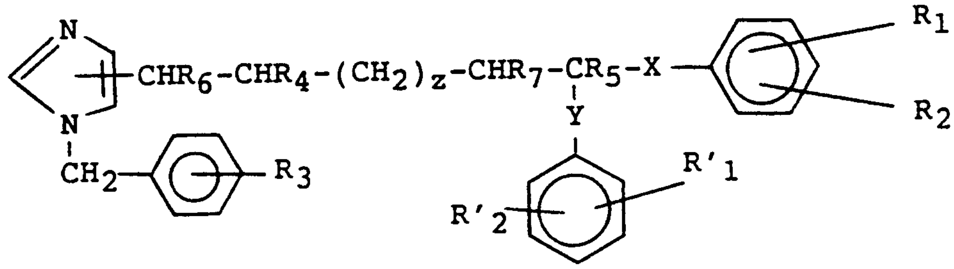

**32.** A process for the preparation of a compound as defined in claim 6 or a non-toxic pharmaceutically acceptable acid addition salt thereof which comprises nitration of a compound of formula (Ia)

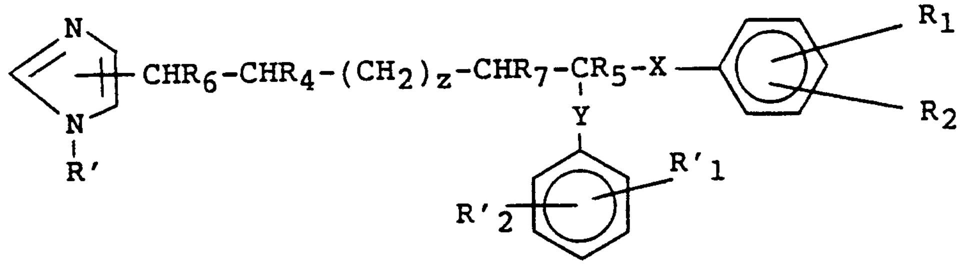

wherein R′, $R_4$, $R_5$, $R_6$, $R_7$, X, Y and z are as defined before and one or more of the substituents $R_1$, $R_2$, $R'_1$ and $R'_2$ are H, to give a compound of formula (Ia) wherein one or more of the substituents $R_1$, $R_2$, $R'_1$ and $R'_2$ are $NO_2$.

**33.** A process for the preparation of a compound as defined in claim 6 or a non-toxic pharmaceutically acceptable acid addition salt thereof which comprises reacting a halogenated hydrocarbon of the formula

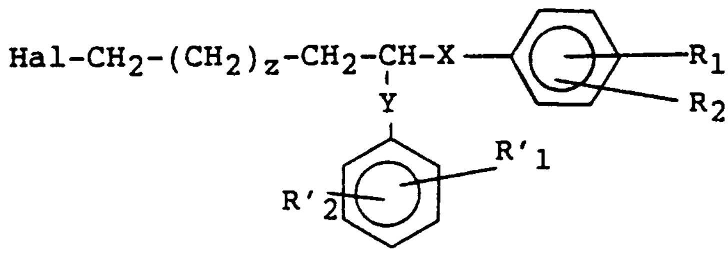

wherein $R_1$, $R_2$, $R'_1$, $R'_2$, X, Y and z are as defined before with triphenylphosphine to give a phosphonium salt of the formula

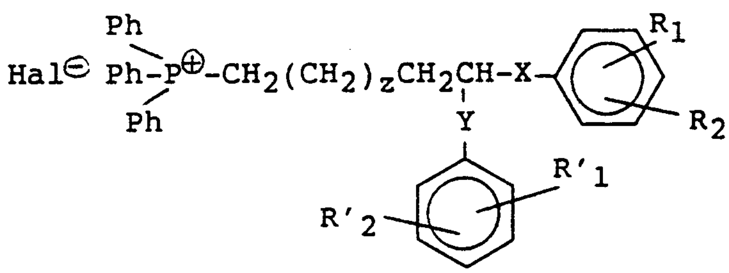

which further is reacted with a strong base and then with an 4(5)-imidazole aldehyde of the formula

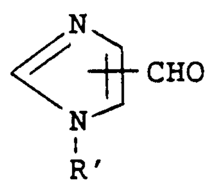

where R' is as defined before to give a compound of formula (Ia) which is

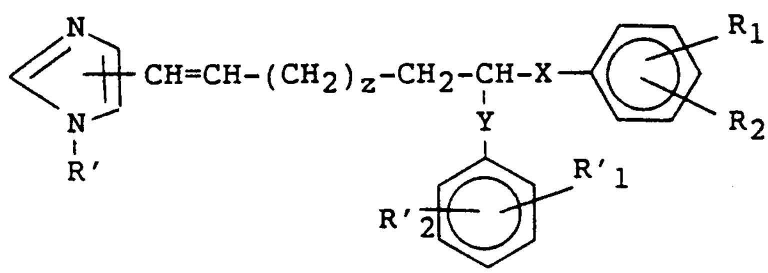

**34.** A process for the preparation of a compound as defined in claim 6 or a non-toxic pharmaceutically acceptable acid addition salt thereof which comprises reacting a halogenated 4(5)-imidazole derivative of the formula

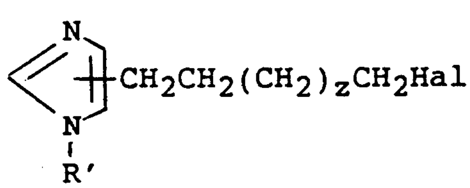

wherein R′ and z are as defined before with triphenylphosphine to give a phosphonium salt of the formula

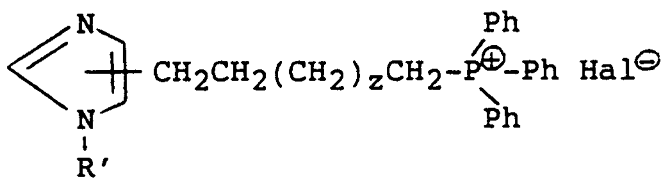

which further is reacted with a strong base and then with a ketone of the formula

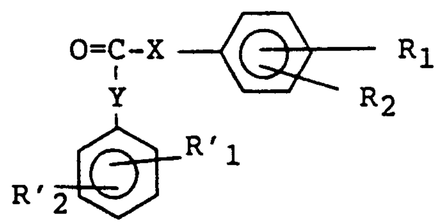

wherein $R_1$, $R_2$, $R'_1$, $R'_2$, X and Y are as defined before to give a compound of formula (Ia) which is

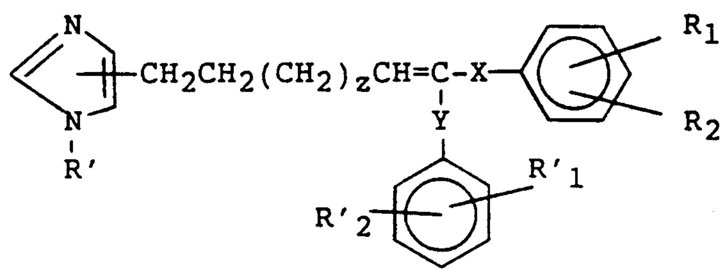

**35.** A process for the preparation of a compound as defined in claim 6 or a non-toxic pharmaceutically acceptable acid addition salt thereof which comprises reacting a halogenated hydrocarbon of the formula

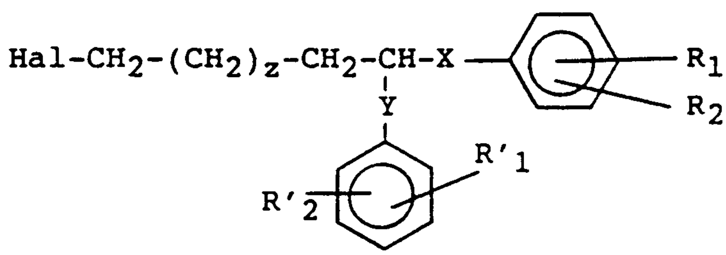

wherein $R_1$, $R_2$, $R'_1$, $R'_2$, X, Y and z are as defined before with a triester of phosphonic acid to give a compound of formula

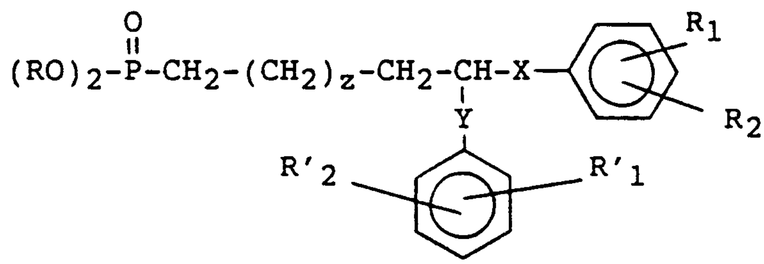

wherein R is alkyl, which further is reacted with a base and then with an 4(5)-imidazole aldehyde of the formula

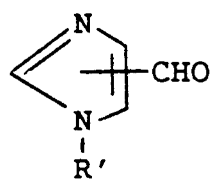

where R′ is as defined before to give a compound of formula (Ia) which is

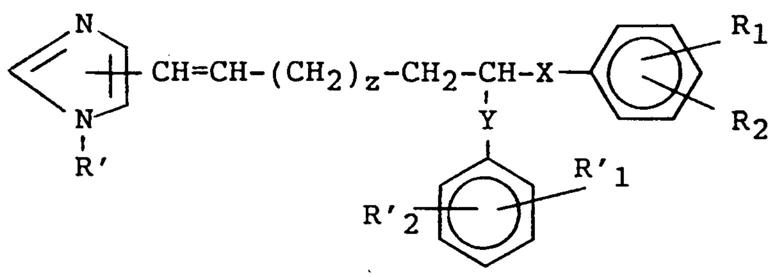

**36.** A process for the preparation of a 4(5)substituted imidazole of the formula

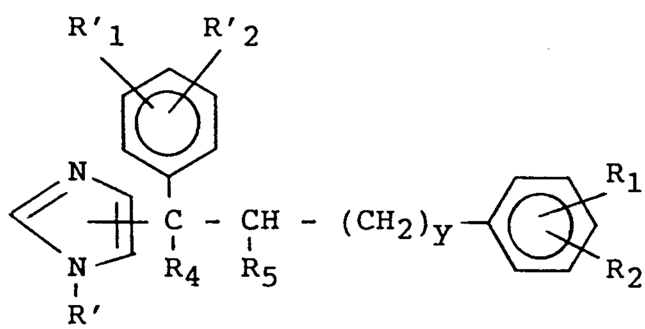

(Ib)

or a non-toxic pharmaceutically acceptable acid addition salt thereof wherein $R_1$, $R_2$, $R'_1$ and $R'_2$, which can be the same or different, are H, $CH_3$, $C_2H_5$, $C_3H_7$, $OCH_3$, $NO_2$, $CF_3$, $CHF_2$, $CH_2F$ or halogen; R′ is H or

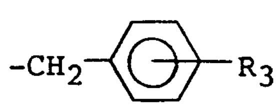

where $R_3$ is H, $CH_3$ or halogen; $R_4$ is H or OH and $R_5$ is H or $R_4$ and $R_5$ together form a bond and y is 0 to 4 which comprises reacting an 4(5)-imidazole aldehyde of formula

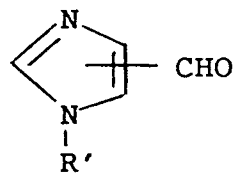

where R′ is as defined before with an appropriate arylalkylmagnesiumhalide of the formula

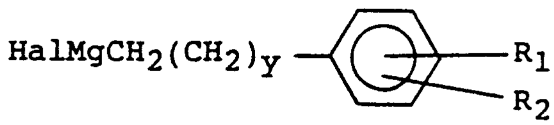

where $R_1$, $R_2$ and y are as defined before to give a compound of formula

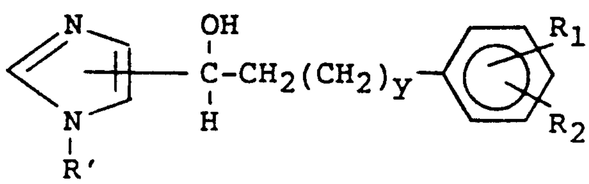

which if R′ is a substituted or unsubstituted benzyl is optionally hydrogenated to remove the benzylic R′, which further is oxidized to give a compound of formula

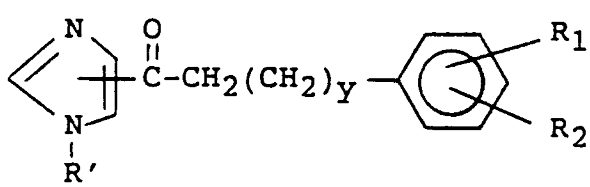

which further is reacted with an arylmagnesiumhalide of the formula

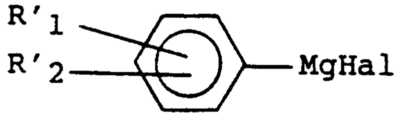

where $R'_1$ and $R'_2$ are as defined before and Hal is halogen to give a compound of formula (Ib) which is

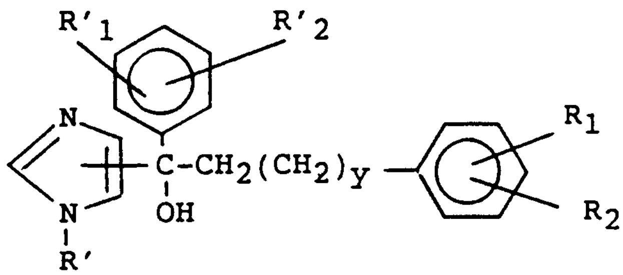

**37.** A process for the preparation of a compound as defined in claim 36 or a non-toxic pharmaceutically acceptable acid addition salt thereof which comprises reacting an 4(5)-imidazole aldehyde of the formula

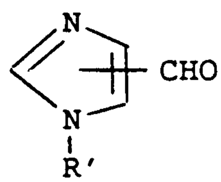

where R' is as defined before with an arylmagnesium halide of the formula

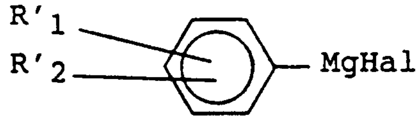

where $R'_1$ and $R'_2$ are as defined before and Hal is halogen to give a compound of the formula

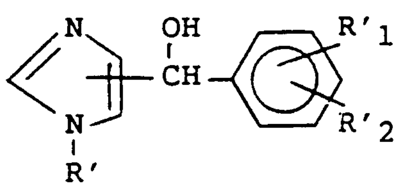

where R', $R'_1$ and $R'_2$ are as defined before, which if R' is a substituted or unsubstituted benzyl is optionally hydrogenated to remove the benzylic R′, which is oxidized to give a compound of the formula

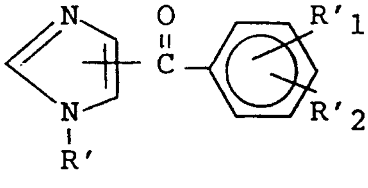

which is further reacted with an appropriate arylalkylmagnesiumhalide of the formula

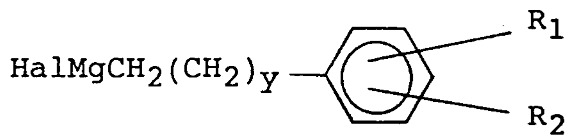

where $R_1$, $R_2$ and y are as defined before to give a compound of formula (Ib) which is

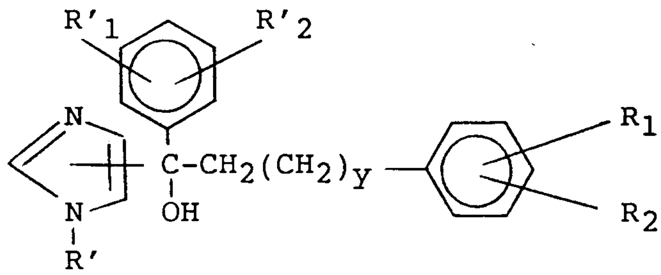

**38.** A process for the preparation of a 4(5)-substituted imidazole of the formula

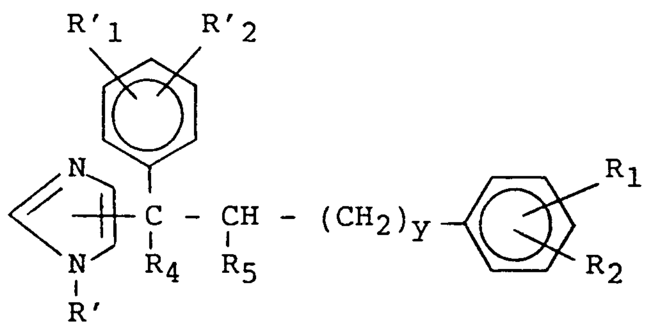

(Ib)

or a non-toxic pharmaceutically acceptable acid addition salt thereof wherein $R_1$, $R_2$, $R'_1$ and $R'_2$, which can be the same or different, are H, $CH_3$, $C_2H_5$, $C_3H_7$, $OCH_3$, OH, $CH_2OH$, $NO_2$, $NH_2$, CN, $CF_3$, $CHF_2$, $CH_2F$ or halogen; R' is H or

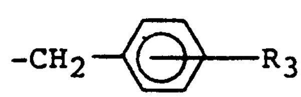

where $R_3$ is H, $CH_3$ or halogen; $R_4$ is H or OH and $R_5$ is H or $R_4$ and $R_5$ together form a bond and y is 0 to 4 which comprises reacting an 4(5)-imidazole aldehyde of the formula

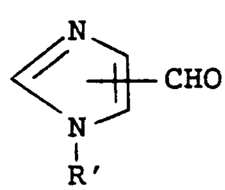

where R' is as defined before with an arylalkylmagnesiumhalide of the formula

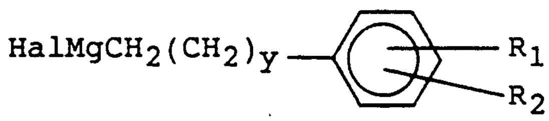

where y is 0 to 4 , Hal is halogen and

i) $R_1$ and/or $R_2$ are $OCH_2Ph$ or OTHP or

ii) neither $R_1$ nor $R_2$ is $OCH_2Ph$ or OTHP

to give a compound of formula

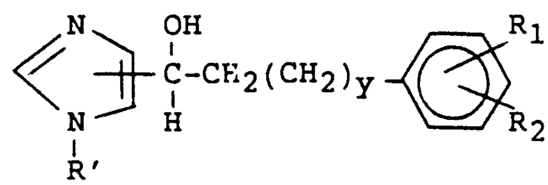

which further is oxidized to give a compound of formula

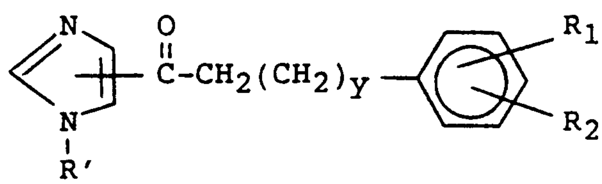

which further is reacted with an arylmagnesiumhalide of the formula

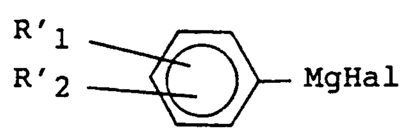

where

iii) $R'_1$ and/or $R'_2$ are $OCH_2Ph$ or OTHP or

iv) neither $R'_1$ nor $R'_2$ is $OCH_2Ph$ or OTHP

provided that that ii) and iv) are not in force at the same time, to give a compound of formula

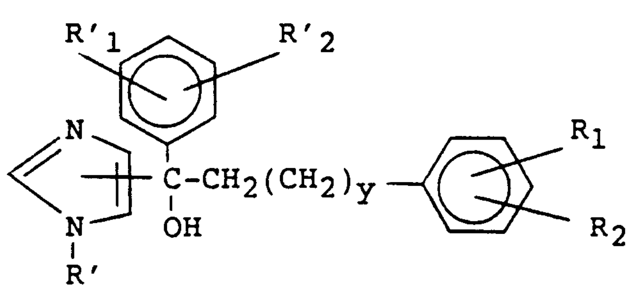

wherein R'and y are as defined before and one or more of the substituents $R_1$, $R_2$, $R'_1$ and $R'_2$ are $OCH_2Ph$ or OTHP, which further is hydrogenated to give a compound of formula (Ib) which is

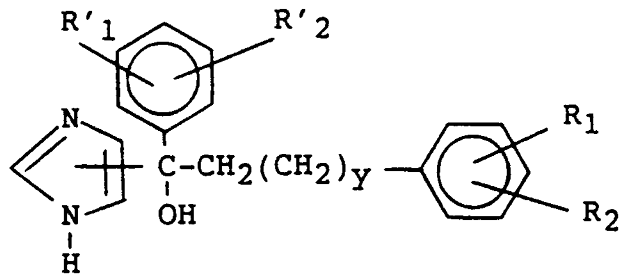

wherein y is 0 to 4 and one or more of the substituents $R_1$, $R_2$, $R'_1$ and $R'_2$ are OH and the substituents that are not OH are H, $CH_3$, $C_2H_5$,$C_3H_7$, $OCH_3$, $NO_2$, $CF_3$, $CHF_2$, $CH_2F$ or halogen.

**39.** A process for the preparation of a compound as defined in claim 38 or a non-toxic pharmaceutically acceptable acid addition salt thereof which comprises reacting an 4(5)-imidazole aldehyde of the formula

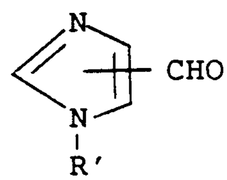

where R′ is as defined before with an arylmagnesiumhalide of the formula

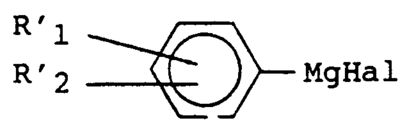

where Hal is halogen and
i) $R'_1$ and/or $R'_2$ are $OCH_2Ph$ or OTHP or
ii) neither $R'_1$ nor $R'_2$ is $OCH_2Ph$ or OTHP
to give a compound of the formula

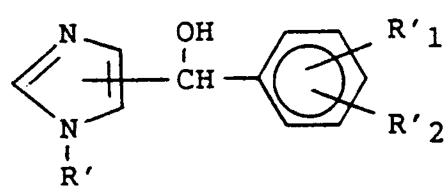

which is oxidized to give a compound of the formula

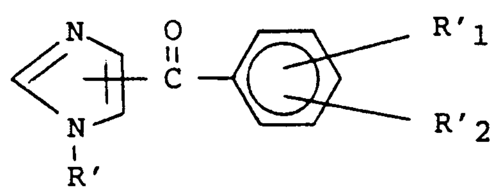

which further is reacted with an arylalkylmagnesiumhalide of the formula

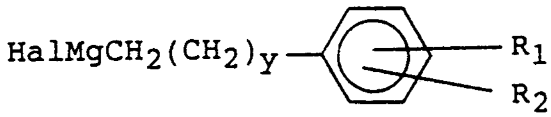

wherein y is as defined before and Hal is halogen and

iii) $R_1$ and/or $R_2$ are $OCH_2Ph$ or OTHP or

iv) neither $R_1$ nor $R_2$ is $OCH_2Ph$ or OTHP

provided that ii) and iv) are not in force at the same time, to give a compound of formula

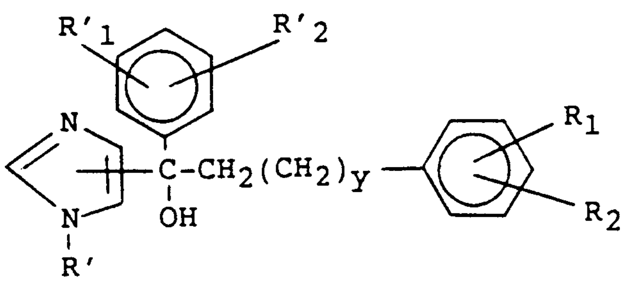

wherein R′ and y are as defined before and one or more of the substituents $R_1$, $R_2$, $R'_1$ and $R'_2$ are $OCH_2Ph$ or OTHP, which further is hydrogenated to give a compound of formula (Ib) which is

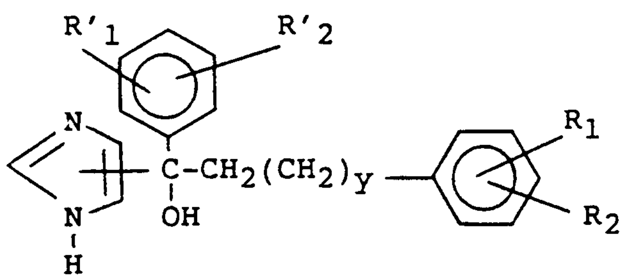

wherein y is 0 to 4 and one or more of the substituents $R_1$, $R_2$, $R'_1$ and $R'_2$ are OH and the substituents that are not OH are H,$CH_3$, $C_2H_5$, $C_3H_7$, $OCH_3$, $NO_2$, $CF_3$, $CHF_2$, $CH_2F$ or halogen.

**40.** A process for the preparation of a compound as defined in claim 38 or a non-toxic pharmaceutically acceptable acid addition salt thereof which comprises dealkylating a compound of formula (Ib)

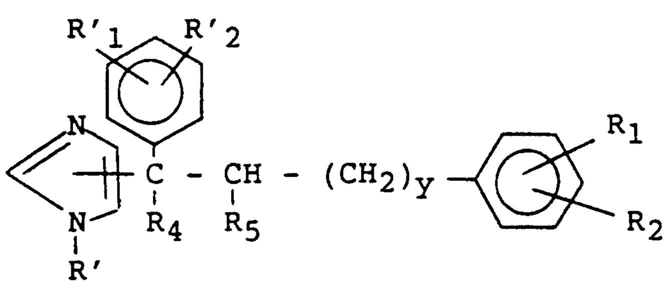

wherein R′, y, $R_4$ and $R_5$ are as defined before and one or more of the substituents $R_1$, $R_2$, $R'_1$ and $R'_2$ are $OCH_3$, to give a compound of formula (Ib) wherein one or more of the substituents $R_1$, $R_2$, $R'_1$ and $R'_2$ are OH.

**41.** A process for the preparation of a compound as defined in claim 38 or a non-toxic pharmaceutically acceptable acid addition salt thereof which comprises hydrolyzing a compound of formula (Ib)

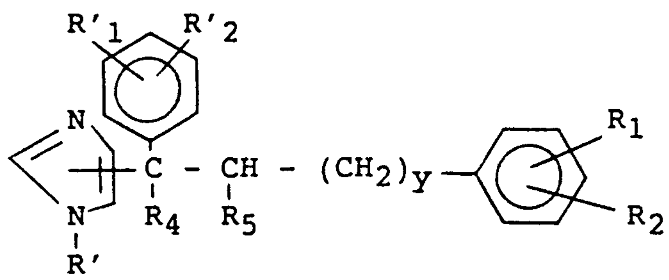

wherein R′, $R_4$, $R_5$ and y are as defined before and one or more of the substituents $R_1$, $R_2$, $R'_1$ and $R'_2$ are CN and further reducing to give a compound of formula (Ib) wherein one or more of the substituents $R_1$, $R_2$, $R'_1$ and $R'_2$ are $CH_2OH$.

**42.** A process for the preparation of a compound as defined in claim 38 or a non-toxic pharmaceutically acceptable acid addition salt thereof which comprises diazotizing a compound of formula (Ib)

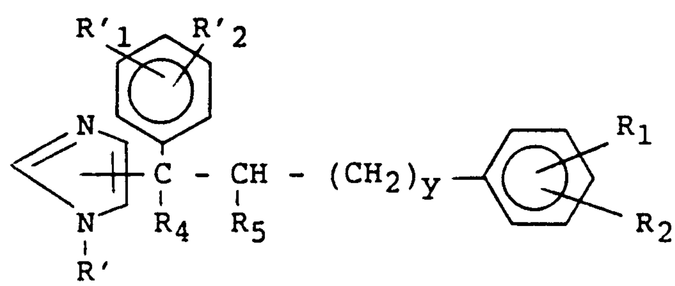

wherein R′, $R_4$, $R_5$ and y are as defined before and one or more of the substituents $R_1$, $R_2$, $R'_1$ and $R'_2$ are $NH_2$ to give a compound of formula (Ib) wherein one or more of the substituents $R_1$, $R_2$, $R'_1$ and $R'_2$ are CN.

**43.** A process for the preparation of a compound as defined in claim 38 or a non-toxic pharmaceutically acceptable acid addition salt thereof which comprises hydrogenating a compound of formula (Ib)

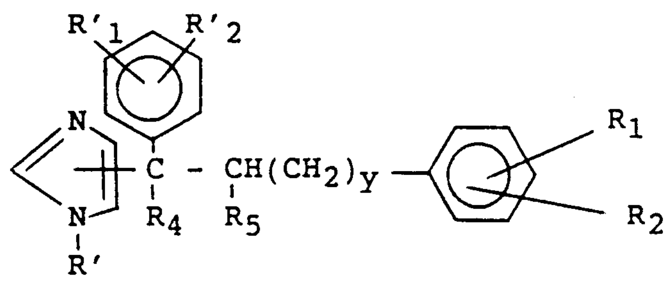

wherein R′, $R_4$, $R_5$ and y are as defined before and one or more of the substituents $R_1$, $R_2$, $R'_1$ and $R'_2$ are $NO_2$ to give a compound of formula (Ib) wherein R′ is H, y is 0 to 4 and one or more of the substituents $R_1$, $R_2$, $R'_1$ and $R'_2$ are $NH_2$.

**44.** A process for the preparation of a compound as defined in claim 38 or a non-toxic pharmaceutically acceptable acid addition salt thereof which comprises dehydrating a compound of formula

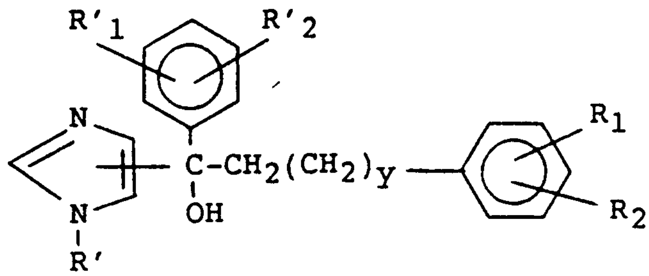

to give a compound of formula (Ib) which is

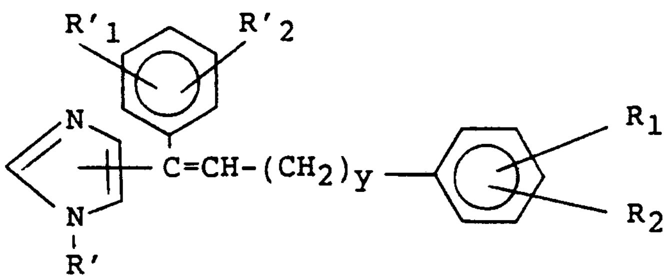

wherein R′, $R_1$, $R_2$, $R'_1$, $R'_2$ and y are as defined before.

**45.** A process for the preparation of a compound as defined in claim 38 or a non-toxic pharmaceutically acceptable acid addition salt thereof which comprises reacting a compound of formula

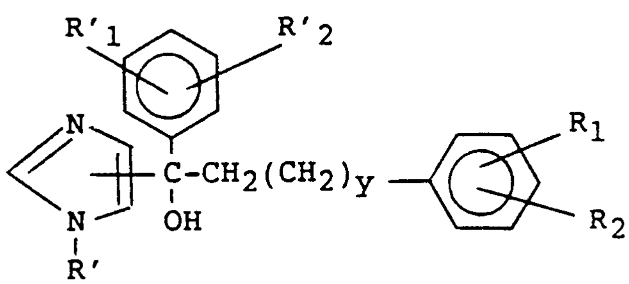

by a hydrogen transfer reaction to give a compound of formula (Ib) which is

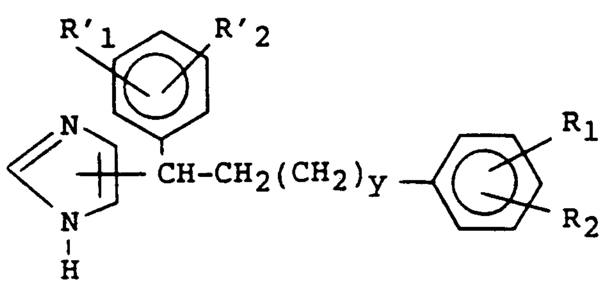

wherein $R_1$, $R_2$, $R'_1$, $R'_2$ and y are as defined before.

**46.** A process for the preparation of a compound as defined in claim 38 or a non-toxic pharmaceutically acceptable acid addition salt thereof which comprises hydrogenating a compound of formula

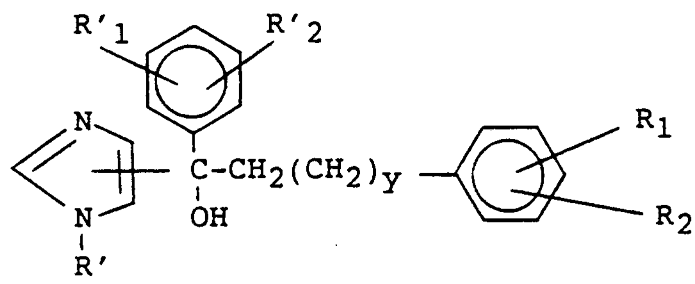

wherein R′ is substituted or unsubstituted benzyl and $R_1$, $R_2$, $R'_1$ and $R'_2$ are as defined before to give a compound of formula (Ib) which is

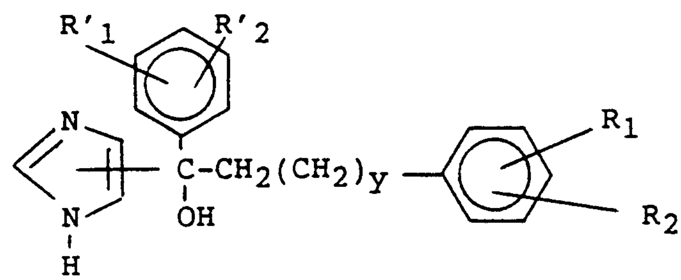

**47.** A process for the preparation of a compound as defined in claim 38 or a non-toxic pharmaceutically acceptable acid addition salt thereof which comprises reacting a compound of formula

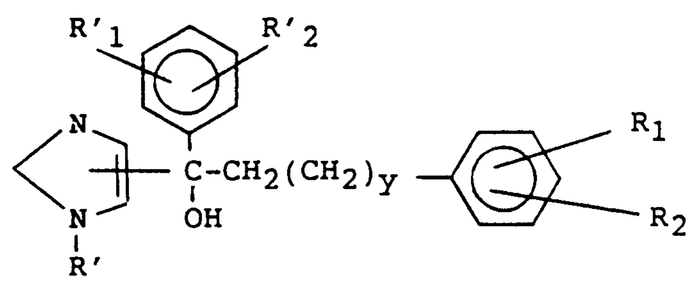

wherein R′ is substituted or unsubstituted benzyl and $R_1$, $R_2$, $R'_1$ and $R'_2$ are as defined before, by a hydrogen transfer reaction to give a compound of formula (Ib) which is

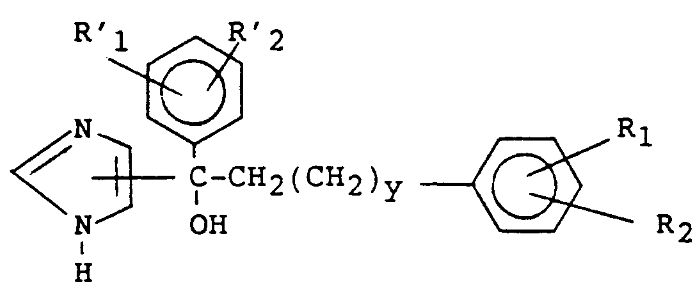

wherein $R_1$, $R_2$, $R'_1$ and $R'_2$ and y are as defined before.

**48.** A process for the preparation of a compound as defined in claim 36 or 38 or a non-toxic pharmaceutically acceptable acid addition salt thereof which comprises reacting a benzoylimidazole of the formula

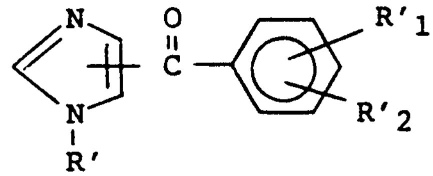

wherein R′, $R'_1$ and $R'_2$ are as defined in claim 36 with an aldehyde of the formula

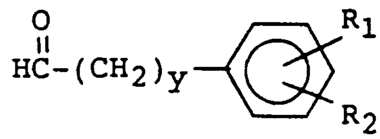

wherein $R_1$, $R_2$ and y are as defined in claim 38 in the presence of a low valent titanium reagent to give a compound of formula (Ib) which is

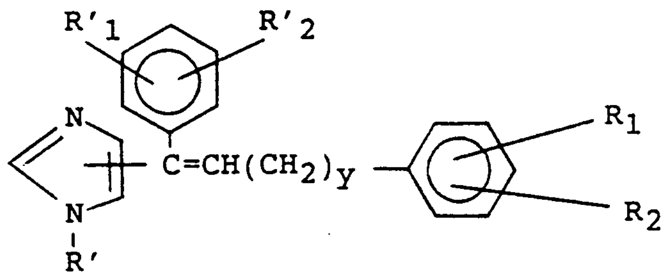

**49.** A process for the preparation of a compound as defined in claim 36 or 38 or a non-toxic pharmaceutically acceptable acid addition salt thereof which comprises reacting a benzoylimidazole of the formula

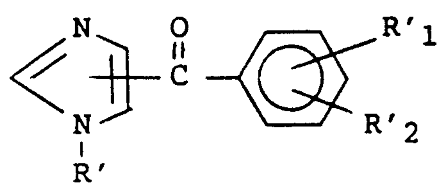

wherein R′ is as defined before and $R'_1$ and/or $R'_2$ are $OCH_2Ph$ or OTHP, with an aldehyde of the formula

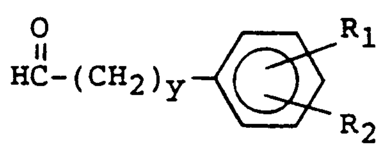

wherein $R_1$, $R_2$ and y are as defined in claim 38 in the presence of a low valent titanium reagent to give a compound of formula

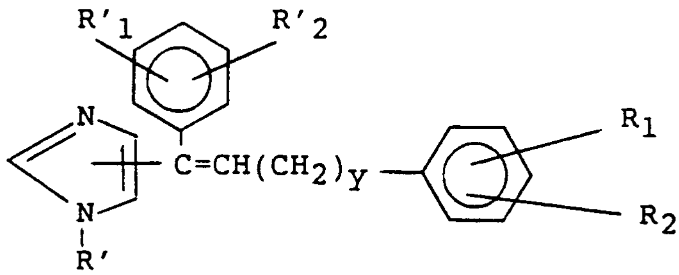

wherein $R'_1$ and/or $R'_2$ are $OCH_2Ph$ or OTHP, which further is hydrogenated to give a compound of formula (Ib) which is

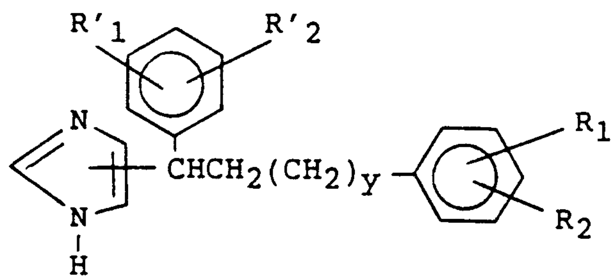

wherein $R'_1$ and/or $R'_2$ are OH, the substituent $R'_1$ or $R'_2$ that is not OH is H, $CH_3$, $C_2H_5$, $C_3H_7$, $OCH_3$, $NO_2$, $CF_3$, $CHF_2$, $CH_2F$ or halogen and $R_1$ and $R_2$ are as defined in claim 38.

**50.** A process for the preparation of a compound as defined in claim 38 or a non-toxic pharmaceutically acceptable acid addition salt thereof which comprises hydrogenating a compound of formula

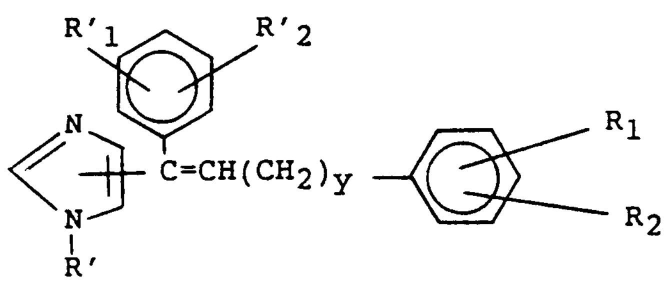

to give a compound of formula (Ib) which is

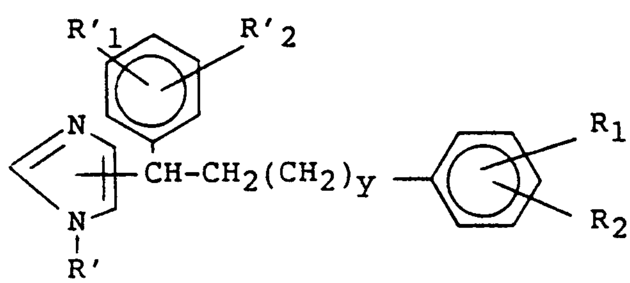

wherein R', $R_1$, $R_2$, $R'_1$, $R'_2$ and y are as defined before.

**51.** A process for the preparation of a compound as defined in claim 38 or a non-toxic pharmaceutically acceptable acid addition salt thereof which comprises hydrogenating a compound of formula

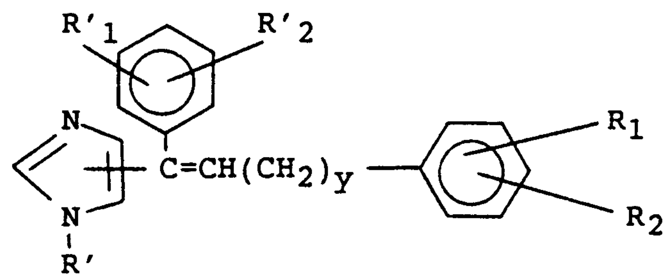

to give a compound of formula (Ib) which is

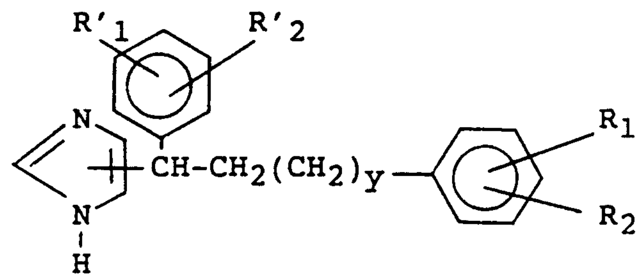

wherein $R_1$, $R_2$, $R'_1$, $R'_2$ and y are as defined before.

**52.** A process for the preparation of a compound as defined in claim 38 or a non-toxic pharmaceutically acceptable acid addition salt thereof which comprises reacting a compound of formula

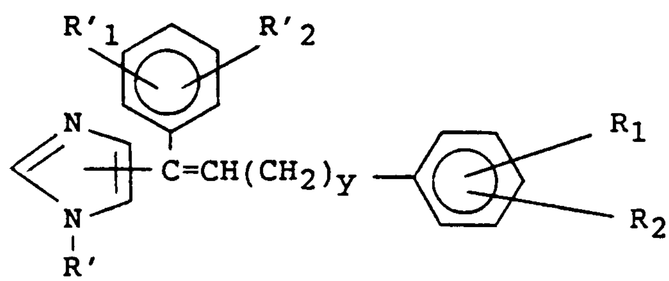

by a hydrogen transfer reaction to give a compound of formula (Ib) which is

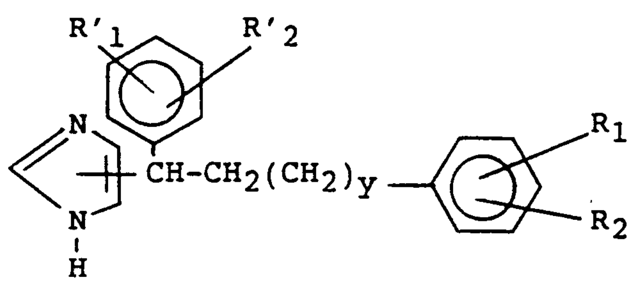

wherein $R_1$, $R_2$, $R'_1$, $R'_2$ and y are as defined before.

53. A process for the preparation of a compound as defined in claim 38 or a non-toxic pharmaceutically acceptable acid addition salt thereof which comprises hydrogenating a compound of formula

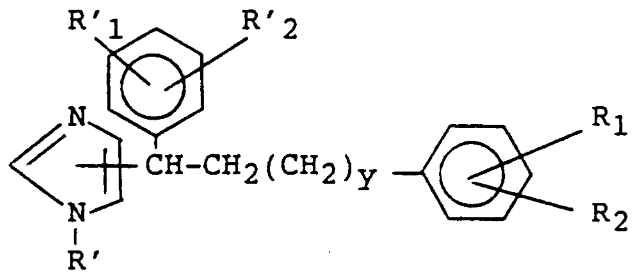

wherein $R_1$, $R_2$, $R'_1$, $R'_2$ and y are as defined before and R′ is a substituted or unsubstituted benzyl to give a compound of formula (Ib) which is

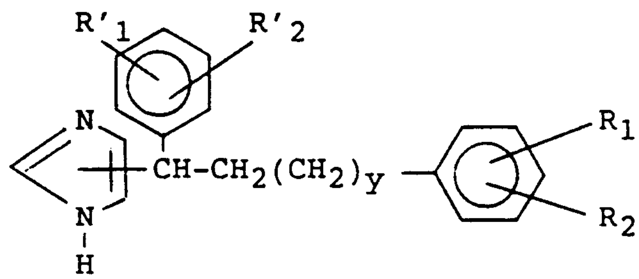

54. A process for the preparation of a compound as defined in claim 38 or a non-toxic pharmaceutically acceptable acid addition salt thereof which comprises reacting a compound of formula

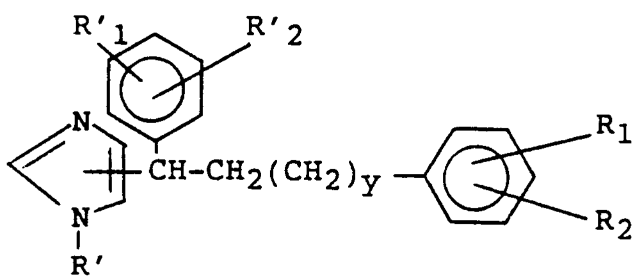

wherein $R_1$, $R_2$, $R'_1$, $R'_2$ and y are as defined before and R′ is substituted or unsubstituted benzyl by a hydrogen transfer reaction to give a compound of formula (Ib) which is

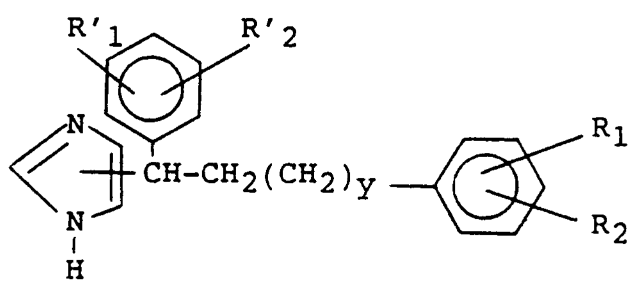

**55.** A process for the preparation of a compound as defined in claim 38 or a non-toxic pharmaceutically acceptable acid addition salt thereof which comprises reacting a compound of formula

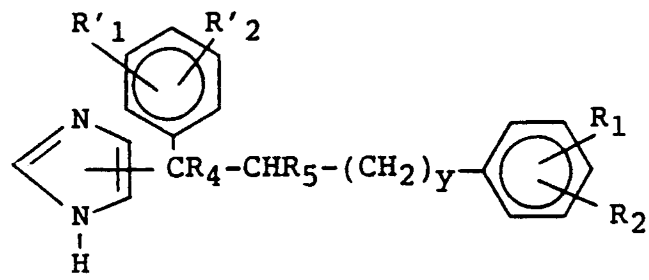

wherein $R_1$, $R_2$, $R'_1$, $R'_2$, $R_4$, $R_5$ and y are as defined before with a strong base and further with a benzyl halide of the formula

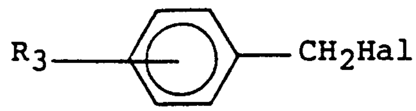

where $R_3$ is as defined before and Hal is halogen to give a compound of formula (Ib) which is

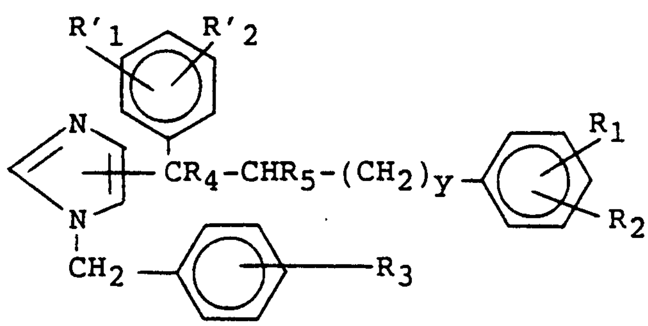

**56.** A process for the preparation of a compound as defined in claim 38 or a non-toxic pharmaceutically acceptable acid addition salt thereof which comprises nitration of a compound of formula (Ib)

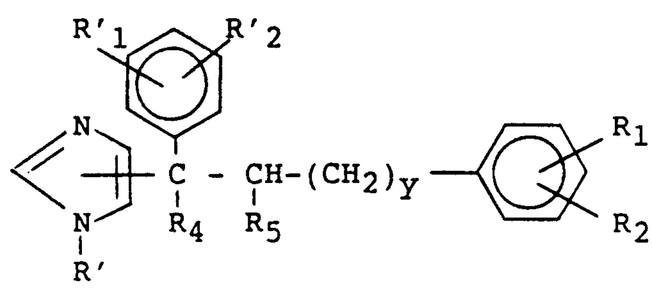

wherein R', $R_4$, $R_5$ and y are as defined before and one or more of the substituents $R_1$, $R_2$, $R'_1$ and $R'_2$ are H to give a compound of formula (Ib) wherein one or more of the substituents $R_1$, $R_2$, $R'_1$ and $R'_2$ are $NO_2$.

**Patentansprüche**
**Patentansprüche für folgende Vertragsstaaten : AT, BE, CH, DE, DK, FR, GB, IT, LI, LU, NL, SE**

1. 4(5)-substituiertes Imidazol der Formel

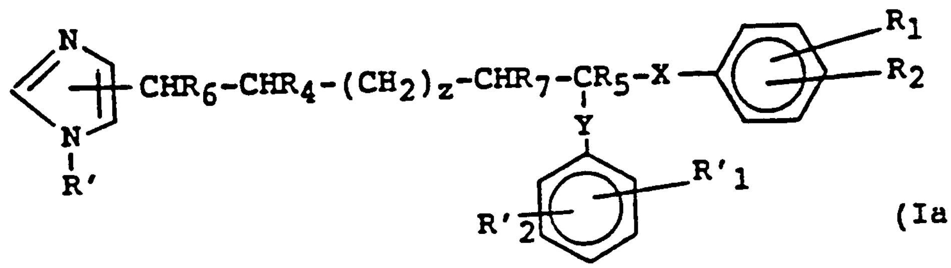

(Ia)

oder ein nicht-toxisches, pharmazeutisch vertragliches Säureadditionssalz davon, worin $R_1$, $R_2$, $R'_1$ und $R'_2$, die gleich oder verschieden sein können, H, $CH_3$, $C_2H_5$, $C_3H_7$, $OCH_3$, OH, $CH_2OH$, $NO_2$, $NH_2$, CN, $CF_3$, $CHF_2$, $CH_2F$ oder Halogen sind; R' H oder

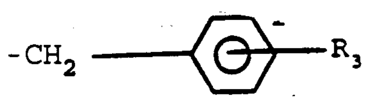

ist, worin $R_3$ H, $CH_3$ oder Halogen ist; $R_4$ H ist, $R_5$ H oder OH ist, $R_6$ H oder OH ist und $R_7$ H ist oder $R_4$ und $R_6$ zusammen eine Bindung bilden oder $R_5$ und $R_7$ zusammen eine Bindung bilden; X und Y, die gleich oder verschieden sein können, eine Bindung, geradkettiges $C_{1-2}$-Alkyl oder das entsprechende Alkenyl sind und z 0 bis 2 ist.

2. Substituiertes Imidazol nach Anspruch 1, worin $R_4$, $R_5$, $R_6$ und $R_7$ H sind.

3. Substituiertes Imidazol nach Anspruch 1 oder 2, worin $R_1$, $R_2$, $R'_1$ und $R'_2$ jeweils H sind.

4. Substituiertes Imidazol nach Anspruch 1 oder 2, worin $R'_1$, $R'_2$ und $R_2$ jeweils H sind und $R_1$, welches wie in Anspruch 1 definiert ist, jedoch nicht H ist, in ortho-, meta- oder para-Stellung der Phenylgruppe steht.

5. Substituiertes Imidazol nach Anspruch 4, worin $R_1$ in para-Stellung der Phenylgruppe steht.

6. Substituiertes Imidazol nach Anspruch 5, worin $R_1$ $OCH_3$ ist.

7. Substituiertes Imidazol nach Anspruch 1 oder 2, worin $R_2$ und $R'_2$ beide H sind und $R_1$ und $R'_1$, welche wie in Anspruch 1 definiert sind, jedoch jeweils nicht H sind, jeweils in para-Stellung der Phenylgruppe stehen.

8. Substituiertes Imidazol nach Anspruch 7, worin $R_1$ und $R'_1$ beide F sind.

9. Substituiertes Imidazol nach Anspruch 1 oder 2, worin $R'_1$ und $R'_2$ beide H sind und $R_1$ und $R_2$, welche wie in Anspruch 1 definiert sind, jedoch jeweils nicht H sind, in den 3- und 5-Stellungen der Phenylgruppe stehen.

10. Substituiertes Imidazol nach Anspruch 1 oder 2, worin $R'_1$ und $R'_2$ H sind und $R_1$ und $R_2$, welche wie in Anspruch 1 definiert sind, jedoch jeweils nicht H sind, in den 3- und 4-Stellungen der Phenylgruppe stehen.

11. Substituiertes Imidazol nach irgendeinem der Ansprüche 1 bis 10, worin $R_4$ und $R_6$ zusammen eine Bindung bilden.

**12.** Substituiertes Imidazol nach irgendeinem der Ansprüche 1 bis 11, worin R' H ist.

**13.** Verbindung nach Anspruch 1, welche 4-[4-(4-Methylphenyl)-4-phenylbutyl]-1H-imidazol oder ein nicht-toxisches pharmazeutisch verträgliches Säureadditionssalz davon ist.

**14.** Verbindung nach Anspruch 1, welche 1-Benzyl-5-[4-(4-methylphenyl)-4-phenylbutyl]-1H-imidazol oder ein nicht-toxisches pharmazeutisch verträgliches Säureadditionssalz davon ist.

**15.** Verbindung nach Anspruch 1, welche 1-Benzyl-5-[4-(3-methylphenyl)-4-phenylbutyl]-1H-imidazol oder ein nicht-toxisches pharmazeutisch verträgliches Säureadditionssalz davon ist.

**16.** Verbindung nach Anspruch 1, welche 4-[4-(3-Methylphenyl)-4-phenylbutyl]-1H-imidazol oder ein nicht-toxisches pharmazeutisch verträgliches Säureadditionssalz davon ist.

**17.** Verbindung nach Anspruch 1, welche 4-[4-(2-Methylphenyl)-4-phenylbutyl]-1H-imidazol oder ein nicht-toxisches pharmazeutisch verträgliches Säureadditionssalz davon ist.

**18.** Verbindung nach Anspruch 1, welche 1-Benzyl-5-[4-(2-methylphenyl)-4-phenylbutyl]-1H-imidazol oder ein nicht-toxisches pharmazeutisch verträgliches Säureadditionssalz davon ist.

**19.** Verbindung nach Anspruch 1, welche 1-Benzyl-5-(4,5-diphenylpentyl)-1H-imidazol oder ein nicht-toxisches pharmazeutisch verträgliches Säureadditionssalz davon ist.

**20.** Verbindung nach Anspruch 1, welche 4-(4,5-Diphenylpentyl)-1H-imidazol oder ein nichttoxisches pharmazeutisch verträgliches Säureadditionssalz davon ist.

**21.** Verbindung nach Anspruch 1, welche 4-(4,4-Diphenylbutyl)-1H-imidazol oder ein nichttoxisches pharmazeutisch verträgliches Säureadditionssalz davon ist.

**22.** Verbindung nach Anspruch 1, welche 1-Benzyl-5-(4,4-diphenylbutyl)-1H-imidazol oder ein nicht-toxisches pharmazeutisch verträgliches Säureadditionssalz davon ist.

**23.** Verbindung nach Anspruch 1, welche 4-[4-(4-Methoxyphenyl)-4-phenylbutyl]-1H-imidazol oder ein nicht-toxisches pharmazeutisch verträgliches Säureadditionssalz davon ist.

**24.** Verbindung nach Anspruch 1, welche 4-[4,4-Bis(4-methoxyphenyl)butyl]-1H-imidazol oder ein nicht-toxisches pharmazeutisch verträgliches Säureadditionssalz davon ist.

**25.** Verbindung nach Anspruch 1, welche 4-[4,4-Bis(3-methylphenyl)butyl]-1H-imidazol oder ein nicht-toxisches pharmazeutisch verträgliches Säureadditionssalz davon ist.

**26.** Verbindung nach Anspruch 1, welche 4-[4-(3,5-Dimethylphenyl)-4-phenylbutyl]-1H-imidazol oder ein nicht-toxisches pharmazeutisch verträgliches Säureadditionssalz davon ist.

**27.** Verbindung nach Anspruch 1, welche 4-[4-(3,4-Dimethylphenyl)-4-phenylbutyl]-1H-imidazol oder ein nicht-toxisches pharmazeutisch verträgliches Säureadditionssalz davon ist.

**28.** Verbindung nach Anspruch 1, welche 4-[4-(3,5-Dimethylphenyl)-4-(3-methylphenyl)butyl]-1H-imidazol oder ein nicht-toxisches pharmazeutisch verträgliches Säureadditionssalz davon ist.

**29.** Verbindung nach Anspruch 1, welche 4-[4,4-Bis(4-methylphenyl)butyl]-1H-imidazol oder ein nicht-toxisches pharmazeutisch verträgliches Säureadditionssalz davon ist.

**30.** Verbindung nach Anspruch 1, welche 4-[5,5-Diphenylpentyl)-1H-imidazol oder ein nichttoxisches pharmazeutisch verträgliches Säureadditionssalz davon ist.

**31.** Verbindung nach Anspruch 1, welche 4-[6,6-Diphenylhexyl)-1H-imidazol oder ein nichttoxisches pharmazeutisch verträgliches Säureadditionssalz davon ist.

**32.** Verbindung nach Anspruch 1, welche 4-[4-(2-Fluorphenyl)-4-phenylbutyl]-1H-imidazol oder ein nicht-toxisches pharmazeutisch vertragliches Säureadditionssalz davon ist.

**33.** Verbindung nach Anspruch 1, welche 4-[4-(4-Fluorphenyl)-4-phenylbutyl]-1H-imidazol oder ein nicht-toxisches pharmazeutisch verträgliches Säureadditionssalz davon ist.

**34.** Verbindung nach Anspruch 1, welche 4-(4,4-Diphenyl-1-butenyl)-1H-imidazol oder ein nichttoxisches pharmazeutisch verträgliches Säureadditionssalz davon ist.

**35.** Verbindung nach Anspruch 1, welche 4-[4,4-Bis(4-fluorphenyl)butyl]-1H-imidazol oder ein nicht-toxisches pharmazeutisch verträgliches Säureadditionssalz davon ist.

**36.** Verbindung nach Anspruch 1, welche 4-[4,4-Bis(4-nitrophenyl)butyl]-1H-imidazol oder ein nicht-toxisches pharmazeutisch verträgliches Säureadditionssalz davon ist.

**37.** Verbindung nach Anspruch 1, welche 4-[4,4-Bis(4-aminophenyl)butyl]-1H-imidazol oder ein nicht-toxisches pharmazeutisch verträgliches Säureadditionssalz davon ist.

**38.** Verbindung nach Anspruch 1, welche 4-[4-(4-Ethylphenyl)-4-phenylbutyl]-1H-imidazol oder ein nicht-toxisches pharmazeutisch verträgliches Säureadditionssalz davon ist.

**39.** Pharmazeutische Zusammensetzung, umfassend ein substituiertes Imidazol, wie in irgendeinem der Ansprüche 1 bis 38 beansprucht, und einen pharmazeutisch verträglichen Träger.

**40.** Imidazolderivat, wie in irgendeinem der Ansprüche 1 bis 39 beansprucht, oder ein nicht-toxisches Säureadditionssalz zur therapeutischen Verwendung als Aromatase-inhibierendes Mittel.

**41.** 4(5)-substituiertes Imidazol der Formel

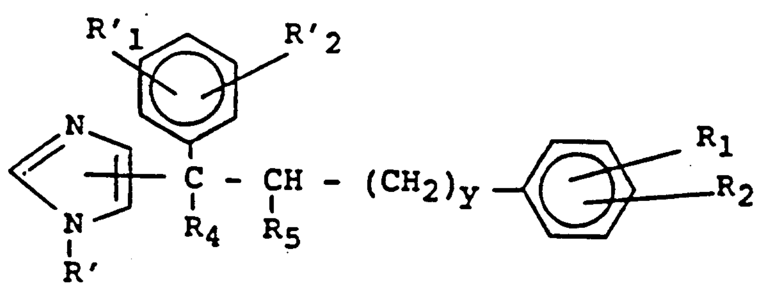

(Ib)

oder ein nicht-toxisches, pharmazeutisch verträgliches Säureadditionssalz davon, worin $R_1$, $R_2$, $R'_1$ und $R'_2$, die gleich oder verschieden sein können, H, $CH_3$, $C_2H_5$, $C_3H_7$, $OCH_3$, OH, $CH_2OH$, $NO_2$, $NH_2$, CN, $CF_3$, $CHF_2$, $CH_2F$ oder Halogen sind; R' H oder

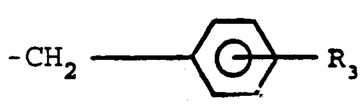

ist, worin $R_3$ H, $CH_3$ oder Halogen ist; $R_4$ H oder OH ist und $R_5$ H ist oder $R_4$ und $R_5$ zusammen eine Bindung bilden und y 0 bis 4 ist.

**42.** Substituiertes Imidazol nach Anspruch 41, worin $R_4$, $R_5$ und R' jeweils H sind.

**43.** Substituiertes Imidazol nach Anspruch 41 oder 42, worin $R_1$, $R_2$ und $R'_2$ jeweils H sind und $R'_1$, welches wie in Anspruch 41 definiert ist, in para-Stellung der Phenylgruppe steht.

**44.** Substituiertes Imidazol nach Anspruch 43, worin $R'_1$ F ist.

**45.** Substituiertes Imidazol nach Anspruch 41 oder 42, worin $R_2$ und $R'_2$ jeweils H sind und $R_1$, welches wie in Anspruch 41 definiert ist, in ortho-, meta- oder para-Stellung der Phenylgruppe steht und $R'_1$, welches wie in Anspruch 41 definiert ist, in para-Stellung der Phenylgruppe steht.

**46.** Substituiertes Imidazol nach Anspruch 45, worin $R_2$ und $R'_2$ beide H sind und $R_1$ und $R'_1$, welche wie in Anspruch 41 definiert sind, beide in para-Stellung der Phenylgruppe stehen.

**47.** Substituiertes Imidazol nach Anspruch 46, worin $R_1$ und $R'_1$ beide F sind.

**48.** Substituiertes Imidazol nach Anspruch 46, worin $R_1$ $CH_3$ ist und $R'_1$ F ist.

**49.** Substituiertes Imidazol nach Anspruch 41 oder 42, worin $R'_2$ H ist $R'_1$, welches wie in Anspruch 41 definiert ist, in para-Stellung der Phenylgruppe steht und $R_1$ und $R_2$, welche wie in Anspruch 41 definiert sind, in den 3- und 5-Stellungen der Phenylgruppe stehen.

**50.** Substituiertes Imidazol nach Anspruch 41 oder 42, worin $R'_2$ H ist, $R'_1$, welches wie in Anspruch 41 definiert ist, in para-Stellung der Phenylgruppe steht und $R_1$ und $R_2$, welche wie in Anspruch 41 definiert sind, in den 2- und 6-Stellungen der Phenylgruppe stehen.

**51.** Verbindung nach Anspruch 41, welche 4-[1-(4-Fluorphenyl)-5-phenylpentyl]-1H-imidazol oder ein nicht-toxisches, pharmazeutisch verträgliches Säureadditionssalz davon ist.

**52.** Verbindung nach Anspruch 41, welche 4-[1-(4-Fluorphenyl)-5-(2-methylphenyl)pentyl]-1H-imidazol oder ein nicht-toxisches, pharmazeutisch verträgliches Säureadditionssalz davon ist.

**53.** Verbindung nach Anspruch 41, welche 4-[1-(4-Fluorphenyl)-5-(3-methylphenyl)pentyl]-1H-imidazol oder ein nicht-toxisches, pharmazeutisch verträgliches Säureadditionssalz davon ist.

**54.** Verbindung nach Anspruch 41, welche 4-[1-(4-Fluorphenyl)-5-(4-methylphenyl)pentyl]-1H-imidazol oder ein nicht-toxisches, pharmazeutisch verträgliches Säureadditionssalz davon ist.

**55.** Verbindung nach Anspruch 41, welche 4-[5-(3,5-Dimethylphenyl)-1-(4-fluorphenyl)pentyl]-1H-imidazol oder ein nicht-toxisches, pharmazeutisch verträgliches Säureadditionssalz davon ist.

**56.** Verbindung nach Anspruch 41, welche 4-(1,5-Diphenylpentyl)-1H-imidazol oder ein nichttoxisches, pharmazeutisch verträgliches Säureadditionssalz davon ist.

**57.** Verbindung nach Anspruch 41, welche 4-(1,3-Diphenylpropyl)-1H-imidazol oder ein nichttoxisches, pharmazeutisch verträgliches Säureadditionssalz davon ist.

**58.** Verbindung nach Anspruch 41, welche 1-Benzyl-5-(1,3-diphenylpropyl)-1H-imidazol oder ein nicht-toxisches, pharmazeutisch verträgliches Säureadditionssalz davon ist.

**59.** Verbindung nach Anspruch 41, welche 4-[1-(4-Fluorphenyl)-3-phenylpropyl]-1H-imidazol oder ein nicht-toxisches, pharmazeutisch verträgliches Säureadditionssalz davon ist.

**60.** Verbindung nach Anspruch 41, welche 4-[1-(4-Fluorphenyl)-4-phenylbutyl]-1H-imidazol oder ein nicht-toxisches, pharmazeutisch verträgliches Säureadditionssalz davon ist.

**61.** Verbindung nach Anspruch 41, welche 4-[1-(4-Fluorphenyl)-5-(3-methoxyphenyl)pentyl]-1H-imidazol oder ein nicht-toxisches, pharmazeutisch verträgliches Säureadditionssalz davon ist.

**62.** Verbindung nach Anspruch 41, welche 4-[1-(4-Fluorphenyl)-5-(4-methoxyphenyl)pentyl]-1H-imidazol oder ein nicht-toxisches, pharmazeutisch verträgliches Säureadditionssalz davon ist.

**63.** Verbindung nach Anspruch 41, welche 4-[1-(4-Fluorphenyl)-5-(2,6-dimethylphenyl)pentyl]-1H-imidazol oder ein nicht-toxisches, pharmazeutisch verträgliches Säureadditionssalz davon ist.

**64.** Verbindung nach Anspruch 41, welche 4-[1,3-Bis(4-fluorphenyl)propyl]-1H-imidazol oder ein nicht-toxisches, pharmazeutisch verträgliches Säureadditionssalz davon ist.

**65.** Verbindung nach Anspruch 41, welche 4-[1,4-Bis(4-fluorphenyl)butyl]-1H-imidazol oder ein nichttoxisches, pharmazeutisch verträgliches Säureadditionssalz davon ist.

**66.** Pharmazeutische Zusammensetzung, umfassend ein substituiertes Imidazol , wie in irgendeinem der Ansprüche 41 bis 65 beansprucht, und einen pharmazeutisch verträglichen Träger.

**67.** Imidazolderivat, wie in irgendeinem der Ansprüche 41 bis 66 beansprucht, oder ein nicht-toxisches Säureadditionssalz davon zur therapeutischen Verwendung als Aromatase-inhibierendes Mittel.

**Patentansprüche für folgende Vertragsstaaten : ES, GR**

**1.** Verfahren zur Herstellung eines 4(5)-substituierten Imidazols der Formel

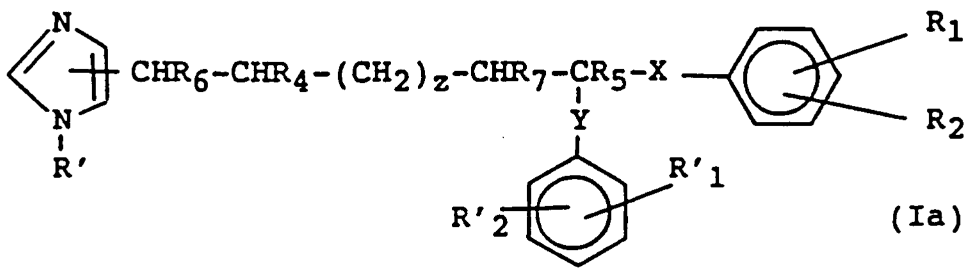

(Ia)

oder eines nicht-toxischen, pharmazeutisch verträgliche Säureadditionssalzes davon, worin $R_1$, $R_2$, $R'_1$ und $R'_2$, die gleich oder verschieden sein können, H, $CH_3$, $C_2H_5$, $C_3H_7$, $OCH_3$, $NO_2$, $CF_3$, $CHF_2$, $CH_2F$ oder Halogen sind; R' H oder

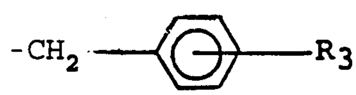

ist, worin $R_3$ H, $CH_3$ oder Halogen ist; $R_4$ H ist, $R_5$ H oder OH ist, $R_6$ H oder OH ist und $R_7$ H ist oder $R_4$ und $R_6$ zusammen eine Bindung bilden oder $R_5$ und $R_7$ zusammen eine Bindung bilden; X und Y, die gleich oder verschieden sein können, eine Bindung, geradkettiges $C_{1-2}$-Alkyl oder das entsprechende Alkenyl sind und z 0 bis 2 ist,
welches umfaßt die Umsetzung eines 4(5)-Imidazol-derivats der Formel

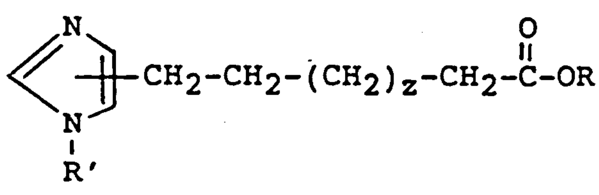

worin R' und z wie oben definiert sind und R Alkyl ist, mit einen Grignard-Reagens der Formel

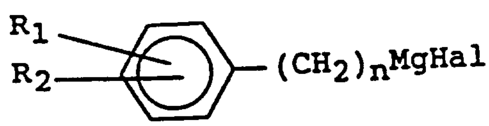

worin $R_1$ und $R_2$ wie oben definiert sind und n 0 bis 2 ist, um eine Verbindung der Formel (Ia) zu ergeben, welche

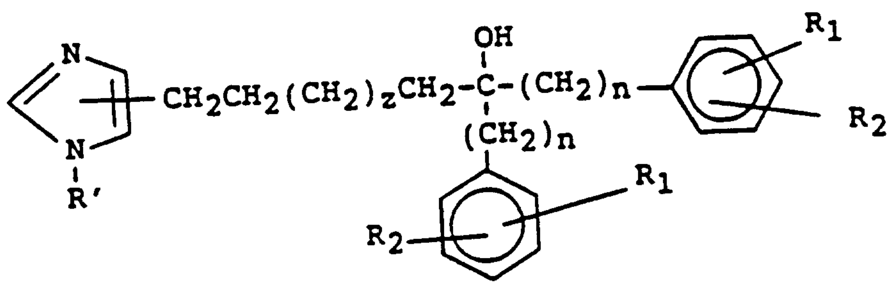

ist.

**2.** Verfahren zur Herstellung einer Verbindung nach Anspruch 1 oder eines nicht-toxischen, pharmazeutische verträglichen Säureadditionssalzes davon, welches umfaßt die Umsetzung eines 4(5)-Imidazolderivats der Formel

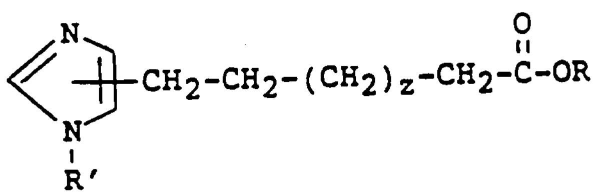

worin R' und z wie oben definiert sind und R Alkyl ist, mit einem Grignard-Reagens

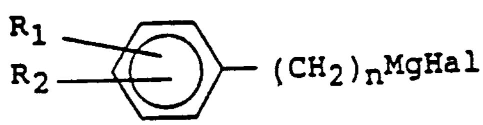

worin $R_1$ und $R_2$ wie oben definiert sind und n 0 bis 2 ist, um eine Verbindung der Formel

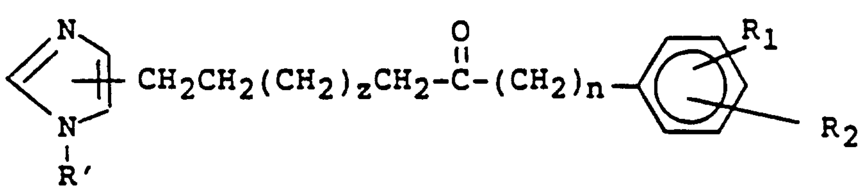

zu ergeben, welche weiter mit einem anderen Grignard-Reagens der Formel

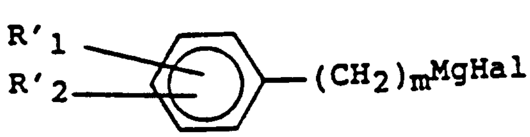

umgesetzt wird, worin $R'_1$ und $R'_2$ wie oben definiert sind und m 0 bis 2 ist, um eine Verbindung der Formel (Ia) zu ergeben, welche

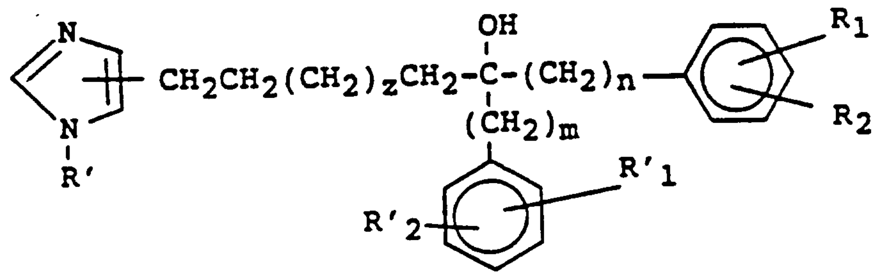

ist.

3. Verfahren nach Anspruch 1 bis 2 zur Herstellung einer Verbindung nach Anspruch 1 oder eines nicht-toxischen, pharmazeutisch verträglichen Säureadditionssalzes davon, worin ein Amid der Formel

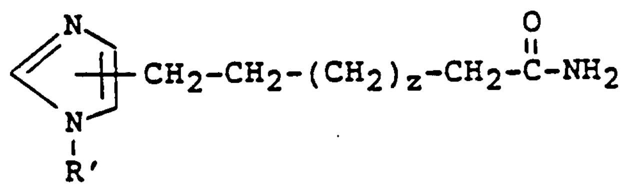

worin R' und z wie oben definiert sind, als Ausgangsmaterial anstelle des entsprechenden Esters verwendet wird.

4. Verfahren nach Anspruch 1 bis 2 zur Herstellung einer Verbindung nach Anspruch 1 oder eines nicht-toxischen, pharmazeutisch verträglichen Säureadditionssalzes davon, worin ein Nitril der Formel

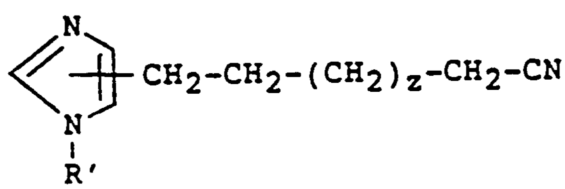

worin R' und z wie oben definiert sind, als Ausgangsmaterial anstelle des entsprechenden Esters verwendet wird.

5. Verfahren zur Herstellung einer Verbindung nach Anspruch 1 oder eines nicht-toxischen, pharmazeutisch verträglichen Säureadditionssalzes davon, welches umfaßt die Umsetzung eines 4(5)-Imidazolaldehyds der Formel

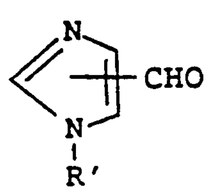

worin R' wie oben definiert ist, mit einem Grignard-Reagens

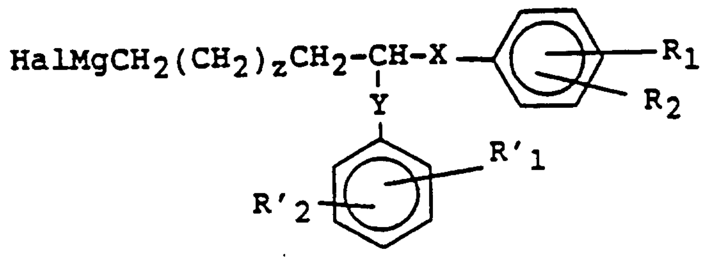

worin $R_1$, $R_2$, $R'_1$, $R'_2$. X, Y und z wie oben definiert sind, um eine Verbindung der Formel (Ia) zu ergeben, welche

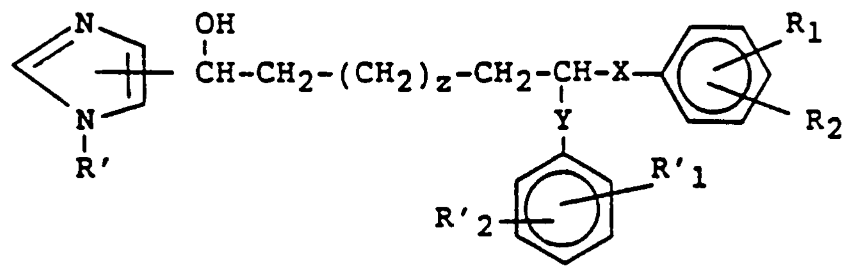

ist.

**6.** Verfahren zur Herstellung eines 4(5)-substituierten Imidazols der Formel

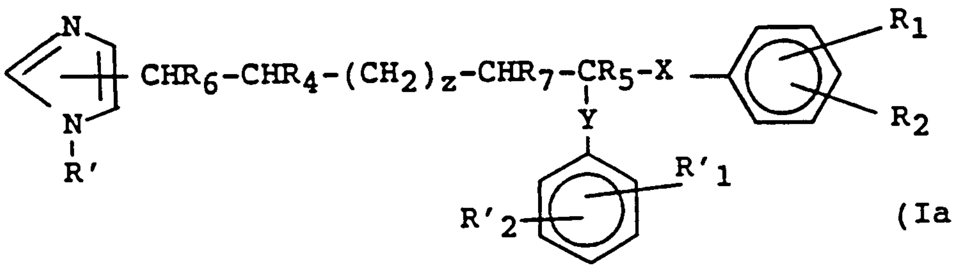

(Ia)

oder eines nicht-toxischen, pharmazeutisch verträglichen Säureadditionssalz davon, worin $R_1$, $R_2$, $R'_1$ und $R'_2$, die gleich oder verschieden sein können, H, $CH_3$, $C_2H_5$, $C_3H_7$, $OCH_3$, OH, $CH_2OH$, $NO_2$, $NH_2$, CN, $CF_3$, $CHF_2$, $CH_2F$ oder Halogen sind; R' H oder

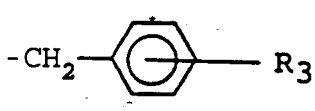

ist, worin $R_3$ H, $CH_3$ oder Halogen ist; $R_4$ H ist, $R_5$ H oder OH ist, $R_6$ H oder OH ist und $R_7$ H ist oder $R_4$ und $R_6$ zusammen eine Bindung bilden oder $R_5$ und $R_7$ zusammen eine Bindung bilden; X und Y, die gleich oder verschieden sein können, eine Bindung, geradkettiges $C_{1-2}$-Alkyl oder das entsprechende Alkenyl sind und z 0 bis 2 ist, welches umfaßt die Umsetzung eines 4(5)-Imidazolderivats der Formel

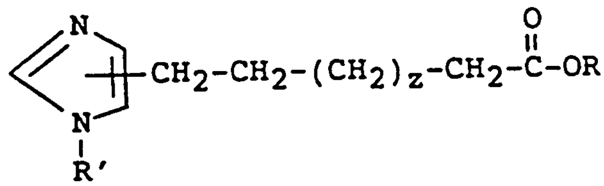

worin R' und z wie oben definiert sind und R Alkyl ist, mit einem Grignard-Reagens

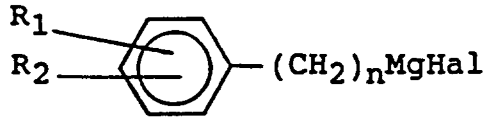

worin n 0 bis 2 ist und $R_1$ und/oder $R_2$ $OCH_2Ph$ oder OTHP sind, um eine Verbindung der Formel

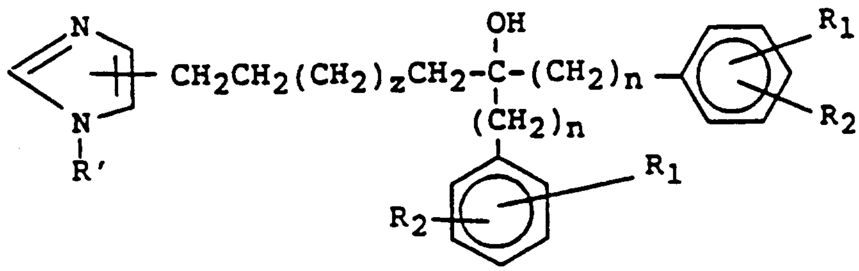

zu ergeben, worin $R_1$ und/oder $R_2$ $OCH_2Ph$ oder OTHP sind, welche weiter hydriert wird, um eine Verbindung der Formel (Ia) zu ergeben, welche

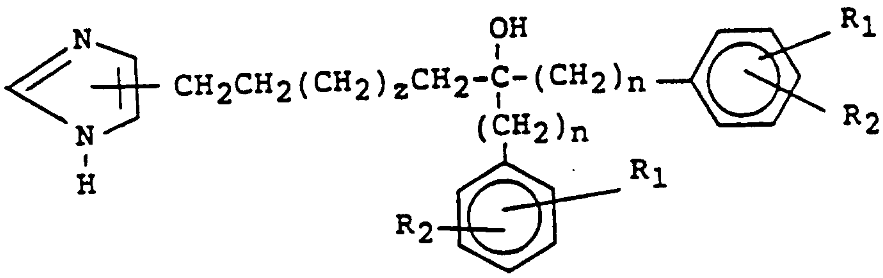

ist, worin z und n wie oben definiert sind und $R_1$ und/oder $R_2$ OH sind und $R_1$ oder $R_2$, welches nicht OH ist, H, $CH_3$, $C_2H_5$, $C_3H_7$, $OCH_3$, $NO_2$, $CF_3$, $CHF_2$, $CH_2F$ oder Halogen ist.

7. Verfahren zur Herstellung einer Verbindung nach Anspruch 6 oder eines nicht-toxischen, pharmazeutisch verträglichen Säureadditionssalzes davon, welches umfaßt die Umsetzung eines 4(5)-Imidazolderivats der Formel

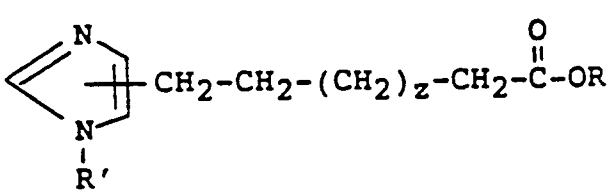

worin R' und z wie oben definiert sind und R Alkyl ist, mit einem Grignard-Reagens

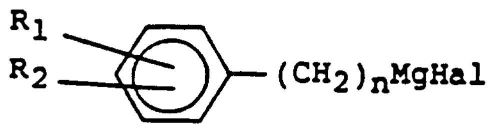

worin n 0 bis 2 ist und

(i) $R_1$ und/oder $R_2$ $OCH_2Ph$ oder OTHP sind oder

(ii) weder $R_1$ noch $R_2$ $OCH_2Ph$ oder OTHP sind,

um eine Verbindung der Formel

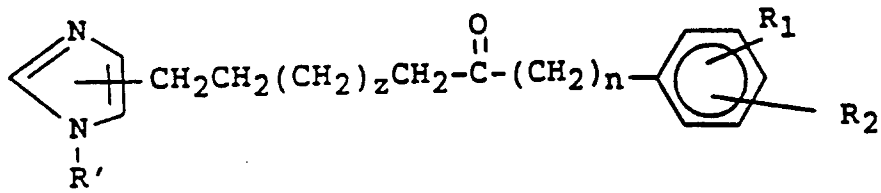

zu ergeben, welche weiter mit einem anderen Grignard-Reagens der Formel

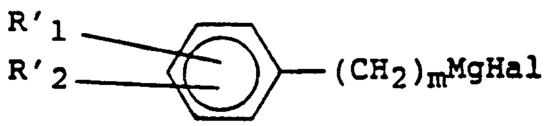

umgesetzt wird, worin m 0 bis 2 ist und

(iii)$R'_1$ und/oder $R'_2$ $OCH_2Ph$ oder OTHP sind oder

(iv) weder $R'_1$ noch $R'_2$ $OCH_2Ph$ oder OTHP sind,

mit der Maßgabe, daß (ii) und (iv) nicht gleichzeitig gelten, um eine Verbindung der Formel

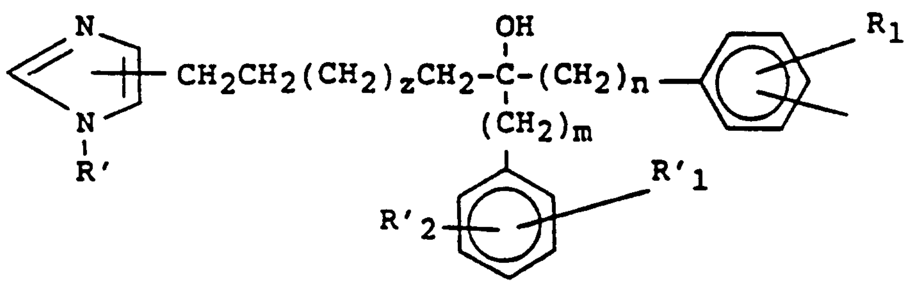

zu ergeben, worin einer oder mehrere der Substituenten $R_1$, $R_2$, $R'_1$ und $R'_2$ $OCH_2Ph$ oder OTHP sind, welche weiter hydriert wird, um eine Verbindung der Formel (Ia) zu ergeben, welche

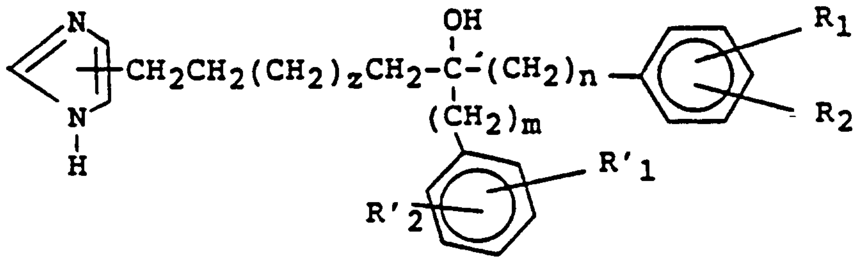

ist, worin z 0 bis 2 ist, m und n, die gleich oder verschieden sein können, 0 bis 2 sind und einer oder mehrere der Substituenten $R_1$, $R_2$, $R'_1$ und $R'_2$ OH sind und die Substituenten, welche nicht OH sind, H, $CH_3$, $C_2H_5$, $C_3H_7$, $OCH_3$, $NO_2$, $CF_3$, $CHF_2$, $CH_2F$ oder Halogen sind.

**8.** Verfahren nach Anspruch 6 bis 7 zur Herstellung einer Verbindung der Formel (Ia) nach Anspruch 6 oder eines nicht-toxischen, pharmazeutisch verträglichen Säureadditionssalzes davon, worin ein Amid der Formel

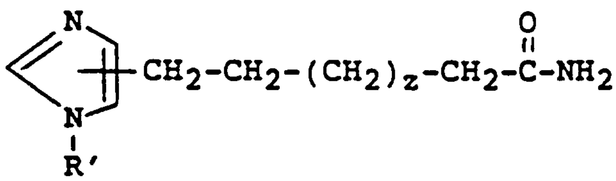

worin R' und z wie oben definiert sind, als Ausgangsmaterial anstelle des entsprechenden Esters verwendet wird.

**9.** Verfahren nach Anspruch 6 bis 7 zur Herstellung einer Verbindung der Formel (Ia) nach Anspruch 6 oder eines nicht-toxischen, pharmazeutisch verträglichen Säureadditionssalzes davon, worin ein Nitril der Formel

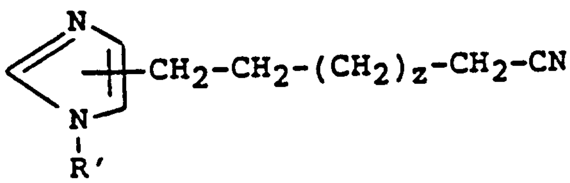

worin R' und z wie oben definiert sind, als Ausgangsmaterial anstelle des entsprechenden Esters verwendet wird.

**10.** Verfahren zur Herstellung einer Verbindung nach Anspruch 6 oder eines nicht-toxischen, pharmazeutisch verträglichen Säureadditionssalzes davon, welches umfaßt die Umsetzung eines 4(5)-Imidazolaldehyds der Formel

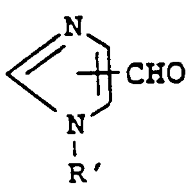

worin R' wie oben definiert ist, mit einem Grignard-Reagens

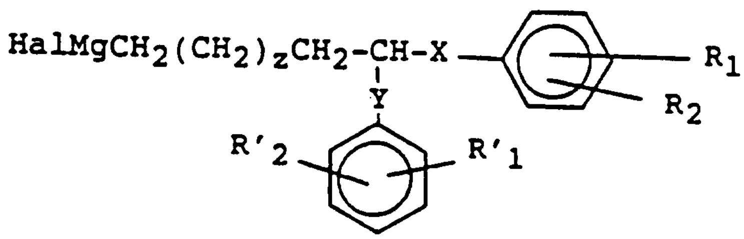

worin X, Y und z wie oben definiert sind und einer oder mehrere der Substituenten $R_1$, $R_2$, $R'_1$ und $R'_2OCH_2Ph$ oder OTHP sind, um eine Verbindung der Formel

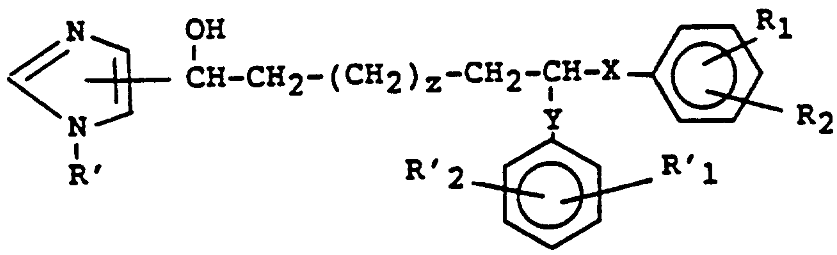

zu ergeben, worin einer oder mehrere der Substituenten $R_1$, $R_2$, $R'_1$ und $R'_2$ $OCH_2Ph$ oder OTHP sind, welche weiter hydriert wird, um eine Verbindung der Formel (Ia) zu ergeben, welche

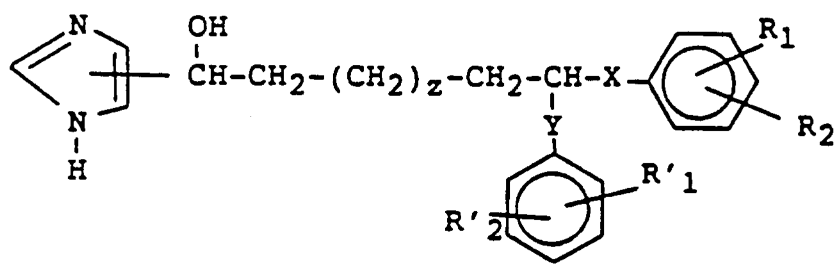

ist, worin X, Y und z wie oben definiert sind und einer oder mehrere der Substituenten $R_1$, $R_2$, $R'_1$ und $R'_2$ OH sind und die Substituenten, welche nicht OH sind, H, $CH_3$, $C_2H_5$, $C_3H_7$, $OCH_3$, $NO_2$, $CF_3$, $CHF_2$, $CH_2F$ oder Halogen sind.

**11.** Verfahren zur Herstellung einer Verbindung nach Anspruch 6 oder eines nicht-toxischen, pharmazeutisch verträglichen Säureadditionssalzes davon, welches umfaßt die Dealkylierung einer Verbindung der Formel (Ia)

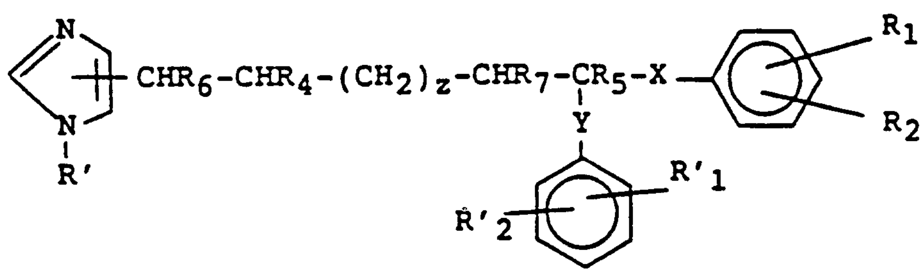

worin R', $R_4$, $R_5$, $R_6$, $R_7$, X, Y und z wie oben definiert sind und einer oder mehrere der Substituenten $R_1$, $R_2$, $R'_1$ und $R'_2$ $OCH_3$ sind, um eine Verbindung der Formel (Ia) zu ergeben, worin einer oder mehrere der Substituenten $R_1$, $R_2$, $R'_1$ und $R'_2$ OH sind.

12. Verfahren zur Herstellung einer Verbindung nach Anspruch 6 oder eines nicht-toxischen, pharmazeutisch verträglichen Säureadditionssalzes davon, welches umfaßt die Hydrolyse einer Verbindung der Formel (Ia)

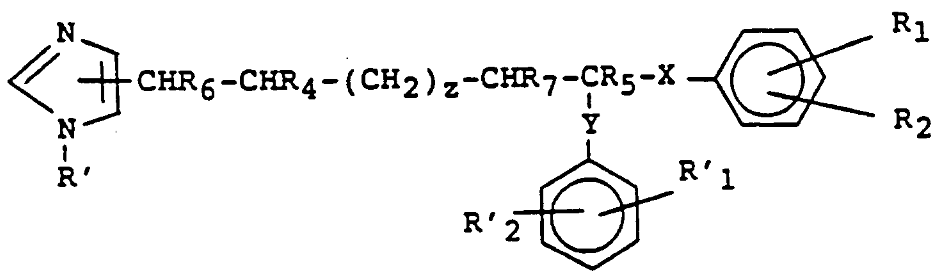

worin R', $R_4$, $R_5$, $R_6$, $R_7$, X, Y und z wie oben definiert sind und einer oder mehrere der Substituenten $R_1$, $R_2$, $R'_1$ und $R'_2$ CN sind, und weitere Reduktion, um eine Verbindung der Formel (Ia) zu ergeben, worin einer oder mehrere der Substituenten $R_1$, $R_2$, $R'_1$ und $R'_2$ $CH_2OH$ sind.

13. Verfahren zur Herstellung einer Verbindung nach Anspruch 6 oder eines nicht-toxischen, pharmazeutisch verträglichen Säureadditionssalzes davon, welches umfaßt die Diazotierung einer Verbindung der Formel (Ia)

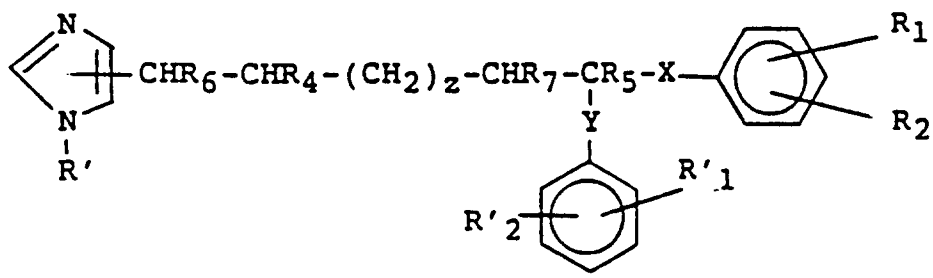

worin R', $R_4$, $R_5$, $R_6$, $R_7$, X, Y und z wie oben definiert sind und einer oder mehrere der Substituenten $R_1$, $R_2$, $R'_1$ und $R'_2$ $NH_2$ sind, um eine Verbindung der Formel (Ia) zu ergeben, worin einer oder mehrere der Substituenten $R_1$, $R_2$, $R'_1$ und $R'_2$ CN sind.

14. Verfahren zur Herstellung einer Verbindung nach Anspruch 6 oder eines nicht-toxischen, pharmazeutisch verträglichen Säureadditionssalzes davon, welches umfaßt die Hydrierung einer Verbindung der Formel (Ia)

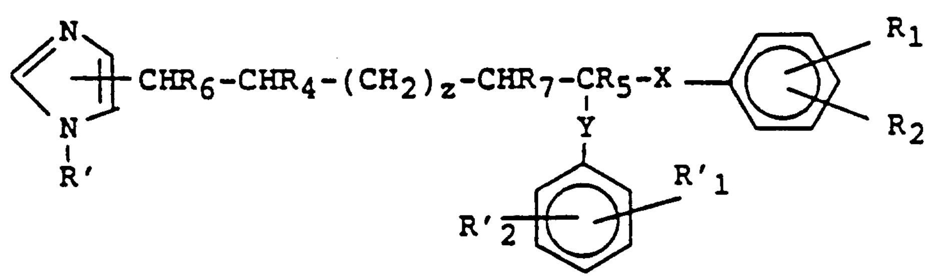

worin R', $R_4$, $R_5$, $R_6$, $R_7$, X, Y und z wie oben definiert sind und einer oder mehrere der Substituenten $R_1$, $R_2$, $R'_1$ und $R'_2$ $NO_2$ sind, um eine Verbindung der Formel (Ia) zu ergeben, worin R' H, $R_4$, $R_5$, $R_6$, $R_7$, X, Y und z wie oben definiert sind und einer oder mehrere der Substituenten $R_1$, $R_2$, $R'_1$ und $R'_2$ $NH_2$ sind.

**15.** Verfahren zur Herstellung einer Verbindung nach Anspruch 6 oder eines nicht-toxischen, pharmazeutisch verträglichen Säureadditionssalzes davon, welches umfaßt die Dehydratisierung einer Verbindung der Formel

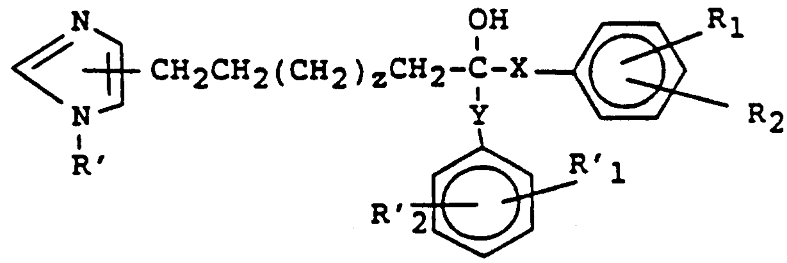

um eine Verbindung der Formel (Ia) zu ergeben, welche

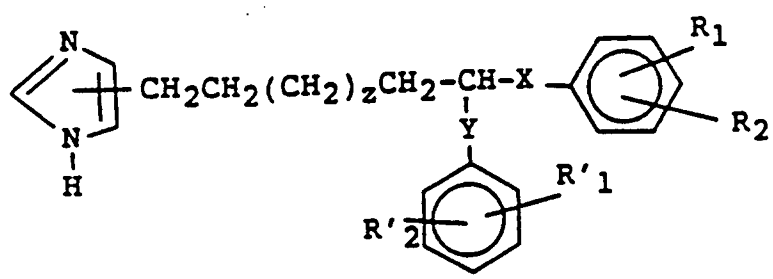

ist, worin R', $R_1$, $R_2$, $R'_1$, $R'_2$ und z wie oben definiert sind.

**16.** Verfahren zur Herstellung einer Verbindung nach Anspruch 6 oder eines nicht-toxischen, pharmazeutisch verträglichen Säureadditionssalzes davon, welches umfaßt die Umsetzung einer Verbindung der Formel

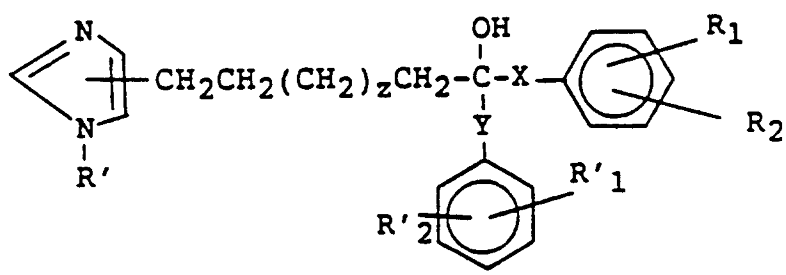

durch eine Wasserstofftransfer-Reaktion, um eine Verbindung der Formel (Ia) zu ergeben, welche

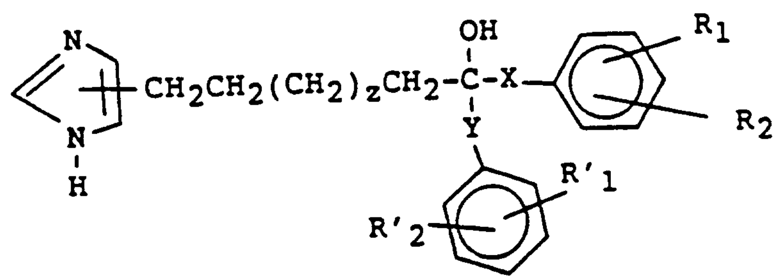

ist, worin $R_1$, $R_2$, $R'_1$, $R'_2$, X, Y und z wie oben definiert sind.

17. Verfahren zur Herstellung einer Verbindung nach Anspruch 6 oder eines nicht-toxischen, pharmazeutisch verträglichen Säureadditionssalzes davon, welches umfaßt die Hydrierung einer Verbindung der Formel

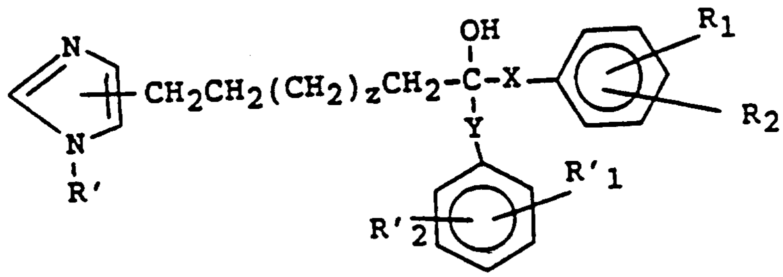

worin R' substituiertes oder unsubstituiertes Benzyl ist und $R_1$, $R_2$, $R'_1$, $R'_2$, X, Y und z wie oben definiert sind, um eine Verbindung der Formel (Ia) zu ergeben, welche

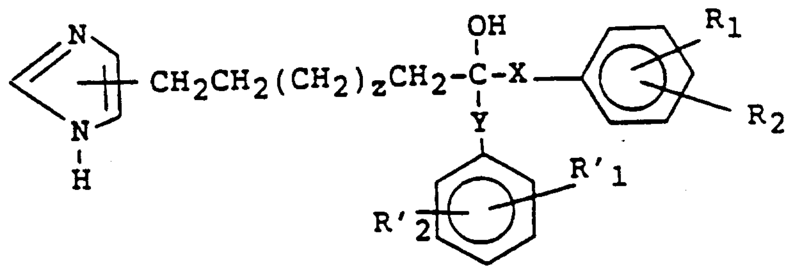

ist.

18. Verfahren zur Herstellung einer Verbindung nach Anspruch 6 oder eines nicht-toxischen, pharmazeutisch verträglichen Säureadditionssalzes davon, welches umfaßt die Umsetzung einer Verbindung der Formel

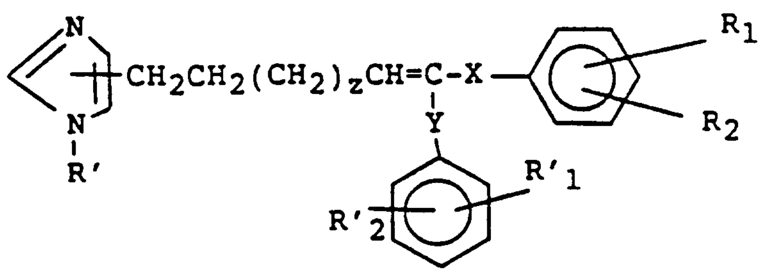

worin R'substituiertes oder unsubstituiertes Benzyl ist und $R_1$, $R_2$, $R'_1$, $R'_2$, X, Y und z wie oben definiert sind, durch eine Wasserstofftransfer-Reaktion, um eine Verbindung der Formel (Ia) zu ergeben, welche

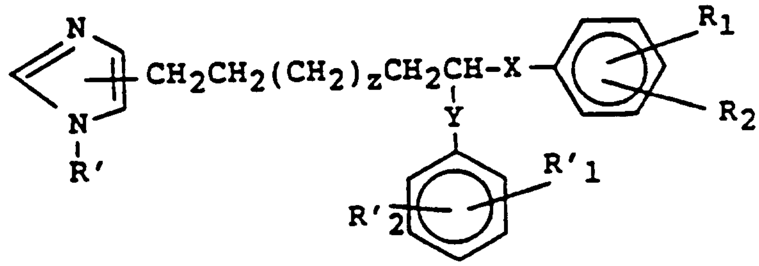

ist.

**19.** Verfahren zur Herstellung einer Verbindung nach Anspruch 6 oder eines nicht-toxischen, pharmazeutisch verträglichen Säureadditionssalzes davon, welches umfaßt die Hydrierung einer Verbindung der Formel

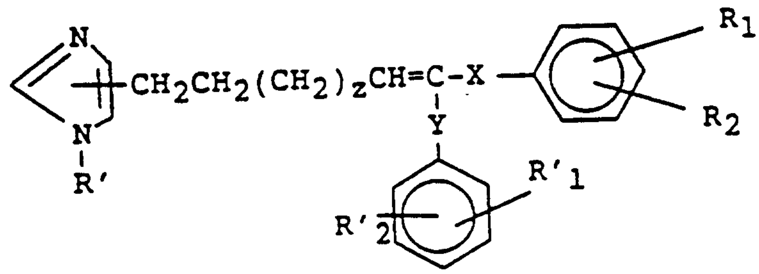

um eine Verbindung der Formel (Ia) zu ergeben, welche

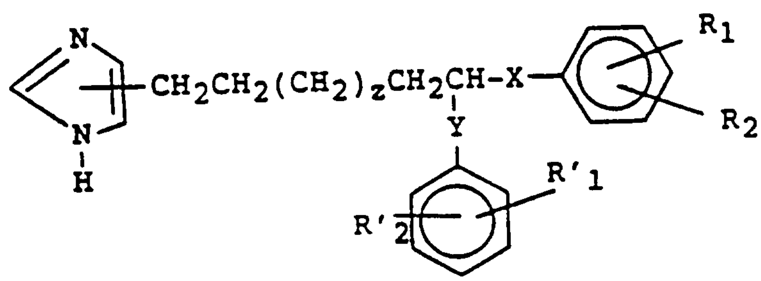

ist, worin R', $R_1$, $R_2$, $R'_1$, $R'_2$ und z wie oben definiert sind.

**20.** Verfahren zur Herstellung einer Verbindung nach Anspruch 6 oder eines nicht-toxischen, pharmazeutisch verträglichen Säureadditionssalzes davon, welches umfaßt die Hydrierung einer Verbindung der Formel

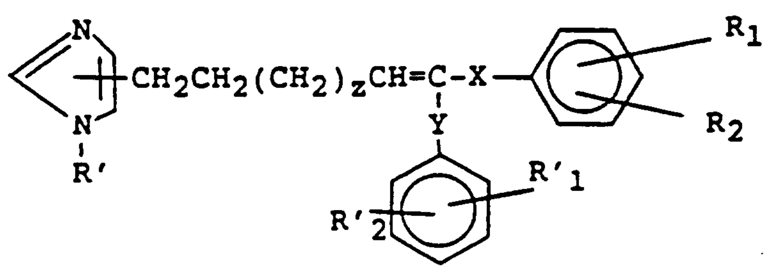

um eine Verbindung der Formel (Ia) zu ergeben, welche

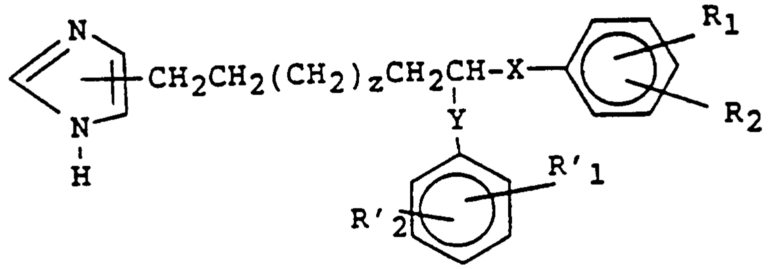

ist, worin $R_1$, $R_2$, $R'_1$, $R'_2$, X, Y und z wie oben definiert sind.

**21.** Verfahren zur Herstellung einer Verbindung nach Anspruch 6 oder eines nicht-toxischen, pharmazeutisch verträglichen Säureadditionssalzes davon, welches umfaßt die Umsetzung einer Verbindung der Formel

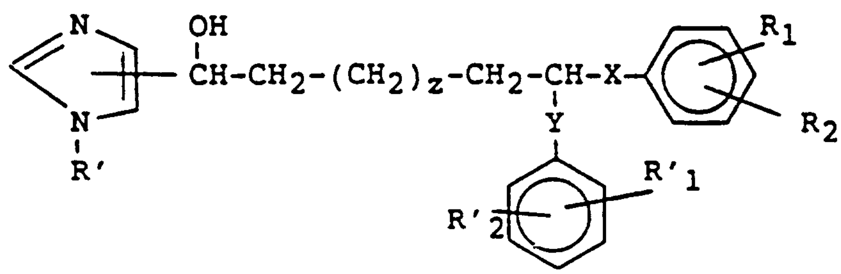

durch eine Wasserstofftransfer-Reaktion, um eine Verbindung der Formel (Ia) zu ergeben, welche

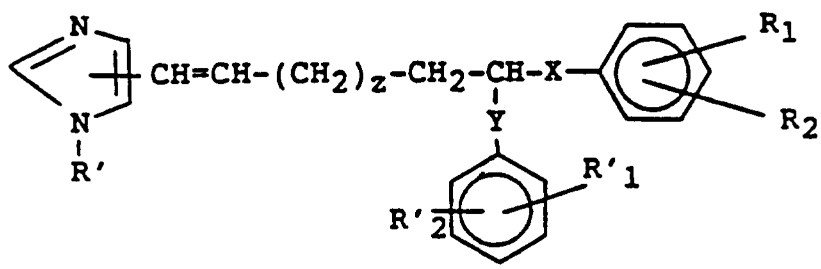

ist, worin $R_1$, $R_2$, $R'_1$, $R'_2$, X, Y und z wie oben definiert sind.

**22.** Verfahren zur Herstellung einer Verbindung nach Anspruch 6 oder eines nicht-toxischen, pharmazeutisch verträglichen Säureadditionssalzes davon, welches umfaßt die Dehydratation einer Verbindung der Formel

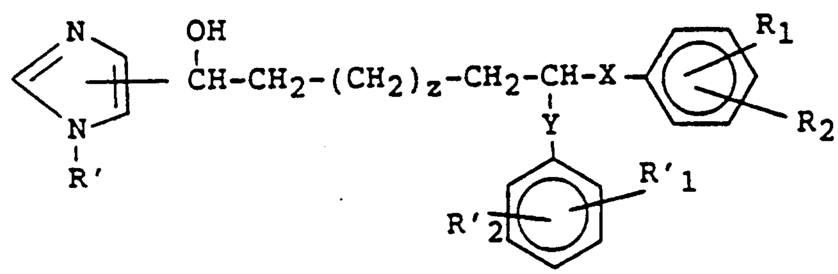

um eine Verbindung der Formel (Ia) zu ergeben, welche

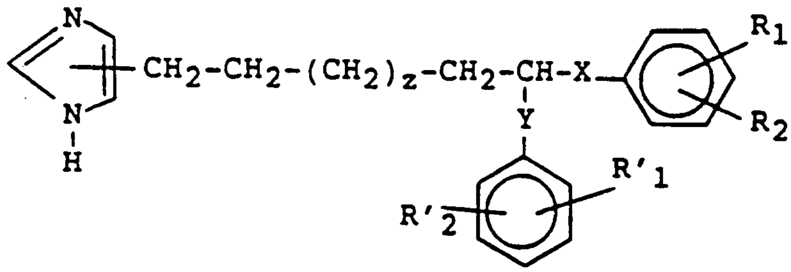

ist, worin R', $R_1$, $R_2$, $R'_1$, $R'_2$ X, Y und z wie oben definiert sind.

**23.** Verfahren zur Herstellung einer Verbindung nach Anspruch 6 oder eines nicht-toxischen, pharmazeutisch verträglichen Säureadditionssalzes davon, welches umfaßt die Umsetzung einer Verbindung der Formel

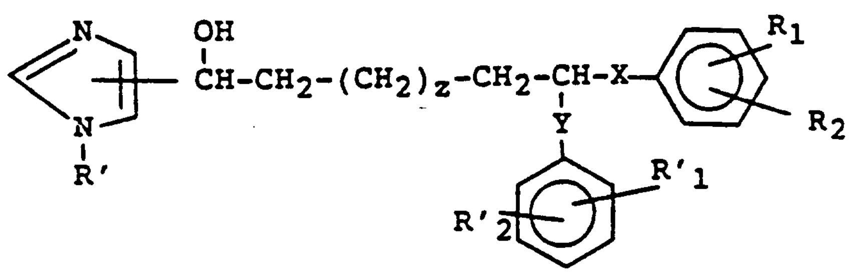

durch eine Wasserstofftransfer-Reaktion, um eine Verbindung der Formel (Ia) zu ergeben, welche

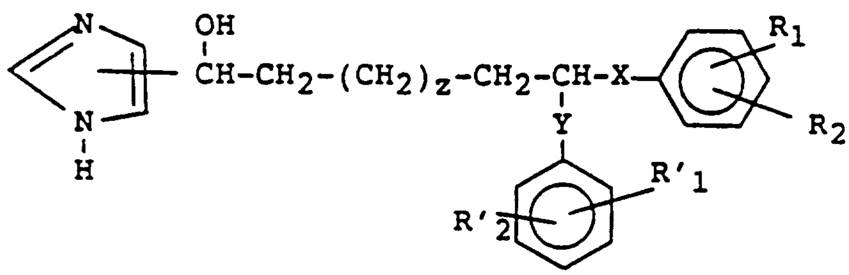

ist, worin $R_1$, $R_2$, $R'_1$, $R'_2$, X, Y und z wie oben definiert sind.

**24.** Verfahren zur Herstellung einer Verbindung nach Anspruch 6 oder eines nicht-toxischen, pharmazeutisch verträglichen Säureadditionssalzes davon, welches umfaßt die Hydrierung einer Verbindung der Formel

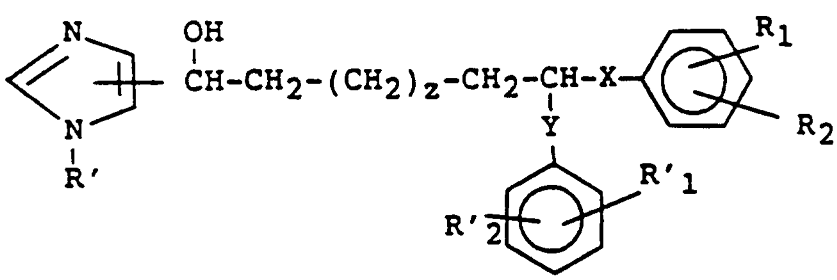

worin R' substituiertes oder unsubstituiertes Benzyl ist und $R_1$, $R_2$, $R'_1$, $R'_2$, X, Y und z wie oben definiert sind, um eine Verbindung der Formel (Ia) zu ergeben, welche

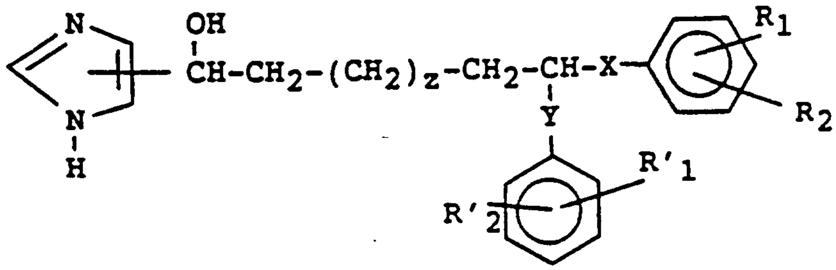

ist.

**25.** Verfahren zur Herstellung einer Verbindung nach Anspruch 6 oder eines nicht-toxischen, pharmazeutisch verträglichen Säureadditionssalzes davon, welches umfaßt die Umsetzung einer Verbindung der Formel

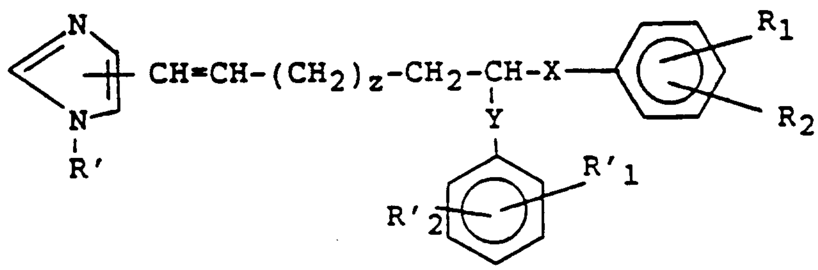

worin R' substituiertes oder unsubstituiertes Benzyl ist ist und $R_1$, $R_2$, $R'_1$, $R'_2$, X, Y und z wie oben definiert sind, durch eine Wasserstofftransfer-Reaktion, um eine Verbindung der Formel (Ia) zu ergeben, welche

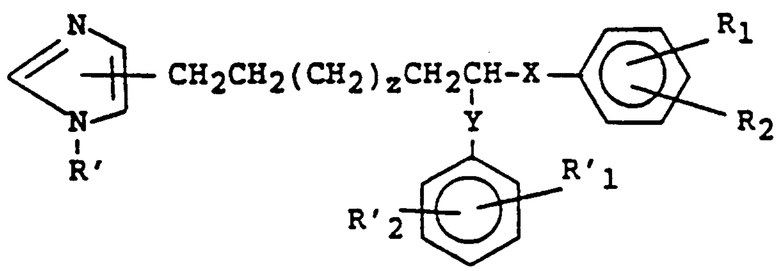

ist.

**26.** Verfahren zur Herstellung einer Verbindung nach Anspruch 6 oder eines nicht-toxischen, pharmazeutisch verträglichen Säureadditionssalzes davon, welches umfaßt die Hydrierung einer Verbindung der Formel

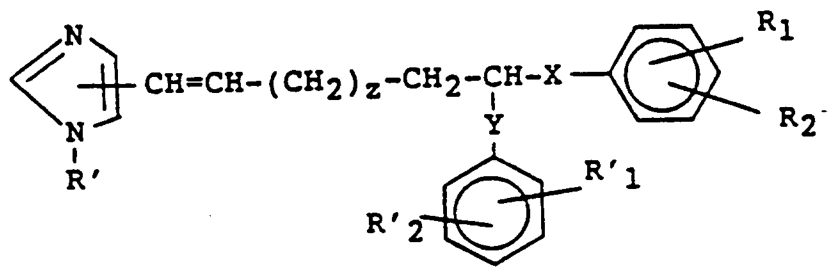

um eine Verbindung der Formel (Ia) zu ergeben, welche

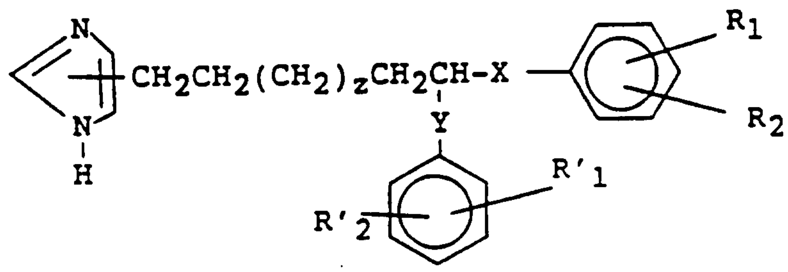

ist, worin R', $R_1$, $R_2$, $R'_1$, $R'_2$, X, Y und z wie oben definiert sind.

27. Verfahren zur Herstellung einer Verbindung nach Anspruch 6 oder eines nicht-toxischen, pharmazeutisch verträglichen Säureadditionssalzes davon, welches umfaßt die Hydrierung einer Verbindung der Formel

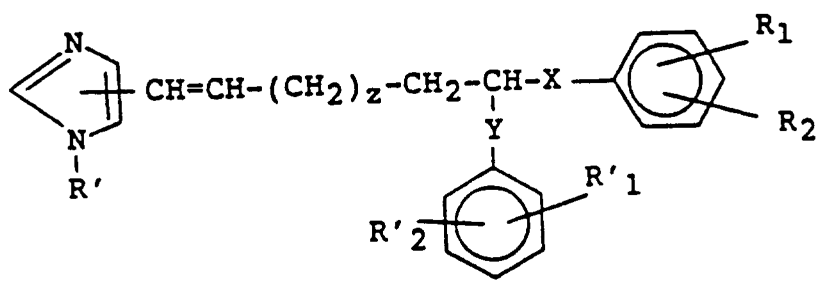

worin R', $R_1$, $R_2$, $R'_1$, $R'_2$, X, Y und z wie oben definiert sind, um eine Verbindung der Formel (Ia) zu ergeben, welche

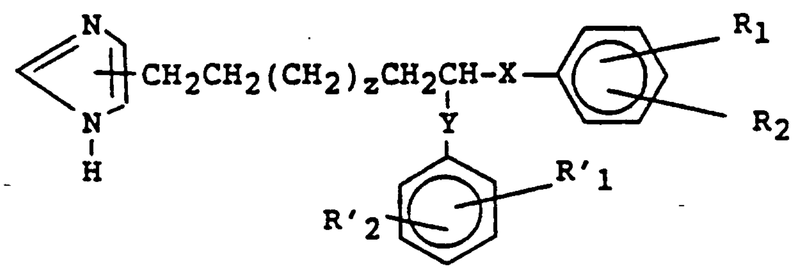

ist.

28. Verfahren zur Herstellung einer Verbindung nach Anspruch 6 oder eines nicht-toxischen, pharmazeutisch verträglichen Säureadditionssalzes davon, welches umfaßt die Umsetzung einer Verbindung der Formel

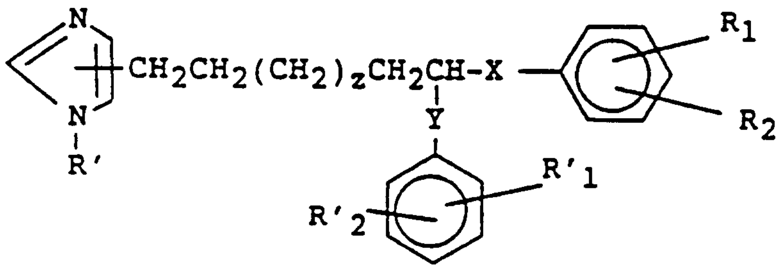

worin R', $R_1$, $R_2$, $R'_1$, $R'_2$, X, Y und z wie oben definiert sind, durch eine Wasserstofftransfer-Reaktion, um eine Verbindung der Formel (Ia) zu ergeben, welche

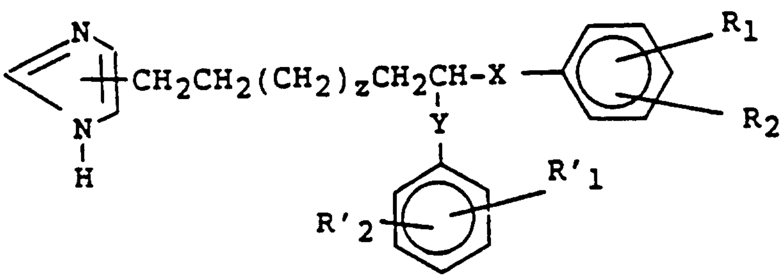

ist.

**29.** Verfahren zur Herstellung einer Verbindung nach Anspruch 6 oder eines nicht-toxischen, pharmazeutisch verträglichen Säureadditionssalzes davon, welches umfaßt die Hydrierung einer Verbindung der Formel

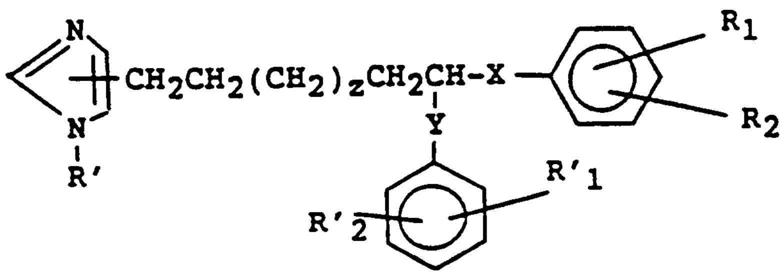

worin $R_1$, $R_2$, $R'_1$, $R'_2$, X, Y und z wie oben definiert sind und R' substituiertes oder unsubstituiertes Benzyl ist, um eine Verbindung der Formel (Ia) zu ergeben, welche

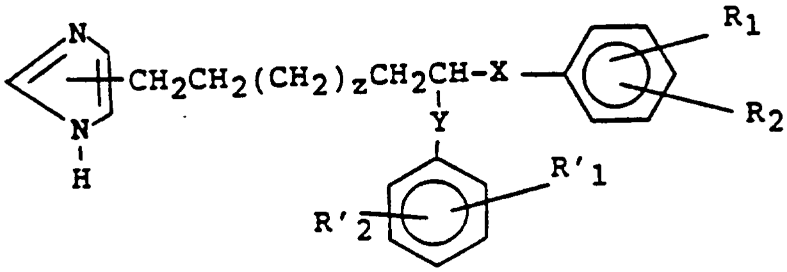

ist.

**30.** Verfahren zur Herstellung einer Verbindung nach Anspruch 6 oder eines nicht-toxischen, pharmazeutisch verträglichen Säureadditionssalzes davon, welches umfaßt die Umsetzung einer Verbindung der Formel

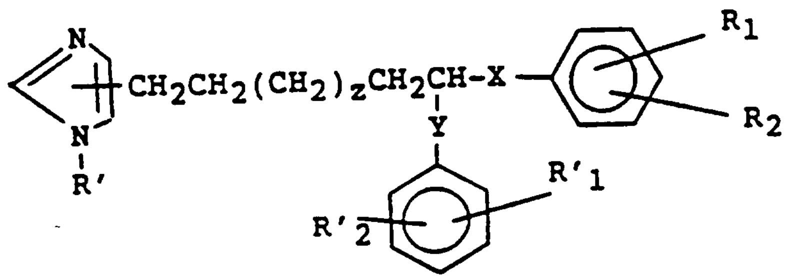

worin $R_1$, $R_2$, $R'_1$, $R'_2$, X, Y und z wie oben definiert sind und R' substituiertes oder unsubstituiertes Benzyl ist, durch eine Wasserstofftransfer-Reaktion, um eine Verbindung der Formel (Ia) zu ergeben, welche

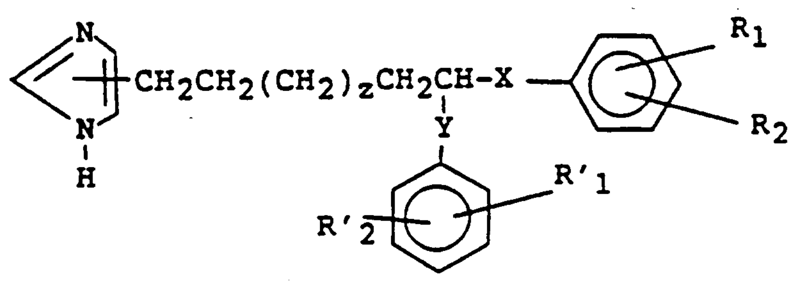

ist.

**31.** Verfahren zur Herstellung einer Verbindung nach Anspruch 6 oder eines nicht-toxischen, pharmazeutisch verträglichen Säureadditionssalzes davon, welches umfaßt die Umsetzung einer Verbindung der Formel

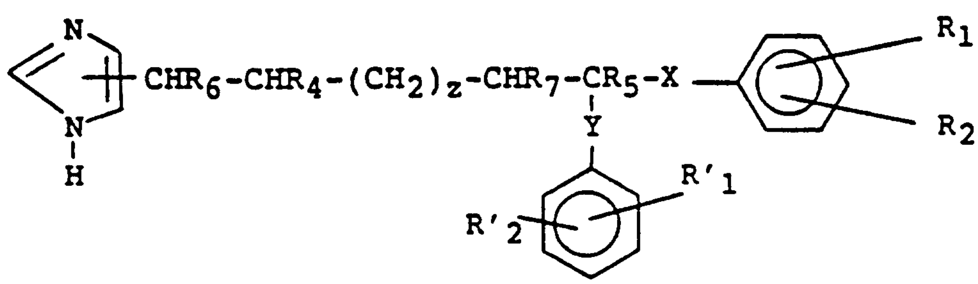

worin $R_1$, $R_2$, $R'_1$, $R'_2$, $R_4$, $R_5$, $R_6$, $R_7$, X, Y und z wie oben definiert sind, mit einer starken Base und weitere Umsetzung mit einem Benzylhalogenid der Formel

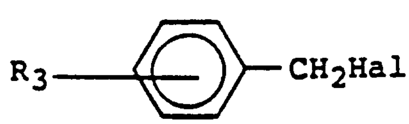

worin $R_3$ wie oben definiert ist, um eine Verbindung der Formel (Ia) zu ergeben, welche

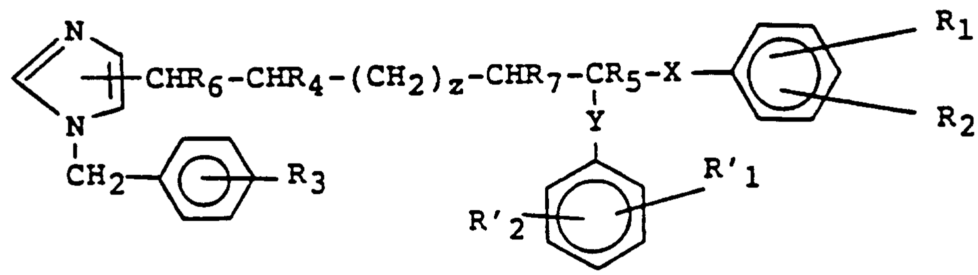

ist.

32. Verfahren zur Herstellung einer Verbindung nach Anspruch 6 oder eines nicht-toxischen, pharmazeutisch verträglichen Säureadditionssalzes davon, welches umfaßt die Nitrierung einer Verbindung der Formel

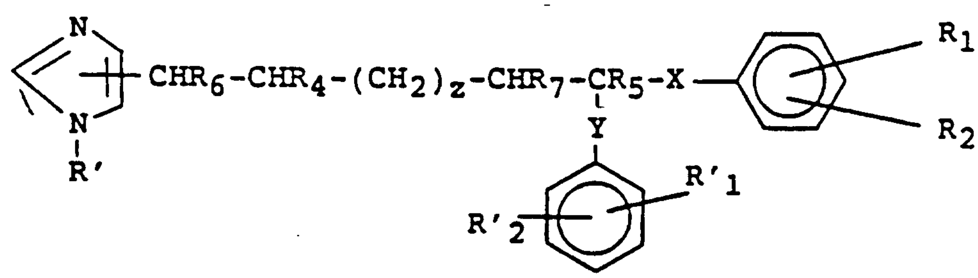

worin R', $R_4$, $R_5$, $R_6$, $R_7$, X, Y und z wie oben definiert sind und einer oder mehrere der Substituenten $R_1$, $R_2$, $R'_1$ und $R'_2$ H sind, um eine Verbindung der Formel (Ia) zu ergeben, worin einer oder mehrere der Substituenten $R_1$, $R_2$, $R'_1$ und $R'_2$ $NO_2$ sind.

33. Verfahren zur Herstellung einer Verbindung nach Anspruch 6 oder eines nicht-toxischen, pharmazeutisch verträglichen Saureadditionssalzes davon, welches umfaßt die Umsetzung eines halogenierten Kohlenwasserstoffs der Formel

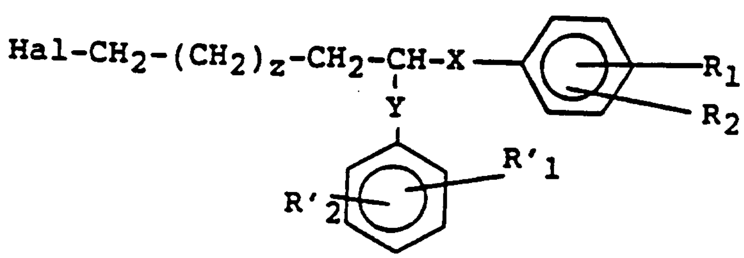

worin $R_1$, $R_2$, $R'_1$, $R'_2$, X, Y und z wie oben definiert sind, mit Triphenylphosphin, um ein Phosphoniumsalz der Formel

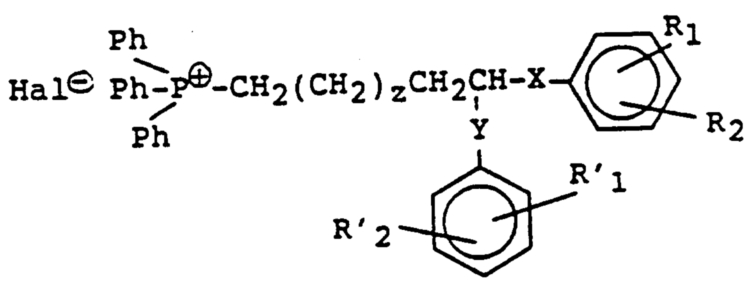

zu ergeben, welches weiter umgesetzt wird mit einer starken Base und dann mit einem 4(5)-Imidazolaldehyd der Formel

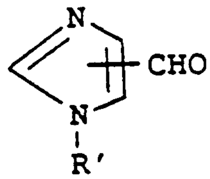

worin R' wie oben definiert ist, um eine Verbindung der Formel (Ia) zu ergeben, welche

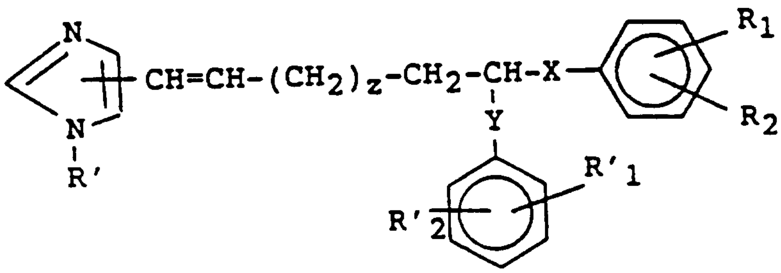

ist.

**34.** Verfahren zur Herstellung einer Verbindung nach Anspruch 6 oder eines nicht-toxischen, pharmazeutisch verträglichen Säureadditionssalzes davon, welches umfaßt die Umsetzung eines halogenierten 4-(5)-Imidazolderivats der Formel

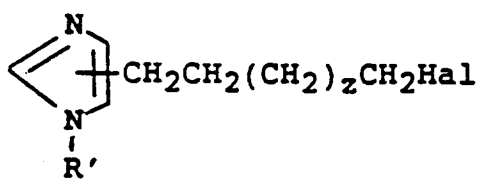

worin R' und z wie oben definiert sind, mit Triphenylphosphin, um ein Phosphoniumsalz der Formel

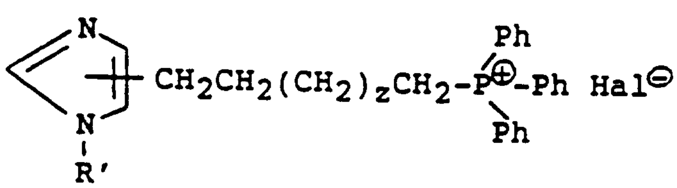

zu ergeben, welches weiter umgesetzt wird mit einer starken Base und dann mit einem Keton der Formel

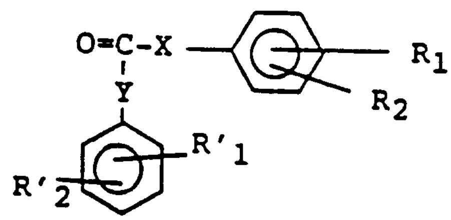

worin $R_1$, $R_2$, $R'_1$, $R'_2$, X und Y wie oben definiert sind, um eine Verbindung der Formel (Ia) zu ergeben, welche

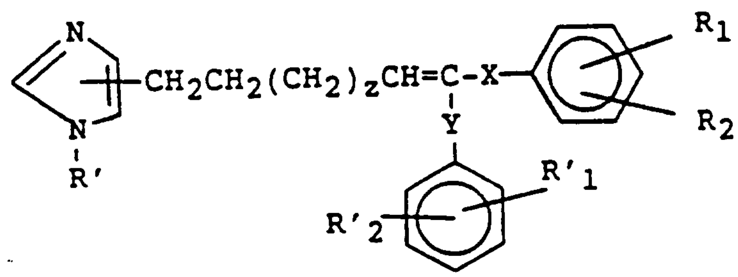

ist.

**35.** Verfahren zur Herstellung einer Verbindung nach Anspruch 6 oder eines nicht-toxischen, pharmazeutisch verträglichen Säureadditionssalzes davon, welches umfaßt die Umsetzung eines halogenierten Kohlenwasserstoffs der Formel

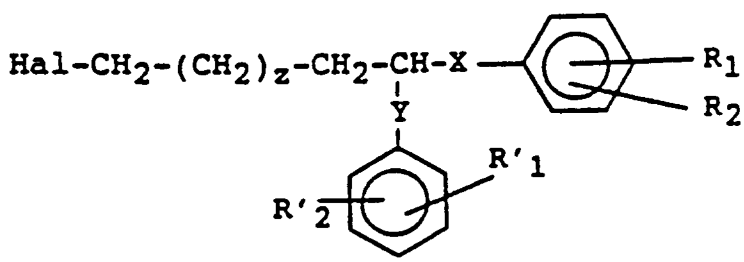

worin $R_1$, $R_2$, $R'_1$, $R'_2$, X, Y und z wie oben definiert sind, mit einem Phosphonsäuretriester, um eine Verbindung der Formel

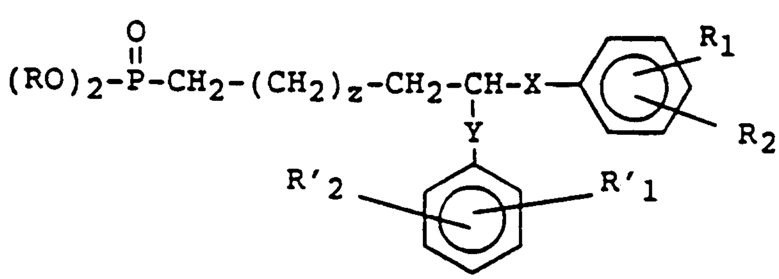

zu ergeben, worin R Alkyl ist, welche weiter umgesetzt wird mit einer Base und dann mit einem 4(5)-Imidazolaldehyd der Formel

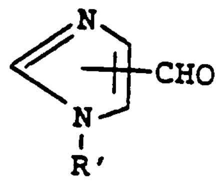

worin R' wie oben definiert ist, um eine Verbindung der Formel (Ia) zu ergeben, welche

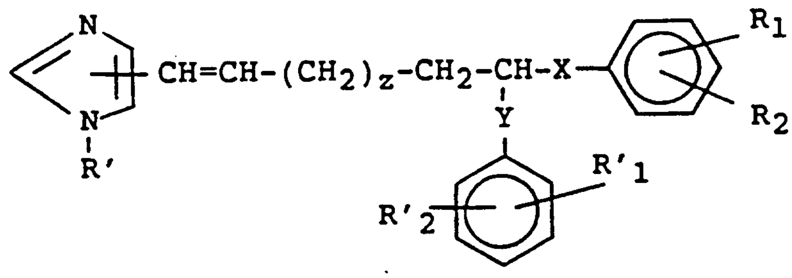

ist.

**36.** Verfahren zur Herstellung eines 4(5)-substituierten Imidazols der Formel

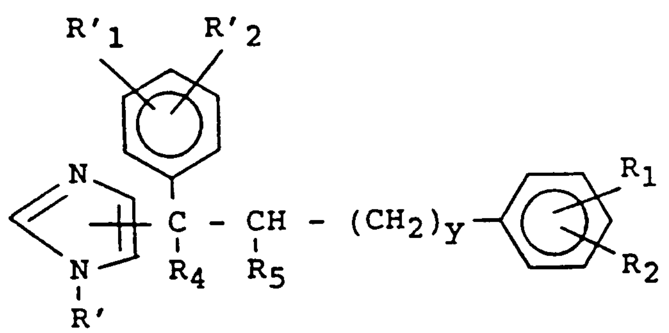

(Ib)

oder eines nicht-toxischen, pharmazeutisch verträglichen Säureadditionssalzes davon, worin $R_1$, $R_2$, $R'_1$ und $R'_2$, die gleich oder verschieden sein können, H, $CH_3$, $C_2H_5$, $C_3H_7$, $OCH_3$, $NO_2$, $CF_3$, $CHF_2$, $CH_2F$ oder Halogen sind; R' H oder

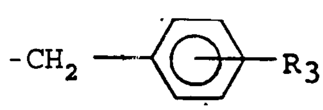

ist, worin $R_3$ H, $CH_3$ oder Halogen ist; $R_4$ H oder OH ist und $R_5$ H ist oder $R_4$ und $R_5$ zusammen eine Bindung bilden und y 0 bis 4 ist,
welches umfaßt die Umsetzung eines 4(5)-Imidazolaldehyds der Formel

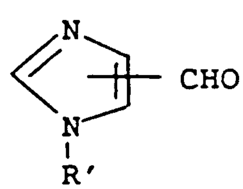

worin R' wie oben definiert ist, mit einem geeigneten Arylalkylmagnesiumhalogenid der Formel

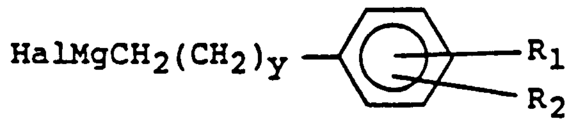

worin $R_1$, $R_2$ und y wie oben definiert sind, um eine Verbindung der Formel

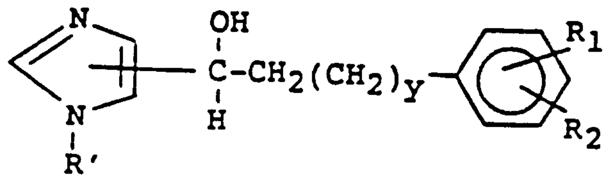

zu ergeben, welche falls R' substituiertes oder unsubstituiertes Benzyl ist, gegebenenfalls hydriert wird, um die Benzylgruppe R' zu entfernen, welche weiter oxidiert wird, um eine Verbindung der Formel

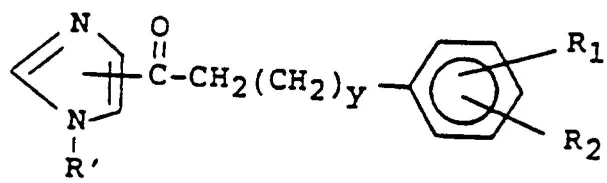

zu ergeben, welche weiter umgesetzt wird mit einem Arylmagnesiumhalogenid der Formel

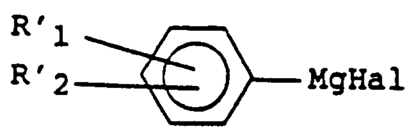

worin $R'_1$ und $R'_2$ wie oben definiert sind und Hal Halogen ist, um eine Verbindung der Formel (Ia) zu ergeben, welche

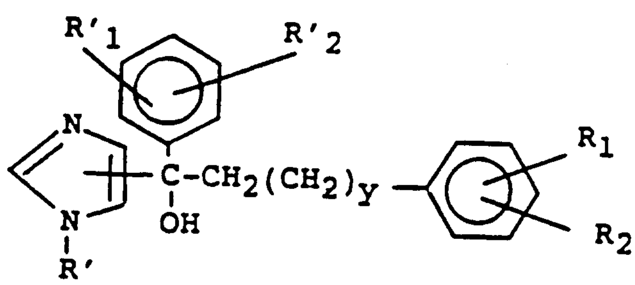

ist.

**37.** Verfahren zur Herstellung einer Verbindung nach Anspruch 36 oder eines nicht-toxischen, pharmazeutisch verträglichen Säureadditionssalzes davon, welches umfaßt die Umsetzung eines 4(5)-Imidazolaldehyds der Formel

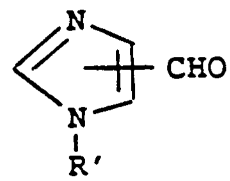

worin R' wie oben definiert ist, mit einem Arylmagnesiumhalogenid der Formel

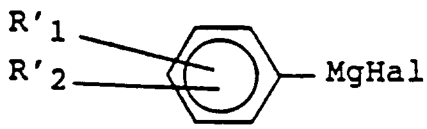

worin $R'_1$ und $R'_2$ wie oben definiert sind und Hal Halogen ist, um eine Verbindung der Formel

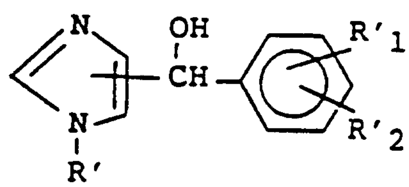

zu ergeben, worin R', $R'_1$ und $R'_2$ wie oben definiert sind, welche falls R' substituiertes oder unsubstituiertes Benzyl ist, gegebenenfalls hydriert wird, um die Benzylgruppe R' zu entfernen, welche oxidiert wird, um eine Verbindung der Formel

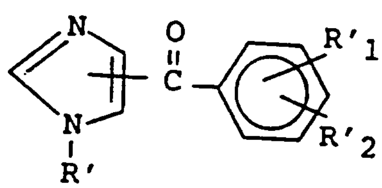

zu ergeben, welche weiter umgesetzt wird mit einem geeigneten Arylalkylmagnesiumhalogenid der Formel

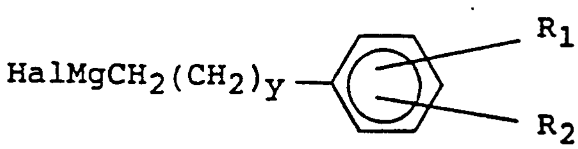

worin $R_1$, $R_2$ und y wie oben definiert sind, um eine Verbindung der Formel (Ib) zu ergeben, welche

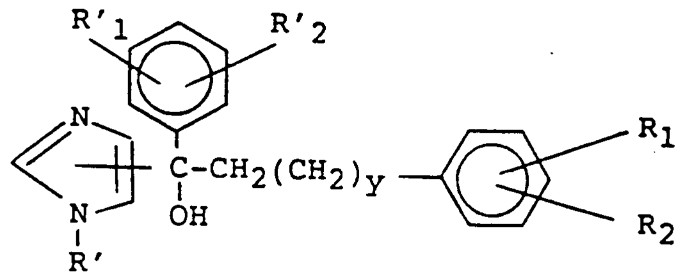

ist.

**38.** Verfahren zur Herstellung eines 4(5)-substituierten Imidazols der Formel

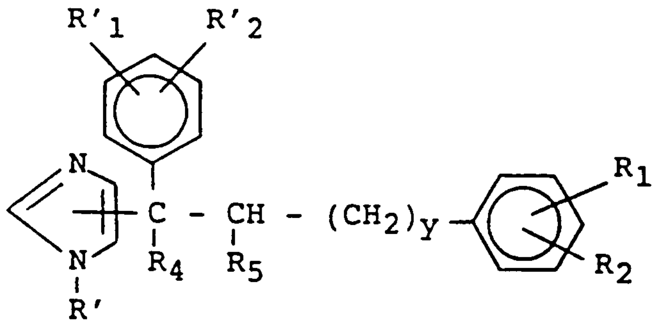

(Ib)

oder eines nicht-toxischen, pharmazeutisch verträglichen Säureadditionssalzes davon, worin $R_1$, $R_2$, $R'_1$ und $R'_2$, die gleich oder verschieden sein können, H, $CH_3$, $C_2H_5$, $C_3H_7$, $OCH_3$, OH, $CH_2OH$, $NO_2$, $NH_2$, CN, $CF_3$, $CHF_2$, $CH_2F$ oder Halogen sind; R' H oder

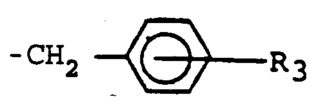

ist, worin $R_3$ H, $CH_3$ oder Halogen ist; $R_4$ H oder OH ist und $R_5$ H ist oder $R_4$ und $R_5$ zusammen eine Bindung bilden und y 0 bis 4 ist, welches umfaßt die Umsetzung eines 4(5)-Imidazolaldehyds der Formel

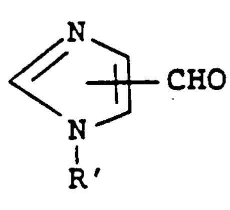

worin R' wie oben definiert ist, mit einen Arylalkylmagnesiumhalogenid der Formel

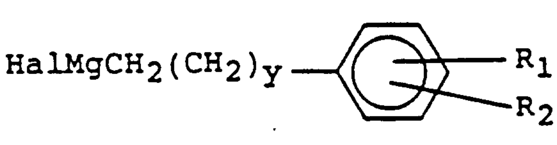

worin y 0 bis 4 ist, Hal Halogen ist und
(i) $R_1$ und/oder $R_2$ $OCH_2Ph$ oder OTHP sind oder

(ii) weder $R_1$ noch $R_2$ $OCH_2Ph$ oder OTHP sind,
um eine Verbindung der Formel

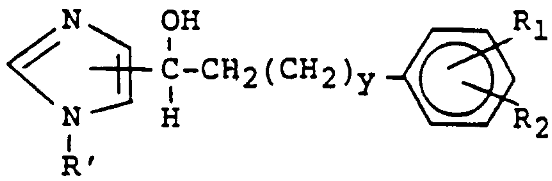

zu ergeben, welche weiter oxidiert wird, um eine Verbindung der Formel

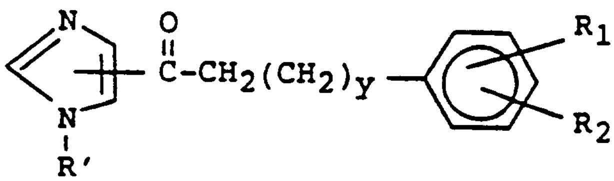

zu ergeben, welche weiter umgesetzt wird mit einem Arylmagnesiumhalogenid der Formel

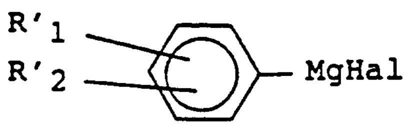

worin
(iii)$R'_1$ und/oder $R'_2$ $OCH_2Ph$ oder OTHP sind oder
(iv) weder $R'_1$ noch $R'_2$ $OCH_2Ph$ oder OTHP sind,
mit der Maßgabe, daß (ii) und (iv) nicht gleichzeitig gelten, um eine Verbindung der Formel

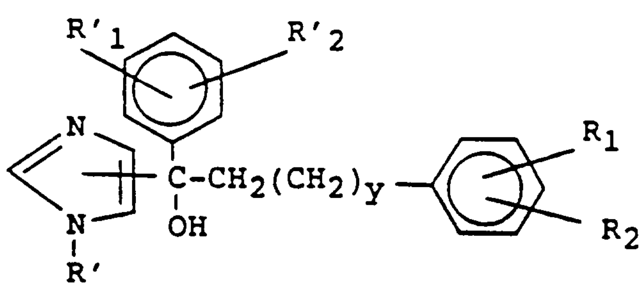

zu ergeben, worin R' und y wie oben definiert sind und einer oder mehrere der Substituenten $R_1$, $R_2$, $R'_1$ und $R'_2$ $OCH_2Ph$ oder OTHP sind, welcher weiter hydriert wird, um eine Verbindung der Formel (Ib) zu ergeben, welche

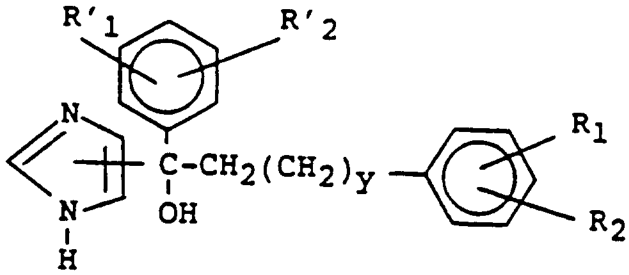

ist, worin y 0 bis 4 ist und einer oder mehrere der Substituenten $R_1$, $R_2$, $R'_1$, $R'_2$, OH sind und die Substituenten, welche nicht OH sind H, $CH_3$, $C_2H_5$, $C_3H_7$, $OCH_3$, $NO_2$, $CF_3$, $CHF_2$, $CH_2F$ oder Halogen sind.

**39.** Verfahren zur Herstellung einer Verbindung nach Anspruch 38 oder eines nicht-toxischen, pharmazeutisch verträglichen Säureadditionssalzes davon, welches umfaßt die Umsetzung eines 4(5)-Imidazolaldehyds der Formel

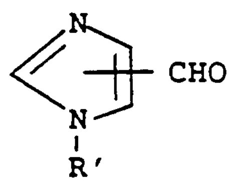

worin R' wie oben definiert ist, mit einem Arylmagnesiumhalogenid der Formel

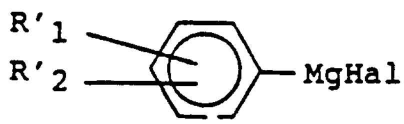

worin Hal Halogen ist, und
(i) $R'_1$ und/oder $R'_2$ $OCH_2Ph$ oder OTHP sind oder
(ii) weder $R'_1$ noch $R'_2$ $OCH_2Oh$ oder OTHP sind,
um eine Verbindung der Formel

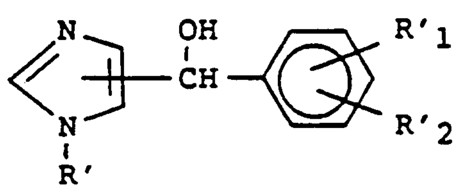

zu ergeben, welche oxidiert wird, um eine Verbindung der Formel

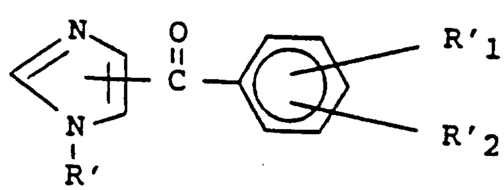

zu ergeben, welche weiter umgesetzt wird mit einem Arylalkylmagnesiumhalogenid der Formel

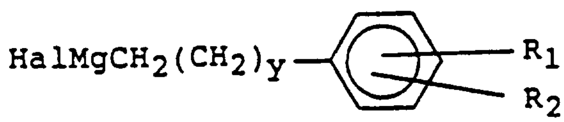

worin y wie oben definiert ist und Hal Halogen ist und
(iii)$R_1$ und/oder $R_2$ $OCH_2Ph$ oder OTHP sind oder
(iv) weder $R_1$ noch $R_2$ $OCH_2Ph$ oder OTHP sind,
mit der Maßgabe, daß (ii) und (iv) nicht gleichzeitig gelten, um eine Verbindung der Formel

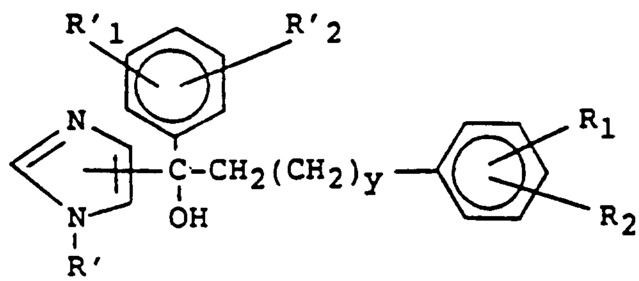

zu ergeben, worin R' und y wie oben definiert sind und einer oder mehrere der Substituenten $R_1$, $R_2$, $R'_1$ und $R'_2$ $OCH_2Ph$ oder OTHP sind, welcher weiter hydriert wird, um eine Verbindung der Formel (Ib) zu ergeben, welche

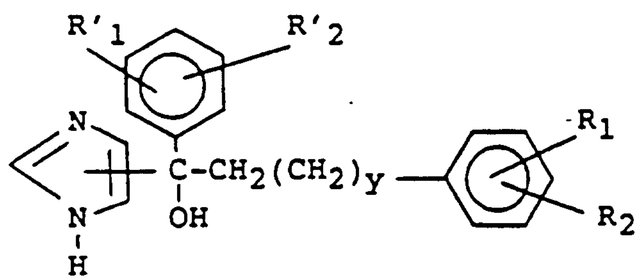

ist, worin y 0 bis 4 ist und einer oder mehrere der Substituenten $R_1$, $R_2$, $R'_1$ und $R'_2$ OH sind und die Substituenten, welche nicht OH sind, H, $CH_3$, $C_2H_5$,$C_3H_7$, $OCH_3$, $NO_2$, $CF_3$, $CHF_2$, $CH_2F$ oder Halogen sind.

**40.** Verfahren zur Herstellung einer Verbindung nach Anspruch 38 oder eines nicht-toxischen, pharmazeutisch verträglichen Saureadditionssalzes davon, welches umfaßt die Dealkylierung einer Verbindung der Formel (Ib)

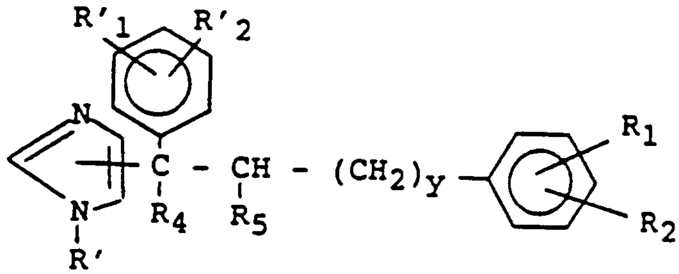

worin R', y, $R_4$ und $R_5$ wie oben definiert sind und einer oder mehrere der Substituenten $R_1$, $R_2$, $R'_1$ und $R'_2$ $OCH_3$ sind, um eine Verbindung der Formel (Ib) zu ergeben, worin einer oder mehrere der Substituenten $R_1$, $R_2$, $R'_1$ und $R'_2$ OH sind.

**41.** Verfahren zur Herstellung einer Verbindung nach Anspruch 38 oder eines nicht-toxischen, pharmazeutisch verträglichen Säureadditionssalzes davon, welches umfaßt die Hydrolyse einer Verbindung der Formel (Ib)

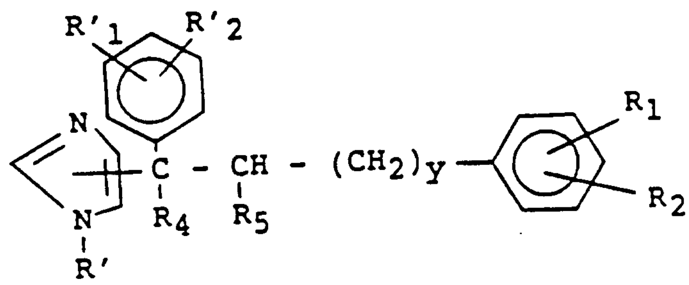

worin R', $R_4$, $R_5$ und y wie oben definiert sind und einer oder mehrere der Substituenten $R_1$, $R_2$, $R'_1$ und $R'_2$ CN sind, und weitere Reduktion, um eine Verbindung der Formel (Ib) zu ergeben, worin einer oder mehrere der Substituenten $R_1$, $R_2$, $R'_1$ und $R'_2$ $CH_2OH$ sind.

**42.** Verfahren zur Herstellung einer Verbindung nach Anspruch 38 oder eines nicht-toxischen, pharmazeutisch verträglichen Säureadditionssalzes davon, welches umfaßt die Diazotierung einer Verbindung der Formel (Ib)

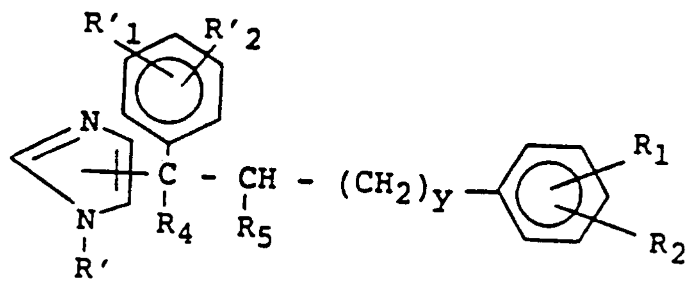

worin R', $R_4$, $R_5$ und y wie oben definiert sind und einer oder mehrere der Substituenten $R_1$, $R_2$, $R'_1$ und $R'_2$ $NH_2$ sind, um eine Verbindung der Formel (Ib) zu ergeben, worin einer oder mehrere der Substituenten $R_1$, $R_2$, $R'_1$ und $R'_2$ CN sind.

**43.** Verfahren zur Herstellung einer Verbindung nach Anspruch 38 oder eines nicht-toxischen, pharmazeutisch verträglichen Säureadditionssalzes davon, welches umfaßt die Hydrierung einer Verbindung der Formel (Ib)

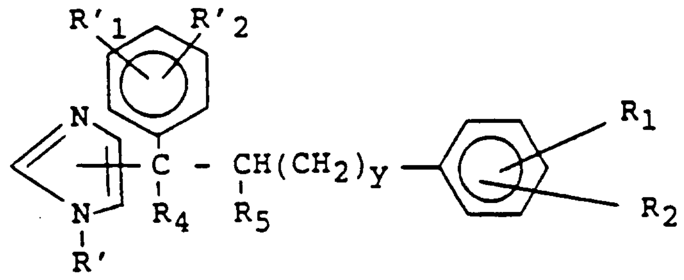

worin R', $R_4$, $R_5$ und y wie oben definiert sind und einer oder mehrere der Substituenten $R_1$, $R_2$, $R'_1$

und $R'_2$ $NO_2$ sind, um eine Verbindung der Formel (Ib) zu ergeben, worin R' H ist, y 0 bis 4 ist und einer oder mehrere der Substituenten $R_1$, $R_2$, $R'_1$ und $R'_2$ $NH_2$ sind.

**44.** Verfahren zur Herstellung einer Verbindung nach Anspruch 38 oder eines nicht-toxischen, pharmazeutisch verträglichen Säureadditionssalzes davon, welches umfaßt die Dehydratisierung einer Verbindung der Formel

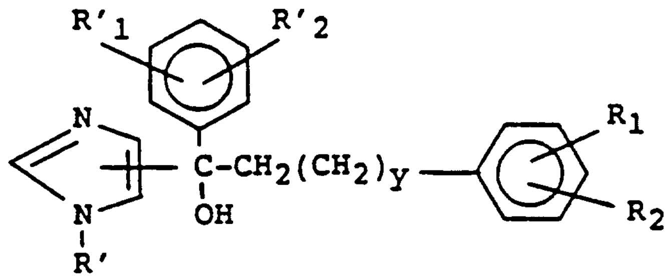

um eine Verbindung der Formel (Ib) zu ergeben, welche

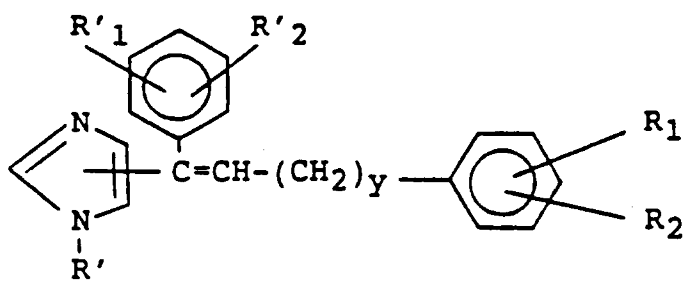

ist, worin R', $R_1$, $R_2$, $R'_1$, $R'_2$ und y wie oben definiert sind.

**45.** Verfahren zur Herstellung einer Verbindung nach Anspruch 38 oder eines nicht-toxischen, pharmazeutisch verträglichen Säureadditionssalzes davon, welches umfaßt die Hydrolyse einer Verbindung der Formel

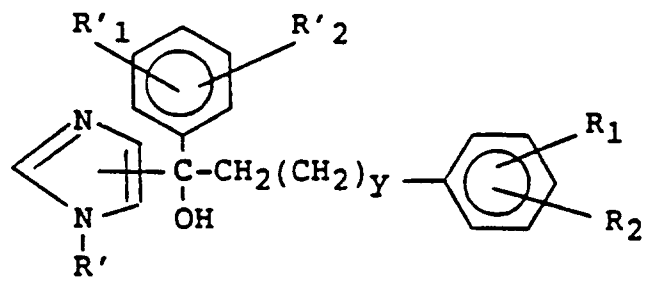

durch eine Wasserstofftransfer-Reaktion, um eine Verbindung der Formel (Ib) zu ergeben, welche

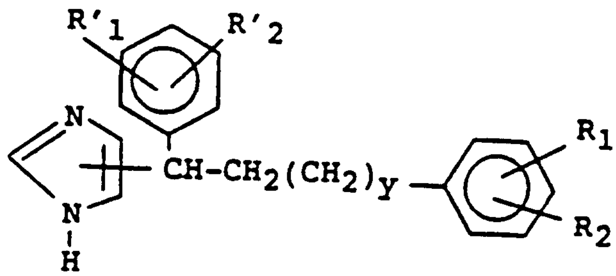

ist, worin $R_1$, $R_2$, $R'_1$, $R'_2$ und y wie oben definiert sind.

**46.** Verfahren zur Herstellung einer Verbindung nach Anspruch 38 oder eines nicht-toxischen, pharmazeutisch verträglichen Säureadditionssalzes davon, welches umfaßt die Hydrierung einer Verbindung der Formel

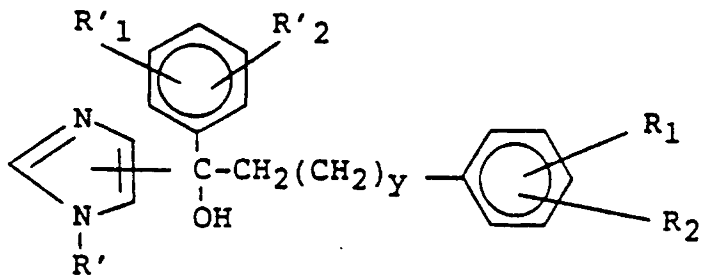

worin R' substituiertes oder unsubstituiertes Benzyl ist und $R_1$, $R_2$, $R'_1$, $R'_2$, wie oben definiert sind, um eine Verbindung der Formel (Ib) zu ergeben, welche

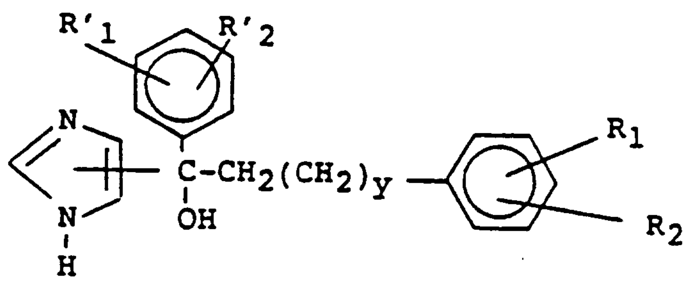

ist.

**47.** Verfahren zur Herstellung einer Verbindung nach Anspruch 38 oder eines nicht-toxischen, pharmazeutisch verträglichen Säureadditionssalzes davon, welches umfaßt die Umsetzung einer Verbindung der Formel

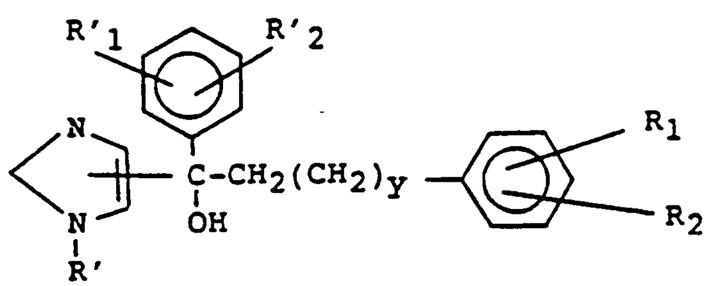

worin R' substituiertes oder unsubstituiertes Benzyl ist und $R_1$, $R_2$, $R'_1$, $R'_2$, wie oben definiert sind, durch eine Wasserstofftransfer-Reaktion, um eine Verbindung der Formel (Ib) zu ergeben, welche

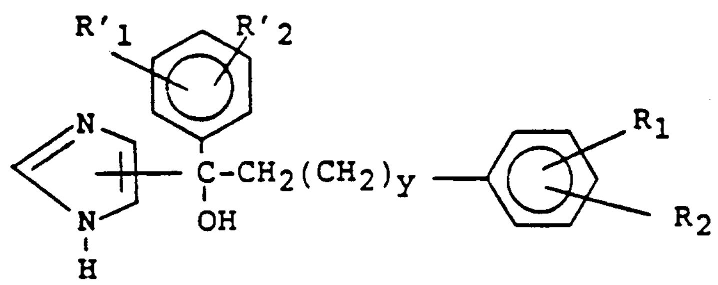

ist, worin $R_1$, $R_2$, $R'_1$, $R'_2$ und y wie oben definiert sind.

**48.** Verfahren zur Herstellung einer Verbindung nach Anspruch 36 oder 38 oder eines nicht-toxischen, pharmazeutisch verträglichen Säureadditionssalzes davon, welches umfaßt die Umsetzung eines Benzoylimidazols der Formel

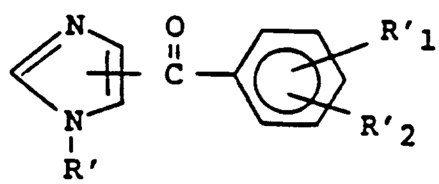

worin R', $R'_1$, $R'_2$ wie in Anspruch 36 definiert sind, mit einem Aldehyd der Formel

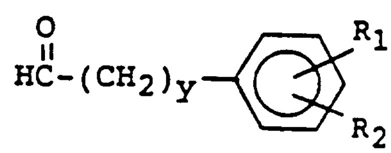

worin $R_1$, $R_2$ und y wie in Anspruch 38 definiert sind, in Gegenwart eines niederwertigen Titan-Reagens, um eine Verbindung der Formel (Ib) zu ergeben, welche

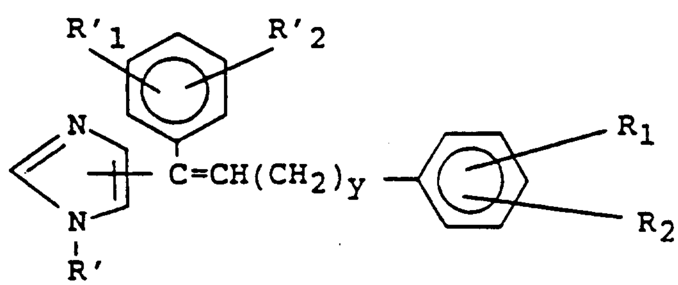

ist.

**49.** Verfahren zur Herstellung einer Verbindung nach Anspruch 36 oder 38 oder eines nicht-toxischen, pharmazeutisch verträglichen Säureadditionssalzes davon, welches umfaßt die Umsetzung eines Benzoylimidazols der Formel

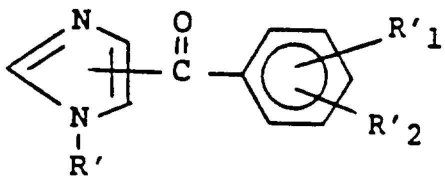

worin R' wie oben definiert ist und $R'_1$ und/oder $R'_2$ $OCH_2Ph$ oder OTHP sind, mit einem Aldehyd der Formel

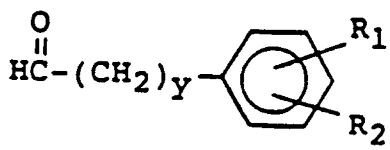

worin $R_1$, $R_2$ und y wie in Anspruch 38 definiert sind, in Gegenwart eines niederwertigen Titan-Reagens, um eine Verbindung der Formel

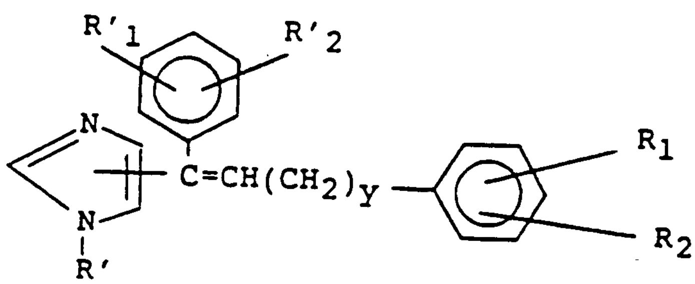

zu ergeben, worin $R'_1$ und/oder $R'_2$ $OCH_2Ph$ oder OTHP sind, welcher weiter hydriert wird, um eine Verbindung der Formel (Ib) zu ergeben, welche

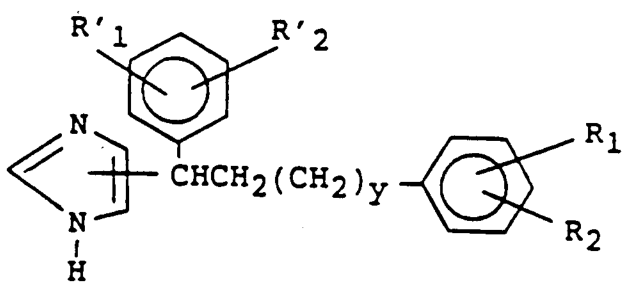

ist, worin $R'_1$ und/oder $R'_2$ OH sind, der Substituent $R'_1$ oder $R'_2$, welcher nicht OH ist, H, $CH_3$, $C_2H_5$, $C_3H_7$, $OCH_3$, $NO_2$, $CF_3$, $CHF_2$, $CH_2F$ oder Halogen ist und $R_1$ und $R_2$ wie in Anspruch 38 definiert sind.

**50.** Verfahren zur Herstellung einer Verbindung nach Anspruch 38 oder eines nicht-toxischen, pharmazeutisch verträglichen Säureadditionssalzes davon, welches umfaßt die Hydrierung einer Verbindung der Formel

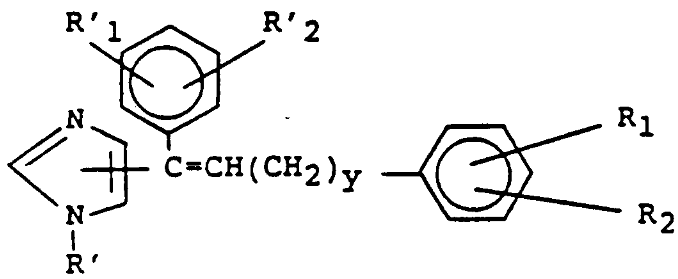

um eine Verbindung der Formel (Ib) zu ergeben, welche

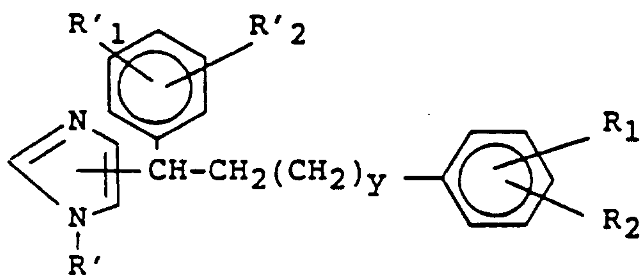

ist, worin R', $R_1$, $R_2$, $R'_1$, $R'_2$ und y wie oben definiert sind.

**51.** Verfahren zur Herstellung einer Verbindung nach Anspruch 38 oder eines nicht-toxischen, pharmazeutisch verträglichen Säureadditionssalzes davon, welches umfaßt die Hydrierung einer Verbindung der Formel

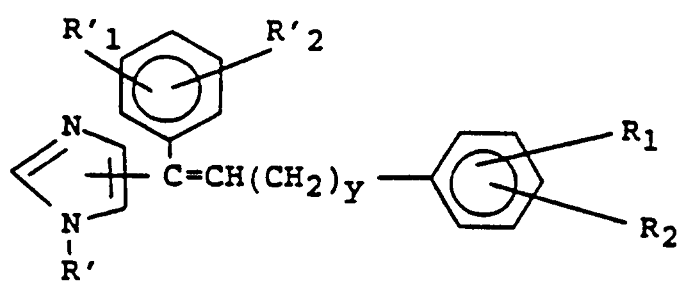

um eine Verbindung der Formel (Ib) zu ergeben, welche

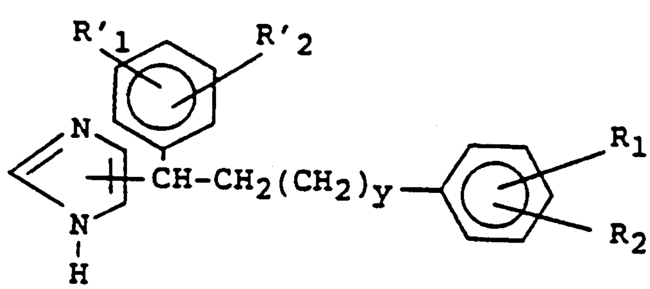

ist, worin $R_1$, $R_2$, $R'_1$, $R'_2$ und y wie oben definiert sind.

**52.** Verfahren zur Herstellung einer Verbindung nach Anspruch 38 oder eines nicht-toxischen, pharmazeutisch verträglichen Säureadditionssalzes davon, welches umfaßt die Umsetzung einer Verbindung der

Formel

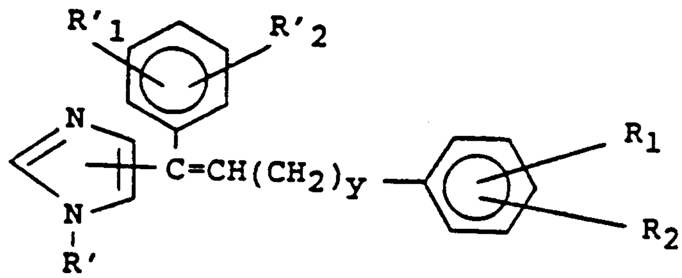

durch eine Wasserstofftransfer-Reaktion, um eine Verbindung der Formel (Ib) zu ergeben, welche

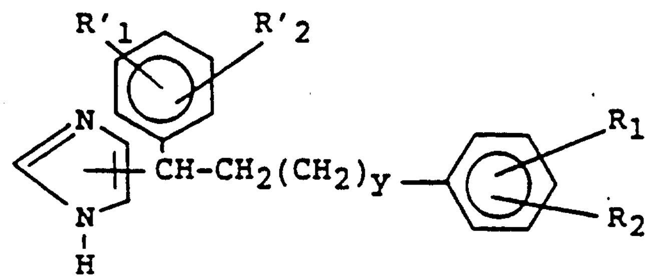

ist, worin $R_1$, $R_2$, $R'_1$, $R'_2$ und y wie oben definiert sind.

**53.** Verfahren zur Herstellung einer Verbindung nach Anspruch 38 oder eines nicht-toxischen, pharmazeutisch vertraglichen Säureadditionssalzes davon, welches umfaßt die Hydrierung einer Verbindung der Formel

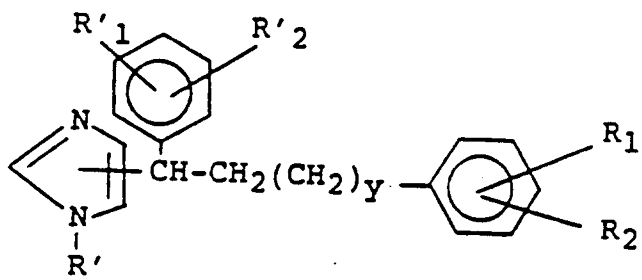

worin $R_1$, $R_2$, $R'_1$, $R'_2$ und y wie oben definiert sind und R' substituiertes oder unsubstituiertes Benzyl ist, um eine Verbindung der Formel (Ib) zu ergeben, welche

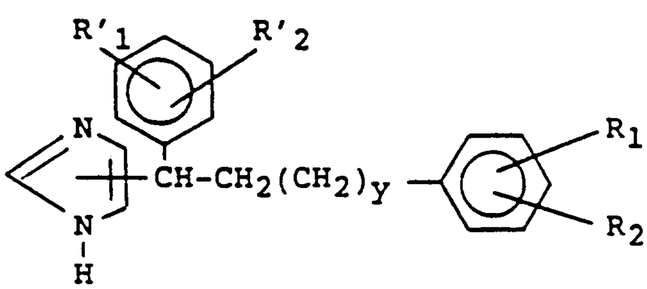

ist.

**54.** Verfahren zur Herstellung einer Verbindung nach Anspruch 38 oder eines nicht-toxischen, pharmazeutisch verträglichen Säureadditionssalzes davon, welches umfaßt die Umsetzung einer Verbindung der Formel

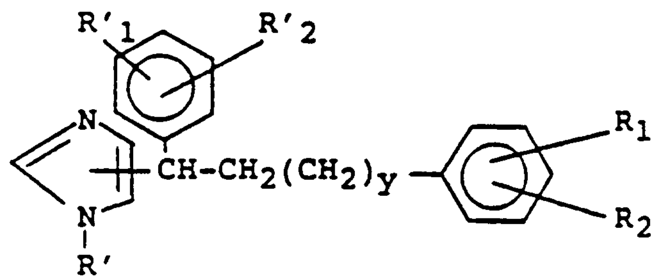

worin $R_1$, $R_2$, $R'_1$, $R'_2$ und y wie oben definiert sind und R' substituiertes oder unsubstituiertes Benzyl ist, durch eine Wasserstofftransfer-Reaktion, um eine Verbindung der Formel (Ib) zu ergeben, welche

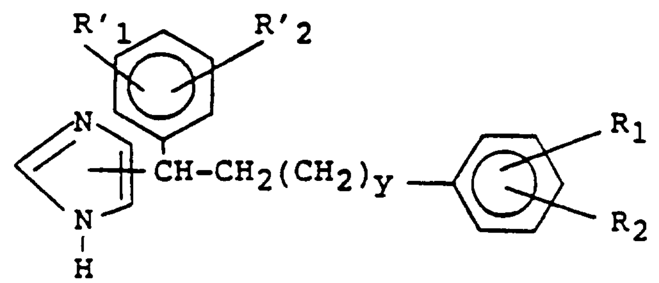

ist.

**55.** Verfahren zur Herstellung einer Verbindung nach Anspruch 38 oder eines nicht-toxischen, pharmazeutisch verträglichen Säureadditionssalzes davon, welches umfaßt die Umsetzung einer Verbindung der Formel

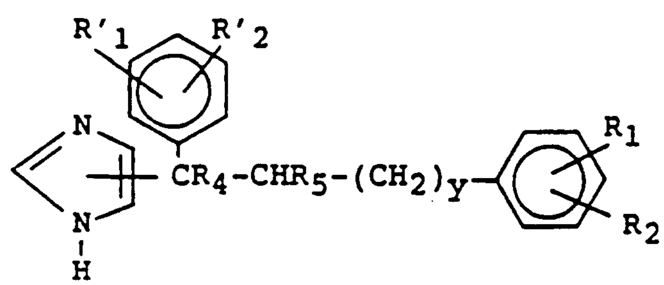

worin $R_1$, $R_2$, $R'_1$, $R'_2$, $R_4$ und $R_5$ und y wie oben definiert sind, mit einer starken Base und weiter mit einem Benzylhalogenid der Formel

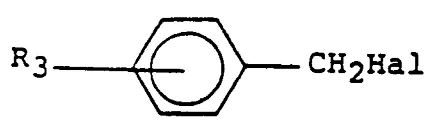

worin $R_3$ wie oben definiert ist und Hal Halogen ist, um eine Verbindung der Formel (ib) zu ergeben, welche

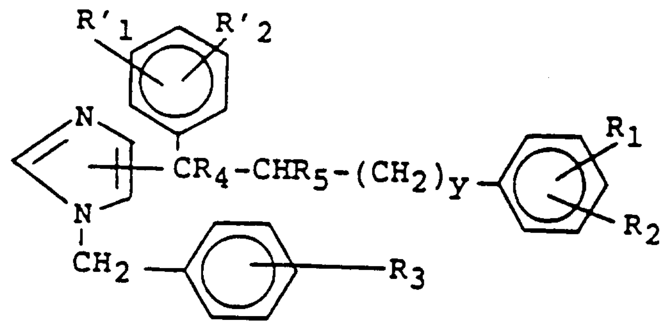

ist.

**56.** Verfahren zur Herstellung einer Verbindung nach Anspruch 38 oder eines nicht-toxischen, pharmazeutisch verträglichen Säureadditionssalzes davon, welches umfaßt die Nitrierung einer Verbindung der Formel (Ib)

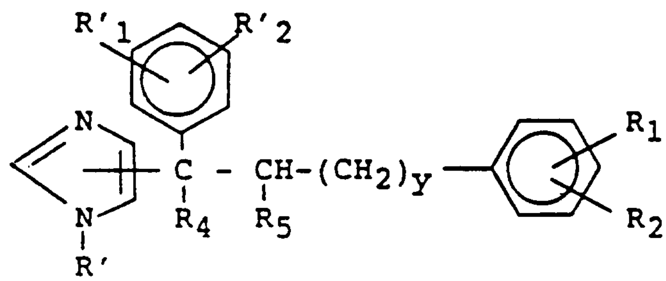

worin R', $R_4$, $R_5$ und y wie oben definiert sind und worin einer oder mehrere der Substituenten $R_1$, $R_2$, $R'_1$ und $R'_2$ H sind, um eine Verbindung der Formel (Ib) zu ergeben, worin einer oder mehrere der Substituenten $R_1$, $R_2$, $R'_1$ und $R'_2$ $NO_2$ sind.

**Revendications**

**Revendications pour les Etats contractants suivants : AT, BE, CH, DE, DK, FR, GB, IT, LI, LU, NL, SE**

**1.** 4-(5)-Imidazole substitué de formule :

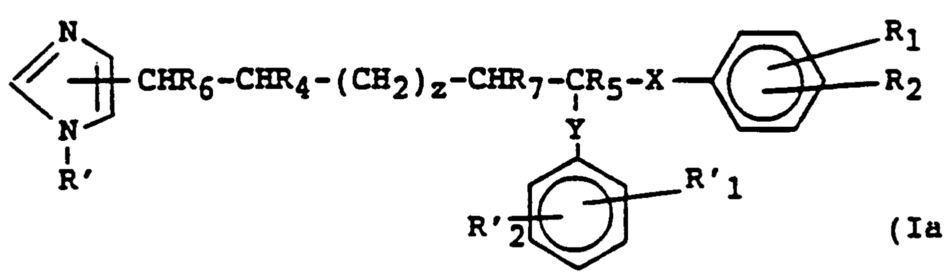

(Ia)

ou sel d'addition d'acide de celui-ci non toxique acceptable du point de vue pharmaceutique, dans laquelle $R_1$, $R_2$, $R'_1$ et $R'_2$, qui peuvent être identiques ou différents, sont un atome d'hydrogène, un groupe $CH_3$, $C_2H_5$, $C_3H_7$, $OCH_3$, OH, $CH_2OH$, $NO_2$, $NH_2$, CN, $CF_3$, $CHF_2$, $CH_2F$ ou un atome d'halogène; R' est un atome d'hydrogène ou un groupe

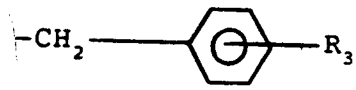

dans lequel $R_3$ est un atome d'hydrogène, d'halogène ou un groupe $CH_3$; $R_4$ est un atome d'hydrogène, $R_5$ est un atome d'hydrogène ou un groupe OH, $R_6$ est un atome d'hydrogène ou un groupe OH et $R_7$ est un atome d'hydrogène, ou $R_4$ et $R_6$ forment ensemble une liaison ou $R_5$ et $R_7$ forment ensemble une liaison; X et Y, qui peuvent être identiques ou différents, sont une liaison, un groupe alkyle en $C_{1-2}$ à chaîne droite ou le groupe alcényle correspondant, et z est 0 à 2.

**2.** Imidazole substitué suivant la revendication 1, dans lequel $R_4$, $R_5$, $R_6$ et $R_7$ sont chacun un atome d'hydrogène.

**3.** Imidazole substitué suivant les revendications 1 ou 2, dans lequel $R_1$, $R_2$, $R'_1$ et $R'_2$ sont chacun un atome d'hydrogène.

**4.** Imidazole substitué suivant les revendications 1 ou 2, dans lequel $R'_1$, $R'_2$ et $R_2$ sont chacun un atome d'hydrogène et $R_1$, qui est tel que défini dans la revendication 1, à l'exception d'un atome d'hydrogène, est en position ortho, méta ou para du groupe phényle.

**5.** Imidazole substitué suivant la revendication 4, dans lequel $R_1$ est en position para du groupe phényle.

**6.** Imidazole substitué suivant la revendication 5, dans lequel $R_1$ est un groupe $OCH_3$.

**7.** Imidazole substitué suivant les revendications 1 ou 2, dans lequel $R_2$ et $R'_2$ sont chacun un atome d'hydrogène, et $R_1$ et $R'_1$, qui sont tels que définis dans la revendication 1 à l'exception d'un atome d'hydrogène, sont chacun en position para du groupe phényle.

**8.** Imidazole substitué suivant la revendication 7, dans lequel $R_1$ et $R'_1$ sont chacun un atome de fluor.

**9.** Imidazole substitué suivant les revendications 1 ou 2, dans lequel $R'_1$ et $R'_2$ sont chacun un atome d'hydrogène, et $R_1$ et $R_2$, qui sont tels que définis dans la revendication 1 à l'exception d'un atome d'hydrogène, sont en positions 3 et 5 du groupe phényle.

**10.** Imidazole substitué suivant les revendications 1 ou 2, dans lequel $R'_1$ et $R'_2$ sont chacun un atome d'hydrogène, et $R_1$ et $R_2$, qui sont tels que définis dans la revendication 1 à l'exception d'un atome d'hydrogène, sont en positions 3 et 4 du groupe phényle.

**11.** Imidazole substitué suivant l'une quelconque des revendications 1 à 10, dans lequel $R_4$ et $R_6$ forment ensemble une liaison.

**12.** Imidazole substitué suivant l'une quelconque des revendications 1 à 11, dans lequel R' est un atome d'hydrogène.

**13.** Composé suivant la revendication 1, qui est le 4-[4-(4-méthylphényl)-4-phénylbutyl]-1H-imidazole ou un sel d'addition d'acide de celui-ci non toxique acceptable du point de vue pharmaceutique.

**14.** Composé suivant la revendication 1, qui est le 1-benzyl-5-[4-(4-méthylphényl)-4-phénylbutyl]-1H-imidazole ou un sel d'addition d'acide de celui-ci non toxique acceptable du point de vue pharmaceutique.

**15.** Composé suivant la revendication 1, qui est le 1-benzyl-5-[4-(3-méthylphényl)-4-phénylbutyl]-1H-imidazole ou un sel d'addition d'acide de celui-ci non toxique acceptable du point de vue pharmaceutique.

**16.** Composé suivant la revendication 1, qui est le 4-[4-(3-méthylphényl)-4-phénylbutyl]-1H-imidazole ou un sel d'addition d'acide de celui-ci non toxique acceptable du point de vue pharmaceutique.

**17.** Composé suivant la revendication 1, qui est le 4-[4-(2-méthylphényl)-4-phénylbutyl]-1H-imidazole ou un sel d'addition d'acide de celui-ci non toxique acceptable du point de vue pharmaceutique.

**18.** Composé suivant la revendication 1, qui est le 1-benzyl-5-[4-(2-méthylphényl)-4-phénylbutyl]-1H-imidazole ou un sel d'addition d'acide de celui-ci non toxique acceptable du point de vue pharmaceutique.

**19.** Composé suivant la revendication 1, qui est le 1-benzyl-5-(4,5-diphénylpentyl)-1H-imidazole ou un sel d'addition d'acide de celui-ci non toxique acceptable du point de vue pharmaceutique.

**20.** Composé suivant la revendication 1, qui est le 4-(4,5-diphénylpentyl)-1H-imidazole ou un sel d'addition d'acide de celui-ci non toxique acceptable du point de vue pharmaceutique.

**21.** Composé suivant la revendication 1, qui est le 4-(4,4-diphénylbutyl)-1H-imidazole ou un sel d'addition d'acide de celui-ci non toxique acceptable du point de vue pharmaceutique.

**22.** Composé suivant la revendication 1, qui est le 1-benzyl-5-(4,4-diphénylbutyl)-1H-imidazole ou un sel d'addition d'acide de celui-ci non toxique acceptable du point de vue pharmaceutique.

**23.** Composé suivant la revendication 1, qui est le 4-[4-(4-méthoxyphényl)-4-phénylbutyl]-1H-imidazole ou un sel d'addition d'acide de celui-ci non toxique acceptable du point de vue pharmaceutique.

**24.** Composé suivant la revendication 1, qui est le 4-[4,4-bis-(4-méthoxyphényl)-butyl]-1H-imidazole ou un sel d'addition d'acide de celui-ci non toxique acceptable du point de vue pharmaceutique.

**25.** Composé suivant la revendication 1, qui est le 4-[4,4-bis-(3-méthylphényl)-butyl]-1H-imidazole ou un sel d'addition d'acide de celui-ci non toxique acceptable du point de vue pharmaceutique.

**26.** Composé suivant la revendication 1, qui est le 4-[4-(3,5-diméthylphényl)-4-phénylbutyl]-1H-imidazole ou un sel d'addition d'acide de celui-ci non toxique acceptable du point de vue pharmaceutique.

**27.** Composé suivant la revendication 1, qui est le 4-[4-(3,4-diméthylphényl)-4-phénylbutyl]-1H-imidazole ou un sel d'addition d'acide de celui-ci non toxique acceptable du point de vue pharmaceutique.

**28.** Composé suivant la revendication 1, qui est le 4-[4-(3,5-diméthylphényl)-4-(3-méthylphényl)-butyl]-1H-imidazole ou un sel d'addition d'acide de celui-ci non toxique acceptable du point de vue pharmaceutique.

**29.** Composé suivant la revendication 1, qui est le 4-[4,4-bis-(4-méthylphényl)-butyl]-1H-imidazole ou un sel d'addition d'acide de celui-ci non toxique acceptable du point de vue pharmaceutique.

**30.** Composé suivant la revendication 1, qui est le 4-[5,5-diphénylpentyl]-1H-imidazole ou un sel d'addition d'acide de celui-ci non toxique acceptable du point de vue pharmaceutique.

**31.** Composé suivant la revendication 1, qui est le 4-[6,6-diphénylhexyl]-1H-imidazole ou un sel d'addition d'acide de celui-ci non toxique acceptable du point de vue pharmaceutique.

**32.** Composé suivant la revendication 1, qui est le 4-[4-(2-fluorophényl)-4-phénylbutyl]-1H-imidazole ou un sel d'addition d'acide de celui-ci non toxique acceptable du point de vue pharmaceutique.

**33.** Composé suivant la revendication 1, qui est le 4-[4-(4-fluorophényl)-4-phénylbutyl]-1H-imidazole ou un sel d'addition d'acide de celui-ci non toxique acceptable du point de vue pharmaceutique.

**34.** Composé suivant la revendication 1, qui est le 4-(4,4-diphényl-1-butényl)-1H-imidazole ou un sel d'addition d'acide de celui-ci non toxique acceptable du point de vue pharmaceutique.

**35.** Composé suivant la revendication 1, qui est le 4-[4,4-bis-(4-fluorophényl)-butyl]-1H-imidazole ou un sel d'addition d'acide de celui-ci non toxique acceptable du point de vue pharmaceutique.

**36.** Composé suivant la revendication 1, qui est le 4-[4,4-bis-(4-nitrophényl)-butyl]-1H-imidazole ou un sel d'addition d'acide de celui-ci non toxique acceptable du point de vue pharmaceutique.

**37.** Composé suivant la revendication 1, qui est le 4-[4,4-bis-(4-aminophényl)-butyl]-1H-imidazole ou un sel d'addition d'acide de celui-ci non toxique acceptable du point de vue pharmaceutique.

**38.** Composé suivant la revendication 1, qui est le 4-[4-(4-éthylphényl)-4-phénylbutyl]-1H-imidazole ou un sel d'addition d'acide de celui-ci non toxique acceptable du point de vue pharmaceutique.

**39.** Composition pharmaceutique comprenant un imidazole substitué suivant l'une quelconque des revendications 1 à 38, et un support acceptable du point de vue pharmaceutique.

**40.** Dérivé d'imidazole suivant l'une quelconque des revendications 1 à 39, ou un sel d'addition non toxique utile en thérapie en tant qu'agent inhibant une aromatase.

**41.** 4-(5)-Imidazole substitué de formule :

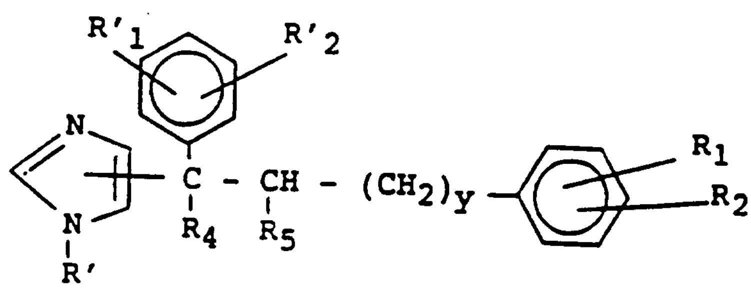

(Ib)

ou sel d'addition d'acide de celui-ci non toxique acceptable du point de vue pharmaceutique, dans laquelle $R_1$, $R_2$, $R'_1$ et $R'_2$, qui peuvent être identiques ou différents, sont un atome d'hydrogène, un groupe $CH_3$, $C_2H_5$, $C_3H_7$, $OCH_3$, OH, $CH_2OH$, $NO_2$, $NH_2$, CN, $CF_3$, $CHF_2$, $CH_2F$ ou un atome d'halogène; R' est un atome d'hydrogène ou un groupe

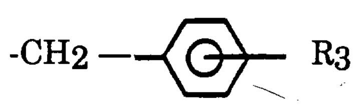

dans lequel $R_3$ est un atome d'hydrogène, d'halogène ou un groupe $CH_3$; $R_4$ est un atome d'hydrogène ou un groupe OH et $R_5$ est un atome d'hydrogène, ou $R_4$ et $R_5$ forment ensemble une liaison et y est 0 à 4.

**42.** Imidazole substitué suivant la revendication 41, dans lequel $R_4$, $R_5$ et R' sont chacun un atome d'hydrogène.

**43.** Imidazole substitué suivant les revendications 41 ou 42, dans lequel $R_1$, $R_2$ et $R'_2$ sont chacun un atome d'hydrogène et $R'_1$, qui est tel que défini dans la revendication 41, est en position para du groupe phényle.

**44.** Imidazole substitué suivant la revendication 43, dans lequel $R'_1$ est un atome de fluor.

**45.** Imidazole substitué suivant les revendications 41 ou 42, dans lequel $R_2$ et $R'_2$ sont chacun un atome d'hydrogène et $R_1$, qui est tel que défini dans la revendication 41, est en position ortho, méta ou para du groupe phényle et $R'_1$, qui est tel que défini dans la revendication 41, est en position para du groupe phényle.

**46.** Imidazole substitué suivant la revendication 45, dans lequel $R_2$ et $R'_2$ sont chacun un atome d'hydrogène et $R_1$ et $R'_1$, qui sont tels que définis dans la revendication 41, sont tous deux en position

para du groupe phényle.

**47.** Imidazole substitué suivant la revendication 46, dans lequel $R_1$ et $R'_1$ sont chacun un atome de fluor.

**48.** Imidazole substitué suivant la revendication 46, dans lequel $R_1$ est un groupe $CH_3$ et $R'_1$ est un atome de fluor.

**49.** Imidazole substitué suivant les revendications 41 ou 42, dans lequel $R'_2$ est un atome d'hydrogène, $R'_1$, qui est tel que défini dans la revendication 41, est en position para du groupe phényle et $R_1$ et $R_2$, qui sont tels que définis dans la revendication 41, sont en positions 3 et 5 du groupe phényle.

**50.** Imidazole substitué suivant les revendications 41 ou 42, dans lequel $R'_2$ est un atome d'hydrogène, $R'_1$, qui est tel que défini dans la revendication 41, est en position para du groupe phényle et $R_1$ et $R_2$, qui sont tels que définis dans la revendication 41, sont en positions 2 et 6 du groupe phényle.

**51.** Composé suivant la revendication 41, qui est le 4-[1-(4-fluorophényl)-5-phénylpentyl]-1H-imidazole ou un sel d'addition d'acide de celui-ci non toxique acceptable du point de vue pharmaceutique.

**52.** Composé suivant la revendication 41, qui est le 4-[1-(4-fluorophényl)-5-(2-méthylphényl)-pentyl]-1H-imidazole ou un sel d'addition d'acide de celui-ci non toxique acceptable du point de vue pharmaceutique.

**53.** Composé suivant la revendication 41, qui est le 4-[1-(4-fluorophényl)-5-(3-méthylphényl)-pentyl]-1H-imidazole ou un sel d'addition d'acide de celui-ci non toxique acceptable du point de vue pharmaceutique.

**54.** Composé suivant la revendication 41, qui est le 4-[1-(4-fluorophényl)-5-(4-méthylphényl)-pentyl]-1H-imidazole ou un sel d'addition d'acide de celui-ci non toxique acceptable du point de vue pharmaceutique.

**55.** Composé suivant la revendication 41, qui est le 4-[5-(3,5-diméthylphényl)-1-(4-fluorophényl)-pentyl]-1H-imidazole ou un sel d'addition d'acide de celui-ci non toxique acceptable du point de vue pharmaceutique.

**56.** Composé suivant la revendication 41, qui est le 4-(1,5-diphénylpentyl)-1H-imidazole ou un sel d'addition d'acide de celui-ci non toxique acceptable du point de vue pharmaceutique.

**57.** Composé suivant la revendication 41, qui est le 4-(1,3-diphénylpropyl)-1H-imidazole ou un sel d'addition d'acide de celui-ci non toxique acceptable du point de vue pharmaceutique.

**58.** Composé suivant la revendication 41, qui est le 1-benzyl-5-(1,3-diphénylpropyl)-1H-imidazole ou un sel d'addition d'acide de celui-ci non toxique acceptable du point de vue pharmaceutique.

**59.** Composé suivant la revendication 41, qui est le 4-[1-(4-fluorophényl)-3-phénylpropyl]-1H-imidazole ou un sel d'addition d'acide de celui-ci non toxique acceptable du point de vue pharmaceutique.

**60.** Composé suivant la revendication 41, qui est le 4-[1-(4-fluorophényl)-4-phénylbutyl]-1H-imidazole ou un sel d'addition d'acide de celui-ci non toxique acceptable du point de vue pharmaceutique.

**61.** Composé suivant la revendication 41, qui est le 4-[1-(4-fluorophényl)-5-(3-méthoxyphényl)-pentyl]-1H-imidazole ou un sel d'addition d'acide de celui-ci non toxique acceptable du point de vue pharmaceutique.

**62.** Composé suivant la revendication 41, qui est le 4-[1-(4-fluorophényl)-5-(4-méthoxyphényl)-pentyl]-1H-imidazole ou un sel d'addition d'acide de celui-ci non toxique acceptable du point de vue pharmaceutique.

63. Composé suivant la revendication 41, qui est le 4-[1-(4-fluorophényl)-5-(2,6-diméthylphényl)-pentyl)-1H-imidazole ou un sel d'addition d'acide de celui-ci non toxique acceptable du point de vue pharmaceutique.

64. Composé suivant la revendication 41, qui est le 4-[1,3-bis-(4-fluorophényl)-propyl]-1H-imidazole ou un sel d'addition d'acide de celui-ci non toxique acceptable du point de vue pharmaceutique.

65. Composé suivant la revendication 41, qui est le 4-[1,4-bis-(4-fluorophényl)-butyl]-1H-imidazole ou un sel d'addition d'acide de celui-ci non toxique acceptable du point de vue pharmaceutique.

66. Composition pharmaceutique comprenant un imidazole substitué suivant l'une quelconque des revendications 41 à 65, et un support acceptable du point de vue pharmaceutique.

67. Dérivé d'imidazole suivant l'une quelconque des revendications 41 à 66, ou sel d'addition d'acide non toxique de celui-ci utilisable en thérapie comme agent inhibant une aromatase.

**Revendications pour les Etats contractants suivants : ES, GR**

1. Procédé pour la préparation d'un 4-(5)-imidazole substitué de formule :

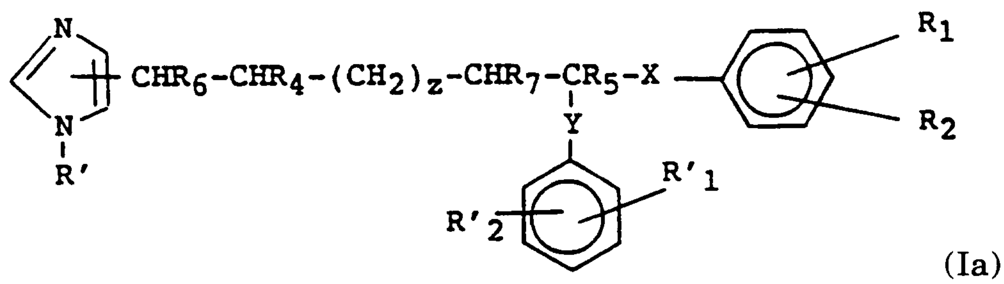

(Ia)

ou d'un sel d'addition d'acide de celui-ci non toxique acceptable du point de vue pharmaceutique, dans laquelle $R_1$, $R_2$, $R'_1$ et $R'_2$, qui peuvent être identiques ou différents, sont un atome d'hydrogène, un groupe $CH_3$, $C_2H_5$, $C_3H_7$, $OCH_3$, $NO_2$, $CF_3$, $CHF_2$, $CH_2F$ ou un atome d'halogène; R' est un atome d'hydrogène ou un groupe

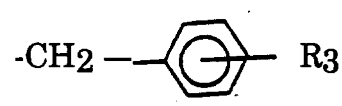

dans lequel $R_3$ est un atome d'hydrogène, d'halogène ou un groupe $CH_3$; $R_4$ est un atome d'hydrogène, $R_5$ est un atome d'hydrogène ou un groupe OH, $R_6$ est un atome d'hydrogène ou un groupe OH et $R_7$ est un atome d'hydrogène, ou $R_4$ et $R_6$ forment ensemble une liaison ou $R_5$ et $R_7$ forment ensemble une liaison; X et Y, qui peuvent être identiques ou différents, sont une liaison, un groupe alkyle en $C_{1-2}$ à chaîne droite ou le groupe alcényle correspondant, et z est 0 à 2,
qui comprend la réaction d'un dérivé de 4(5)-imidazole de formule :

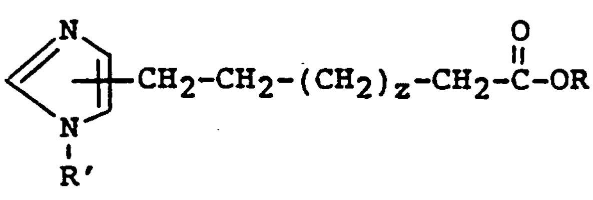

dans laquelle R' et z sont tels que définis plus haut et R est un groupe alkyle, avec un réactif de Grignard de formule :

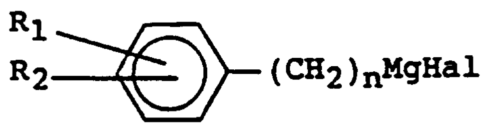

dans laquelle $R_1$ et $R_2$ sont tels que définis plus haut et n est 0 à 2, pour donner un composé de formule (Ia) qui est :

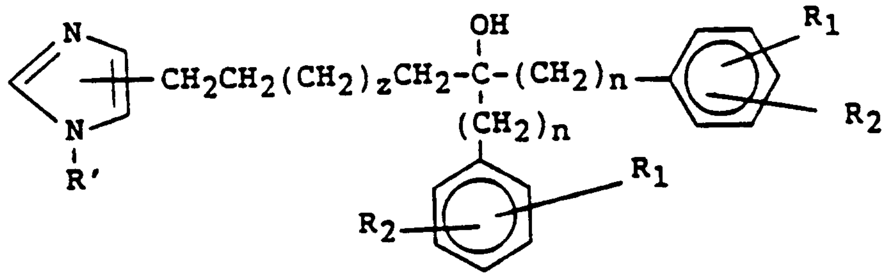

2. Procédé pour la préparation d'un composé suivant la revendication 1 ou d'un sel d'addition d'acide de celui-ci non toxique acceptable du point de vue pharmaceutique, qui comprend la réaction d'un dérivé de 4(5)-imidazole de formule :

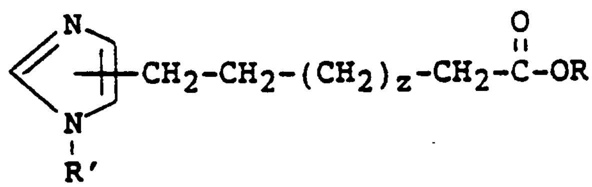

dans laquelle R' et z sont tels que définis plus haut et R est un groupe alkyle, avec un réactif de Grignard de formule :

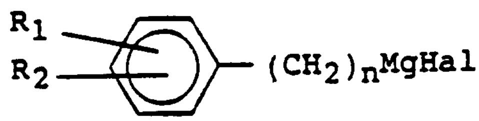

dans laquelle $R_1$ et $R_2$ sont tels que définis plus haut et n est 0 à 2, pour donner un composé de formule qui est :

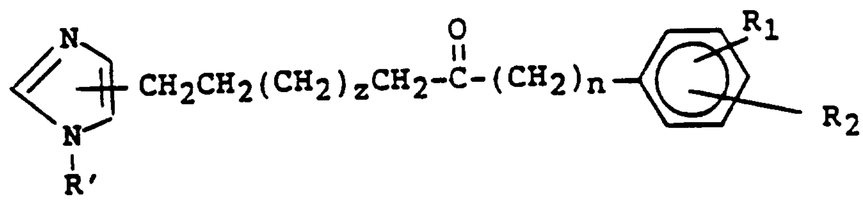

qui est soumis à une réaction supplémentaire avec un autre réactif de Grignard de formule :

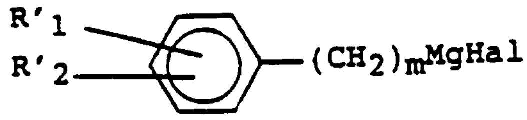

dans laquelle R'1 et R'2 sont tels que définis plus haut et m est 0 à 2, pour donner un composé de formule (Ia) qui est :

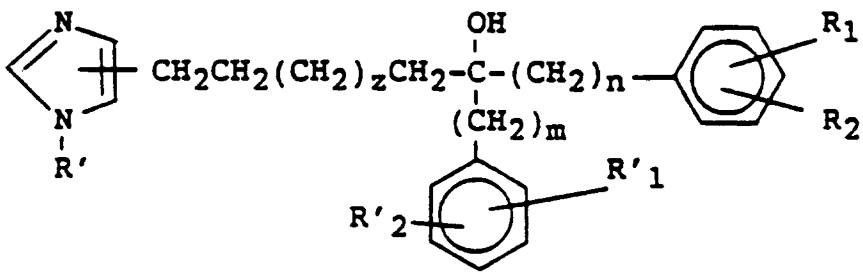

3. Procédé suivant la revendications 1 ou 2 pour la préparation d'un composé suivant la revendication 1 ou d'un sel d'addition d'acide de celui-ci non toxique acceptable du point de vue pharmaceutique, dans lequel un amide de formule :

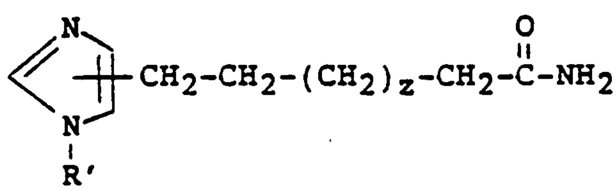

dans laquelle R' et z sont tels que définis plus haut, est utilisé comme matière de départ à la place de l'ester correspondant.

4. Procédé suivant la revendications 1 ou 2 pour la préparation d'un composé suivant la revendication 1 ou d'un sel d'addition d'acide de celui-ci non toxique acceptable du point de vue pharmaceutique, dans lequel un nitrile de formule :

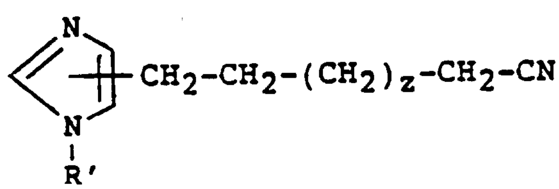

dans laquelle R' et z sont tels que définis plus haut, est utilisé comme matière de départ à la place de l'ester correspondant.

5. Procédé pour la préparation d'un composé suivant la revendication 1 ou d'un sel d'addition d'acide de celui-ci non toxique acceptable du point de vue pharmaceutique, qui comprend la réaction d'un 4(5)-imidazole aldéhyde de formule :

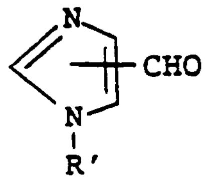

dans laquelle R' est tel que défini plus haut, avec un réactif de Grignard de formule :

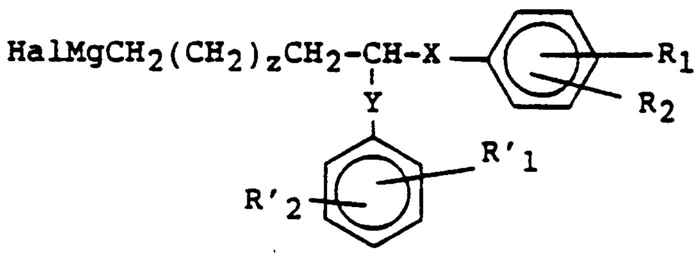

dans laquelle $R_1$, $R_2$, $R'_1$, $R'_2$, X, Y et z sont tels que définis plus haut, pour donner un composé de formule (Ia) qui est :

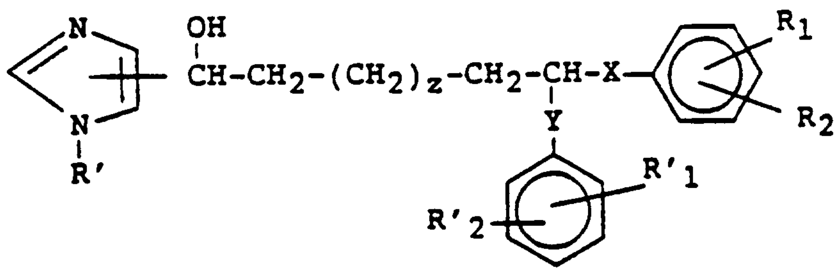

6. Procédé pour la préparation d'un 4-(5)-imidazole substitué de formule :

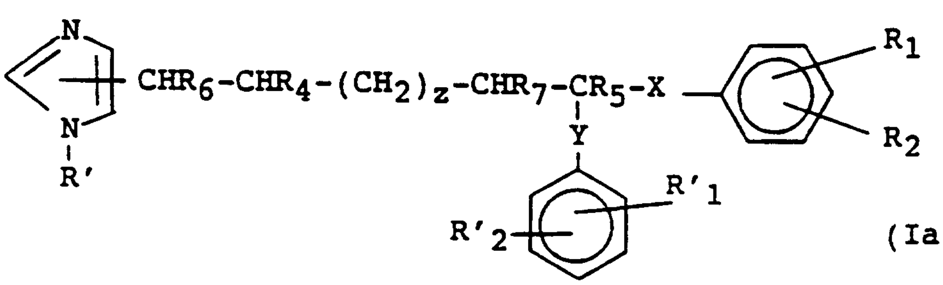

(Ia)

ou d'un sel d'addition d'acide de celui-ci non toxique acceptable du point de vue pharmaceutique, dans laquelle $R_1$, $R_2$, $R'_1$ et $R'_2$, qui peuvent être identiques ou différents, sont un atome d'hydrogène, un groupe $CH_3$, $C_2H_5$, $C_3H7$, $OCH_3$, OH, $CH_2OH$, $NO_2$, $NH_2$, CN, $CF_3$, $CHF_2$, $CH_2F$ ou un atome d'halogène; R' est un atome d'hydrogène ou un groupe

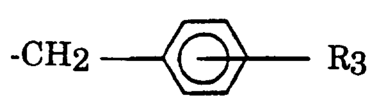

dans lequel $R_3$ est un atome d'hydrogène, d'halogène ou un groupe $CH_3$; $R_4$ est un atome d'hydrogène, $R_5$ est un atome d'hydrogène ou un groupe OH, $R_6$ est un atome d'hydrogène ou un groupe OH et $R_7$ est un atome d'hydrogène, ou $R_4$ et $R_6$ forment ensemble une liaison ou $R_5$ et $R_7$ forment ensemble une liaison; X et Y, qui peuvent être identiques ou différents, sont une liaison, un groupe alkyle en $C_{1-2}$ à chaîne ou le groupe alcényle correspondant et z est 0 à 2,
qui comprend la réaction d'un dérivé de 4(5)-imidazole de formule :

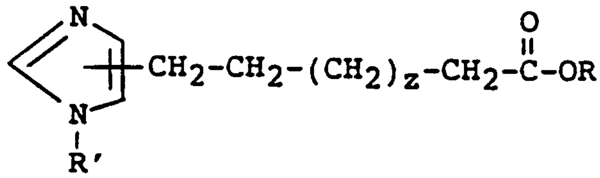

dans laquelle R' et z sont tels que définis plus haut et R est un groupe alkyle, avec un réactif de Grignard de formule :

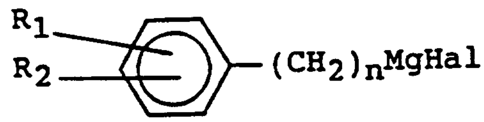

dans laquelle n est 0 à 2 et $R_1$ et/ou $R_2$ sont un groupe $OCH_2Ph$ ou OTHP, pour donner un composé de formule :

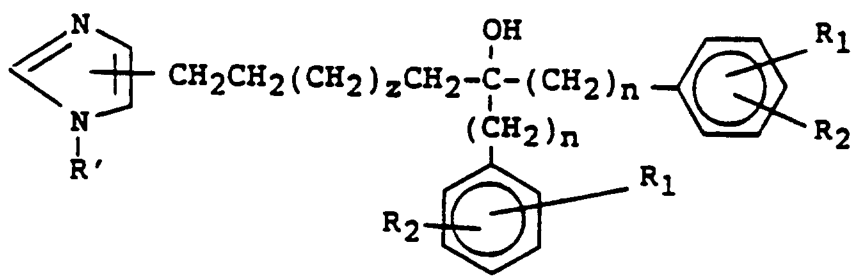

dans laquelle $R_1$ et/ou $R_2$ sont un groupe $OCH_2Ph$ ou OTHP, qui est de plus hydrogéné pour donner un composé de formule (Ia) qui est :

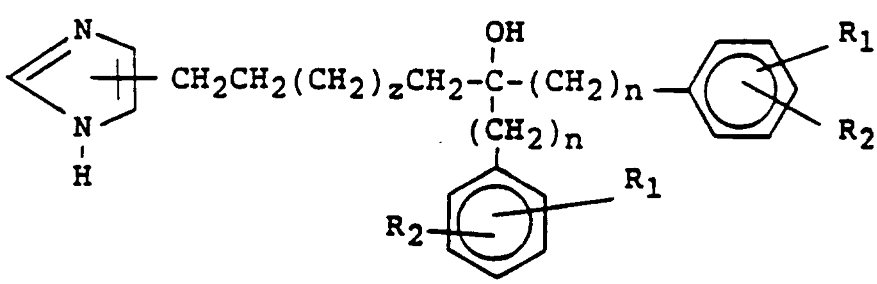

dans laquelle z et n sont tels que définis plus haut et $R_1$ et/ou $R_2$ sont un groupe OH et le groupe $R_1$ ou $R_2$ qui n'est pas un groupe OH est un atome d'hydrogène, un groupe $CH_3$, $C_2H_5$, $C_3H_7$, $OCH_3$, $NO_2$, $CF_3$, $CHF_2$, $CH_2F$ ou un atome d'halogène.

**7.** Procédé pour la préparation d'un composé suivant la revendication 6 ou d'un sel d'addition d'acide de celui-ci non toxique acceptable du point de vue pharmaceutique, qui comprend la réaction d'un dérivé

de 4(5)-imidazole de formule :

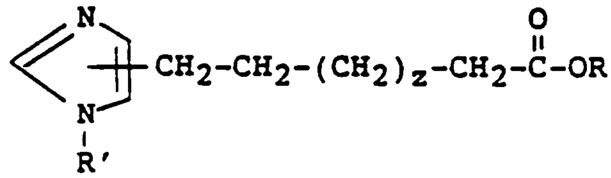

dans laquelle R' et z sont tels que définis plus haut et R est un groupe alkyle, avec un réactif de Grignard de formule :

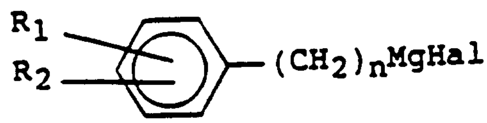

dans laquelle n est 0 à 2 et

i) $R_1$ et/ou $R_2$ sont un groupe $OCH_2Ph$ ou OTHP ou

ii) aucun des $R_1$ ou $R_2$ n'est un groupe $OCH_2Ph$ ou OTHP,

pour donner un composé de formule

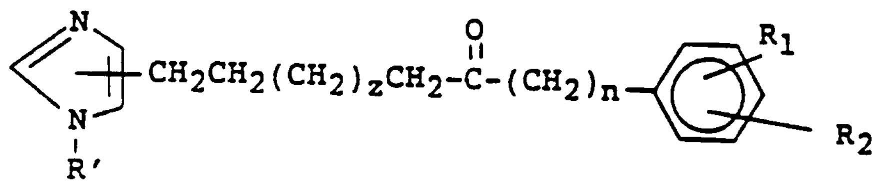

qui est soumis à une réaction supplémentaire avec un autre réactif de Grignard de formule :

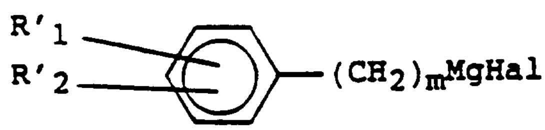

dans laquelle m est 0 à 2 et

iii) $R'_1$ et/ou $R'_2$ sont un groupe $OCH_2Ph$ ou OTHP ou

iv) aucun des $R'_1$ ou $R'_2$ n'est un groupe $OCH_2Ph$ ou OTHP,

à condition que ii) et iv) ne soient pas valables simultanément, pour donner un composé de formule

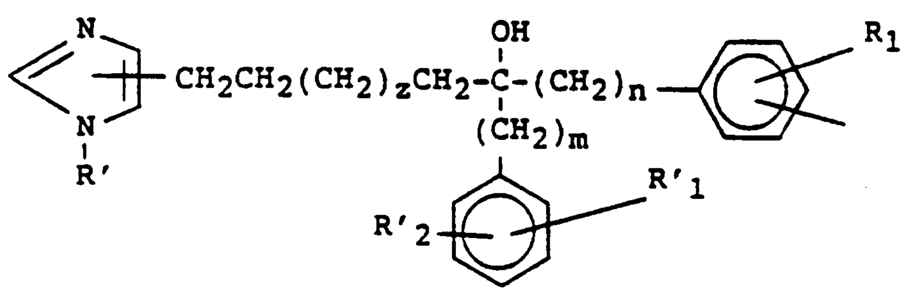

dans laquelle l'un ou plusieurs des substituants $R_1$, $R_2$, $R'_1$ et $R'_2$ sont un groupe $OCH_2Ph$ ou OTHP,

qui est de plus hydrogéné pour donner un composé de formule (Ia) qui est :

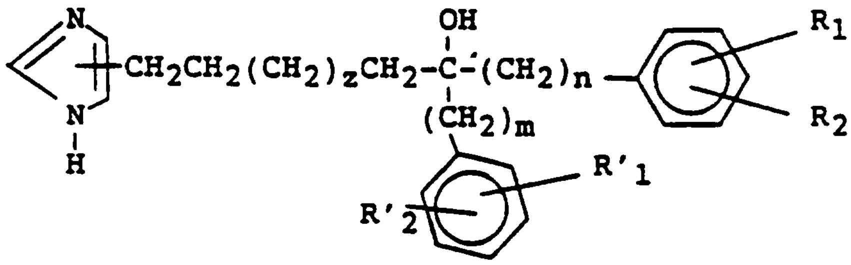

dans laquelle z est 0 à 2, m et n, qui peuvent être identiques ou différents, sont 0 à 2, et l'un ou plusieurs des substituants $R_1$, $R_2$, $R'_1$ et $R'_2$ sont un groupe OH et les substituants qui ne sont pas un groupe OH sont un atome d'hydrogène, un groupe $CH_3$, $C_2H_5$, $C_3H_7$, $OCH_3$, $NO_2$, $CF_3$, $CHF_2$, $CH_2F$ ou un atome d'halogène.

8. Procédé suivant les revendications 6 ou 7 pour la préparation d'un composé de formule (Ia) suivant la revendication 6 ou d'un sel d'addition d'acide de celui-ci non toxique acceptable du point de vue pharmaceutique, dans lequel un amide de formule :

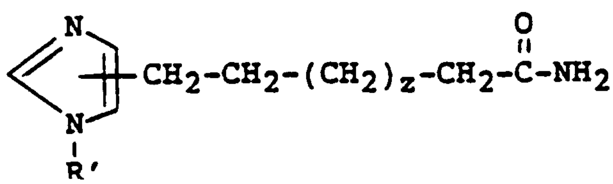

dans laquelle R' et z sont tels que définis plus haut, est utilisé comme matière de départ à la place de l'ester correspondant.

9. Procédé suivant les revendications 6 ou 7 pour la préparation d'un composé de formule (Ia) suivant la revendication 6 ou d'un sel d'addition d'acide de celui-ci non toxique acceptable du point de vue pharmaceutique, dans lequel un nitrile de formule :

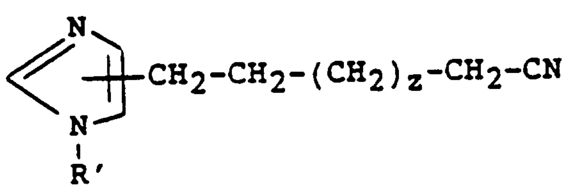

dans laquelle R' et z sont tels que définis plus haut, est utilisé comme matière de départ à la place de l'ester correspondant.

10. Procédé pour la préparation d'un composé suivant la revendication 6 ou d'un sel d'addition d'acide de celui-ci non toxique acceptable du point de vue pharmaceutique, qui comprend la réaction d'un 4(5)-imidazole aldéhyde de formule :

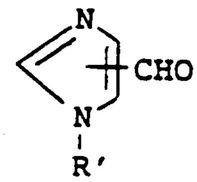

dans laquelle R' est tel que défini plus haut, avec un réactif de Grignard de formule :

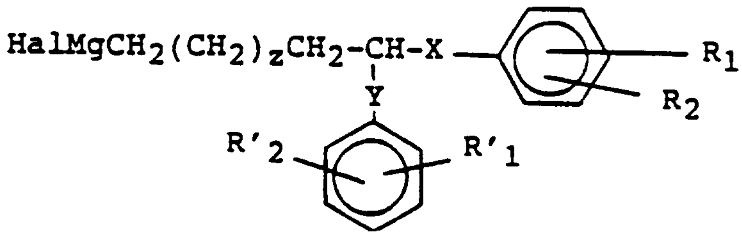

dans laquelle X, Y et z sont tels que définis plus haut et l'un ou plusieurs des substituants $R_1$, $R_2$, $R'_1$ et $R'_2$ sont un groupe $OCH_2Ph$ ou OTHP, pour donner un composé de formule

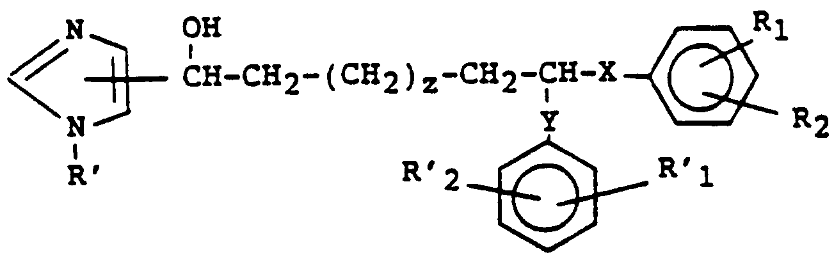

dans laquelle l'un ou plusieurs des substituants $R_1$, $R_2$, $R'_1$ et $R'_2$ sont un groupe $OCH_2Ph$ ou OTHP, qui est de plus hydrogéné pour donner un composé de formule (Ia) qui est :

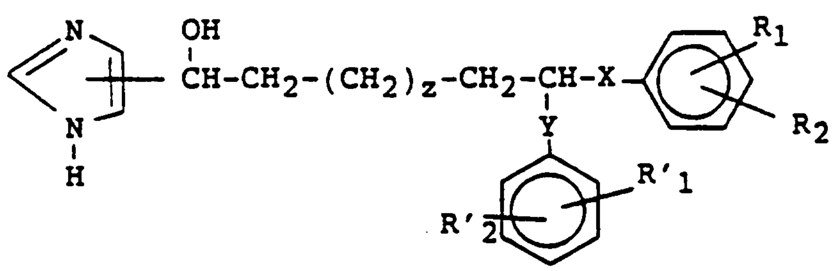

dans laquelle X, Y et z sont tels que définis plus haut et l'un ou plusieurs des substituants $R_1$, $R_2$, $R'_1$ et $R'_2$ sont un groupe OH et les substituants qui ne sont pas un groupe OH sont un atome d'hydrogène, un groupe $CH_3$, $C_2H_5$, $C_3H_7$, $OCH_3$, $NO_2$, $CF_3$, $CHF_2$, $CH_2F$ ou un atome d'halogène.

**11.** Procédé pour la préparation d'un composé suivant la revendication 6 ou d'un sel d'addition d'acide de celui-ci non toxique acceptable du point de vue pharmaceutique, qui comprend la désalkylation d'un composé de formule (Ia) :

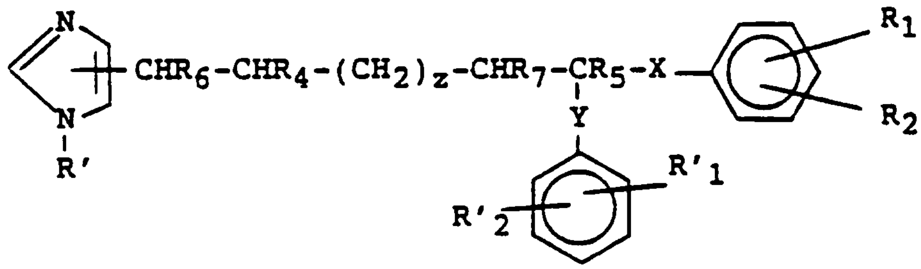

dans laquelle R', $R_4$, $R_5$, $R_6$, $R_7$, X, Y et z sont tels que définis plus haut et l'un ou plusieurs des substituants $R_1$, $R_2$, $R'_1$ et $R'_2$ sont un groupe $OCH_3$ pour donner un composé de formule (Ia) dans laquelle l'un ou plusieurs des substituants $R_1$, $R_2$, $R'_1$ et $R'_2$ sont un groupe OH.

**12.** Procédé pour la préparation d'un composé suivant la revendication 6 ou d'un sel d'addition d'acide de celui-ci non toxique acceptable du point de vue pharmaceutique, qui comprend l'hydrolyse d'un composé de formule (Ia) :

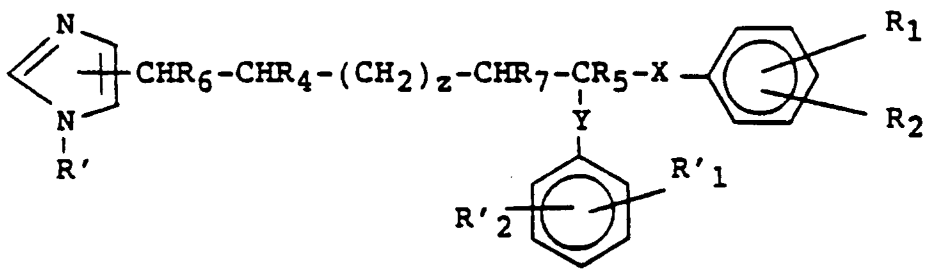

dans laquelle R', $R_4$, $R_5$, $R_6$, $R_7$, X, Y et z sont tels que définis plus haut et l'un ou plusieurs des substituants $R_1$, $R_2$, $R'_1$ et $R'_2$ sont un groupe CN, puis la réduction pour donner un composé de formule (Ia) dans laquelle l'un ou plusieurs des substituants $R_1$, $R_2$, $R'_1$ et $R'_2$ sont un groupe $CH_2OH$.

**13.** Procédé pour la préparation d'un composé suivant la revendication 6 ou d'un sel d'addition d'acide de celui-ci non toxique acceptable du point de vue pharmaceutique, qui comprend la diazotation d'un composé de formule (Ia) :

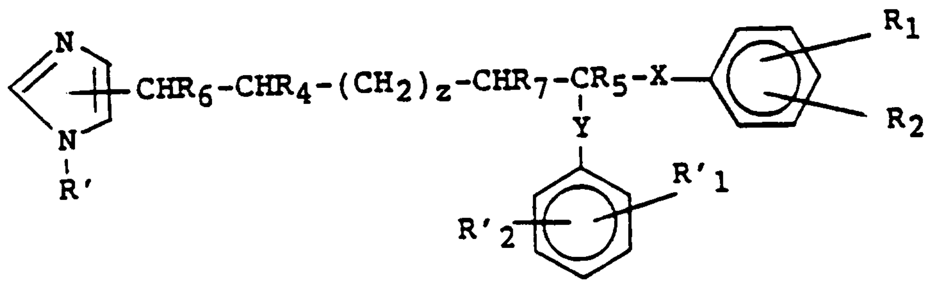

dans laquelle R', $R_4$, $R_5$, $R_6$, $R_7$, X, Y et z sont tels que définis plus haut et l'un ou plusieurs des substituants $R_1$, $R_2$, $R'_1$ et $R'_2$ sont un groupe $NH_2$ pour donner un composé de formule (Ia) dans laquelle l'un ou plusieurs des substituants $R_1$, $R_2$, $R'_1$ et $R'_2$ sont un groupe CN.

**14.** Procédé pour la préparation d'un composé suivant la revendication 6 ou d'un sel d'addition d'acide de celui-ci non toxique acceptable du point de vue pharmaceutique, qui comprend l'hydrogénation d'un composé de formule (Ia) :

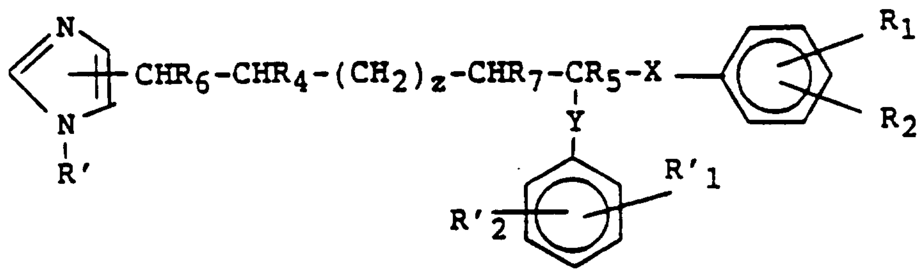

dans laquelle R', $R_4$, $R_5$, $R_6$, $R_7$, X, Y et z sont tels que définis plus haut et l'un ou plusieurs des substituants $R_1$, $R_2$, $R'_1$ et $R'_2$ sont un groupe $NO_2$ pour donner un composé de formule (Ia) dans laquelle R' est un atome d'hydrogène, $R_4$, $R_5$, $R_6$, $R_7$, X, Y et z sont tels que définis plus haut et l'un ou plusieurs des substituants $R_1$, $R_2$, $R'_1$ et $R'_2$ sont un groupe $NH_2$.

**15.** Procédé pour la préparation d'un composé suivant la revendication 6 ou d'un sel d'addition d'acide de celui-ci non toxique acceptable du point de vue pharmaceutique, qui comprend la déshydratation d'un composé de formule :

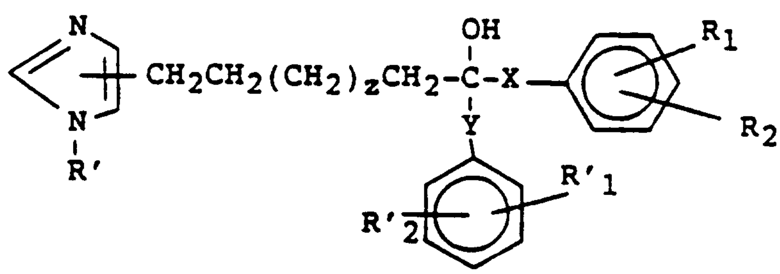

pour donner un composé de formule (Ia) qui est :

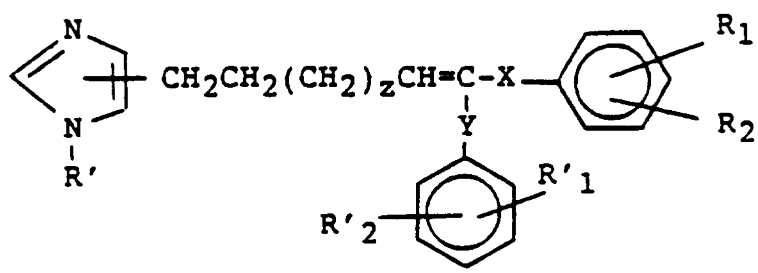

dans laquelle R', $R_1$, $R_2$, $R'_1$, $R'_2$, X, Y et z sont tels que définis plus haut.

**16.** Procédé pour la préparation d'un composé suivant la revendication 6 ou d'un sel d'addition d'acide de celui-ci non toxique acceptable du point de vue pharmaceutique, qui comprend la réaction d'un composé de formule :

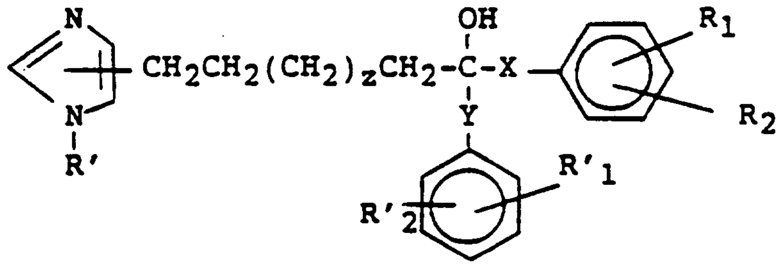

par réaction de transfert d'hydrogène, pour donner un composé de formule (Ia) qui est :

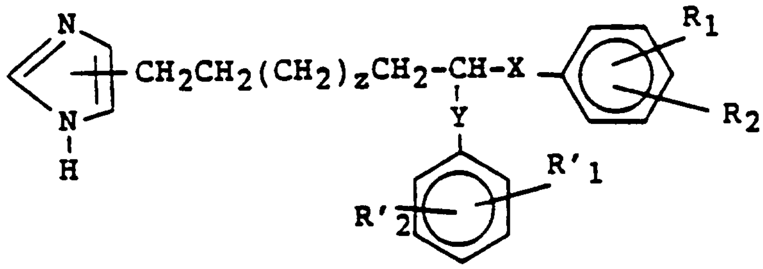

dans laquelle $R_1$, $R_2$, $R'_1$, $R'_2$, X, Y et z sont tels que définis plus haut.

17. Procédé pour la préparation d'un composé suivant la revendication 6 ou d'un sel d'addition d'acide de celui-ci non toxique acceptable du point de vue pharmaceutique, qui comprend l'hydrogénation d'un composé de formule :

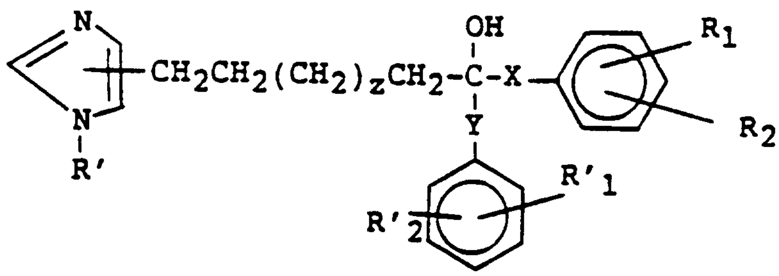

dans laquelle R' est un groupe benzyle substitué ou non substitué, et $R_1$, $R_2$, $R'_1$, $R'_2$, X, Y et z sont tels que définis plus haut, pour donner un composé de formule (Ia) qui est :

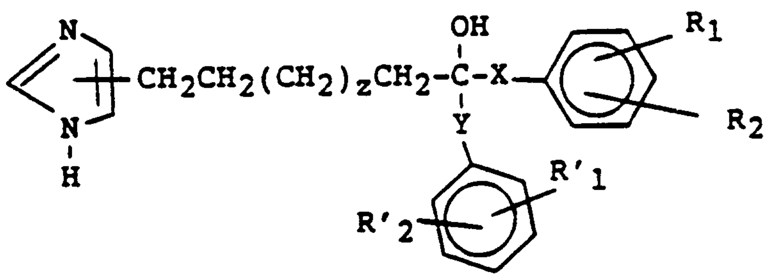

18. Procédé pour la préparation d'un composé suivant la revendication 6 ou d'un sel d'addition d'acide de celui-ci non toxique acceptable du point de vue pharmaceutique, qui comprend la réaction d'un composé de formule :

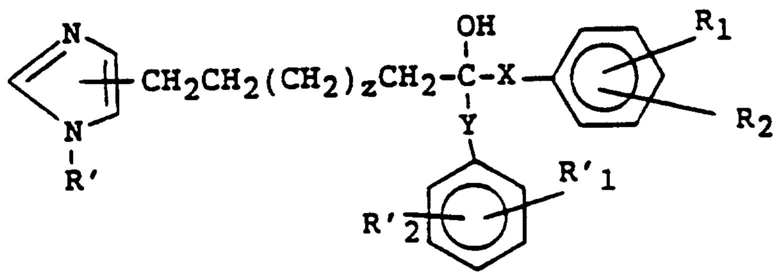

dans laquelle R' est un groupe benzyle substitué ou non substitué, et $R_1$, $R_2$, $R'_1$, $R'_2$, X, Y et z sont tels que définis plus haut, par une réaction de transfert d'hydrogène pour donner un composé de formule (Ia) qui est :

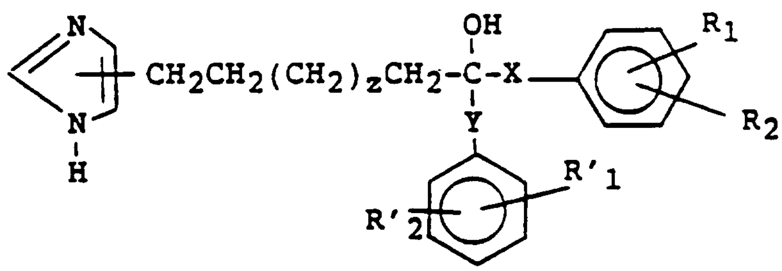

**19.** Procédé pour la préparation d'un composé suivant la revendication 6 ou d'un sel d'addition d'acide de celui-ci non toxique acceptable du point de vue pharmaceutique, qui comprend l'hydrogénation d'un composé de formule :

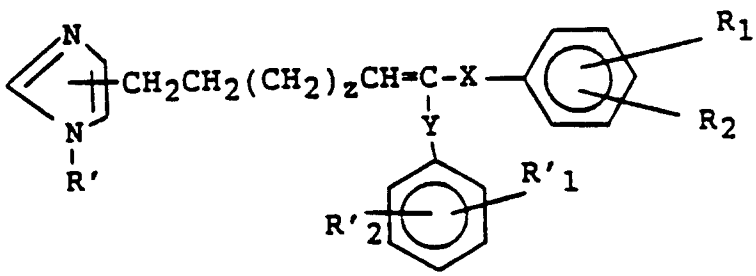

pour donner un composé de formule (Ia) qui est :

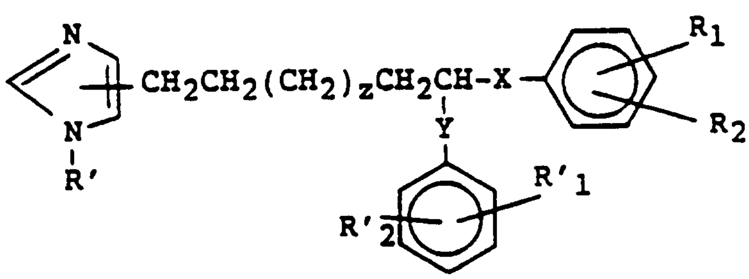

dans laquelle R, $R_1$, $R_2$, $R'_1$, $R'_2$, X, Y et z sont tels que définis plus haut.

**20.** Procédé pour la préparation d'un composé suivant la revendication 6 ou d'un sel d'addition d'acide de celui-ci non toxique acceptable du point de vue pharmaceutique, qui comprend l'hydrogénation d'un composé de formule :

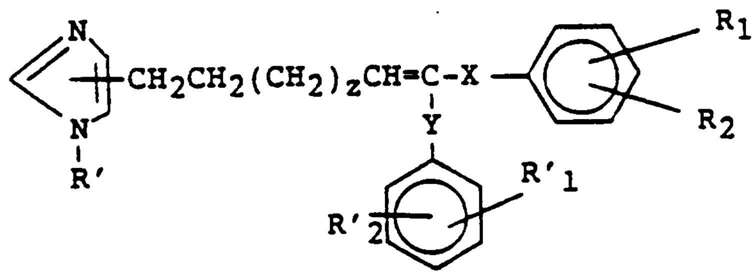

pour donner un composé de formule (Ia) qui est :

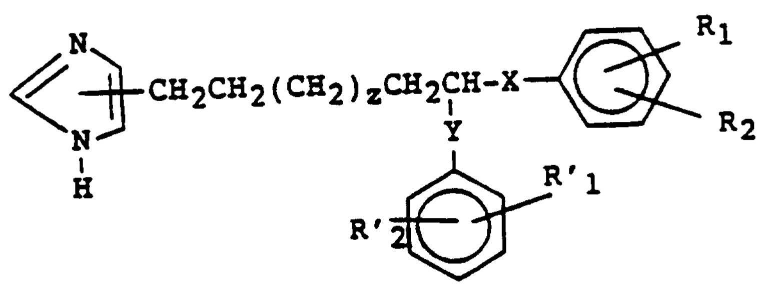

dans laquelle $R_1$, $R_2$, $R'_1$, $R'_2$, X, Y et z sont tels que définis plus haut.

**21.** Procédé pour la préparation d'un composé suivant la revendication 6 ou d'un sel d'addition d'acide de celui-ci non toxique acceptable du point de vue pharmaceutique, qui comprend la réaction d'un composé de formule :

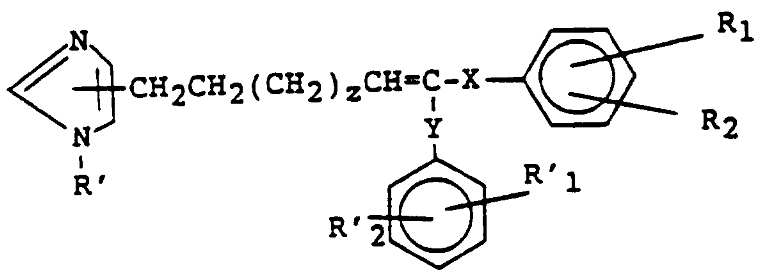

par une réaction de transfert d'hydrogène pour donner un composé de formule (Ia) qui est :

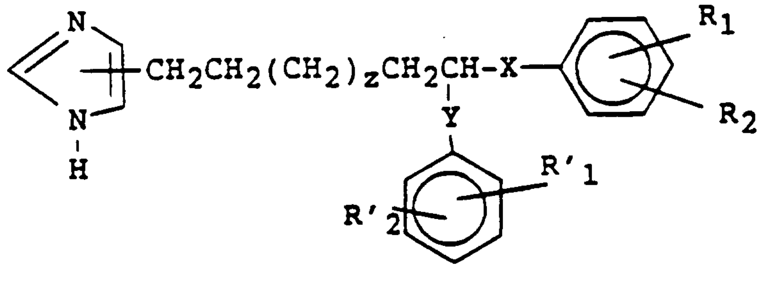

dans laquelle $R_1$, $R_2$, $R'_1$, $R'_2$, X, Y et z sont tels que définis plus haut.

22. Procédé pour la préparation d'un composé suivant la revendication 6 ou d'un sel d'addition d'acide de celui-ci non toxique acceptable du point de vue pharmaceutique, qui comprend la déshydratation d'un composé de formule :

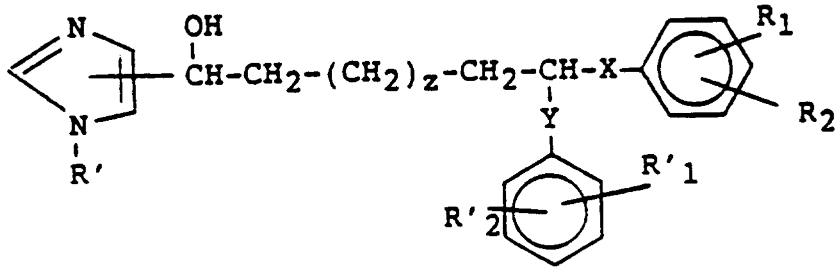

pour donner un composé de formule (Ia) qui est :

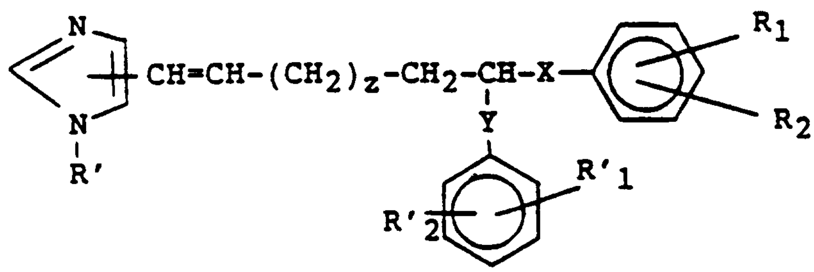

dans laquelle R', $R_1$, $R_2$, $R'_1$, $R'_2$, X, Y et z sont tels que définis plus haut.

23. Procédé pour la préparation d'un composé suivant la revendication 6 ou d'un sel d'addition d'acide de celui-ci non toxique acceptable du point de vue pharmaceutique, qui comprend la réaction d'un composé de formule :

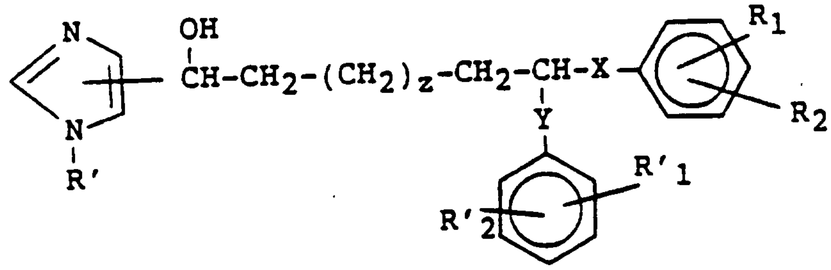

par réaction de transfert d'hydrogène pour donner un composé de formule (Ia) qui est :

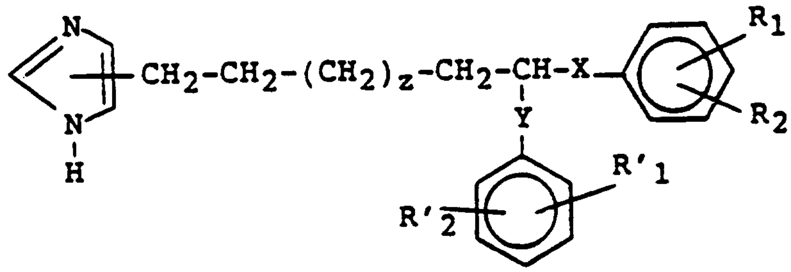

dans laquelle $R_1$, $R_2$, $R'_1$, $R'_2$, X, Y et z sont tels que définis plus haut.

**24.** Procédé pour la préparation d'un composé suivant la revendication 6 ou d'un sel d'addition d'acide de celui-ci non toxique acceptable du point de vue pharmaceutique, qui comprend l'hydrogénation d'un composé de formule :

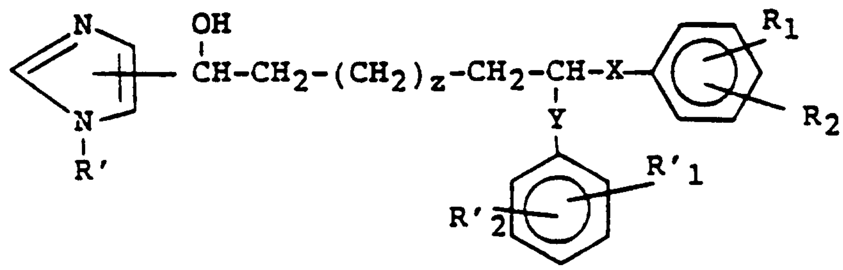

dans laquelle R' est un groupe benzyle substitué ou non substitué et $R_1$, $R_2$, $R'_1$, $R'_2$, X, Y et z sont tels que définis plus haut, pour donner un composé de formule (Ia) qui est :

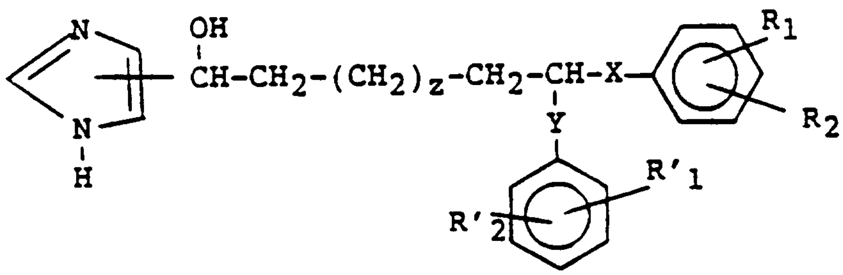

**25.** Procédé pour la préparation d'un composé suivant la revendication 6 ou d'un sel d'addition d'acide de celui-ci non toxique acceptable du point de vue pharmaceutique, qui comprend la réaction d'un composé de formule :

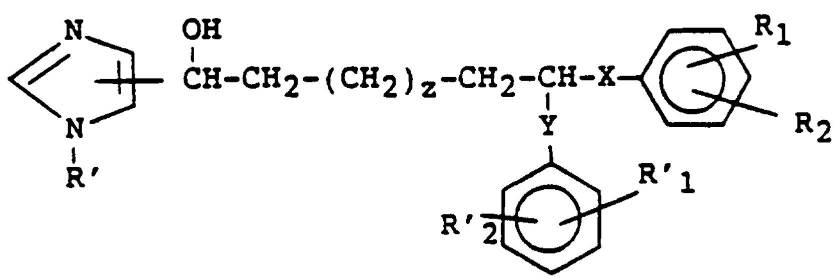

dans laquelle R' est un groupe benzyle substitué ou non substitué et $R_1$, $R_2$, $R'_1$, $R'_2$, X, Y et z sont tels que définis plus haut, par une réaction de transfert d'hydrogène, pour donner un composé de formule (Ia) qui est :

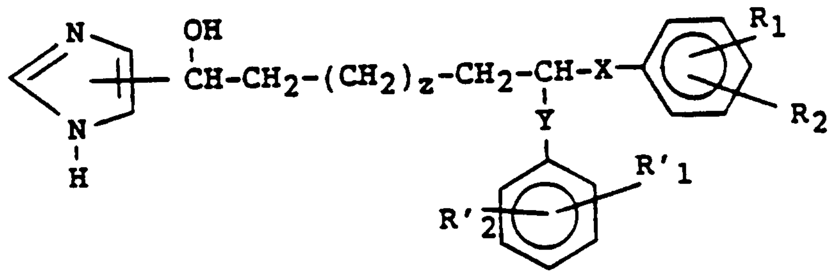

26. Procédé pour la préparation d'un composé suivant la revendication 6 ou d'un sel d'addition d'acide de celui-ci non toxique acceptable du point de vue pharmaceutique, qui comprend l'hydrogénation d'un composé de formule :

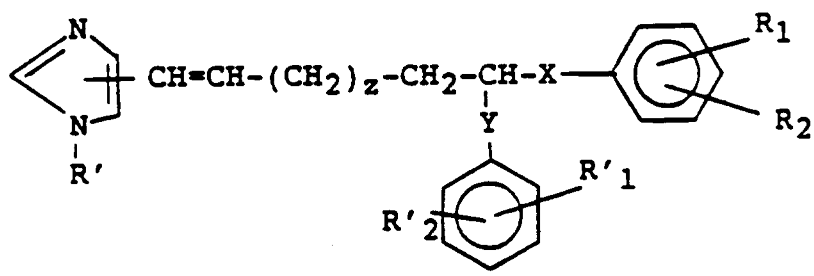

pour donner un composé de formule (Ia) qui est :

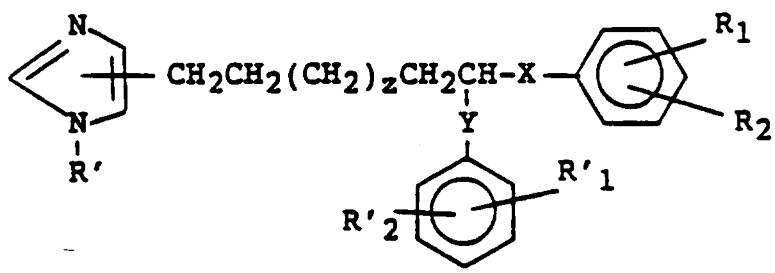

dans laquelle R, $R_1$, $R_2$, $R'_1$, $R'_2$, X, Y et z sont tels que définis plus haut.

27. Procédé pour la préparation d'un composé suivant la revendication 6 ou d'un sel d'addition d'acide de celui-ci non toxique acceptable du point de vue pharmaceutique, qui comprend l'hydrogénation d'un composé de formule :

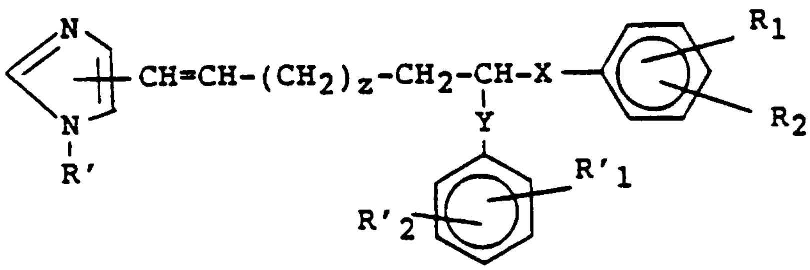

dans laquelle R', $R_1$, $R_2$ $R'_1$, $R'_2$, X, Y et z sont tels que définis plus haut, pour donner un composé de

formule (Ia) qui est :

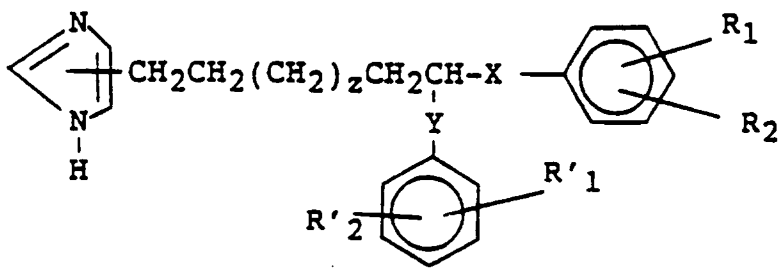

**28.** Procédé pour la préparation d'un composé suivant la revendication 6 ou d'un sel d'addition d'acide de celui-ci non toxique acceptable du point de vue pharmaceutique, qui comprend la réaction d'un composé de formule :

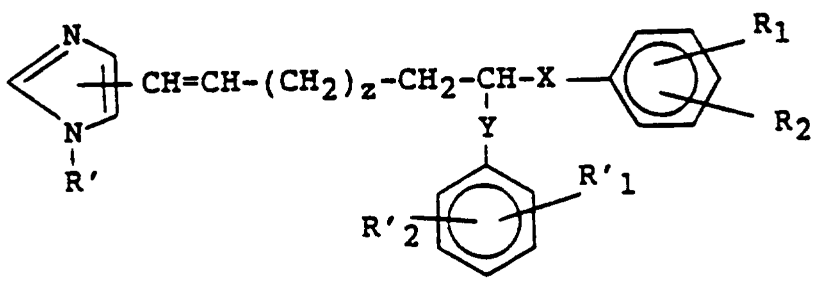

dans laquelle R', $R_1$, $R_2$, $R'_1$ $R'_2$, X, Y et z sont tels que définis plus haut, par une réaction de transfert d'hydrogène, pour donner un composé de formule (Ia) qui est :

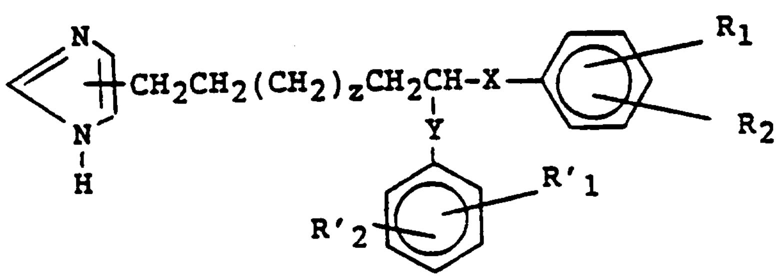

**29.** Procédé pour la préparation d'un composé suivant la revendication 6 ou d'un sel d'addition d'acide de celui-ci non toxique acceptable du point de vue pharmaceutique, qui comprend l'hydrogénation d'un composé de formule :

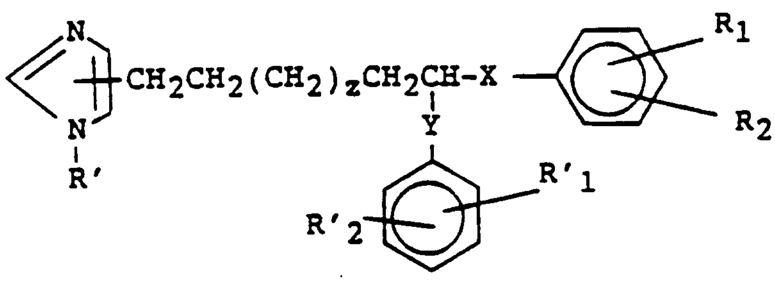

dans laquelle R' est un groupe benzyle substitué ou non substitué et $R_1$, $R_2$, $R'_1$, $R'_2$, X, Y et z sont tels que définis plus haut, pour donner un composé de formule (Ia) qui est :

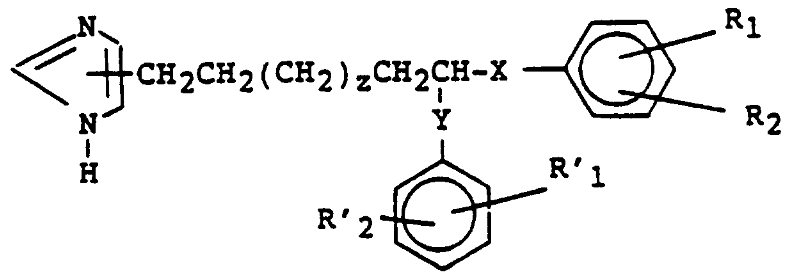

**30.** Procédé pour la préparation d'un composé suivant la revendication 6 ou d'un sel d'addition d'acide de celui-ci non toxique acceptable du point de vue pharmaceutique, qui comprend la réaction d'un composé de formule :

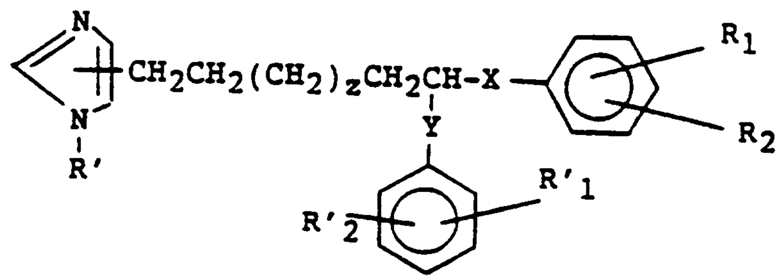

dans laquelle R' est un groupe benzyle substitué ou non substitué et $R_1$, $R_2$, $R'_1$, $R'_2$, X, Y et z sont tels que définis plus haut, par une réaction de transfert d'hydrogène, pour donner un composé de formule (Ia) qui est :

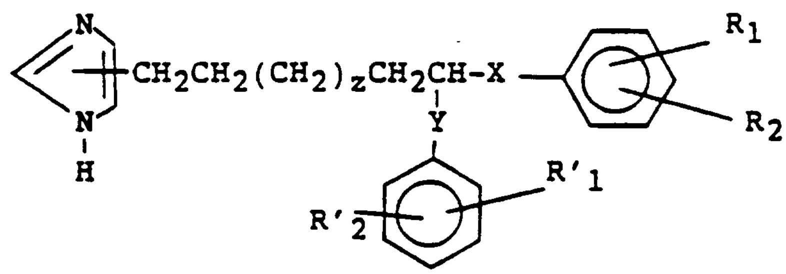

**31.** Procédé pour la préparation d'un composé suivant la revendication 6 ou d'un sel d'addition d'acide de celui-ci non toxique acceptable du point de vue pharmaceutique, qui comprend la réaction d'un composé de formule :

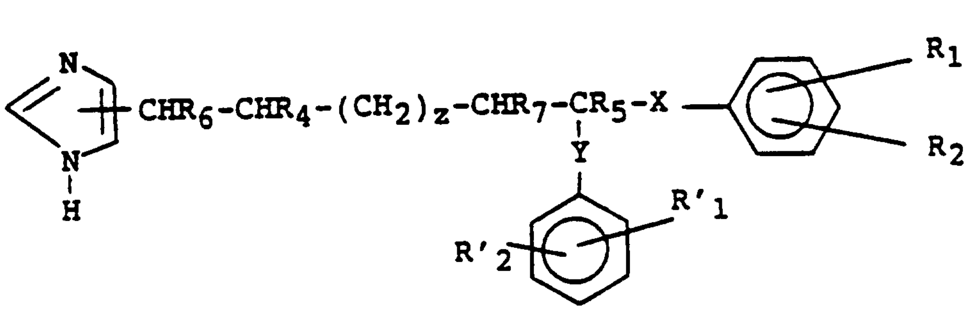

dans laquelle $R_1$, $R_2$, $R'_1$ $R'_2$, $R_4$, $R_5$, $R_6$, $R_7$, X Y et z sont tels que définis plus haut, avec une base

forte et ensuite avec un halogénure de benzyle de formule :

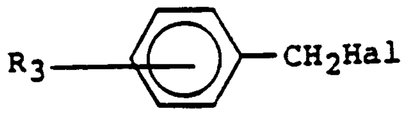

dans laquelle $R_3$ est tel que défini plus haut, pour donner un composé de formule (Ia) qui est :

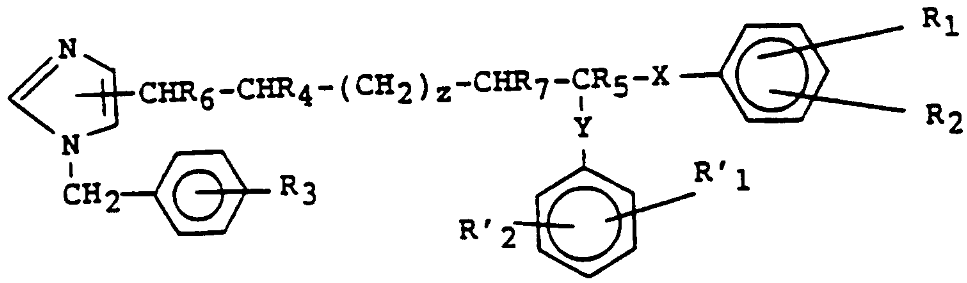

**32.** Procédé pour la préparation d'un composé suivant la revendication 6 ou d'un sel d'addition d'acide de celui-ci non toxique acceptable du point de vue pharmaceutique, qui comprend la nitration d'un composé de formule (Ia) :

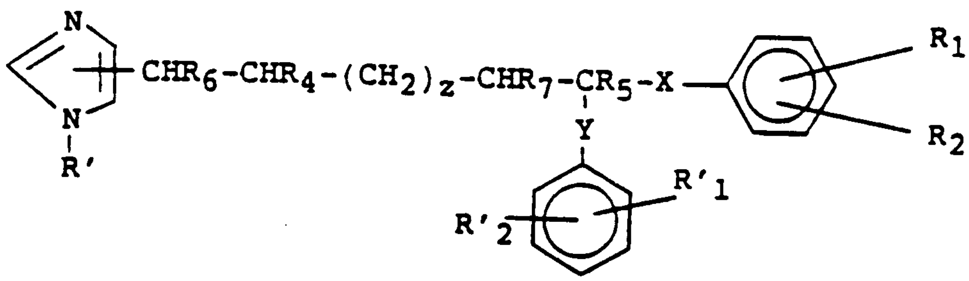

dans laquelle R', $R_4$, $R_5$, $R_6$, $R_7$, X, Y et z sont tels que définis plus haut, et l'un ou plusieurs des substituants $R_1$, $R_2$, $R'_1$, $R'_2$ sont un atome d'hydrogène, pour donner un composé de formule (Ia) dans laquelles l'un ou plusieurs des substituants $R_1$, $R_2$, $R'_1$, $R'_2$ sont un groupe $NO_2$.

**33.** Procédé pour la préparation d'un composé suivant la revendication 6 ou d'un sel d'addition d'acide de celui-ci non toxique acceptable du point de vue pharmaceutique, qui comprend la réaction d'un hydrocarbure halogéné de formule :

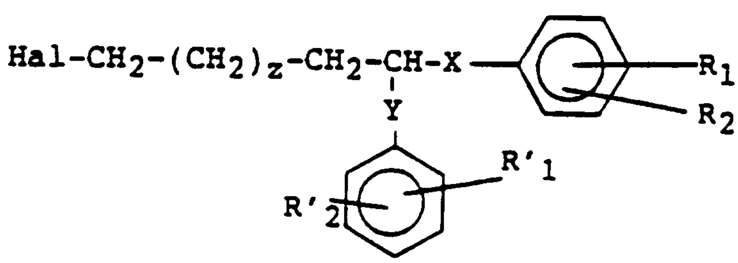

dans laquelle $R_1$, $R_2$, $R'_1$, $R'_2$, X, Y et z sont tels que définis plus haut, avec de la triphénylphosphine pour donner un sel de phosphonium de formule :

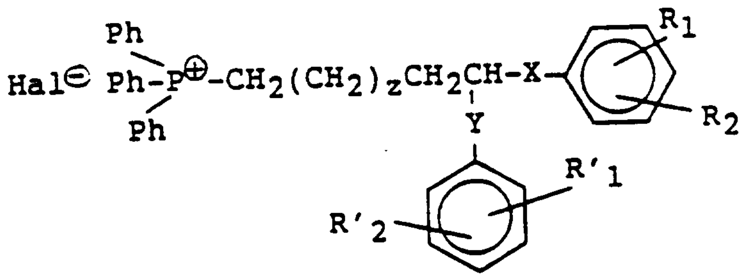

qui est ensuite traité avec une base forte, puis avec un 4(5)-imidazole aldéhyde de formule :

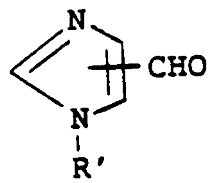

dans laquelle R' est tel que défini plus haut, pour donner un composé de formule (Ia) qui est :

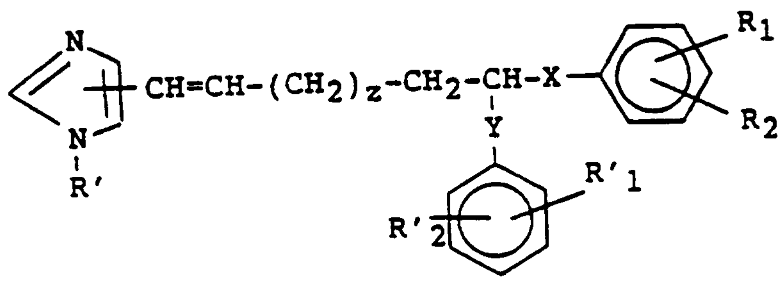

**34.** Procédé pour la préparation d'un composé suivant la revendication 6 ou d'un sel d'addition d'acide de celui-ci non toxique acceptable du point de vue pharmaceutique, qui comprend la réaction d'un dérivé de 4(5)-imidazole halogéné de formule :

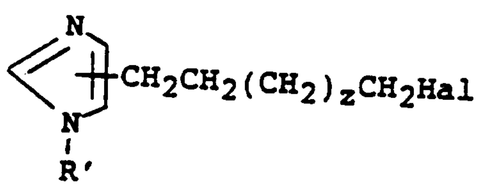

dans laquelle R' et z sont tels que définis plus haut, avec de la triphénylphosphine pour donner un sel de phosphonium de formule :

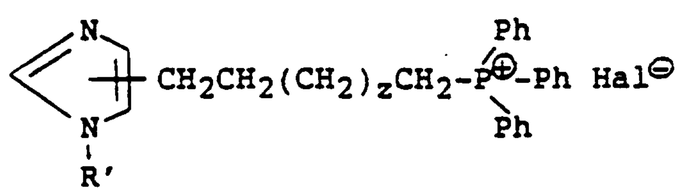

qui est ensuite traité avec une base forte, puis avec une cétone de formule :

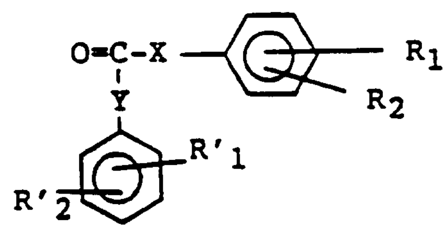

dans laquelle $R_1$, $R_2$, $R'_1$, $R'_2$, X et Y sont tels que définis plus haut, pour donner un composé de formule (Ia) qui est :

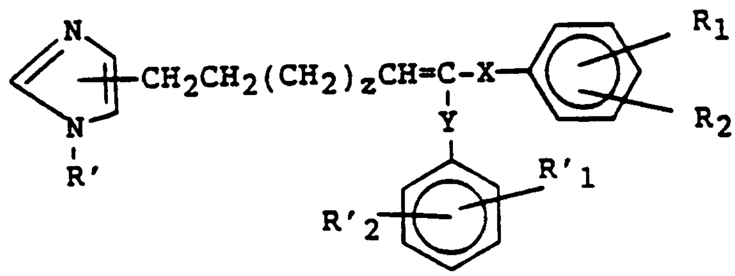

**35.** Procédé pour la préparation d'un composé suivant la revendication 6 ou d'un sel d'addition d'acide de celui-ci non toxique acceptable du point de vue pharmaceutique, qui comprend la réaction d'un hydrocarbure halogéné de formule :

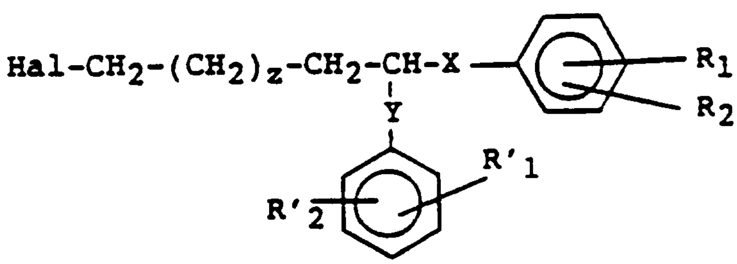

dans laquelle $R_1$, $R_2$, $R'_1$, $R'_2$, X, Y et z sont tels que définis plus haut, avec un triester d'acide phosphonique, pour donner un composé de formule :

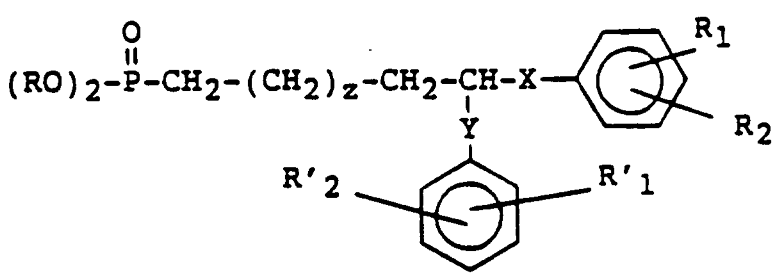

dans laquelle R est un groupe alkyle, qui est ensuite traité avec une base, puis avec un 4(5)-imidazole aldéhyde de formule :

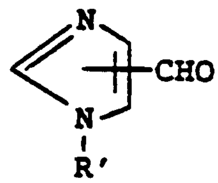

dans laquelle R' est tel que défini plus haut, pour donner un composé de formule (Ia) qui est :

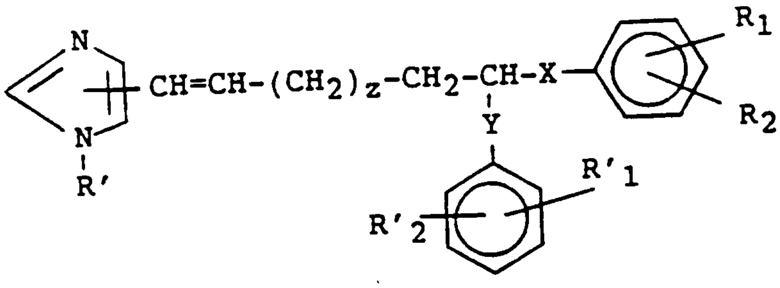

**36.** Procédé pour la préparation d'un 4-(5)-imidazole substitué de formule :

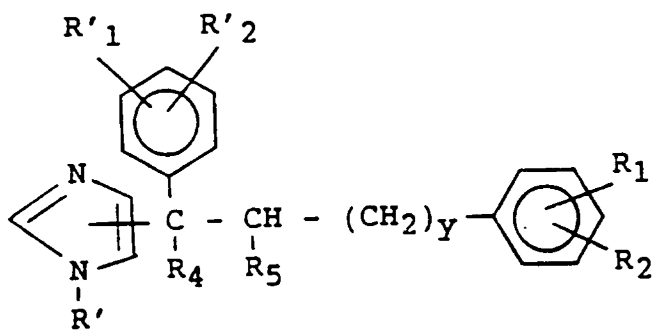

(Ib)

ou d'un sel d'addition d'acide de celui-ci non toxique acceptable du point de vue pharmaceutique, dans laquelle $R_1$, $R_2$, $R'_1$, et $R'_2$, qui peuvent être identiques ou différents, sont un atome d'hydrogène, un groupe $CH_3$, $C_2H_5$, $C_3H7$, $OCH_3$, $NO_2$, $CF_3$, $CHF_2$, $CH_2F$ ou un atome d'halogène; R' est un atome d'hydrogène ou un groupe

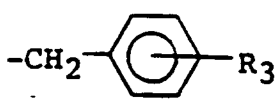

dans lequel $R_3$ est un atome d'hydrogène, d'halogène ou un groupe $CH_3$; $R_4$ est un atome d'hydrogène ou un groupe OH, $R_5$ est un atome d'hydrogène, ou $R_4$ et $R_5$ forment ensemble une liaison et y est 0 à 4,
qui comprend la réaction d'un 4(5)-imidazole aldéhyde de formule :

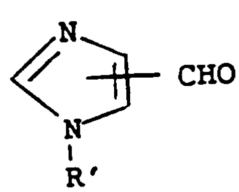

dans laquelle R' est tel que défini plus haut, avec un halogénure d'arylalkylmagnésium approprié de formule :

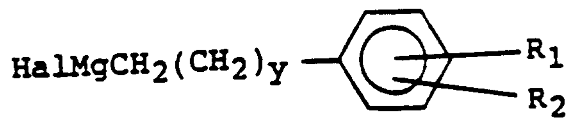

dans laquelle $R_1$, $R_2$ et y sont tels que définis plus haut, pour donner un composé de formule :

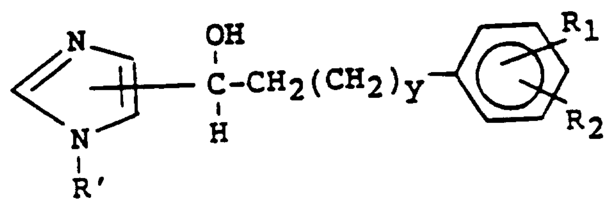

qui, si R' est un groupe benzyle substitué ou non substitué, est éventuellement hydrogéné pour éliminer le groupe R' benzylique, puis ensuite oxydé pour donner un composé de formule :

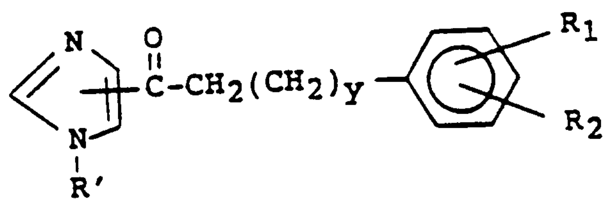

qui est ensuite traité avec un halogénure d'arylmagnésium de formule :

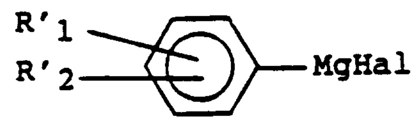

dans laquelle $R'_1$ et $R'_2$ sont tels que définis plus haut et Hal est un atome d'halogène, pour donner un composé de formule (Ib) qui est :

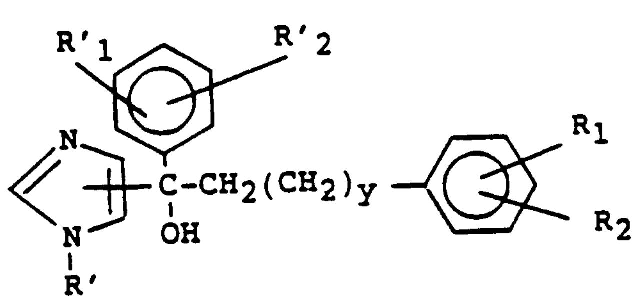

**37.** Procédé pour la préparation d'un composé suivant la revendication 36 ou d'un sel d'addition d'acide de celui-ci non toxique acceptable du point de vue pharmaceutique, qui comprend la réaction d'un 4(5)-imidazole aldéhyde de formule :

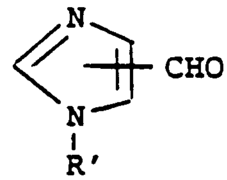

dans laquelle R' est tel que défini plus haut, avec un halogénure d'arylmagnésium de formule :

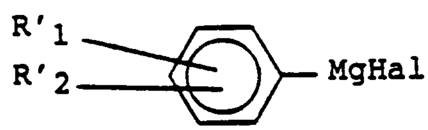

dans laquelle $R'_1$ et $R'_2$ sont tels que définis plus haut et Hal est un atome d'halogène, pour donner un composé de formule :

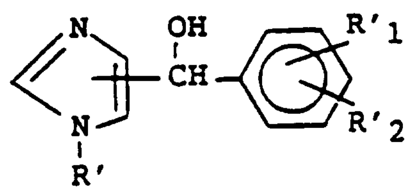

dans laquelle R', $R'_1$ et $R'_2$ sont tels que définis plus haut, qui si R' est un groupe benzyle substitué ou non substitué, est éventuellement hydrogéné pour éliminer le groupe R' benzylique, puis ensuite oxydé pour donner un composé de formule :

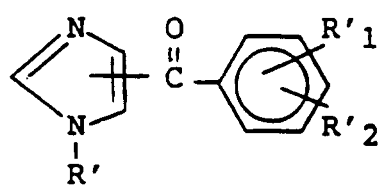

qui est ensuite traité avec un halogénure d'arylalkylmagnésium approprié de formule :

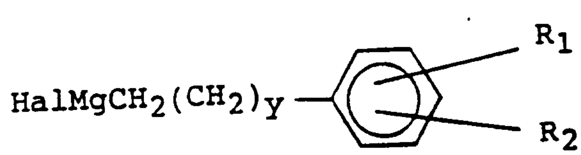

dans laquelle $R_1$, $R_2$ et y sont tels que définis plus haut, pour donner un composé de formule (Ib) qui est :

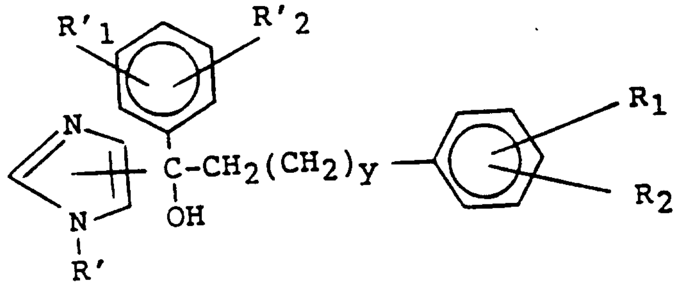

**38.** Procédé pour la préparation d'un 4-(5)-imidazole substitué de formule :

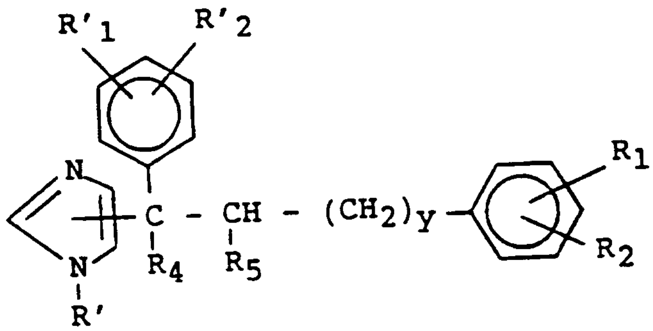

(Ib)

ou d'un sel d'addition d'acide de celui-ci non toxique acceptable du point de vue pharmaceutique, dans laquelle $R_1$, $R_2$, $R'_1$, et $R'_2$, qui peuvent être identiques ou différents, sont un atome d'hydrogène, un groupe $CH_3$, $C_2H_5$, $C_3H7$, $OCH_3$, OH, $CH_2OH$, $NO_2$, $NH_2$, CN, $CF_3$, $CHF_2$, $CH_2F$ ou un atome d'halogène; R' est un atome d'hydrogène ou un groupe

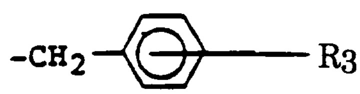

dans lequel $R_3$ est un atome d'hydrogène, d'halogène ou un groupe $CH_3$; $R_4$ est un atome d'hydrogène ou un groupe OH, $R_5$ est un atome d'hydrogène, ou $R_4$ et $R_5$ forment ensemble une liaison et y est 0 à 4,
qui comprend la réaction d'un 4(5)-imidazole aldéhyde de formule :

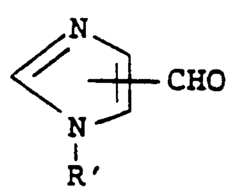

dans laquelle R' est tel que défini plus haut, avec un halogénure d'arylalkylmagnésium de formule :

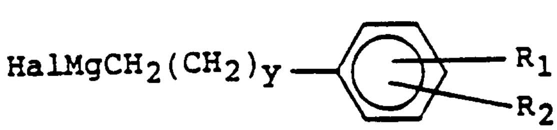

dans laquelle y est 0 à 4, Hal est un atome d'halogène et

i) $R_1$ et/ou $R_2$ sont un groupe $OCH_2Ph$ ou OTHP ou

ii) aucun des $R_1$ ou $R_2$ n'est un groupe $OCH_2Ph$ ou OTHP,

pour donner un composé de formule

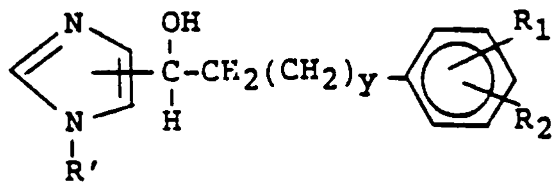

qui est oxydé pour donner un composé de formule :

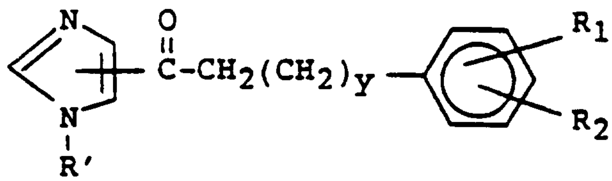

qui est ensuite traité avec un halogénure d'arylmagnésium de formule :

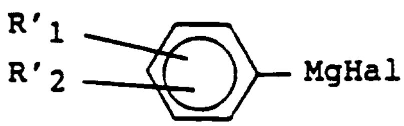

dans laquelle :

iii) $R'_1$ et/ou $R'_2$ sont un groupe $OCH_2Ph$ ou OTHP ou

iv) aucun des $R'_1$ ou $R'_2$ n'est un groupe $OCH_2Ph$ ou OTHP,

à condition que ii) et iv) ne soient pas valables simultanément, pour donner un composé de formule

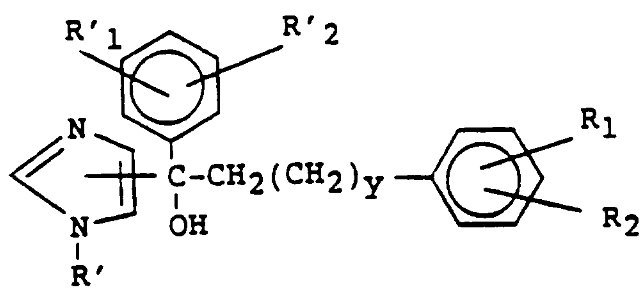

dans laquelle R' et y sont tels que définis plus haut et l'un ou plusieurs des substituants $R_1$, $R_2$, $R'_1$ et $R'_2$ sont un groupe $OCH_2Ph$ ou OTHP, qui est de plus hydrogéné pour donner un composé de formule (Ib) qui est :

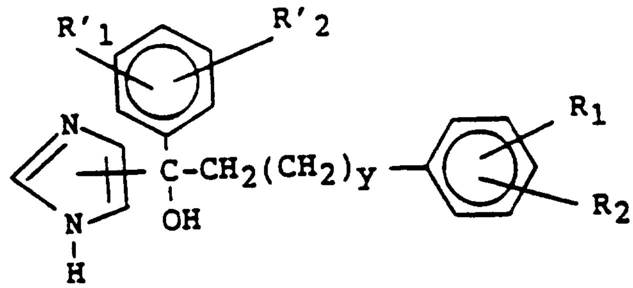

dans laquelle y est 0 à 4 et l'un ou plusieurs des substituants $R_1$, $R_2$, $R'_1$ et $R'_2$ sont un groupe OH et les substituants qui ne sont pas un groupe OH sont un atome d'hydrogène, un groupe $CH_3$, $C_2H_5$, $C_3H_7$, $OCH_3$, $NO_2$, $CF_3$, $CHF_2$, $CH_2F$ ou un atome d'halogène.

**39.** Procédé pour la préparation d'un composé suivant la revendication 38 ou d'un sel d'addition d'acide de celui-ci non toxique acceptable du point de vue pharmaceutique, qui comprend la réaction d'un 4(5)-imidazole aldéhyde de formule :

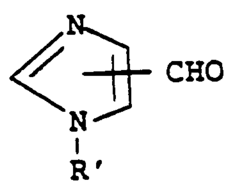

dans laquelle R' est tel que défini plus haut, avec un halogénure d'arylmagnésium de formule :

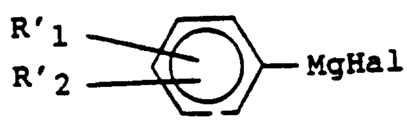

dans laquelle Hal est un atome d'halogène et

i) $R'_1$ et/ou $'R_2$ sont un groupe $OCH_2Ph$ ou OTHP ou

ii) aucun des $R'_1$ ou $R'_2$ n'est un groupe $OCH_2Ph$ ou OTHP,

pour donner un composé de formule

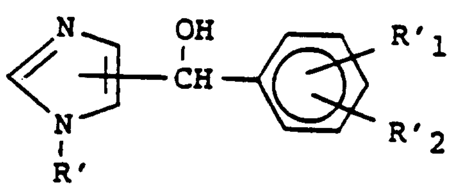

qui est oxydé pour donner un composé de formule :

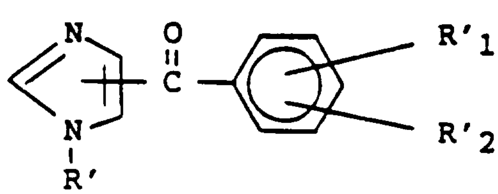

qui est ensuite traité avec un halogénure d'arylalkylmagnésium de formule :

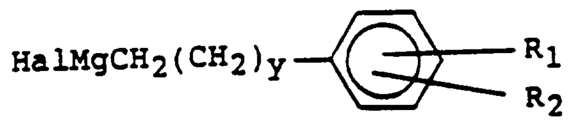

dans laquelle Y est tel que défini plus haut et Hal est un atome d'halogène et:

iii) $R_1$ et/ou $R_2$ sont un groupe $OCH_2Ph$ ou OTHP ou

iv) aucun des $R_1$ ou $R_2$ n'est un groupe $OCH_2Ph$ ou OTHP,

à condition que ii) et iv) ne soient pas valables simultanément, pour donner un composé de formule

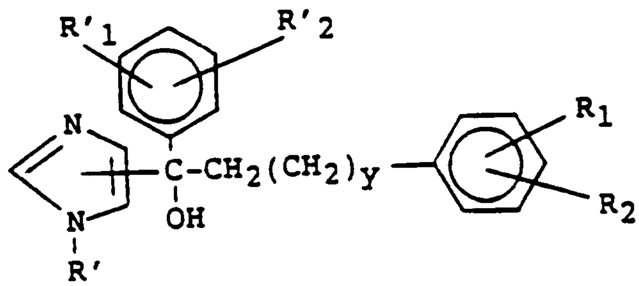

dans laquelle R' et y sont tels que définis plus haut et l'un ou plusieurs des substituants $R_1$, $R_2$, $R'_1$, et $R'_2$, sont un groupe $OCH_2Ph$ ou OTHP, qui est de plus hydrogéné pour donner un composé de formule (Ib) qui est :

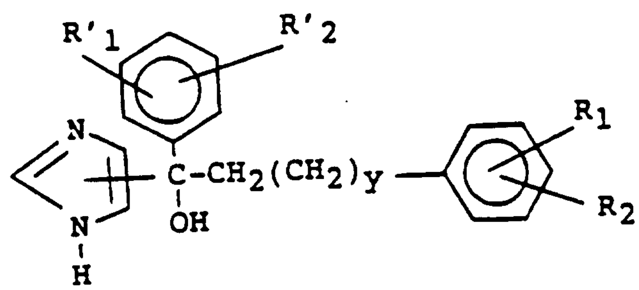

dans laquelle y est 0 à 4 et l'un ou plusieurs des substituants $R_1$, $R_2$, $R'_1$ et $R'_2$ sont un groupe OH et les substituants qui ne sont pas un groupe OH sont un atome d'hydrogène, un groupe $CH_3$, $C_2H_5$, $C_3H_7$, $OCH_3$, $NO_2$, $CF_3$, $CHF_2$, $CH_2F$ ou un atome d'halogène.

**40.** Procédé pour la préparation d'un composé suivant la revendication 38 ou d'un sel d'addition d'acide de celui-ci non toxique acceptable du point de vue pharmaceutique, qui comprend la désalkylation d'un composé de formule (Ib) :

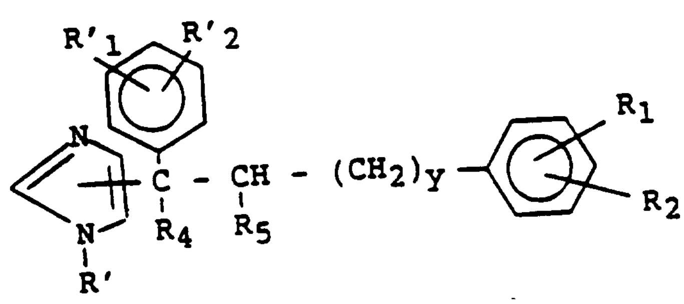

dans laquelle R', $R_4$, $R_5$ et y sont tels que définis plus haut et l'un ou plusieurs des substituants $R_1$, $R_2$, $R'_1$ et $R'_2$ sont un groupe $OCH_3$ pour donner un composé de formule (Ib) dans laquelle l'un ou plusieurs des substituants $R_1$, $R_2$, $R'_1$ et $R'_2$ sont un groupe OH.

**41.** Procédé pour la préparation d'un composé suivant la revendication 38 ou d'un sel d'addition d'acide de celui-ci non toxique acceptable du point de vue pharmaceutique, qui comprend l'hydrolyse d'un composé de formule (Ib) :

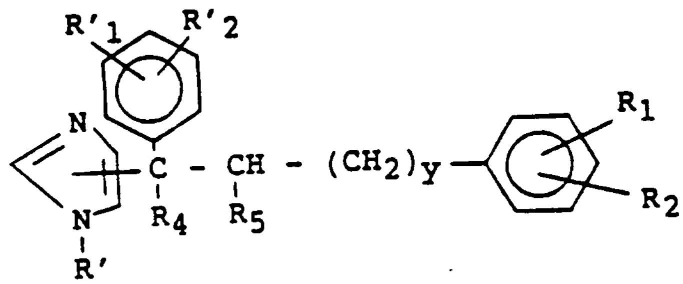

dans laquelle R', $R_4$, $R_5$ et y sont tels que définis plus haut et l'un ou plusieurs des substituants $R_1$, $R_2$, $R'_1$ et $R'_2$ sont un groupe CN, puis la réduction pour donner un composé de formule (Ib) dans laquelle l'un ou plusieurs des substituants $R_1$, $R_2$, $R'_1$ et $R'_2$ sont un groupe $CH_2OH$.

**42.** Procédé pour la préparation d'un composé suivant la revendication 38 ou d'un sel d'addition d'acide de celui-ci non toxique acceptable du point de vue pharmaceutique, qui comprend la diazotation d'un composé de formule (Ib) :

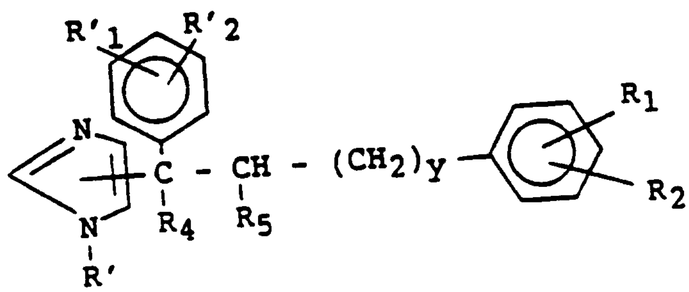

dans laquelle R', $R_4$, $R_5$ et y sont tels que définis plus haut et l'un ou plusieurs des substituants $R_1$, $R_2$, $R'_1$ et $R'_2$ sont un groupe $NH_2$ pour donner un composé de formule (Ib) dans laquelle l'un ou plusieurs des substituants $R_1$, $R_2$, $R'_1$ et $R'_2$ sont un groupe CN.

**43.** Procédé pour la préparation d'un composé suivant la revendication 38 ou d'un sel d'addition d'acide de celui-ci non toxique acceptable du point de vue pharmaceutique, qui comprend l'hydrogénation d'un composé de formule (Ib) :

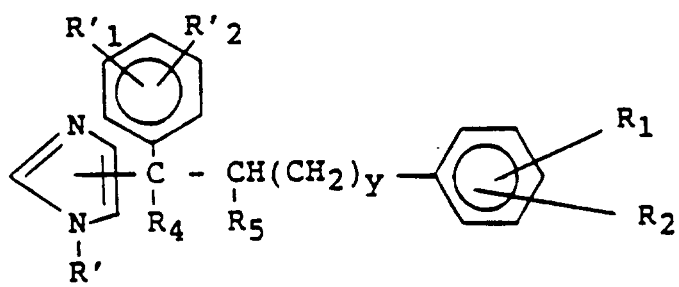

dans laquelle R', $R_4$, $R_5$ et y sont tels que définis plus haut et l'un ou plusieurs des substituants $R_1$, $R_2$,

$R'_1$ et $R'_2$ sont un groupe $NO_2$ pour donner un composé de formule (Ib) dans laquelle R' est un atome d'hydrogène, y est 0 à 4 et l'un ou plusieurs des substituants $R_1$, $R_2$, $R'_1$ et $R'_2$ sont un groupe $NH_2$.

**44.** Procédé pour la préparation d'un composé suivant la revendication 38 ou d'un sel d'addition d'acide de celui-ci non toxique acceptable du point de vue pharmaceutique, qui comprend la déshydratation d'un composé de formule :

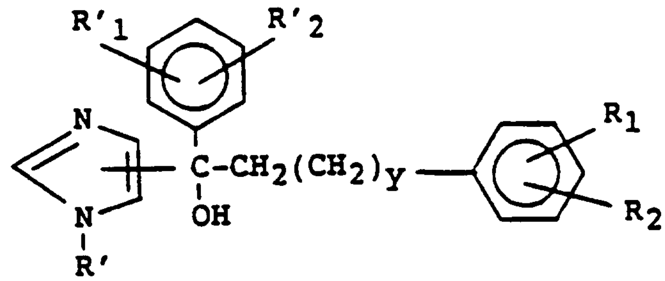

pour donner un composé de formule (Ib) qui est :

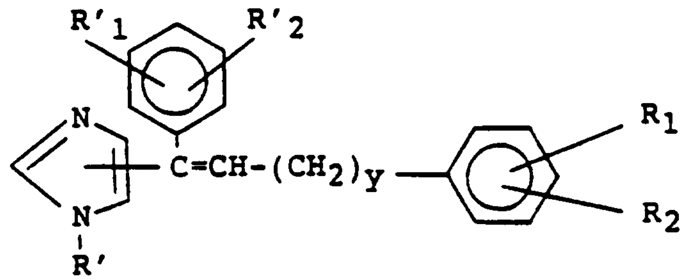

dans laquelle R', $R_1$, $R_2$, $R'_1$, $R'_2$ et y sont tels que définis plus haut.

**45.** Procédé pour la préparation d'un composé suivant la revendication 38 ou d'un sel d'addition d'acide de celui-ci non toxique acceptable du point de vue pharmaceutique, qui comprend la réaction d'un composé de formule :

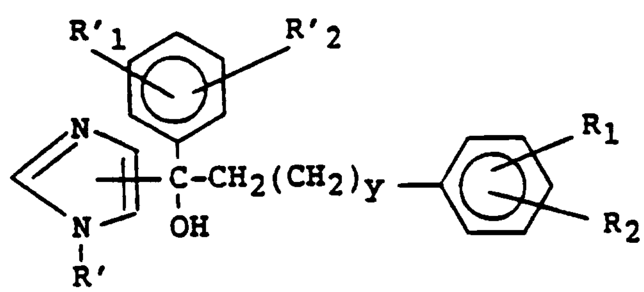

par réaction de transfert d'hydrogène, pour donner un composé de formule (Ib) qui est :

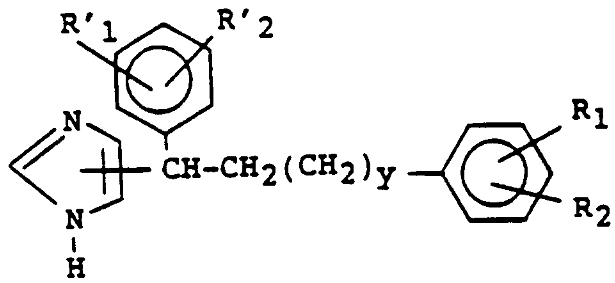

dans laquelle $R_1$, $R_2$, $R'_1$, $R'_2$ et y sont tels que définis plus haut.

**46.** Procédé pour la préparation d'un composé suivant la revendication 38 ou d'un sel d'addition d'acide de celui-ci non toxique acceptable du point de vue pharmaceutique, qui comprend l'hydrogénation d'un composé de formule :

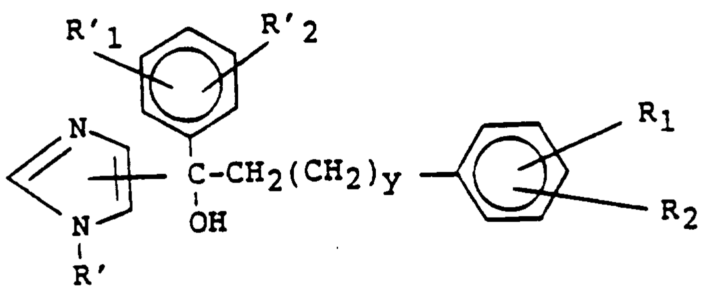

dans laquelle R' est un groupe benzyle substitué ou non substitué, et $R_1$, $R_2$, $R'_1$, $R'_2$ et y sont tels que définis plus haut, pour donner un composé de formule (Ib) qui est :

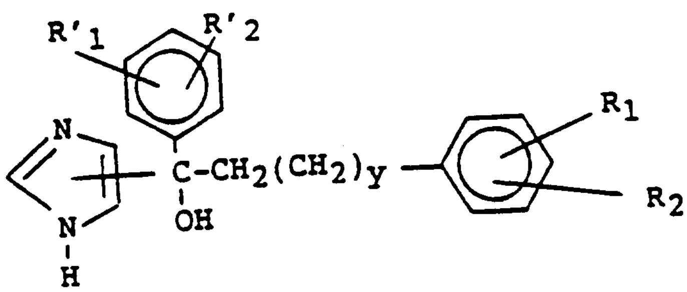

**47.** Procédé pour la préparation d'un composé suivant la revendication 38 ou d'un sel d'addition d'acide de celui-ci non toxique acceptable du point de vue pharmaceutique, qui comprend la réaction d'un composé de formule :

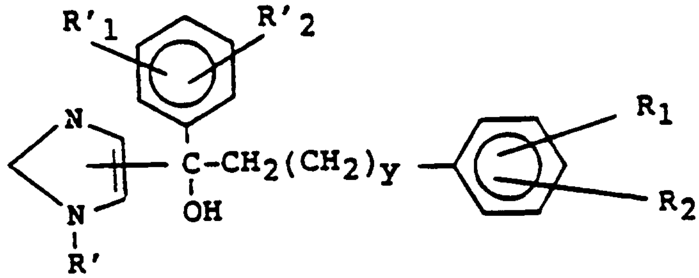

dans laquelle R' est un groupe benzyle substitué ou non substitué, et $R_1$, $R_2$, $R'_1$, $R'_2$ et y sont tels que définis plus haut, par une réaction de transfert d'hydrogène pour donner un composé de formule (Ib) qui est:

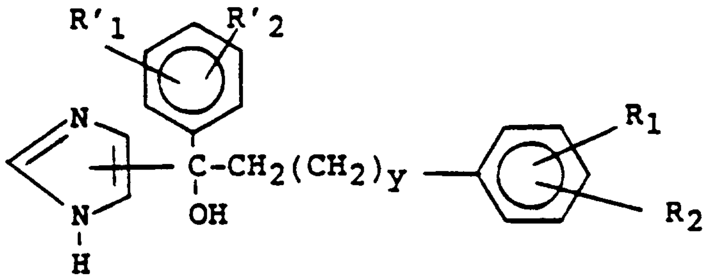

dans laquelle $R_1$, $R_2$, $R'_1$, $R'_2$ et y sont tels que définis plus haut.

**48.** Procédé pour la préparation d'un composé suivant les revendications 36 ou 38 ou d'un sel d'addition d'acide de celui-ci non toxique acceptable du point de vue pharmaceutique, qui comprend la réaction d'un benzoylimidazole de formule :

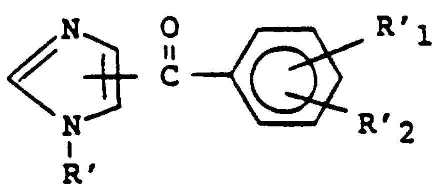

dans laquelle R', $R'_1$ et $R'_2$ sont tels que définis dans la revendication 36 avec un aldéhyde de formule :

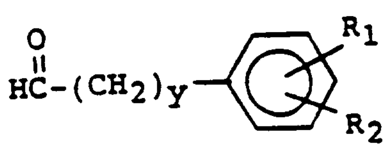

dans laquelle $R_1$, $R_2$ et y sont tels que définis dans la revendication 38, en présence d'un réactif au titane de faible valence pour donner un composé de formule (Ib) qui est :

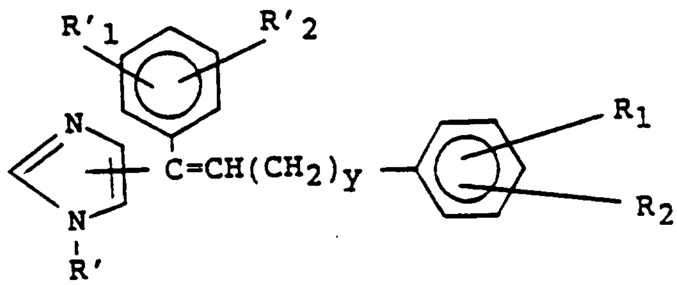

**49.** Procédé pour la préparation d'un composé suivant les revendications 36 ou 38 ou d'un sel d'addition d'acide de celui-ci non toxique acceptable du point de vue pharmaceutique, qui comprend la réaction d'un benzoylimidazole de formule :

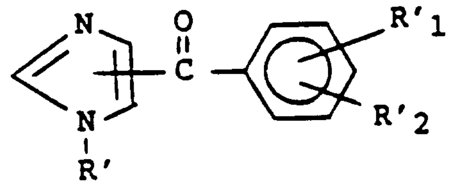

dans laquelle R' est tel que défini plus haut et $R'_1$ et/ou $R'_2$ sont des groupes $OCH_2Ph$ ou OTHP avec un aldéhyde de formule :

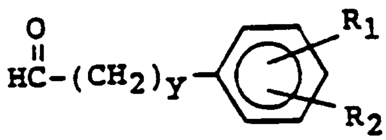

dans laquelle $R_1$, $R_2$ et y sont tels que définis dans la revendication 38, en présence d'un réactif au titane de faible valence pour donner un composé de formule :

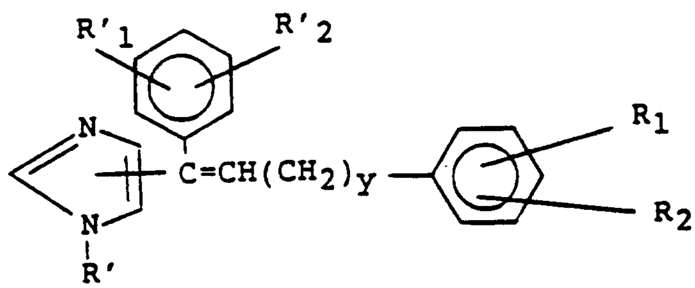

dans laquelle $R'_1$ et/ou $R'_2$ sont des groupes $OCH_2Ph$ ou OTHP, qui est ensuite hydrogéné pour donner un composé de formule (Ib) qui est:

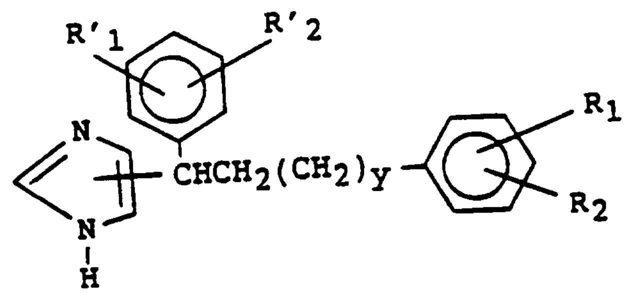

dans laquelle R'$_1$ et/ou R'$_2$ sont des groupes OH, le substituant R'$_1$ ou R'$_2$ qui n'est pas un groupe OH est un atome d'hydrogène, un groupe $CH_3$, $C_2H_5$, $C_3H_7$, $OCH_3$, $NO_2$, $CF_3$, $CHF_2$, $CH_2F$ ou un atome d'halogène et $R_1$ et $R_2$ sont tels que définis dans la revendication 38.

**50.** Procédé pour la préparation d'un composé suivant la revendication 38 ou d'un sel d'addition d'acide de celui-ci non toxique acceptable du point de vue pharmaceutique, qui comprend l'hydrogénation d'un composé de formule :

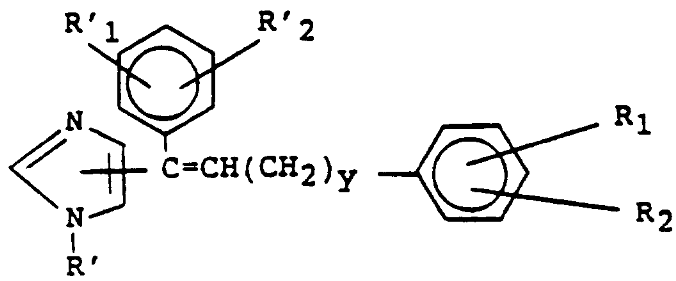

pour donner un composé de formule (Ib) qui est :

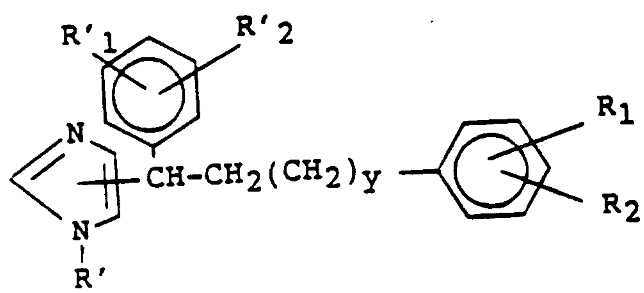

dans laquelle R', $R_1$, $R_2$, R'$_1$, R'$_2$ et y sont tels que définis plus haut.

**51.** Procédé pour la préparation d'un composé suivant la revendication 38 ou d'un sel d'addition d'acide de celui-ci non toxique acceptable du point de vue pharmaceutique, qui comprend l'hydrogénation d'un composé de formule :

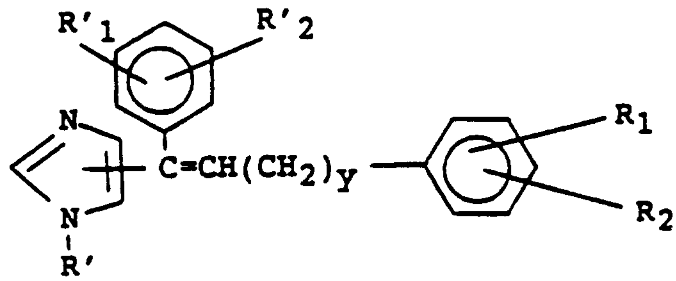

pour donner un composé de formule (Ib) qui est :

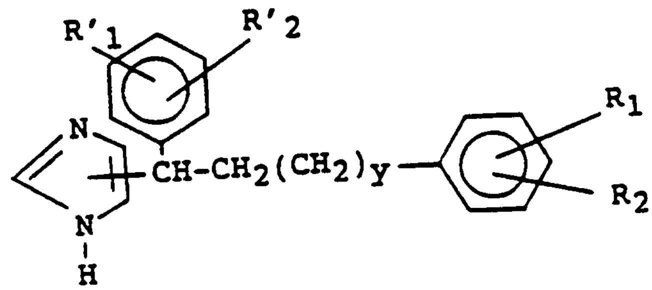

dans laquelle $R_1$, $R_2$, $R'_1$, $R'_2$ et y sont tels que définis plus haut.

**52.** Procédé pour la préparation d'un composé suivant la revendication 38 ou d'un sel d'addition d'acide de celui-ci non toxique acceptable du point de vue pharmaceutique, qui comprend la réaction d'un composé de formule :

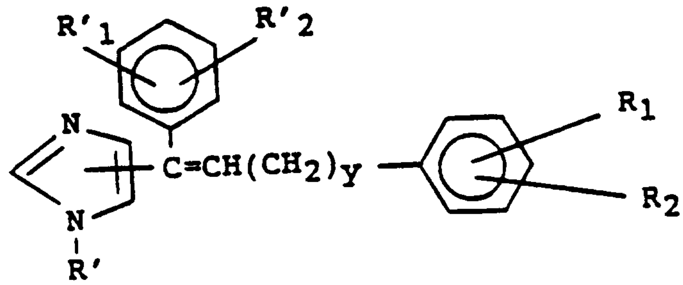

par une réaction de transfert d'hydrogène pour donner un composé de formule (Ib) qui est :

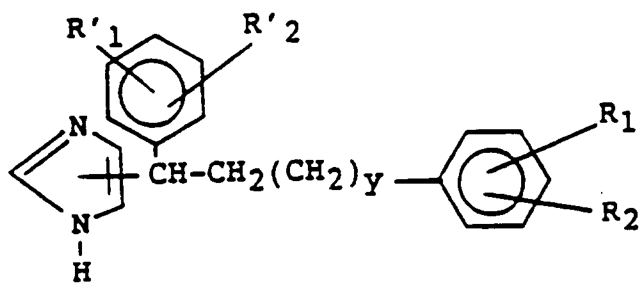

dans laquelle $R_1$, $R_2$, $R'_1$, $R'_2$ et y sont tels que définis plus haut.

**53.** Procédé pour la préparation d'un composé suivant la revendication 38 ou d'un sel d'addition d'acide de celui-ci non toxique acceptable du point de vue pharmaceutique, qui comprend l'hydrogénation d'un composé de formule :

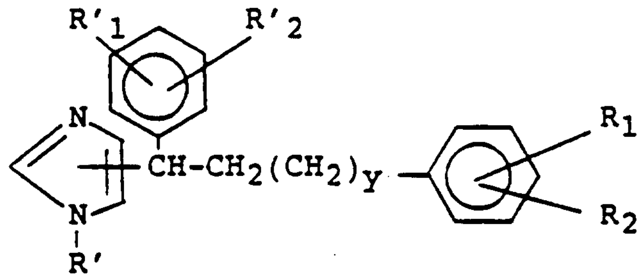

dans laquelle $R_1$, $R_2$, $R'_1$, $R'_2$ et y sont tels que définis plus haut et R' est un groupe benzyle substitué ou non substitué, pour donner un composé de formule (Ib) qui est :

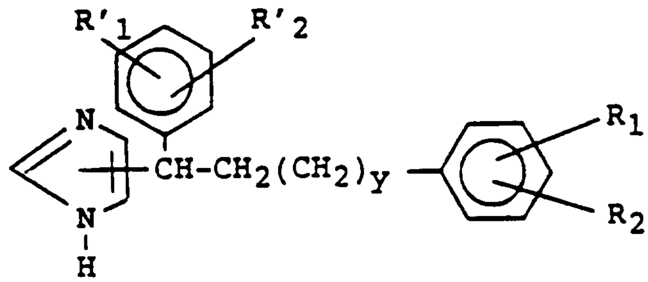

**54.** Procédé pour la préparation d'un composé suivant la revendication 38 ou d'un sel d'addition d'acide de celui-ci non toxique acceptable du point de vue pharmaceutique, qui comprend la réaction d'un composé de formule :

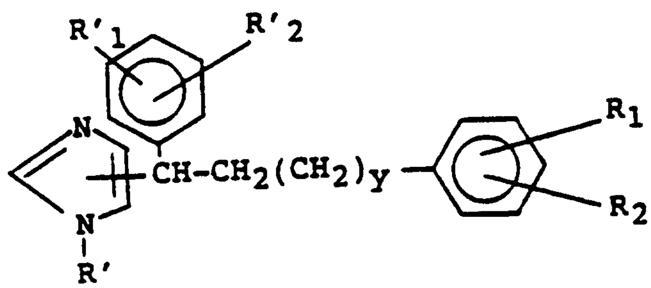

dans laquelle $R_1$, $R_2$, $R'_1$, $R'_2$ et y sont tels que définis plus haut et R' est un groupe benzyle substitué ou non substitué, par une réaction de transfert d'hydrogène pour donner un composé de formule (Ib) qui est:

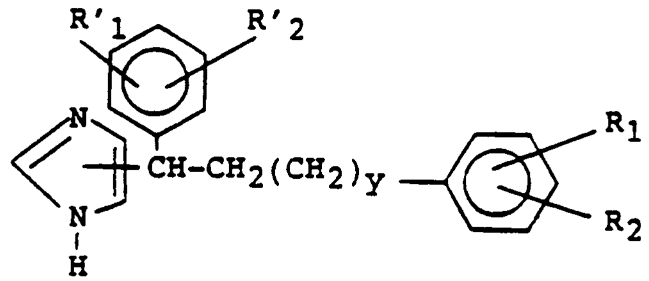

55. Procédé pour la préparation d'un composé suivant la revendication 38 ou d'un sel d'addition d'acide de celui-ci non toxique acceptable du point de vue pharmaceutique, qui comprend la réaction d'un composé de formule :

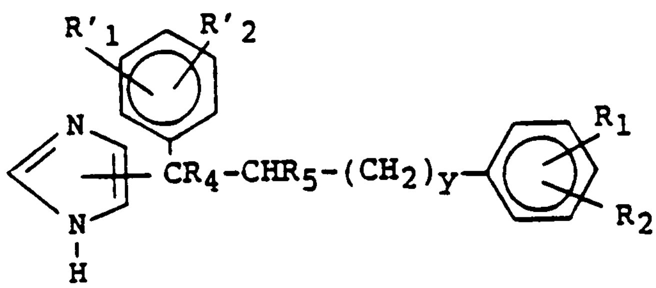

dans laquelle $R_1$, $R_2$, $R'_1$, $R'_2$, $R_4$, $R_5$ et y sont tels que définis plus haut, avec un base forte et ensuite avec un halogénure de benzyle de formule :

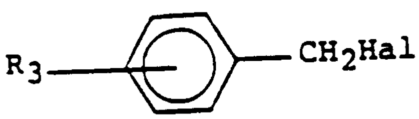

dans laquelle $R_3$ est tel que défini plus haut et Hal est un atome d'halogène pour donner un composé de formule (Ib) qui est :

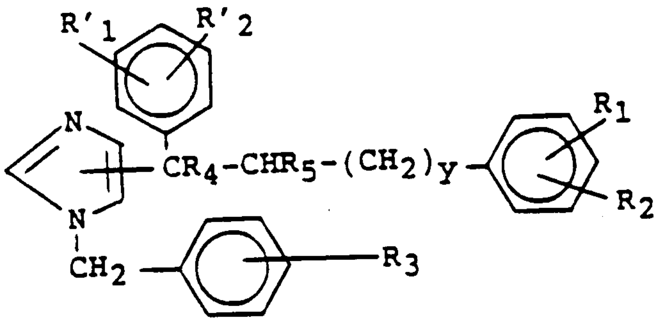

56. Procédé pour la préparation d'un composé suivant la revendication 38 ou d'un sel d'addition d'acide de celui-ci non toxique acceptable du point de vue pharmaceutique, qui comprend la nitration d'un composé de formule (Ib) :

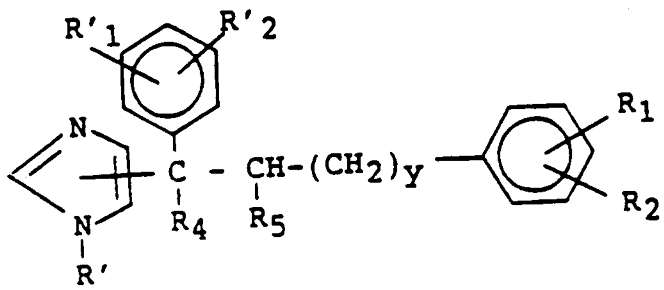

dans laquelle R', $R_4$, $R_5$ et y sont tels que définis plus haut, et l'un ou plusieurs des substituants $R_1$, $R_2$, $R'_1$, $R'_2$ sont un atome d'hydrogène, pour donner un composé de formule (Ib) dans laquelle l'un ou plusieurs des substituants $R_1$, $R_2$, $R'_1$, $R'_2$ sont un groupe $NO_2$.